Europäisches Patentamt

European Patent Office

Office européen des brevets

(19)

(11) Numéro de publication : **0 239 425**
**B1**

(12) FASCICULE DE BREVET EUROPÉEN

(45) Date de publication du fascicule du brevet :
02.11.89

(21) Numéro de dépôt : 87400151.4

(22) Date de dépôt : 22.01.87

(51) Int. Cl.⁴ : **C 12 N 7/00**, A 61 K 39/21,
G 01 N 33/569,
C 07 K 15/06, C 12 N 5/00,
C 12 N 15/00, C 12 Q 1/70,
C 12 P 21/02

(54) Rétrovirus du type HIV-2 susceptible de provoquer le SIDA, et ses constituants antigéniques et nucléiques.

Une requête en addition d'une page 36 a été présentée selon la Règle 88 CBE. Une décision sera prise par la Division d'Examen compétente.

(30) Priorité : 22.01.86 FR 8600910
22.01.86 FR 8600911
06.02.86 FR 8601635
13.02.86 FR 8601985
18.03.86 FR 8603881
24.03.86 FR 8604215
03.03.86 US 835228
06.10.86 US 916080
21.11.86 US 933184

(43) Date de publication de la demande :
30.09.87 Bulletin 87/40

(45) Mention de la délivrance du brevet :
02.11.89 Bulletin 89/44

(84) Etats contractants désignés :
AT BE CH DE ES FR GB GR IT LI LU NL SE

(56) Documents cités :
WO—A—85 /048 97
WO—A—86 /023 83
THE LANCET, no. 8469/70, 21/28 décembre 1985, pages 1387-1389, London, GB; F. BARIN et al.: "Serological evidence for virus related to simian T-lymphotropic retrovirus III in residents of West Africa"
SCIENCE, vol. 228, 7 juin 1985, pages 1199-1201; P.J. KANKI et al.: "Serologic identification and characterization of a macaque T-lymphotropic retrovirus closely related to HTLV-III"

(73) Titulaire : INSTITUT PASTEUR
25-28, rue du Docteur Roux
F-75724 Paris Cedex 15 (FR)

(72) Inventeur : Montagnier, Luc
21, rue de Malabry
F-92350 Le Plessis Robinson (FR)
Inventeur : Chamaret, Solange
324 rue Lecourbe
F-75015 Paris (FR)
Inventeur : Guetard, Denise
4B, rue Anselme Payen
F-75015 Paris (FR)
Inventeur : Alizon, Marc
71, rue du Cardinal Lemoine
F-75005 Paris (FR)
Inventeur : Clavel, François
83, rue de l'Assomption
F-75016 Paris (FR)
Inventeur : Brun-Vezinet, Françoise
24 boulevard Saint-Germain
F-75005 Paris (FR)
Inventeur : Rey, Marianne
10 rue du Temple
F-75004 Paris (FR)
Inventeur : Rouzioux, Christine
21 rue de Dantzig
F-75015 Paris (FR)
Inventeur : Katlama, Christine
8 rue de Jarente
F-75004 Paris (FR)
Inventeur : GUYADER, Mireille
2 Square Rocamadoure
F-75016 Paris (FR)
Inventeur : SONIGO Pierre
23, rue Gutenberg
F-75015 Paris (FR)

SCIENCE, vol. 228, 7 juin 1985, pages 1201-1203; M.D. DANIEL et al.: "Isolation of T-cell tropic HTLV-III-like retrovirus form macaques"

SCIENCE, vol. 230, 22 novembre 1985, pages 951-954; P.J. KANKI et al.: "Isolation of T-lymphotropic retrovirus related to HTLV-III/LAV from wild-caught African green monkeys"

COMPTE RENDUS DE L'ACADEMIE DES SCIENCES PARIS, vol. 302, série III, no. 13, 7 avril 1986, pages 485-488, Académie des Sciences; F. CLAVEL et al.: "LAV type II: un second rétrovirus associé au SIDA en Afrique de l'Ouest"

SCIENCE, vol. 233 - 11 Juillet 1986, 209-212,"NEUTRALIZATION OF THE AIDS RETROVIRUS BY ANTIBODIES TO A RECOMBINANT ENVELOPE GLYCOPROTEIN"

CELL, vol. 50, 975-985 - 15 Septembre 1987 "DELINEATION OF A REGION OF THE HUMAN IMMUNODEFICIENCY VIRUS TYPE 1 pg120 GLYCOPROTEIN CRITICAL FOR INTERACTION WITH THE CD4 RECEPTOR"

BIOTECHNOLOGY, vol. 4 - Septembre 1986, 790-795, "AIDS VIRUS ENV PROTEIN EXPRESSED FROM A RECOMBINANT VACCINIA VIRUS"

JOURNAL OF VIROLOGY, Nov. 1987, vol. 61, no. 11, 3617-3620,"EXPRESSION OF ENVELOPE CLYCOPROTEINS OF HUMAN IMMUNODEFICIENCY VIRUS BY AN INSECT VIRUS VECTOR"

(74) Mandataire : **Gutmann, Ernest et al**
**S.C. Ernest Gutmann - Yves Plasseraud 67, boulevard Haussmann**
**F-75008 Paris (FR)**

## Description

L'invention est relative à une nouvelle classe de virus, ayant la capacité de provoquer des lymphadénopathies susceptibles d'être relayées ensuite par le syndrome d'immuno-déficence acquise (SIDA) chez l'homme. L'invention concerne également des antigènes susceptibles d'être reconnus par des anticorps induits chez l'homme par cette nouvelle classe de virus. Elle concerne également les anticorps induits par des antigènes obtenus à partir de ces virus.

Cette invention concerne, en outre, des séquences d'ADN cloné soit présentant une analogie de séquence, soit une complémentarité avec l'ARN génomique du virus précité. Elle concerne aussi des procédés de préparation de ces séquences d'ADN cloné.

L'invention est également relative à des polypeptides contenant des séquences d'amino-acides codées par les séquences d'ADN cloné.

De plus, l'invention concerne des applications de ces antigènes au diagnostic in vitro chez l'homme de potentialités de certaines formes du SIDA et, en ce qui concerne certains d'entre eux, à la production de compositions immunogènes et de compositions vaccinantes contre ce rétrovirus. De même l'invention concerne les applications aux mêmes fins des susdits anticorps et, pour certains d'entre eux, leur application à la production de principes actifs de médicaments contre ces SIDAS humains.

L'invention concerne enfin l'application des séquences d'ADN cloné comme sondes dans des kits de diagnostic.

L'isolement et la caractérisation d'un premier rétrovirus, dénommé LAV, dont avait été reconnue la responsabilité dans le développement du SIDA, a fait l'objet d'une description dans un article de F. BARRE-SINOUSSI et al. dès 1983 (Science, vol. 220, n° 45-99, 20, p. 868-871. L'application au diagnostic de la présence d'anticorps contre le virus de certains extraits de ce dernier, et plus particulièrement de certaines de ses protéines, a été plus spécialement décrite dans la demande de brevet européen n° 138.667. Depuis, d'autres souches similaires et des variants du LAV ont été isolés. On rappellera pour mémoire ceux connus sous les désignations HTLV-III et ARV.

En application des nouvelles règles de nomenclature publiées dans Nature, en mai 1986, les rétrovirus susceptibles d'induire chez l'homme les susdites lymphadénopathies et le SIDA, seront désignés globalement par « HIV », abréviation de l'expression anglaise « Human immunodeficiency Virus » (ou virus d'immunodéficience humaine). Le sous-ensemble des rétrovirus formés par LAV et de ces variants a initialement été désigné par les expressions « LAV type I » ou « LAV-I ». Le même sous-ensemble sera désigné ci-après par la désignation HIV-1, étant entendu que l'expression LAV sera encore conservée pour désigner celle parmi les souches de rétrovirus (notamment LAAV, IDAV-1 et IDAV-2) appartenant à la classe des virus HIV-1 décrite dans la susdite demande de brevet européen 138.667, qui a été utilisée dans les essais comparatifs décrits plus loin, à savoir LAV$_{BRU}$, qui a été déposée à la Collection des Cultures Nationales de Microorganismes (CNCM) de l'Institut Pasteur de Paris, France, le 15 juillet 1983, sous le n° I-232.

Le nouveau rétrovirus faisant l'objet du présent brevet et les souches de virus qui en sont proches et qui, comme lui sont susceptibles de se multiplier dans des lymphocytes humains, antérieurement appelées « LAV type II » ou « LAV II » sont désormais appelées « HIV-2 », étant entendu que les désignations de certains isolats de HIV-2 décrits plus loin seront suivies des trois lettres faisant référence aux malades à partir desquels ils ont été isolés.

On peut définir l'ensemble « HIV-2 » comme un ensemble de virus ayant in vitro un tropisme pour des lymphocytes T4 humains, et qui ont un effet cytopathogène à l'égard de ces lymphocytes, lorsqu'ils s'y multiplient, pour alors causer soit des poly-adénopathies généralisées et persistantes, soit un SIDA. Les rétrovirus HIV-2 se sont révélés distincts des virus de type HIV-1 dans les conditions évoquées plus loin. Comme ces derniers, ils sont distincts des autres rétrovirus humains déjà connus (HTLV-I et HTLV-II).

Bien qu'il y ait une assez grande variabilité génétique du virus, les différentes souches HIV-1 isolées à ce jour, à partir des malades américains, européens, haïtiens et africains ont en commun des sites antigéniques conservés sur leurs principales protéines : protéine du noyau (core) p25 et glycoprotéine d'enveloppe gp110 et protéine transmembranaire gp41-43. Cette parenté permet par exemple à la souche prototype LAV d'être utilisée comme souche d'antigènes pour la détection d'anticorps contre tous les virus de la classe HIV-1, chez toutes les personnes qui les portent, quelle que soit leur origine. Cette souche est donc utilisée actuellement pour la détection d'anticorps anti-HIV-1 chez les donneurs de sang et les malades par les techniques d'immunofluorescence, notamment par les techniques dites ELISA, « Western Blot » (ou immuno-empreintes) et « RIPA » abréviation anglaise de « Radio Immunoprecipitation Assay » (test de radio-immunoprécipitation).

Cependant dans une étude sérologique effectuée avec un lysat de HIV-1 sur des malades originaires de l'Afrique de l'Ouest, il a été observé que certains d'entre eux donnaient des réactions séro-négatives ou très faiblement positives, alors qu'ils présentaient les signes cliniques et immunologiques du SIDA.

C'est à partir des lymphocytes mis en culture de l'un de ces malades, que l'on a isolé un premier rétrovirus HIV-2, dont la structure en microscopie électronique et le profil des protéines en gel d'électrophorèse SDS ont une similitude avec ceux de HIV-1. Mais ce nouveau rétrovirus HIV-2 ne présente globalement qu'une faible parenté avec HIV-1, tant du point de vue de l'homologie antigénique

3

de ses protéines que de l'homologie de son matériel génétique.

Ce nouveau rétrovirus ou des rétrovirus ayant des propriétés antigéniques et immunologiques équivalentes, peuvent donc constituer des sources d'antigènes pour le diagnostic de l'infection par ce virus et des variants qui induisent un SIDA, du type de celui que l'on avait observé pour les premières fois chez des malades africains ou des personnes ayant séjourné en Afrique.

Le premier isolement de ce virus a été réalisé à partir du sang, prélevé sous héparine, d'un malade de 28 ans, hétérosexuel, qui n'avait jamais été transfusé et qui n'était pas toxicomane. Il présentait depuis 1983 une importante diarrhée chronique, un amaigrissement important (17 kg) avec de la fièvre par intermittence. Plus récemment il avait présenté des infections à Candida et Serratia, dont une candidose œsophagienne, typique du SIDA.

Ce patient présentait également une anémie, une anergie cutanée, une lymphopénie, un rapport lymphooytes T4/lymphocytes T8 de 0,15, avec un taux en lymphocytes T4 inférieur à 100 par $mm^3$ de sérum. Ses lymphocytes en culture ne répondaient pas à la stimulation par la phytohémoglutinine et la concanavaline A. On a également diagnostiqué chez ce malade des bactériémies récurrentes dues à S. enteriditis, des cryptosporidioses, des infections dues à Isospora belli et des toxoplasmoses cérébrales.

L'ensemble de ces signes témoignait des symptômes « complexes liés au SIDA » ou « ARC » (abréviation anglaise de « AIDS Related Complex »), du type de ceux causés par le virus HIV-1. Ces différentes observations étaient également conformes aux critères appliqués par le Centre de Contrôle des Maladies (Center of Disease Control ou CDC) d'Atlanta, Etats-Unis.

La mise en culture des lymphocytes de ces malades et l'isolement du rétrovirus ont été effectués selon la technique déjà décrite pour l'isolement du HIV-1 (1) dans l'article de BARRE-SINOUSSI et Coll. et la demande de brevet européen n° 84 401834/0 138 667. On les rappelle brièvement ci-après. Des lymphocytes stimulés pendant 3 jours par de la phytohémagglutinine (PHA) ont été cultivés en milieu de culture RPM1-16-40 additionné de 10 % de sérum de veau fœtal et de $10^{-5}$ M β-mercaptoéthanol, d'interleukine-2 et de sérum anti-interféron α humain.

La production de virus a été suivie par son activité transcriptase inverse. Dans le surnageant de culture, le pic d'activité virale est apparu entre le $14^e$ et le $22^e$ jour, puis a diminué. Le déclin et la mort de la culture cellulaire ont suivi. Comme avec le HIV-1, des coupes de lymphocytes infectés par HIV-2 révèlent en microscopie électronique des virions parvenus à maturité et des particules virales bourgeonnant à la surface des cellules infectées. Les lignées cellulaires utilisées pour réaliser les cultures de ces virus isolés peuvent être selon les cas, les lignées cellulaires du type HUT, CEM, MOLT ou de toute lignée lymphocytaire immortalisée portant des récepteurs T4.

Le virus a ensuite été propagé sur des cultures de lymphocytes de donneurs de sang, puis sur des lignées continues d'origine leucémique, telles que HUT 78. Il a été caractérisé comme étant sensiblement distinct du HIV-I par ses antigènes et son acide nucléique. Le virus a été purifié comme décrit dans les documents antérieurs déjà mentionnés. Un premier isolat de ce virus a été déposé à la CNCM le 19 décembre 1985, sous le n° I-502. Il a été désigné postérieurement sous le nom LAV-II MIR. Un second isolat a été déposé à la CNCM le 21 février 1986 sous le n° I-532. Ce second isolat a pour nom de référence LAV-II ROD. Il sera quelquefois fait simplement référence plus loin, à MIR ou à ROD.

D'une façon générale l'invention concerne tout variant des virus précédents ou tout virus équivalent (par exemple tel que HIV-2 IRMO et HIV-2 EHO déposés à la CNCM le 19 décembre 1986 sous les numéros I-642 et I-643 respectivement) contenant des protéines de structure qui présentent les mêmes propriétés immunologiques que celles des virus·HIV-2 déposés à la CNCM sous les numéros I-502, ou I-532. Il sera encore revenu plus loin sur les définitions critères d'équivalence.

En d'autres termes, l'invention concerne un rétrovirus humain HIV-2 ayant la capacité d'infecter des lymphocytes T4 humains et susceptible de provoquer un SIDA chez l'homme, ce rétrovirus étant constitué par l'un des rétrovirus déposés à la CNCM sous les n° I-502, I-532, I-642 et I-643 et à l'ECACC sous les n° 87011001 et 87011002 (correspondant respectivement aux n° I-502 et I-532) ou un variant de l'un de ces rétrovirus comprenant tous les antigènes qui sont reconnus par les anticorps formés contre les antigènes correspondants de l'un quelconque des rétrovirus ayant fait l'objet des susdits dépôts à la CNCM ou à l'ECACC.

L'invention concerne encore un procédé de production du virus HIV-2 ou de variants de celui-ci dans les lignées cellulaires permanentes dérivées de lymphocytes T4, ou portant le phénotype T4, c procédé consistant à cultiver ces lignées préalablement infectées avec le virus HIV-2, et notamment lorsque le niveau d'activité de la transcriptase inverse (ou reverse-transcriptase) a atteint un seuil déterminé, à récupérer les quantités de virus libérées dans le milieu de culture.

Une lignée permanente préférée en vue de la culture de HIV-2 est, par exemple, du type cellules HUT 78. Une lignée de HUT 78, infectée par HIV-2, a été déposée le 6 février 1986 à la CNCM sous le n° I-519. La culture est, par exemple, réalisée comme suit :

Les cellules HUT 78 ($10^6$/ml) sont mises en co-cultures avec des lymphocytes humains normaux infectés ($10^6$/ml). Le milieu de culture est du RPMI 1640 avec 10 % de sérum de veau fœtal. Au bout de 15 à 21 jours, on observe un effet cytopathogène des cellules HUT 78. On dose la reverse transcriptase une semaine après cette observation, dans le surnageant de culture. On peut alors commencer à récupérer le virus à partir de ce surnageant.

Une autre lignée préférée pour la culture appartient aux lignées des cellules connues sous la désignation CEM.

L'infection, puis la culture des cellules CEM infectées peuvent notamment être réalisées comme suit.

On procède à la co-culture de lymphocytes T4 préalablement infectés avec le virus HIV-II et de cellules non infectées de la lignée CEM pendant le temps nécessaire à l'infection des CEM. On poursuit d'ailleurs ensuite les conditions de culture dans un milieu approprié, par exemple celui décrit ci-après et, lorsque l'activité transcriptase inverse des cellules infectées a atteint un niveau suffisant, on récupère le virus produit à partir du milieu de culture.

On a notamment réalisé une co-culture dans les conditions précisées ci-après de lymphocytes T4 humains qui avaient été infectés cinq jours auparavant avec une souche de virus HIV-II provenant d'un patient désigné ci-après « ROD », d'une part, et de CEM, d'autre part.

Les lymphocytes T4 infectés, préalablement activés par la phytohémaglutinine, se sont révélés posséder une activité de transcriptase inverse de 5 000 cpm/$10^6$ lymphocytes T normaux trois jours après le début de l'infection. La culture a été poursuivie jusqu'à ce que l'activité transcriptase inverse mesurée ait atteint 100 000 cpm dans le surnageant. Ces lymphocytes T4 ont alors été mis en contact avec les cellules CEM ($3.10^6$ lymphocytes T normaux infectés) et incubées dans le milieu de culture suivant : RPMI 1640 contenant 2,92 mg/ml de L glutamine, 10 % de sérum de veaux fœtal décomplémenté, 2 µg/ml de polybrène, 0,05 % de sérum anti-interféron-alpha, 100 000 µg/ml de pénicilline, 10 µg/ml de streptomycine et 10 000 µg/ml de néomycine.

Le milieu de culture est changé deux fois par semaine.

Les mesures d'activité transcriptase inverse mesurée dans le surnageant ont été les suivantes :

| | |
|---|---|
| — au jour  0 | 1 000 (bruit de fond) |
| — au jour 15 | 20 000 |
| — au jour 21 | 200 000 |
| — au jour 35 | 1 000 000 |

Une culture CEM infectée par le virus HIV-2 a été déposée à la Collection Nationale de Culture de Micro-organismes de l'Institut Pasteur (CNCM) sous le n° I-537 le 24 mars 1986.

Quelques caractéristiques des antigènes et des acides nucléiques, entrant dans la constitution de HIV-2, résultent des essais réalisés dans les conditions indiquées ci-après. Elles seront souvent mieux appréciées par comparaison avec des caractéristiques du même type se rapportant à d'autres types de rétrovirus, notamment HIV-1 et SIV.

Il sera fait référence dans ce qui suit aux dessins dans lesquels :

— les figures 1a, 1b et 1c se rapportent à des essais d'immunoprécipitation croisée entre des sérums respectivement de patients infectés par HIV-1 et HIV-2, et de macaques rhésus infectés avec STLV-III, d'une part, et des extraits viraux de HIV-1, d'autre part ;

— les figures 2a et 2b présentent des résultats comparatifs quant aux mobilités électrophorétiques des protéines de HIV-1, HIV-2 et STLV-III respectivement, dans des gels de polyacrylamide-SDS ;

— la figure 3 présente des résultats d'hybridation croisée entre des séquences génomiques de HIV-1, HIV-2 et STLV-III, d'une part, et des sondes contenant différentes séquences subgénomiques du virus HIV-1, d'autre part ;

— la figure 4 est une carte de restriction de l'ADNc dérivé de l'ARN de HIV-2 ROD ;

— la figure 5 est une carte de restriction d'un fragment E2 de l'ADNc dérivé de HIV-2, ce fragment contenant une région correspondant à la région LTR 3' de HIV-2 ;

— la figure 6 est la séquence nucléotidique d'une partie de E2, cette séquence correspondant à la région U3/R de HIV-2 ;

— la figure 7 fait apparaître :

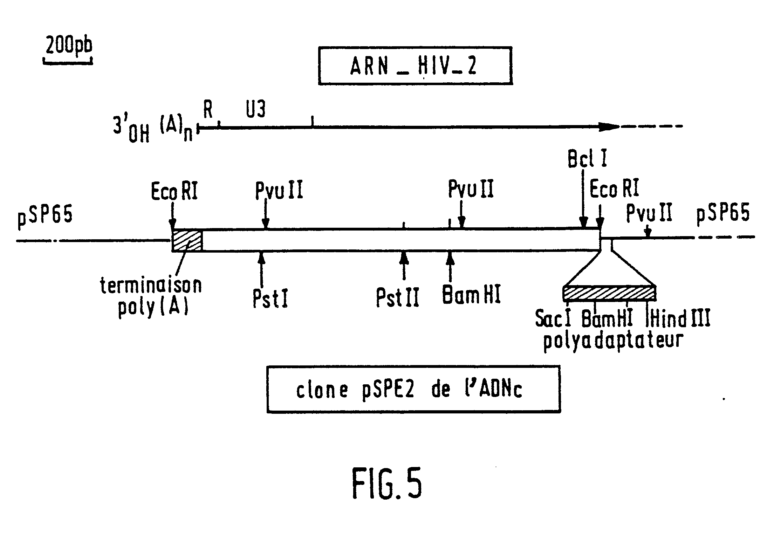

— d'une part et schématiquement, des éléments de structure de HIV-1 (figure 5A) et, alignée avec une région contenant le LTR 3' de HIV-1, la séquence dérivée de la région E2 de l'ADNc de HIV-2 et,

— d'autre part, les mises en alignement au prix d'un certain nombre de délétions et d'insertions, des nucléotides communs, contenus respectivement dans la séquence dérivée de la région E2 de HIV-2, et dans la séquence correspondante de HIV-1 (fig. 5B) ;

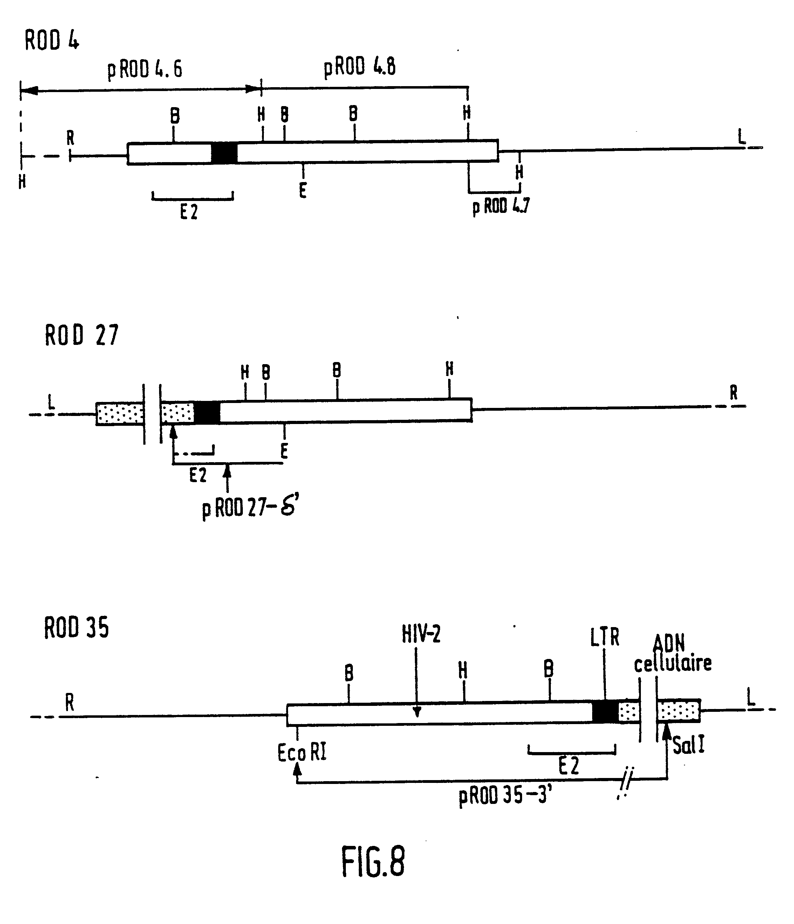

— la figure 8 montre schématiquement les structures de plusieurs clones d'un phage λ modifié par divers insérats issus de l'ADNc dérivé d'HIV-2 (clones ROD4, ROD27 et ROD35) ; on y a également représenté schématiquement des séquences dérivées à leur tour de ROD4, ROD27 et ROD35, sous-clonées dans un plasmide PUC18, ces dernières séquences étant placées en correspondance avec les régions de ROD4, ROD27 et ROD35 dont elles sont respectivement issues ;

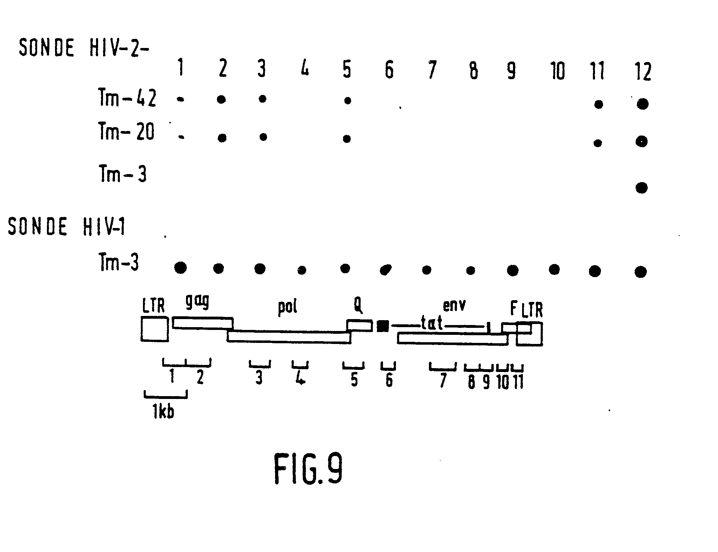

— la figure 9 fait apparaître les intensités relatives d'hybridation entre

a) onze fragments prélevés à partir de différentes régions du génome complet de HIV-1 (schématiquement représentés dans la partie inférieure de la figure d'une part, et l'ADNc de HIV-2, contenu dans ROD4, d'autre part, et

5

b) des fragments issus de HIV-II avec le même ADNc.

D'une façon générale, les antigènes de HIV-2 utilisés dans les tests comparatifs dont la description suit sont issus de la souche de HIV-2 MIR déposée à la CNCM sous le n° I-502, et les séquences d'ADN dérivés de l'ADN génomique de HIV-2 sont issus de la souche HIV-2 ROD déposée à la CNCM sous le n° I-532.

I — Antigènes, notamment protéines et glycoprotéines

Le virus initialement cultivé dans HUT 78 a été marqué métaboliquement par la $^{35}$S-cystéine et la $^{35}$S-méthionine, les cellules infectées étant incubées en présence de ces acides aminés radioactifs en milieu de culture dépourvu de l'acide aminé non marqué correspondant, pour une période de 14 à 16 heures, en particulier selon la technique décrite dans l'article répertorié en (21) dans la bibliographie présentée à la fin de la description, pour ce qui est du marquage à la $^{35}$S-cystéine. Le surnageant est ensuite clarifié, puis le virus ultracentrifugé une heure à 100 000 g sur un coussin de 20 % de saccharose. Les principaux antigènes du virus sont séparés par électrophorèse dans un gel de polyacrylamide (12,5 %), dans des conditions dénaturantes (SDS) ou dans un gel de polyacrylamide (10 %) + bis-acrylamide (0,13 %) avec SDS (0,1 % en concentration finale). On utilise comme poids moléculaire de référence les marqueurs colorés suivants :

BRL :

| | |
|---|---|
| myosine | 200 Kd |
| phosphorylase B | 97,4 Kd |
| BSA | 68 Kd |
| ovalbumine | 43 Kd |
| α chymotrypsine | 25,7 Kd |
| β lactoglobuline | 18,4 Kd |
| lysozyme | 14,3 Kd |

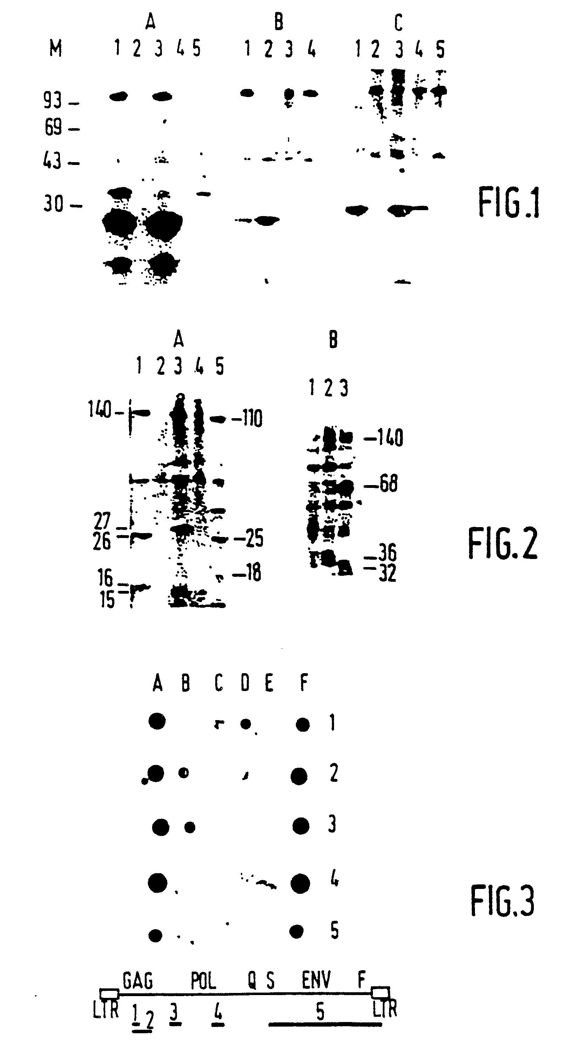

D'autres marqueurs de poids moléculaires ont été utilisés dans d'autres essais. Il est en particulier fait référence aux figures 1a, 1b et 1c qui renvoient à d'autres marqueurs de poids moléculaires connus (sous la lettre M. dans ces figures). Les antigènes sont encore mieux distingués après immunoprécipitation (RIPA) ou par immunoempreintes (Western blot) en utilisant les anticorps présents dans le sérum du malade : leurs poids moléculaires apparents déterminés par leurs migrations apparentes sont très proches de ceux des antigènes du HIV-1.

D'une façon générale il est précisé que dans ce qui suit les nombres qui suivaient les indications « p » et/ou « gp » correspondent aux poids moléculaires approximatifs des protéines et/ou glycoprotéines correspondantes divisés par 1 000. Par exemple la p36 a un poids moléculaire de l'ordre de 36 000. Il est cependant entendu que ces valeurs de poids moléculaires peuvent varier dans un intervalle pouvant atteindre 5 %, voire 10 % ou même plus, selon les techniques utilisées pour les déterminations de ces poids moléculaires.

La répétition des essais a permis de mieux cerner les poids moléculaires apparents des antigènes de HIV-2. Ainsi on a constaté que les poids moléculaires des trois protéines du noyau (core), auxquelles on avait initialement attribué des poids moléculaires de l'ordre de 13 000, 18 000 et 25 000 respectivement, avaient en fait des poids moléculaires apparents plus proches des valeurs suivantes : 12 000, 16 000 et 26 000, respectivement. Ces protéines sont ci-après respectivement désignées par les abréviations p12, p16 et p26.

De même l'existence de bandes protéine ou glycoprotéine dont les poids moléculaires apparents ont été appréciés à des valeurs qui pouvaient s'étager de 32 000 à 42 000-45 000. La répétition des mesures a finalement permis de préciser une bande correspondant à un poids moléculaire apparent de 36 000. Dans ce qui suit, cette bande est désignée par l'abréviation p36. Une autre bande à 42 000-45 000 (p42) est constamment observée aussi. L'une ou l'autre des p36 ou p42 constitue vraisemblablement une glycoprotéine transmembranaire du virus.

Une glycoprotéine majeure d'enveloppe ayant un poids moléculaire de l'ordre de 130-140 Kd est constamment observée : cette glycoprotéine est ci-après désignée par l'expression gp140. Il convient de noter que, d'une façon générale, les poids moléculaires sont appréciés avec une précision de plus ou moins 5 %, cette précision pouvant même devenir un peu moindre pour les antigènes de haut poids moléculaire, comme on l'a constaté pour la gp140 (poids moléculaire de 140 Kd ± 10 %). L'ensemble de ces antigènes sont peu ou pas reconnus (lorsqu'ils sont marqués par la $^{35}$S-cystéine) par des sérums de malades contenant des anticorps anti-HIV-1 dans les systèmes de détection utilisés au laboratoire ou par utilisation des tests mettant en œuvre des lysats de HIV-1, tels que ceux commercialisés par DIAGNOSTICS PASTEUR sous la marque « ELAVIA ». Seule la protéine p26 a été faiblement immunoprécipitée par de tels sérums. La protéine d'enveloppe ne l'a pas été. Le sérum du malade infecté par le

6

nouveau virus (HIV-2) reconnaît faiblement une protéine p34 du HIV-1. Dans le système de détection utilisé, les autres protéines du HIV-1 n'ont pas été reconnues.

En revanche le HIV-2 possède certaines protéines présentant une certaine parenté immunologique avec des protéines ou glycoprotéines structurales semblables, séparables dans des conditions analogues, à partir d'un rétrovirus récemment isolé à partir de singes macaques rhésus captifs, alors que cette parenté immunologique tend à s'effacer pour d'autres protéines ou glycoprotéines. Ce dernier rétrovirus, qui est présumé être l'agent étiologique de SIDAs chez les singes, a été désigné par les chercheurs qui l'ont isolé (références bibliographiques (16-18) ci-après) sous l'appellation « STLV-III$_{mac}$ ». Pour la commodité du langage, il ne sera plus désigné dans ce qui suit que par l'expression « STLV-III » (ou encore par l'expression SIV, abréviation anglaise de « Simian Immunodeficiency Virus » (virus d'immuno-déficience du singe)).

Un autre rétrovirus, désigné « STLV-III$_{AGM}$ », (ou SIV$_{AGM}$) a été isolé chez des singes verts sauvages. Mais, contrairement au virus présent chez le singe macaque rhésus, la présence de « STLV-III$_{AGM}$ » ne semble pas induire une maladie du type SIDA chez le singe vert d'Afrique.

Toutefois, la parenté immunologique des protéines et des glycoprotéines structurales, et par conséquent la parenté de leurs séquences nucléiques, de HIV-II d'une part, et des rétrovirus STLV-III$_{mac}$ et STLV-III$_{AGM}$ d'autre part, reste limitée. Des expériences ont permis d'établir une première distinction entre les rétrovirus capables d'infecter l'homme ou le singe, il en ressort ceci :

— le virus HIV-II ne se multiplie pas de façon chronique dans les lymphocytes du singe macaque rhésus, lorsqu'il a été injecté in vivo et dans des conditions opératoires permettant le développement du virus STLV-III$_{mac}$ telles qu'elles ont été décrites par N. L. Letvin et al., Science (1985) vol. 230, 71-75.

Cette apparente inaptitude de HIV-2 à se développer chez le singe dans des conditions naturelles, permet de différencier biologiquement le virus HIV-II d'une part, et les isolats du virus STLV-III, d'autre part.

En mettant en œuvre les mêmes techniques que celles rappelées plus haut, il a été constaté que l'on pouvait également obtenir à partir de STLV-III :

— une protéine principale du noyau p27, ayant un poids moléculaire de l'ordre de 27 kilodaltons,

— une glycoprotéine majeure d'enveloppe, gp140,

— une protéine vraisemblablement transmembranaire p32, qui n'est pas observée en RIPA lorsque le virus a au préalable été marqué par la ³⁵S-cystéine, mais qui peut être observée dans les essais d'immunoempreintes (Western blots), sous forme de bandes larges.

La glycoprotéine majeure d'enveloppe de HIV-2 s'est révélée être plus proche immunologiquement de la glycoprotéine majeure d'enveloppe de STLV-III que de la glycoprotéine majeure d'enveloppe de HIV-1.

Ces constatations s'imposent non seulement au niveau des poids moléculaires : 130-140 kilodaltons pour les glycoprotéines majeures de HIV-2 et de STLV-III, contre environ 110 pour la glycoprotéine majeure d'enveloppe de HIV-1, mais aussi au niveau des propriétés immunologiques, puisque des sérums prélevés à partir de malades infectés par HIV-2, et plus particulièrement des anticorps formés contre la gp140 de HIV-2 reconnaissent la gp140 de STLV-III, alors que dans des essais semblables les mêmes sérums et les mêmes anticorps de HIV-2 ne reconnaissent pas la gp10 de HIV-1. Mais les sérums anti-HIV-1 qui n'ont jamais réagi avec la gp140 de HIV-2 précipitent une protéine de 26 Kdal marquée par la ³⁵S-cystéine, contenue dans les extraits de HIV-II.

La protéine majeure du noyau (core) de HIV-2 semble présenter un poids moléculaire moyen (environ 26.000) intermédiaire entre celui de la p25 de HIV-1 et la p27 de STLV-III.

Ces observations résultent des essais réalisés avec des extraits viraux obtenus à partir du HIV-2 isolé à partir de l'un des patients susmentionnés. Des résultats similaires ont été obtenus avec des extraits viraux du HIV-2 isolé à partir du second patient.

Des cellules infectées respectivement avec HIV-1, HIV-2 et STLV-III ont été incubées dans un milieu contenant 200 µCi/ml de ³⁵S-cystéine dans un milieu exempt de cystéine non marquée pendant 16 heures. Les surnageants clarifiés ont été centrifugés à 60.000 g pendant 90 minutes. Les culots ont été lysés dans un tampon RIPA(1), immunoprécipités avec différents sérums, puis soumis à une électrophorèse sur gel de polyacrylamide chargé en dodécylsulfate de sodium (SDS-PAGE).

Les résultats observés sont illustrés par les figures 1a, 1b et 1c.

Dans la figure 1a, sont présentés les résultats d'immunoprécipitation observés entre un extrait viral de HIV-1 obtenu à partir d'une lignée cellulaire CEM C1.13 et les sérums respectifs suivants :

— sérum positif anti-HIV-1 (bande 1),

— sérum obtenu du premier patient mentionné plus haut (bande 2),

— sérum d'un porteur africain sain d'anticorps anti-HIV-1 (bande 3),

— sérum obtenu à partir d'un macaque infecté avec STLV-III (bande 4) et

— sérum du deuxième patient susmentionné (bande 5).

Dans la figure 1b, sont rapportés les résultats d'immunoprécipitation observés entre les antigènes de HIV-2 obtenus à partir du premier patient, après culture préalable sur des cellules HUT-78, et différents sérums, plus particulièrement le sérum du premier patient sus-mentionné (bande 1), le sérum positif anti-HIV-1 (bande 2), le sérum du macaque infecté par STLV-III (bande 3), le sérum du second patient susdit (bande 4).

Enfin, la figure 1c illustre les résultats d'immunoprécipitation observés entre les antigènes d'un isolat de STLV-III obtenu à partir d'un macaque présentant un SIDA de singe. Les sérums utilisés et auxquelles se réfèrent les bandes 1 à 5 sont les mêmes que ceux rappelés plus haut en rapport avec la figure 1a.

M se réfère aux marqueurs myosine (200 Kd) galactosidase (130 Kd), sérum albumine bovine (69 Kd), phosphorylase B (93 Kd), ovalbumine (46 Kd) et carbonate anhydrase (30 Kd).

Les figures 2a et 2b présentent les résultats comparatifs quant aux mobilités électrophorétiques des protéines de HIV-1, HIV-2 et STLV-III.

La figure 2a se rapporte aux essais réalisés avec des extraits de virus marqués par la $^{35}$S-cystéine, après immunoprécipitation sur SDS-PAGE. Les différentes bandes se rapportent aux extraits de virus suivants : virus obtenu à partir du patient 1 et immunoprécipité par le sérum provenant du même patient (bande 1), extrait du même virus immunoprécipité avec un sérum témoin négatif provenant d'une personne ne portant pas d'anticorps anti-HIV-1 ou anti-HIV-2 (bande 2), extrait de virus STLV-III immunoprécipité par un sérum provenant d'un macaque infecté par STLV-III (bande 3), immunoprécipitations observées entre des extraits du même virus et d'un sérum témoin négatif (bande 4), extrait de HIV-1 immunoprécipité par un sérum d'un patient européen infecté par le SIDA.

La figure 1b présente les résultats obtenus dans des essais de Western-blot (immunoempreinte). Des lysats cellulaires provenant de cellules HUT-78 non infectées ou infectées ont été soumis à une électrophorèse sur SDS-PAGE, puis transférés électrophorétiquement sur un filtre de nitrocellulose, avant d'être mis à réagir avec le sérum du premier patient susmentionné (sérum dilué au 1/100e). Le filtre de nitrocellulose a ensuite été lavé et la détection des anticorps fixés révélée avec des IgG antihumaines de chèvre marquées par $^{125}$I.

Les taches observées dans les bandes 1, 2 et 3 se rapportent respectivement aux essais d'agglutination entre le susdit sérum et des extraits de cellules HUT-78 non infectées (bande 1), des extraits de cellules HUT-78 infectées avec un virus HIV-2 (bande 2) et des extraits de cellules HUT-78 infectées par STLV-III (bande 3). Les nombres qui apparaissent dans les marges de chacune des bandes correspondent aux poids moléculaires approximatifs des protéines virales les plus représentatives (poids moléculaires en kilodaltons).

## II — Acides nucléiques

### 1) les ARNs du rétrovirus HIV-2

L'ARN du virus, déposé sur filtre d'après la technique du « spot blot » n'a pas hybridé, dans des conditions stringentes avec l'ADN du HIV-1.

Par « conditions stringentes » on entend : les conditions dans lesquelles la réaction d'hybridation entre l'ARN du HIV-2 et la sonde choisie marquée radioactivement au p$^{32}$ (ou marquée de façon différente), puis les lavages de la sonde sont faits. L'hybridation, sur membrane, est faite à 42 °C en présence d'une solution aqueuse 0.1 % SDS/5 × SSC, contenant 50 % de formamide (volume/volume) pendant 18 heures. La membrane sur laquelle a été faite la réaction d'hybridation est lavée ensuite à 65 °C dans un tampon contenant 0,1 % de SDS et 0,1 × SSC.

Par « conditions non stringentes », on entend les conditions dans lesquelles la réaction d'hybridation et les lavages sont faits. L'hybridation est réalisée en mettant en contact avec la sonde choisie marquée au p$^{32}$ (ou marquée autrement), à savoir à 42 °C dans un tampon 5× SSC, 0,1 % SDS contenant 30 % de formamide pendant 18 heures. Le lavage de la membrane est réalisé à 50 °C avec un tampon contenant 0,1 % de SDS et 2 × SSC.

On a également réalisé des essais d'hybridation avec une sonde d'hybridation constituée par un plasmide recombinant pBT1 obtenu par clonage de l'ADN du HIV-1 provenant de λJ19 (Cell 1985, vol. 40, p. 9) dans le vecteur pUC18. En conditions non stringentes, on n'a observé qu'une très faible hybridation entre l'ARN de HIV-2 et l'ADN cloné dérivé de HIV-1.

D'autres sondes contenant des séquences clonées de HIV-1 ont été utilisées :

a) Des sondes simple brin de l'ADN du HIV-1 subgénomique élaborées à partir de sous-clones du génome du HIV-1 et insérées dans le phage M13. Les régions clonées concernaient le gène protéase ou le gène « endonucléase ».

Seule une sonde de la région endonucléase de HIV-1 (séquence de nucléotides comprise entre les bases n° 3760 et 4130) a donné une hybridation faible en conditions non stringentes avec le HIV-2. La sonde « protéase » (séquence de nucléotides de HIV-1 comprise entre les bases n° 1680 et 1804) n'a pas hybridé, même en conditions non stringentes avec le HIV-2.

b) Une sonde pRS3 constituée par la séquence codant pour la région « enveloppe » du HIV-1 (sous-clonage dans pUC18) n'a pas donné d'hybridation en conditions non stringentes avec HIV-2.

La technique du « spot blot » est appelée aussi « dot blot » (transfert par taches).

Des résultats d'hybridation supplémentaires entre des ARNs génomiques de HIV-1, HIV-2 et STLV-III, d'une part, et des sondes contenant différentes séquences sub-génomiques du virus HIV-1, d'autre part, apparaissent dans la figure 3.

Les surnageants des différents milieux de culture (à raison de 0,5 à 1 ml pour chaque tache) ont été centrifugés pendant 5 minutes à 45.000 tours par minute ; les culots ont été remis en suspension dans un tampon NTE contenant 0,1 % de SDS et déposés sur filtre de nitrocellulose. Celui-ci a été prétrempé dans un milieu 2 × SSC (NaCl 0,3 M, citrate de sodium 0,03 M). Après cuisson (pendant 2 heures à 80 °C), les filtres ont été hybridés avec diverses sondes contenant des sous-fragments génomiques de HIV-1, dans des conditions non stringentes (30 % de formamide, 5 × SSC, 40 °C), lavés avec une solution 2 × SSC et contenant 0,1 % de SDS, à 50 °C, puis autoradiographiés pendant 48 heures à — 70 °C avec des écrans d'amplification.

Les sondes 1-4· sont des sondes à brin unique obtenues par la méthode de « coupe principale » (prime cut method) telle que décrite en (25). En bref des fragments mono-brins issus du virus M13 et portant des insérats de HIV-I subgénomiques (30) ont été ligaturés à des fragments d'oligomères (17 nucléotides) issus de M13 (BIOLABS). Le brin complémentaire a été ensuite synthétisé avec l'enzyme de Klenow dans un tampon TM (Tris 10 mM, pH 7,5, $MgCl_2$, 10 mM) en présence de dATP, dGTP, dTTP et dCTP marqués par du $^{32}$P en alpha (Amersham, 3000 Ci/mMol). L'ADN a ensuite été digéré par les enzymes de restriction appropriés, dénaturé par la chaleur et soumis à électrophorèse sur un gel dénaturant de polyacrylamide (contenant 6 % d'acrylamide, de l'urée 8 M dans un tampon TDE). Le gel a ensuite été autoradiographié pendant 5 minutes. La sonde a ensuite été découpée et éluée dans un tampon de NaCl 300 mM, SDS 0,1 %. Des activités spécifiques (AS) de ces sondes à brin unique ont été estimées à $5.10^8$-$10^9$ désintégrations par minute/microgramme (dpm/µg).

Les séquences caractéristiques contenues dans les différentes sondes étaient les suivantes :

sonde 1 : nucléotides 990-1070,
sonde 2 : nucléotides 990-1260,
sonde 3 : nucléotides 2170-2240,
sonde 4 : nucléotides 3370-3640.

Les numérotations des nucléotides qui précèdent sont celles envisagées dans l'article référencé en (30).

Enfin la sonde 5 consiste en un plasmide pUC-18 portant le fragment EcoR1-Sac1 du HIV cloné dans λJ19 (31), lequel a fait l'objet d'une translation de coupure (nick-translation) pour obtenir un AS d'approximativement $10^8$ dpm/µg.

Les dispositions relatives des fragments subgénomiques contenus dans les sondes vis-à-vis du génome entier de HIV-1 sont schématisées à la figure 3. Les différentes taches correspondent respectivement :

— tache A : virus obtenus à partir d'une culture de cellules CEM C1.13 infectées par HIV-1,
— tache B : virus obtenus à partir de cellules HUT-78 infectées par STLV-III,
— taches C et D : isolats respectivement obtenus à partir des virus des deux patients africains susmentionnés,
— tache E : extrait cellulaire témoin négatif obtenu à partir de cellules HUT-78 non infectées,
— tache F : virus obtenu à partir d'un patient du Zaïre affecté par le SIDA et ayant été cultivé sur des lymphocytes T normaux en présence de TCGF.

Toutes les taches ont été obtenues avec une quantité de virus correspondant à 25.000 dpm en activité transcriptase inverse, sauf pour les taches C : 15.000 dpm.

Les observations faites ont été les suivantes :

Les ARNs génomiques des deux isolats de HIV-2, obtenus à partir de particules virales purifiées, n'ont hybridé avec aucune des sondes dans les conditions stringentes sus-indiquées, bien que les particules virales aient été isolées et purifiées à partir de surnageants de cultures de cellules hautement infectées témoignant d'une activité transcriptase inverse élevée.

Dans les conditions non stringentes sus-indiquées, les observations suivantes ont été faites.

Toutes les sondes ont hybridé de façon intense avec les ARNs génomiques obtenus des préparations témoins de HIV-1 et d'un autre isolat obtenu à partir d'un patient du Zaïre souffrant du SIDA.

Deux des sondes obtenues (nucléotides 990-1070 et 990-1260, toutes deux issues de la région gag de HIV-1) ont hybridé légèrement avec les taches des extraits des rétrovirus HIV-2 ; l'une seulement de ces deux sondes (nucléotides 990-1260) a aussi montré une légère hybridation avec la tache de STLV-III (figure 3). En ce qui concerne la sonde contenant un fragment de la région pol (nucléotides 2170-2240), on a observé une hybridation avec STLV-III et, mais de façon beaucoup plus faible, avec l'ARN de HIV-2. L'autre sonde de la région pol (nucléotides 3370-3640) n'a donné d'hybridation avec aucune des taches de HIV-2 et de STLV-III.

Enfin, la sonde modifiée par translation de coupure et contenant la totalité du gène env et le LTR (nucléotides 5290-9130) de HIV-1 n'a hybridé ni avec les ARNs de STLV-III, ni avec ceux de HIV-2.

On remarquera encore qu'une autre sonde, qui contenait l'extrémité 5' du cadre de lecture de pol de HIV-1 (correspondant à la région protéase) n'a hybridé ni avec les ARNS de HIV-2, ni avec les ARNs de STLV-III.

Il résulte donc encore de ce qui précède que le virus HIV-2 paraît plus éloigné, sur le plan structural, du virus HIV-1 qu'il ne l'est de STLV-III. HIV-2 diffère néanmoins de façon significative de STLV-III, ce qui vient à l'appui des résultats différents observés quant aux capacités infectieuses des virus HIV-2 pratiquement nulles chez les singes, comparés aux capacités infectieuses certaines des STLV-III chez les mêmes espèces de singes.

Les cartes de restriction et les séquences de l'ARN génomique de HIV-2 ou des cADNs obtenus à partir de ces ARNs génomiques sont accessible à l'homme du métier, dès lors que les souches de HIV-2 déposées de la C.N.C.M. peuvent, après multiplication appropriée, mettre en ses mains le matériel génétique nécessaire aux déterminations de ces cartes de restriction et séquences nucléotidiques. Les conditions dans lesquelles la carte de restriction du génome de l'un des isolats de HIV-2 de cette invention ont été établies et les conditions dans lesquelles certaines des parties de ADNc dérivées de ces génomes ont été séquencées sont décrites ci-après.

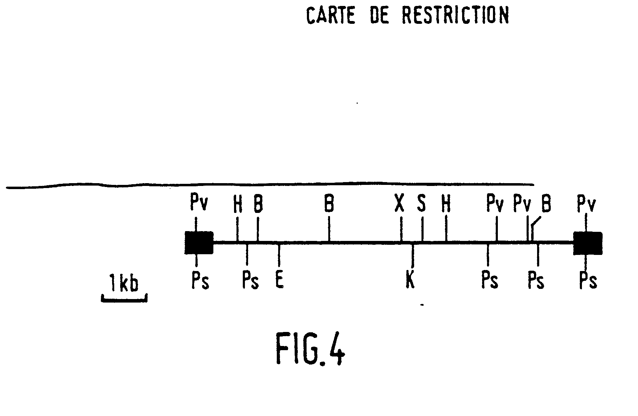

La carte de restriction du génome d'un rétrovirus représentatif des rétrovirus HIV-2 est représentée dans la figure 4. La carte de restriction d'un fragment important de cet ADNc apparaît à la figure 5. Enfin une partie de ce dernier fragment a été séquencé.

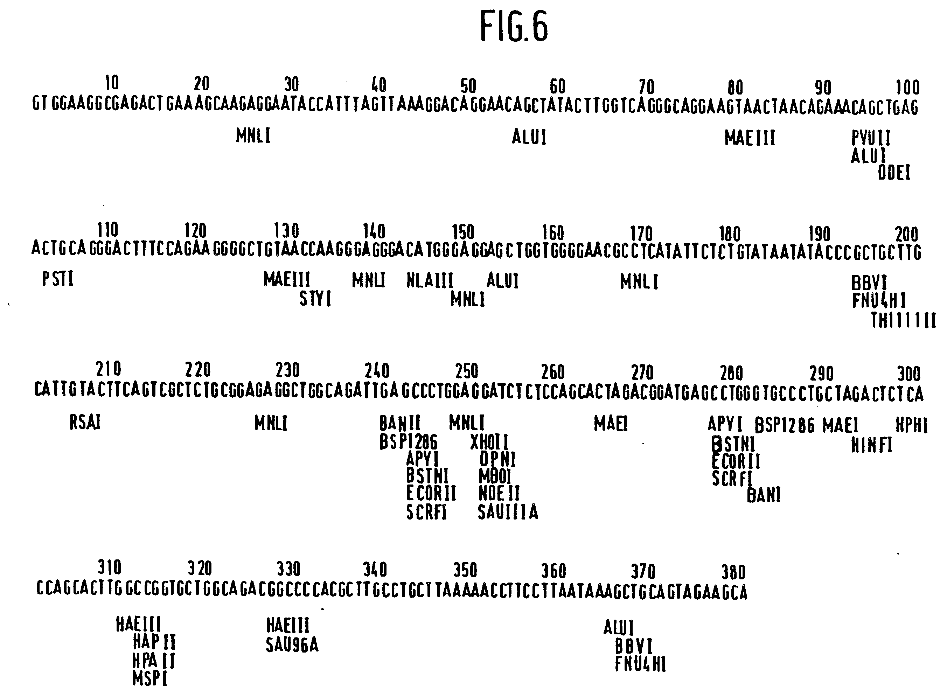

Cette séquence et un certain nombre des sites de restriction qu'elle contient apparaissent à la figure 6. L'ADNc entier cloné — ou des fragments clonés de cet ADNc — peuvent eux-mêmes être utilisés comme sondes d'hybridation spécifiques.

2) ADNc et fragments de cet ADNc, respectivement dérivés de l'ARN de HIV-2

Les conditions dans lesquelles l'ADNc susdit a été obtenu, sont décrites ci-après.

La première étape de fabrication de cet ADNc a compris la production d'un oligo (dT) servant d'amorce ou de brins d'ADNc initiateurs, pour la réalisation d'une réaction endogène activée par un détergent, utilisant la transcriptase inverse de HIV-2, sur des virions purifiés, obtenus à partir de surnageants de cellules CEM infectées. La lignée cellulaire CEM était une lignée cellulaire lymphoblastoïde CD4+ décrite par G. E. Foley et al. dans Cancer 18 : 522-529 (1965), dont le contenu est considéré comme faisant partie de la présente description. Ces cellules CEM utilisées sont infectées avec un isolat ROD, qui s'est révélé produire de façon continue des quantités importantes de HIV-2.

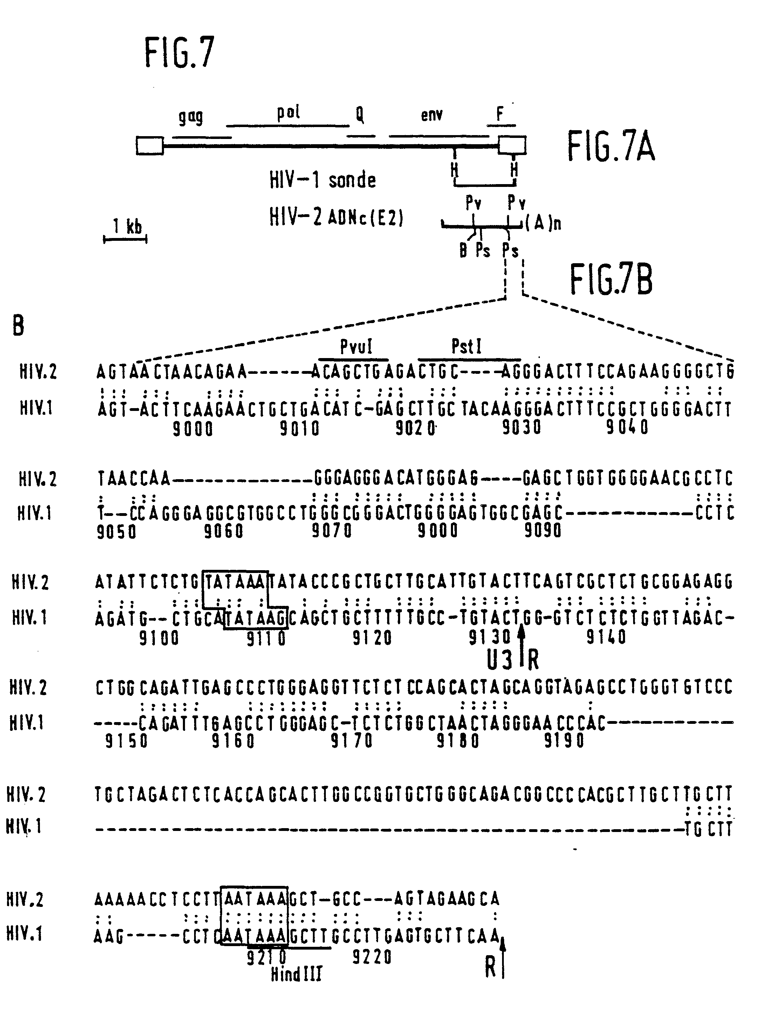

Après la synthèse du second brin (en présence de nucléotides et d'une ADN polymérase bactérienne), les cADNs double-brins ont été insérés dans un vecteur de phage bactérien M13 TG130. Une banque de phages de $10^4$ phages recombinants M13 a été obtenue et soumise à un tri in situ avec une sonde HIV-1. Celle-ci contient un fragment de 1.5 kb issu de l'extrémité 3' de l'ADNc dérivée de l'ARN de l'isolat LAV (montré dans la figure 7 A). Environ 50 plaques positives ont été détectées, purifiées et caractérisées par une hybridation croisée des insérats et séquençage des extrémités.

Cette procédure a permis l'isolement de différents clones contenant des séquences approximativement complémentaires de l'extrémité 3' de l'ARN polyadénylé de la région de « répétition longue terminale » LTR (abréviation anglaise de « long terminal repeat » de HIV-1, décrite par S. Wain Hobson et al. dans Ceil 40 ; 9-17 (1985)) dont le contenu est considéré ici comme faisant partie de la description.

Le plus important des insérats du groupe concerné de clones de M13 hybridant avec la région LTR3' de HIV-1, est un clone désigné par E2, d'environ 2 kb. Comme la région LTR3' de HIV-1, le clone E2 contient un signal AATAAA situé à environ 20 nucléotides en amont d'une partie terminale poly A, et une région LTR3' correspondant à celle de HIV-2. Après séquençage partiel, cette région LTR3' de HIV-2 s'est révélée présenter une parenté éloignée avec le domaine homologue de HIV-1.

La figure 5 est une carte de restriction du fragment de E2 (zone rectangulaire allongée) incorporé dans le plasmide pSPE2 qui le contient. Il comprend une partie de la région R, la région U3 de HIV-2. Le dessin ne fait pas apparaître les limites des régions R et U3.

La séquence d'une partie de E2 apparaît à la figure 6. Les positions de sites de restriction spécifiques y sont indiquées. Le faible degré de parenté entre les régions LTR3' de HIV-1 et HIV-2 est illustré par la figure 7. En effet, à peu près 50 % seulement des nucléotides des deux LTR peuvent être mis en alignement (homologie de séquence d'environ 50 %) au prix d'une centaine d'insertions ou de délétions. En comparaison, l'homologie de séquence des régions correspondantes des isolats américains et africains différents variants de HIV-1 est supérieure à 95 %, sans insertion ni délétion.

Le clone E2 a été utilisé comme sonde spécifique d'HIV-2, pour l'identification sur filtre d'hybridation des séquences issues de HIV-2, contenues dans d'autres clones.

Cette sonde détecte également l'ARN génomique de HIV-2 dans des conditions stringentes. Elle permet, de même, la détection par la méthode dite de « Southern blot » sur l'ADN de cellules CEM ou analogues infectées avec un isolat ROD ou d'autres isolats de HIV-2. Aucun signal n'est détecté dans les mêmes conditions de stringence dans des essais d'hybridation de cette sonde avec des ADNc issus de cellules non infectées ou de cellules infectées par HIV-1. Ces résultats ont confirmé la nature exogène de HIV-2 vis-à-vis de HIV-1. Une espèce d'environ 10 kb, correspondant probablement à l'ADN viral non intégré, a été détecté à titre principal dans l'ADN non digéré de cellules infectées par HIV-2. Un autre ADN ayant une taille apparente de 6 kb correspondant peut être à une forme circulaire de l'ADN viral, a été détecté aussi.

Les autres parties du génome de HIV-2 ont été identifiées également. A cet effet, une banque génomique a été construite dans le phage lambda L47. Le phage lambda L47.1 a été décrit par W. A. M. Loenen et al. dans Gène 10 :249-259 (1980), publication dont le contenu est considéré comme faisant partie de la présente description.

La banque génomique est construite avec des fragments obtenus par digestion de l'ADN provenant de la lignée cellulaire CEM infectée avec HIV-2$_{ROD}$, après digestion avec l'enzyme Sau3A.

Environ $2 \times 10^6$ plaques de recombinants ont été triées in situ avec un clone contenant l'insérat E2 marqué de l'ADNc de HIV-2. Dix phages recombinants ont été détectés sur plaques et purifiés. Les cartes de restriction de trois de ces phages, caractérisés par leur capacité d'hybridation en « Southern blot » avec l'insérat E2 dans des conditions stringentes, ainsi qu'avec des sondes subgénomiques de HIV-1 dans des conditions non-stringentes.

Un clone portant un insérat de 9.5 kb et dérivant de l'ADN viral circulaire entier, contenant le génome complet de HIV-2, a été identifié. Il a été désigné « Lambda ROD 4 ». Les deux autres clones, Lambda ROD 27 et Lambda ROD 35 dérivant de provirus intégrés, portant des séquences LTR des séquences codantes virales et des séquences LTR des séquences codantes virales et des séquences adjacentes d'ADN cellulaire. Les différentes séquences découlent de la figure 8.

Des fragments des clones Lambda ont été sous-clonés dans le vecteur plasmidique pUC 18. Les fragments issus de λ ROD 4, λ ROD 27 et λ ROD 35 et respectivement sous-clonés dans le susdit vecteur plasmide apparaissant également à la figure 8. Les sous-clones suivants ont été obtenus.

— pROD 27-5 dérive de Lambda ROD 27, contient une région de 5,2 kb du génome de HIV-II et des séquences cellulaires adjacentes (5'LTR et séquence virale codante 5' autour d'un site ECo RI).

— pROD 4.8 dérivé de Lambda ROD 4, contient un fragment Hind III d'environ 5 kb. Ce fragment correspond à la partie centrale du génome HIV-2.

— pROD 27-5' et pROD 4.8 contiennent des insérats de HIV-2 qui se chevauchent mutuellement.

— pROD 4.7 contient un fragment HIND III de 1.8 kb de Lambda ROD 4 ; ce fragment est placé dans la direction 3 vis-à-vis du fragment sous-cloné dans p ROD 4.8 et contient environ 0.8 kb de séquences virales codantes et une partie située entre les sites de clonage Bam H1 et Hind III du bras gauche du phage Lambda (Lambda L 47.1).

— pROD 35 contient toutes les séquences HIV-2 codantes dans la direction 3, vis-à-vis du site Eco R1, l'extrémité 3' LTR et environ 4 kb de séquences nucléiques adjacentes d'origine cellulaire.

— pROD 27-5' et pROD 35 contenus dans E. Coli HB 101, ont été déposés à la CNCM sous les numéros respectifs I-626 et I-633.

pROD 4.7 et pROD 4.8, contenus dans E. Coli TG1, ont été déposés à la CNCM respectivement sous les numéros 1-627 et 1-628.

Le génome complet de HIV-2 ROD, dont la carte de restriction apparaît à la figure 4, a été reconstitué à partir de pROD 35, linéarisé au préalable avec Eco R1, et de pROD 27-5'. L'insérat Eco R1 de pROD 27-5 a été ligaturé dans l'orientation correcte dans le site EcoRI de pROD 35.

Le degré de parenté entre HIV-2 et les autres rétrovirus humains ou simiens a été apprécié par des expériences d'hybridation mutuelle. L'homologie relative entre les différentes régions de génomes de HIV-1 et de HIV-2 a été déterminée par des essais d'hybridation de fragments respectivement issus de génome de HIV-1 cloné et de Lambda ROD 4 marqué radioactivement. Les positions relatives de ces fragments (numérotés de 1 à 11) vis-à-vis du génome de HIV-1 apparaissant au bas de la figure 9.

Même dans des conditions de très faible stringence (TM-42 °C) les génomes de HIV-1 et HIV-2 n'hybrident qu'aux niveaux de leurs gènes respectifs gag (taches 1 et 2), des régions reverse transcriptase dans pol (tache 3), des régions d'extrémités de pol, des gènes Q (ou sor) (tache 5) et des gènes F (ou 3' orf) et 3' LTR (tache 11). Le fragment HIV-1 utilisé pour détecter les premiers clones ADNc de HIV-2 correspond au sous-clone de la tache 11, qui hybride relativement bien avec HIV-2 dans des conditions non stringentes. Un signal provenant de la tache 5 est le seul persistant après un lavage stringent. Le gène d'enveloppe, la région du gène tat et une partie de pol apparaissent très divergents. Ces données, ainsi que la séquence obtenue avec LTR (figure 3) démontrent que HIV-2 n'est pas (en tous les cas au niveau de son enveloppe) un variant de HIV-1.

On observe que HIV-2 est plus proche de SIV (décrit par M. D. Daniel et al. dans Science 228 : 1201-1204 (1985), dont le contenu doit être considéré comme faisant partie de la présente description) qu'il ne l'est de HIV-1.

Toutes les protéines de SIV, y compris la protéine de l'enveloppe, sont immuno-précipitées par des sérums de patients infectés par HIV-2, tandis que la réactivité sérologique croisée de HIV-1 et HIV-2 est limitée aux protéines du noyau. Cependant SIV et HIV-2 peuvent être distingués par les différences mentionnées plus haut au niveau des poids moléculaires de leurs protéines.

En ce qui concerne les séquences nucléotidiques, on remarque aussi que HIV-2 a une parenté avec SIV.

De plus, la caractérisation de HIV-2 permet aussi de délimiter le domaine de la glycoprotéine d'enveloppe qui est responsable de la fixation du virus à la surface des cellules cibles et de l'internalisation ultérieure du virus. L'interaction se fait par l'intermédiaire de la molécule CD4 elle-même et il semble que HIV-1 et HIV-2 utilisent le même récepteur. Aussi, bien qu'il existe de grandes différences entre les gènes env de HIV-1 et HIV-2, les domaines homologues restreints des enveloppes des deux HIV peuvent-ils être considérés comme constituants de fixation à un récepteur commun des lymphocytes T4.

Ces sites sont appelés à constituer des épitopes porteurs de l'immunogénicité de peptides qui pourraient être utilisés pour susciter chez l'homme une réponse immunitaire protectrice contre les virus HIV.

Des séquences avantageuses pour la constitution de sondes dans des réactions d'hybridation avec le matériel génétique de patients porteurs de virus ou de provirus, notamment pour détecter la présence d'ARN de virus HIV-2 dans leurs lymphocytes contiennent une séquence nucléotidique résultant de combinaison des fragments de 5 kb de HindIII de ROD 4 et de l'ADNc E2. Les essais peuvent être réalisés selon toutes méthodes notamment selon les techniques de « Northern blot », « Southern blot » et « dot blot ».

Des caractéristiques supplémentaires de l'invention apparaîtront encore de façon non limitative au cours de la description qui suit d'exemples d'identification de certaines parties du génome rétroviral et de la production d'un certain nombre d'ADNs recombinants mettant en jeu diverses parties d'un ADNc dérivé du génome rétroviral de HIV-2.

Exemples

Exemple I

Sonde ADN, pour l'utilisation dans les kits de diagnostic de HIV-2.

Un ADNc complémentaire de l'ARN du génome obtenu à partir de virions purifiés, a été préparé selon la méthode suivante :

Le surnageant obtenu après 48 heures de culture de cellules CEM infectées par un isolat HIV-2 ROD de HIV-2 a été ultracentrifugé. Le culot de centrifugation contenant le virion a été centrigué sur gradient de sucrose pour former un nouveau culot de centrifugation substantiellement par la même méthode que celle décrite dans la demande de brevet européen EP-A-0.138.667 déjà mentionnée.

La préparation de HIV-2 purifié a été utilisée pour la synthèse d'ADNc en mettant en œuvre une réaction endogène, activée par un détergent.

En résumé, la préparation du virion a été ajoutée à un mélange de réaction contenant 50 mM Tris-HCl, 5 mM $MgCl_2$, 10 mM DTT, 0.025 % du détergent commercialisé sous la marque TRITON®, et 50 μM de chacun des 4 déoxynucleoside-triphosphates et un initiateur oligo (dT). La réaction a été conduite pendant 90 minutes à 37 °C.

Après l'extraction au phénol des protéines présentes dans le premier milieu de réaction, la seconde chaîne de l'ADNc a été synthétisée en présence de RNAse, ADN polymerase 1 de E. coli et des 4 déoxynucléotides, pendant 1 heure à 15 °C et 1 heure à 22 °C. Des extrémités franches ont été créées sur cette double chaîne ADNc, par l'action de la T4 ADN polymérase. Tous les réactifs de ce procédé sont disponibles dans le commerce (kit ADNc de la Société AMERSHAM) et ont été utilisés comme recommandé par le fournisseur.

Après (1) ligation d'adaptateurs (linkers) contenant un site EcoR1 (commercialisés par Pharmacia) aux extrémités franches de l'ADNc en présence d'une ligase ADN T4 (commercialisée par BIOLABS) (2), digestion de ces « linkers » par l'endonucléase de restriction EcoR1, et (3) élimination des fragments de « linker » par une filtration sur gel (colonne du gel commercialisé sous la marque ULTROGEL®) AcA 34 (LKB-IBF), l'ADNc est inséré dans un vecteur M 13 TG 130 clivé par EcoR1. Une banque d'ADNcs a été obtenue après transformation de la souche de E. coli TG1. On a obtenu approximativement $10^4$ plaque de recombinants M13.

Pour sélectionner dans la banque d'ADNcs des clones M13 recombinants contenant l'ADNc de HIV-2, la technique d'hybridation sur plaque a été utilisée. L'ADN des plaques M13 a été transféré sur des filtres de nitrocellulose, et hybridé à des sondes de HIV-1 subgénomiques dérivées du clone « lambda J19 » d'un virus LAV (ou HIV) décrit dans la demande de brevet européen. Cette sonde comprenait un insérat constitué par une partie ayant une longueur approximative de 1500 paires de bases (pb) de l'ADN de HIV-1. Cet insérat était délimité par deux sites de restriction HindIII respectivement à l'intérieur du cadre de lecture ouverte du gène « env » et dans le segment R de l'extrémité 3' LTR de HIV-1. Cette sonde contenait l'extrémité 3' du gène env, la totalité du gène F, le segment U3 et une partie du segment R de LTR, d'une longueur approximative de 1500 paires de bases (pb).

La sonde contenant l'insérat HindIII de 1,5 kb a été marquée avec du $^{32}P$ dCTP et dTTP (3000 Ci × 10$^{-3}$ mole) par incubation de la sonde en présence d'initiateurs et d'ADN polymerase I de Klenow, pendant 4 heures à 15 °C (en utilisant un kit de AMERSHAM). Les essais d'hybridation des clones d'ADNc de la banque ont été conduits pendant toute la nuit dans des conditions de faible stringence, au sein d'une solution d'un milieu d'hybridation contenant 5X SSC, 5X Denhart, 25 % de formamide, 100 μg/ml d'ADN de sperme de saumon dénaturé et la sonde marquée ($2 × 10^7$ cpm avec une spécificité de $10^9$ cpm/ug) à 37 °C. Les filtres ont été soumis à trois étapes de lavage, successivement en présence des trois solutions dont les compositions sont indiquées ci-après : Lavage n° 1 :5XSSC, 0.1 % SDS, à 25 °C pendant 4 × 15 minutes. Lavage n° 2 :2XSSC, 0.1 % SDS, à 42 °C pendant 2 × 30 minutes. Lavage n° 3 :0.1XSSC, 0.1 % SDS, à 65 °C pendant 2 × 30 minutes. Chaque lavage est suivi par une autoradiographie des filtres.

Plusieurs clones positifs ont été détectés après le lavage n° 1 et l'ont encore été après le lavage n° 2. Cependant, tous les signaux disparaissaient après le lavage n° 3. Ceci indique que les clones positifs

12

n'avaient avec le génome de HIV-1 qu'une faible parenté, néanmoins suffisante pour effectuer la susdite sélection. Les clones positifs ont été repiqués, redéposés sur des plaques et de nouveau hybridés avec la même sonde dans les conditions de stringence correspondant au lavage n° 1. La majorité d'entre-eux étaient encore positifs.

Les clones ont aussi été sélectionnés en mettant en œuvre une sonde d'ADN humain total dans des conditions de stringence moyenne et par hybridation dans 5X SSC, 5X Denhart, et 40 % de formamide, suivie d'un lavage dans 1X SSC, 0.1 % SDS, à 50 °C. Aucun des clones préalablement positifs n'a été détecté, par conséquent ne correspondait pas à l'ADN répétitif spécifique ou à l'ADNc de l'ARN ribosomal.

Les clones recombinants M13 positifs ont été cultivés dans un milieu liquide et caractérisés :

(1) Taille de leur insertion :

Un ADN type simple brin de M13 a été obtenu à partir de chaque clone individuel, et la synthèse du second brin a été conduite avec une séquence initiatrice 17-mer M13 et l'enzyme de Klenow. Les insérats ont été excisés à l'aide d'EcoR1 (BOEHRINGER) et analysés par électrophorèse sur gel d'agarose. La plupart des insérats contenaient de 200 à 600 et 200 pb, à l'exception du clone dénommé E2.1, qui avait approximativement une longueur de 2 Kpb.

(2) Analyse de la séquence nucléotidique :

Plusieurs clones ont été partiellement séquencés, en utilisant la méthode dideoxy de Sanger et al. décrite dans Proc. Natl. Acad. Sci. 74 :5463-7 (1977), faisant partie de la présente description. Différents clones indépendants contenaient des séquences nucléotidiques similaires, à l'exception des chaînes poly-A à leurs extrémités 3′ dont les longueurs étaient différentes. Ces résultats démontrent que ces clones ADNc étaient dérivés dans la matrice d'ARN. L'analyse de séquence détaillée de ces clones ADNc, comprenant l'extrémité 3′ du génome de HIV-2, a montré une parenté limitée avec HIV-1.

(3) Hybridation avec l'ARN et l'ADN génomique de HIV-2.

(a) Obtention de l'ARN génomique de HIV-2 : Un surnageant infecté a été centrifugé (50.000 rotations, 30 minutes). Le culot du dépôt à été resuspendu dans 10 mM Tris pH 7.5, 1 mM EDTA, 0.1 % SDS. L'un des clones d'insertion F1.1 a été marqué et utilisé comme sonde pour l'hybridation avec l'ARN génomique de différents isolats viraux, conformément à la technique de « dot-blot ».

La technique « dot-blot » comprenait les étapes suivantes :

(i) déposer l'échantillon (lysat HIV-2) en taches sur une membrane de nitrocellulose préalablement trempée dans 20X SSC (3M NaCl, 0.3M de citrate de sodium) et séchée à l'air, (ii) faire cuire la membrane pendant 2 heures à 80 °C, et (iii) effectuer l'hybridation.

Cette hybridation a été conduite dans des conditions de haute stringence (5X SSC, 5X Denhart, 50 % formamide à 42 °C). Elle a été suivie d'un lavage dans 0.1X SSC, 0.1 % SDS à 65 °C. Dans ces conditions, la sonde hybride fortement aux taches provenant de deux isolats indépendants de HIV-2, incluant le LAV-II ROD, dont l'ADNc cloné était issu. Un faible signal d'hybridation a été détecté avec la tache formée par STLV-IIImac (Virus T-Lymphotropique de Singe (également connu comme « SIV ») de type III, macaque), et aucune hybridation n'a été détectée avec les isolats de HIV-1.

Les expériences de « Southern Blot », mettant en jeu de clone E-2.1 contenant l'inserat de 2 Kb en tant que sonde marquée au $^{32}$P, ne manifestèrent aucune hybridation avec des ADNs de cellules non infectées, mais détectaient des bandes dans des cellules détachées infectées par HIV-2 dans des conditions de forte stringence. HIV-2 montre un polymorphisme à deux niveaux de sa carte de restriction équivalents à ceux des cartes de restriction de HIV-1. Avec de l'ADN cellulaire complet de cellules infectées, deux sortes de signaux sont détectés par la méthode de « Southern Blot » : (1) dans des fractions d'ADN ayant des poids moléculaires PM d'environ 20 kb et plus dans le cas de formes intégrées du virus, et (2) dans les fractions de PM plus faibles (9, 10 kb), dans le cas de virus non intégré au génome.

Ces caractéristiques sont hautement spécifiques d'un rétrovirus.

Certaines expériences effectuées avec STLV-III (SIV-3) de cellules infectées ont permis de constater que le rétrovirus simien est relativement distant de HIV-2 (le signal est uniquement détecté dans des conditions de faible stringence). Ces expériences montrent que les susdites sondes permettent la détection spécifique de HIV-2.

(4) Sous-clonage de l'ADNc de HIV-2 dans un vecteur plasmide bactérien :

Le clone M13 positif, E 2-1 a été sélectionné et sous-cloné dans un vecteur plasmidique. L'ADN du phage M13 (TG 130) recombinant E-2 a été purifié sous forme d'un ADN simple brin (M-13-ROD-E2) contenant l'inserat de 2 kb, contenant la portion 3′ du génome de HIV-2 (obtenu à partir de HIV-2 ROD). Cet inserat a été transféré dans le plasmide pSP65, décrit par Melton, D. A. dans 357 Nucleic Acid Res. 12 :035-7056 (1984).

Une seconde chaîne a été construite in vitro en présence de la séquence d'initiateur 17 mer (AMERSHAM), des quatre nucléotides A, C, T, G, et de l'ADN polymerase I (Klenow). L'inserat EcoR1 a été excisé par digestion EcoR1 et purifiée sur gel d'agarose, puis ligué à pSP65, lui-même préalablement digéré avec EcoR1. Le mélange de ligation a été utilisé pour transformer la souche E. coli DH1 et des recombinants ont été sélectionnés grâce à leur capacité de résistance à l'ampicilline. Les recombinants identifiés ont été cultivés sur milieu LB (Luria medium) contenant 50 μ/ml d'ampicilline. Ces plasmides recombinants ont été purifiés et contrôlés quant à la présence du fragment inséré correct.

L'un des clones obtenus désigné par la référence pSPE2, a été déposé à la CNCM à PARIS, France, sous le n° d'accès I-595 le 5 septembre 1986.

Les insérats dérivés des ADNc de HIV-2 et qui se trouvaient insérés dans la sonde susdite contenaient la séquence de nucléotides qui a été définie plus haut, en rapport avec une partie de E2.

### Exemple II

Clonage d'un ADNc complémentaire à l'ADN complémentaire à l'ADN génomique de virions de HIV-2.

Des virions HIV-2 ont été purifiés à partir de 5 litres d'un surnageant d'une culture d'une lignée CEM infectée avec un isolat ROD. Un premier brin d'ADNc a été synthétisé au contact de virus purifié sédimenté, en présence d'un initiateur oligo (dT) et en mettant en œuvre une réaction endogène activée par un détergent, selon la technique décrite par Alizon et al., Nature 312 : 757-760 (1984). Les hybrides ARN-ADNc ont été purifiés par une extraction par un mélange phénolchloroforme et par une précipitation à l'éthanol. Le second brin d'ADNc a été produit en présence d'ADN polymérase-I/RNAse H selon la méthode décrite par Gubler et Hoffman Gene 25 : 263-269 (1983). La description de cet article est considérée comme faisant partie de la présente description.

L'ADNc double brin a été muni d'extrémités franches en présence d'ADN polymérase T4 en utilisant les constituants d'un kit de synthèse d'ADNc commercialisé par AMERSHAM.

Des adaptateurs (linker) Eco R1 commercialisés par PHARMACIA, ont été fixés à l'extrémité de l'ADNc ; l'ADNc ainsi modifié a été inséré après digestion en présence de Eco R1 dans un vecteur de phage déphosphorilé M13tg130 lui-même digéré par Eco R1, également commercialisé par AMERSHAM. Une bande d'ADNc a été obtenu après transformation de la souche E. coli TG1. Des plaques de recombinants ($10^4$) ont été triés in situ sur des filtres permettant des répliques par hybridation avec le clone J19 contenant le fragment Hind III de 1,5 kb déjà mentionné plus haut, issu de HIV-1.

Les filtres ont été préhybridés en présence d'un milieu contenant 5 × SSC, 5 × solution DENHARDT, formamide à 25 %, de l'ADN dénaturé de sperme de saumon (100 microgrammes/mml) à 37 °C, pendant 4 heures, puis hybridé pendant 16 heures dans le même tampon (Tm — 42 °C) en présence d'un supplément de sonde marquée ($4 \times 10^7$ cbm), pour fournir une solution finale de tampon d'hybridation contenant $10^6$ cpm/ml). Le lavage a été fait avec une solution 5 × SSC, 0,1 % SDS à 25 °C pendant 2 heures (étant entendu que 20 × SSC correspond à une solution NaCl 3M et de citrate de sodium 0,3M. Les plaques répondant positivement ont été purifiées et les ADNs mono-brins de M13 ont été préparés et leurs extrémités ont été séquencées selon la méthode de Sanger et al.

Hybridation d'un ADN de cellules infectées par HIV-1 et HIV-2 et d'ARNs de HIV-1, HIV-2 et de virions SIV respectivement avec une sonde dérivée d'un ADNc cloné de HIV-2.

Les ADNs ont été extraits de cellules CEM infectées produisant de façon continue respectivement HIV-1 et HIV-2. Des échantillons d'ADN de ces deux rétrovirus, digérés pour certains par 20 ug de Pstl, non digérés pour d'autres ont été soumis à une électrophorèse sur gel d'agarose à 0,8 % et transféré par la méthode « Southern » sur membrane de nylon. Des petits volumes de surnageant infecté, repris dans un tampon NTE contenant 0,1 % de SDS ayant les mêmes activités de transcriptase inverse ont été déposés sur nitrocellulose, qui avait été trempé auparavant dans une solution 2 × SSC.

Une préhybridation a été faite dans une solution contenant 50 % de formamide, 5 × SSC, 5 × Denhardt, et 100 mg/ml d'ADN de sperme de saumon dénaturé pendant 4 heures à 42 °C. Une hybridation a été conduite dans le même tampon, auquel avait été ajouté 10 % de sulphate de dextran, et $10^6$ cpm/ml. d'insérat marqué E2 (activité spécifique $10^9$ cpm/ug) pendant 16 heures à 42 °C. Deux lavages ont ensuite été faits avec une solution 0.1 × SSC, 0,1 % SDS pendant 30 mn chacun. Après l'exposition pendant 16 heures avec un écran d'intensification la membrane de Southern Blot est déshybridée dans 0.4 N NaOH, neutralisée et réhybridée dans les mêmes conditions à la sonde HIV-1 marquée avec $10^9$ cpm/ug.

### Exemple III

Clonage dans le Phage Lamdba de l'ADN complet du provirus de HIV-2.

L'ADN des cellules CEM infectées par HIV-IIROD (figure 2, bandes a et c) est partiellement digéré avec Sau3A. La fraction 9-15 kb a été sélectionnée sur gradient de sucrose a 5-40 % et liguée aux bras BamHI du vecteur lambda L47.1. Les plaques ($2 \times 10^6$) obtenues après empaquetage in vitro et dépôt sur souché E. coli LA 101 ont été triées in situ par hybridation avec l'insérat de l'ADNc cloné E2. Approximativement, 10 clones positifs ont été purifiés sur plaque et propagés sur E. coli C600 recBC. Les clones lambda ROD 4, ROD 27 et ROD 35 ont été amplifiés et leurs ADN caractérisés par établissement de leurs cartes de restriction et par hybridation par la méthode de « Southern » avec le clone ADNc de HIV-II dans des conditions stringentes et avec les ondes gag-pol de HIV-1 dans des conditions non stringentes.

La figure 8 présente de façon schématique les structures de 3 des phages recombinants obtenus ROD 4, ROD 27 et ROD 35.

Les parties rectangulaires allongées de ces schémas correspondent à des séquences pro-virales issues des ADNs des cellules CEM initialement infectées, les parties blanches correspondant à des séquences rétrovirales, les parties grisées à des parties des ADNs cellulaires et les parties en noir au LTR dans lesdites fréquences virales.

Les traits fins désignés par les lettres L et R correspondent aux bras issus du vecteur de phage Lambda L47.1 ayant servi au clonage.

Certains des sites des restrictions ont également été indiqués : il s'agit plus particulièrement des sites suivants :

B : BamHI ; E : EcoRI ; H : HindIII ; K : KpnI ; Ps : PstI ; Pv : PvuII ; S : SacI ; X : XbaI.

Ces séquences virales ont des parties en commun avec la séquence E2. Les positions relatives de ces parties déterminées par hybridation avec E2, apparaissent également dans les figures.

ROD 4 est dérivé d'un ADN viral circulaire. ROD 27 et ROD 35 sont dérivés de provirus intégrés dans une structure ADN cellulaire.

Enfin, on voit apparaître dans ces figures les insérats sous-clonés dans les conditions qui ont été indiquées plus haut et leurs positions relatives vis-à-vis des séquences correspondantes de ROD 4, ROD 27 et ROD 35.

Il s'agit plus particulièrement des insérats des plasmides pROD 27-5′, pROD 35-3′, pROD 4.6, pROD 4.8 et pROD 4.7.

La figure 9 est une représentation des intensités relatives des taches d'hybridation qui ont été réalisées entre ROD-4 et des sous-fragments 1 à 11 respectivement issus des différentes parties d'un ADN à chaîne unique issu de sous-clone de M13 contenant un acide nucléique dérivé du génome entier de LAV. Les positions relatives de ces divers fragments vis-à-vis du génome entier de LAV (déterminé par séquençage) sont indiquées dans la partie inférieure de la figure. Le point 12 correspond à une tache témoin réalisé en mettant en œuvre un ADN témoin du phage Lambda.

Les essais d'hybridation dans la méthode de transfert par tache (dot-blot) ont été réalisés dans les conditions de faible stringence de l'exemple II. En utilisant à titre de sonde, le recombinant Lambda ROD 4 contenant l'ADNc total de HIV 2. Les lavages ont ensuite été faits successivement dans les conditions suivantes : $2 \times$ SSC, 0.1 % SDS à 25 °C. (Tm — 42 °C), $1 \times$ SSC, 0.1 % SDS à 60 °C. (Tm — 20 °C), et 0.1 $\times$ SSC, 0.1 % SDS à 60 °C. (Tm — 3 °C).

Les taches représentées ont été obtenues après exposition radiographique pendant 1 nuit.

Exemple IV

Test de diagnostic in vitro de la présence du virus HIV-2 dans un milieu biologique

Matériel et méthodes

Patients

Les patients infectés par HIV-2 ont été sélectionnés parmi des personnes admises à l'hôpital Egas Moniz de LISBONNE pour hospitalisation ou pour consultation, entre septembre 1985 et septembre 1986.

Pour réaliser cette sélection, tous les individus d'origine africaine ou ayant séjourné en Afrique ont subi un test sérologique à la fois pour rechercher la présence d'anticorps contre HIV-I (immunofluorescence-IFA-et/ou ELISA) et contre HIV-2 (IFA). Seuls les patients pour lesquels on a prouvé sérologiquement l'infection avec HIV-2 ont été retenus dans cette étude.

Isolation du virus

Chez 12 patients, l'isolation de HIV a été conduite comme on le rappelle brièvement. Les lymphocytes périphériques sanguins des patients (PBL) ont été stimulés avec PHA, cultivés en coculture avec des PBLs normaux humains stimulés par PHA et maintenus en présence d'interleukine-2 (IL-2). Les cultures ont été contrôlées afin de repérer la présence éventuelle d'un effet cytopathogénique (CPE) et afin de mesurer l'activité de transcriptase inverse (RT) dans le surnageant.

Test d'immunofluorescence (IFA)

Des plaques IFA ont été préparées comme suit : des cellules MOLT-4 infectées avec HIV-2 ont été lavées 2 fois dans PBS et étalées sur des lames de verre IFA ($10^4$ cellules par puits), puis séchées à l'air et fixées par de l'acétone à froid. Pour le test IFA, ces cellules ont été soumises à une réaction, le sérum de test étant dilué à 1/10, pendant 45' à 37 °C, ont été lavées, séchées et soumises à une réaction avec de la fluorescéine conjuguée à des IgG, A, M anti-humaines de chèvre (dilué à 1/100) pendant 30 minutes à 37 °C. Après le lavage, les cellules ont été colorées avec du bleu Evans, à 0,006 %, montées dans un mélange de 90 % de glycérol, 10 % de PBS et examinées au microscope à fluorescence.

Elisa

Certains sérums de patients ont été examinés pour déterminer la présence d'anticorps anti-HIV-1 en utilisant les tests sérologiques disponibles dans le commerce sous la dénomination ELAVIA (Pasteur Diagnostics) ou ABBOTT.

Test de radio-immunoprécipitation (RIPA)

Des cellules CEM infectées avec HIV-1 ou HIV-2 ont été cultivées en présence de $^{35}S$ cystéine (200 microCi/ml) pendant 16 heures. Le surnageant a été recueilli, les particules virales ont été centrifugées et lysées sur un tampon RIPA (Tris-HC1 50mM pH 7.5, NaC1 150mM, EDTA 1mM, 1 % Triton X100 ®, Sodium deoxycholate 0.1 %, SDS 0.1 %). Pour chaque réaction, 50 microlitres de lysat dilué correspondant à $10^5$ cpm ont été mis en réaction avec 5 microlitres d'un sérum test pendant 18 heures à 4 °C. Des complexes immuns ont été liés grâce à une protéine A fixée sur du Sépharose ® PHARMACIA lavés, et élués pendant une ébullition de 2 minutes. Les antigènes élués ont été analysés par électrophorèse sur gel SDS-polyacrylamide et par autographie.

Hybridation Dot-blot

Des virus isolés dans des PBLs de patients ont été centrifugés et lysés dans Tris-HCl 10mM pH 7.5, NaCl 10mM, EDTA 1 mM, SDS 0.5 %. Un microlitre de chaque lysat, correspondant à une activité RT de 50.000 cpm a été déposé sur un support de nitrocellulose. Une hybridation et un lavage ont été réalisés dans des conditions de forte stringence : hybridation dans 6X SSC, 5X Denhart, 50 % de formamide, pendant 18 heures à 42 °C ; et lavage dans 0.1X SSC, 0.1 % SDS à 65 °C. On a utilisé des sondes HIV-1 et HIV-2, marquées au 32p (activité spécifique de $10^8$ cpm/microgramme). La sonde HIV-1 correspond au génome complet de l'isolat LAV$_{BRU}$, et la sonde HIV-2 dérive d'un ADN cloné de 2 kilobases de l'isolat LAV-2$_{ROD}$.

Résultats

Population des patients

30 patients chez lesquels on décelait une infection par HIV-2 par mise en évidence sérologique ou virale, ont été étudiés. Parmi ces 30 patients on comptait 12 hommes et 18 femmes. La moyenne d'âge était de 35 ans pour un intervalle de 11 à 55 ans. Tous les patients excepté 1, étaient restés plusieurs années en Afrique de l'Ouest : 26 étaient nés et vivaient en Guinée-Bissau et 2 étaient originaires des Iles du Cap-Vert. 1 patient était un garçon de 11 ans de l'Angola, qui avait vécu dans les Iles du Cap-Vert pendant plusieurs années. Le seul européen de la population étudiée était un Portugais de 40 ans qui avait vécu pendant 8 ans au Zaïre, mais démentait tout séjour en Afrique de l'Ouest.

Présentation clinique

Parmi les 30 patients, 17 avaient un SIDA, conformément aux critères reconnus CDC. Le symptôme majeur chez ces patients était la diarrhée chronique, accompagnée dans la plupart des cas d'une perte de poids de plus de 10 kg. en 1 an. Chez 10 patients la diarrhée était associée avec la présence d'une infection intestinale soudaine ; pour 7 cas, l'agent pathogène était le seul Isospora belli, 1 patient était atteint uniquement par Cryptosporidium et 2 avaient à la fois les 2 agents pathogènes. Dans 3 cas, aucun agent pathogène intestinal inattendu n'a été révélé. Parmi les 17 cas de malades atteints de SIDA, on a diagnostiqué chez 8 des candidoses œsophagiennes. 6 patients atteints du SIDA. présentaient des symptômes respiratoires. Une tuberculose pulmonaire a été diagnostiquée chez 2 d'entre eux et une microbactérie différente non identifiée a été décelée chez l'un d'entre eux.

2 patients souffraient d'une aspergilose pulmonaire à la suite d'une tuberculose. 2 autres patients atteints du SIDA présentaient des phases récurrentes de pneumonie sans identification de l'agent pathogène, et 1 patient avait une pneumonie du type pneumocystis carinii, qui fut diagnostiquée uniquement post-mortem. Quatre des 17 patients atteints du SIDA avaient un sarcome de Kaposis : dans 3 cas il est apparu comme limité à la peau et chez un autre patient un examen post-mortem a révélé des lésions disséminées au niveau viscéral. Des désordres du système nerveux central sont apparus chez 2 patients atteints du SIDA : 1 avait un lymphome cérébral, et l'autre avait une encéphalite subaiguë de cause inconnue.

4 patients présentaient les symptômes du complexe lié au SIDA (ARC) :

2 avaient des lymphadénopathies diffuses avec fièvre persistante, 1 avait une diarrhée chronique avec perte de poids, et 1 avait des phases récurrentes de bronchopneumonie et des lymphadénopathies multiples.

Parmi les 9 patients restants, 6 n'avaient aucun symptôme rattachable à l'infection par HIV, 1 était atteint uniquement d'une tuberculose pulmonaire, 1 avait uniquement des lymphadénopathies diffuses persistantes et 1 présentait une syphillis neurologique. Pendant la période d'étude de 12 mois, 7 patients sont morts, tous présentant un SIDA.

Etude sérologique

Chaque patient fait l'objet d'au moins un test sérologique pour rechercher la présence d'anticorps contre à la fois HIV-1 et HIV-2. Tous les sérums des patients ont été testés par test IFA afin de repérer les anticorps contre HIV-2 et tous se sont révélés positifs. Parmi eux, 21 furent aussi testés pour les anticorps contre HIV-2 par un test RIPA : tous ont précipité de façon claire la glycoprotéine de haut poids moléculaire de l'enveloppe du virus, désignée par gp140, et 16 d'entre eux ont réagi également avec la protéine majeure du noyau p26, alors qu'une seulement réagit avec une autre protéine virale du noyau, désignée par p16.

Dans ces sérums on a recherché la présence d'anticorps présentant des réactions croisées avec HIV-I en utilisant les différents tests. Un test IFA a été utilisé pour 24 sérums : 12 sont négatifs, 10 sont faiblement réactifs, et 2 positifs. Dans un test ELISA, 21 sérums ont été testés : 16 étaient négatifs, et 5 étaient positifs. Finalement, on a recherché dans 11 sérums la présence d'anticorps contre les protéines de HIV-1 par la méthode RIPA.

3 d'entre eux n'ont absolument pas réagi, et ceci avec aucune protéine virale, 2 seulement ont précipité avec la protéine p34, issue du gène pol, et 5 ont réagi avec la protéine p25, protéine majeure du noyau. 2 sérums ont réagi seulement faiblement avec la glyco-protéine gp110 de l'enveloppe du virus HIV-1. Ces deux sérums ainsi que tous les sérums positifs lors des tests IFA ou ELISA concernant les anticorps anti HIV-1 ont réagi fortement avec la gp140 de HIV-2 dans un test RIPA, indiquant une infection avec HIV-2 plutôt qu'avec HIV-1. Seulement 1 patient, qui n'est pas inclu dans la population étudiée, a été reconnu sérologiquement comme étant infecté par HIV-1 et non par HIV-2. Ce patient était une femme de 21 ans de l'Afrique Centrale (Ile Sao Tome) présentant un SIDA.

Isolation du virus

L'isolation des virus dans les lymphocytes périphériques du sang a été pratiquée sur 12 patients. Un HIV a été isolé chez 11 d'entre eux, grâce à la présence d'un effet cytopathogénique, et à la mesure d'un pic des particules associées à l'activité de transcriptase inverse dans les surnageants de culture.

Ces 11 isolats ont été identifiés comme étant des isolats de HIV-2 en utilisant la technique d'hybridation « dot-blot ». Les taches virales obtenues à partir de 10 isolats ont montré une hybridation forte dans les conditions stringentes d'hybridation et de lavage avec une sonde HIV-2 dérivée d'un EDNc cloné de HIV-2, alors qu'aucune d'entre-elles n'hybridait avec les sondes HIV-1 dans les mêmes conditions. Un isolat hybridait seulement faiblement avec une sonde HIV-2, mais n'hybridait pas avec une sonde HIV-1.

Evaluation immunologique

13 patients ont été étudiés pour évaluer le nombre de leurs lymphocytes circulant identifiés comme étant des cellules T auxiliaires (helper) (CD4+) et le rapport de : cellules T auxiliaires/cellules T suppresseur. Parmi ces patients, 11 avaient un SIDA : leurs nombres de cellules T auxiliaires était de 243 à 300 par microlitre, et leur rapport cellules T auxiliaires/cellules T suppresseur moyen était de 0,25 à 0,15. 1 patient présentait des signes cliniques de ARC, avait un nombre de lymphocytes T helper de 240/microlitre et un ratio de 0,18. Un autre patient, présentant une syphillis neurologique et aucun symptôme lié de façon évidente à HIV, avait un nombre de lymphocytes T helper de 690 par microlitre et un ratio de 0.82.

Discussion

Dans cette étude, l'infection par HIV-2 a été mise en évidence chez 30 patients africains d'Afrique de l'Ouest présentant soit un SIDA, soit des symptômes du complexe ARC ou n'ayant aucun signe clinique rattaché au SIDA. Les résultats permettent néanmoins de conclure que HIV-2 doit être considéré comme un agent étiologique majeur du SIDA chez les patients d'Afrique de l'Ouest. Les résultats sérologiques et viraux qui ont été observés indiquent que l'infection par HIV-2 n'était pas souvent associée avec une infection par HIV-1 parmi ces patients.

17

En dépit de différences antigéniques et génétiques importantes, HIV-1 et HIV-2 présentent un tropisme similaire pour les lymphocytes T CD4+, ont des effets cytopathogènes similaires, une morphologie similaire, et partagent des épitopes d'immunoréaction communs pour certaines de leurs protéines constitutives.

Dans la mesure où tous les patients d'Afrique de l'Ouest, infectés avec HIV et étudiés ici ont été reconnus comme étant infectés par HIV-2 alors qu'aucun n'était infecté par HIV-1, le nouveau virus HIV-2 pourrait être la cause majeure du SIDA en Afrique de l'Ouest.

Les symptômes du SIDA liés à HIV-2 ne sont pas différents de ceux du SIDA liés à HIV-1 en Afrique Centrale : Le symptôme le plus commun consiste dans des diarrhées chroniques, avec perte importante de poids, troubles dûs la plupart du temps à Isospora belli et/ou Cryptosporidium. La fréquence d'autres infections soudaines, telles que des candidoses, des microbactéries (incluant M. tuberculosis) et des toxoplamoses comparable à ce que l'on reconnaissait chez les Africains atteints d'un SIDA lié à HIV-1. La pneumonie du type Pneumocystis carinii, considérée comme une complication très commune chez les malades atteints du SIDA aux USA et en Europe, a été décelée seulement une fois lors de notre étude, et des méningites de type cryptococcal n'ont pas été détectées.

Néanmoins, les anomalies immunologiques décelées parmi des patients atteints du SIDA lié à une infection par HIV-2 sont identiques à celles décrites lors de SIDA lié à HIV-1.

Parmi les 30 patients qui possédaient dans leur sérum des anticorps réagissant avec les antigènes HIV-2, seuls 7 d'entre eux avaient des anticorps spécifiques contre HIV-1 détectables par des tests IFA ou ELISA. Lors d'un test RIPA, on observe que ces 7 patients présentent des anticorps réagissant contre les protéines majeures du noyau p25 (HIV-1) ou p26 (HIV-2), qui ont en commun des épitopes fortement immunoréactifs. 5 patients ne présentent pas de tels anticorps : ces 5 patients ont des réponses négatives à des tests ELISA vis-à-vis de HIV-1. 2 d'entre eux ont des réponses négatives à des tests IFA et 3 d'entre eux ont des réponses très faibles. Cependant, bien que certain d'entre eux n'aient pas été totalement étudiés, 9 patients ont été observés qui possédaient dans leur sérum des anticorps de la protéine virale p26 du noyau de HIV-2 et qui présentaient une réaction faible ou une réaction négative à des tests IFA ou ELISA spécifiques de HIV-1. Ces résultats font ressortir l'importance de l'incorporation d'antigène HIV-2 dans des tests sérologiques HIV utilisés en Afrique et peut-être dans d'autres endroits.

Un rétrovirus a été isolé des lymphocytes périphériques de 11 patients. Dans tous les cas la croissance virale a été obtenue en 2 semaines, caractérisée par la présence d'une activité de transcriptase inverse dans le surnageant de culture et par un effet cytopatogénique. Cependant, cet effet cytopatogène est apparu d'importance variable pour les différents isolats : certains isolats présentaient un nombre important de syncytia de grande taille accompagné d'une lyse cellulaire importante, alors que d'autres présentaient seulement peu de syncytia de taille limitée, et affectant la viabilité de la culture.

Tous les ARNs sauf celui d'un unique isolat hybrident avec une sonde dérivée d'un ADNc cloné de HIV-2 représentant la terminaison 3' du génome, dans des conditions de fortes stringences. Aucun n'hybride avec une sonde HIV-1 dans les mêmes conditions. Ceci démontre que les isolats infectant ces patients appartiennent au même type de virus. Un isolat seulement hybride mais rarement avec une sonde HIV-2 à la fois dans des conditions de forte et de faible stringence, et n'hybride pas avec HIV-1. Ce virus a cependant été isolé chez un patient possédant dans son sérum des anticorps capables de réagir avec tous les antigènes de HIV-2 lors d'un test RIPA.

D'une façon générale, l'invention concerne, outre le virus HIV-2 et ses variants, tout virus équivalent infectieux pour l'homme présentant des caractéristiques immunologiques propres au HIV-2. D'une façon générale, l'invention concerne tout virus qui, outre les propriétés que possède les virus HIV-2 déposés à la CNCM, présente encore les caractéristiques suivantes.

La cible préférentielle du rétrovirus HIV-2 est constituée par les cellules Leu 3 (ou lymphocytes T4) humaines et pour des cellules « immortalisées » dérivées de ces lymphocytes T4, par exemple les cellules des lignées HUT-78 considérées dans le cadre de cette demande. En d'autres termes, il a un tropisme spécifique pour ces cellules. Il peut être cultivé dans des lignées permanentes du type HUT, CEM, MOLT ou analogue exprimant la protéine T4. Il n'est pas infectieux pour les lymphocytes T8. Il est cytotoxique pour les lymphocytes T4 humains. Le caractère cytopathique des virus HIV-2 à l'égard des lymphocytes T4 se manifeste notamment par l'apparition de cellules multinuclées. Il a une activité de transcriptase inverse nécessitant la présence d'ions $Mg^{2+}$ et présentant une forte affinité pour le poly(adénylate-oligo-déoxythymidylase) (poly(A)-oligo (dT) 12-18). Il a une densité d'environ 1,16 dans un gradient de sucrose. Il a un diamètre moyen de 140 nanomètres et un noyau ayant un diamètre moyen de 41 nanomètres. Les lysats de ce virus contiennent une protéine p26 qui ne croise pas immunologiquement avec la protéine p24 du virus HTLV-I ou du virus HTLV-II. Ces protéines p26 présentent donc un poids moléculaire un peu plus élevée (d'environ 1000) que les p25 correspondantes des HIV-1 et un peu moins élevée (de nouveau de l'ordre d'environ 1000) que les p27 correspondantes des SIV. Les lysats de HIV-2 contiennent en outre une protéine p16 qui n'est pas reconnue immunologiquement par la protéine p19 de HTLV-I ou de HTLV-II dans des essais de radioimmunoprécipitation RIPA (abréviation de l'expression anglaise « radioimmunoprecipitation assay »). Ils contiennent en outre une glycoprotéine d'enveloppe ayant un poids moléculaire de l'ordre de 130.000-140.000 qui ne croise pas immunologiquement avec la gp110 du HIV-1, mais qui, par contre, croise immunologiquement avec la glycoprotéine d'enveloppe gp140 de STLV-III. Ces lysats contiennent encore des protéines ou glycoprotéines, marquables par la $^{35}$S-

cystéine, ayant des poids moléculaires respectivement de l'ordre de 36.000 et 42.000-45.000. L'ARN génomique de HIV-2 n'hybride pas avec l'ARN génomique de HIV-1 dans des conditions stringentes. Dans des conditions non stringentes, il n'hybride, ni avec la séquence nucléotidique dérivée de HIV-1 et contenant le gène env et le LTR qui le jouxte. En particulier il n'hybride pas avec la séquence de nucléotides 5290-9130 de HIV-1, ni avec des séquences de la région pol du génome de HIV-1, en particulier avec la séquence de nucléotides 2170-2240. Dans des conditions non stringentes, il hybride faiblement avec des séquences de nucléotides de la région de HIV-1, notamment les séquences de nucléotides 990-1070 et 990-1260.

Il est à remarquer que tout rétrovirus infectieux pour l'homme susceptible d'induire un SIDA ayant les propriétés essentielles susdites et dont l'ARN génomique est susceptible de s'hybrider dans les conditions stringentes avec ceux des souches virales de HIV-2 déposées à la C.N.C.M. (ou avec un cADN ou fragment de cADN dérivés de ces ARNs génomiques doit être considéré comme un équivalent de HIV-2.

L'invention concerne encore chacun des antigènes, notamment protéines et glycoprotéines à l'état purifié, tels qu'ils peuvent être obtenus à partir de HIV-2. On parle de protéines ou glycoprotéines « purifiées » lorsque ces protéines ou glycoprotéines ne conduisent respectivement qu'à des bandes uniques en électrophorèse sur gel de polyacrylamide, notamment dans les conditions expérimentales qui ont été indiquées plus haut. Tout procédé approprié de séparation et/ou de purification pour obtenir chacune d'entre elles peut être utilisé. On mentionnera à titre d'exemple de technique pouvant être mise en œuvre celle décrite par R. C. MONTELARO et Coll., J. of Virology, juin 1982, pp. 1029-1038.

D'une façon générale, l'invention concerne tous antigènes, notamment protéines, glycoprotéines ou polypeptides issus d'un HIV-2 et ayant des propriétés immunologiques équivalentes à ceux ou celles du HIV-2. Deux antigènes sont dits « équivalents » dans le cadre de cet exposé, dès lors qu'ils sont reconnus par les mêmes anticorps, notamment d'anticorps isolables à partir d'un sérum obtenu à partir d'un patient ayant fait une infection avec un HIV-2, ou dès lors qu'ils répondent aux conditions « d'équivalence immunologique » indiquées ci-après.

Parmi les polypeptides, protéines ou glycoprotéines équivalents, doivent être rangés des fragments des antigènes qui précèdent (ou des peptides reconstitués par synthèse chimique), dès lors qu'ils donnent lieu à des réactions immunologiques croisées avec les antigènes dont ils sont dérivés. En d'autres termes, l'invention concerne tout polypeptide ayant, en commun avec les susdits antigènes, des épitopes identiques ou semblables, susceptibles d'être reconnus par les mêmes anticorps. Font partie de ce dernier type de polypeptides les produits d'expression de séquences correspondantes des ADNs codant pour les séquences polypeptidiques correspondantes.

Le virus HIV-2 s'est avéré utilisable comme source d'antigènes pour la détection d'anticorps chez toutes personnes ayant été mises en contact avec le virus HIV-2.

D'une façon générale, l'invention concerne toute composition utilisable pour le diagnostic de la présence dans un sérum fluide biologique, notamment de personnes ayant été mises au contact de HIV-2, d'anticorps contre l'un au moins des antigènes de HIV-2. Cette composition peut être appliquée au diagnostic sélectif de la variété correspondante du SIDA, en mettant en œuvre les techniques de diagnostic, telles que décrites dans la demande de brevet européen citée ci-dessus, si ce n'est que l'on utilise des extraits, lysats ou encore antigènes purifiés de HIV-2 au lieu de ceux de HIV-I. A cet égard, l'invention concerne plus particulièrement des compositions contenant l'une au moins des protéines p12, p16, p26, s'agissant des protéines internes, ou l'une au moins des glycoprotéines p36 ou gp140. On mentionnera à titre d'exemples de compositions, celles qui contiennent simultanément :

— la p26 et la gp36,
— la p26, les p36 et gp140,
— les p12, p16 et p26,
— les p16, p26 et gp140...

Il va de soi que ces compositions n'ont que valeur d'exemples. En particulier, l'invention concerne les extraits ou lysats viraux contenant l'ensemble de ces protéines et/ou glycoprotéines ou toutes fractions dont auront au préalable été séparées une ou plusieurs des protéines ou glycoprotéines susdites.

L'invention concerne encore des compositions associant des protéines et/ou glycoprotéines d'un HIV-2, avec des protéines et/ou glycoprotéines d'un HIV-1, par exemple :
— soit des protéines du noyau (core) de HIV-1 et de HIV-2, notamment les p25 d'un HIV-1 et p26 d'un HIV-2, ou encore soit la p18 d'un HIV-1 et la p16 d'un HIV-2,
— soit des glycoprotéines d'enveloppe d'un HIV-1 et des glycoprotéines d'enveloppe d'un HIV-2, notamment la gp110 de HIV-1 et la gp140 de HIV-2, ou encore la p42 de HIV-1 et la p36 ou p42-45 de HIV-2,
— soit naturellement des mélanges de protéines et/ou glycoprotéines d'un HIV-1 et de protéines et/ou glycoprotéines d'un HIV-2.

De telles compositions, utilisées pour le diagnostic, permettent par conséquent des opérations de diagnostic du SIDA ou des symptômes qui lui sont associés, qui s'étendent sur un plus large spectre des agents étiologiques responsables. Il va sans dire que l'utilisation pour les opérations de diagnostic de compositions qui ne contiennent que des protéines et/ou glycoprotéines de HIV-2 n'en reste pas moins utile pour des diagnostics plus sélectifs de la catégorie de rétrovirus qui peut être tenue pour responsable de la maladie.

L'invention concerne encore les ADNs ou fragments d'ADNs, plus particulièrement ADNs et fragments d'ADNs clonés obtenus à partir de l'ARN ou de cADNs dérivés de l'ARN du rétrovirus HIV-2. L'invention concerne encore particulièrement tous ADNs équivalents, notamment tout ADN présentant des homologies de séquences avec l'ADN du HIV-2, en particulier avec les séquences codant pour les régions env et pol de la souche de HIV-2 déposée à la C.N.C.M., au moins égales à 50 %, de préférence à 70 %, et plus avantageusement encore à 90 %. D'une façon générale, on dira encore que l'invention concerne tout ADN (ou ARN) équivalent capable d'hybrider avec l'ADN ou l'ARN du HIV-2 dans la technique du « spot blot » dans des conditions non stringentes telles que définies ci-dessus.

L'invention concerne de la même façon les sérums susceptibles d'être produits chez l'animal par inoculation à celui-ci de HIV-2. L'invention concerne donc plus particulièrement les anticorps polyclonaux plus spécifiquement orientés contre chacun des antigènes, notamment protéines ou glycoprotéines du virus. Elle concerne aussi les anticorps monoclonaux qui peuvent être produits par des techniques classiques, ces anticorps monoclonaux étant orientés respectivement plus spécifiquement contre les différentes protéines du HIV-2.

Ces anticorps polyclonaux ou monoclonaux sont utilisables dans différentes applications. On mentionnera essentiellement leur utilisation pour neutraliser les protéines correspondantes, voir inhiber l'infectivité du virus entier. Ils peuvent également être utilisés, par exemple, pour mettre en évidence des antigènes viraux dans les préparations biologiques ou pour réaliser des opérations de purification des protéines et/ou glycoprotéines correspondantes, par exemple par leur utilisation dans des colonnes de chromatographie d'affinité.

Il est entendu que, d'une façon générale, la littérature technique disponible (notamment celle dont les références bibliographiques sont rappelées dans le cadre de la présente description) en ce qui concerne le HIV-1 et le virus dénommé HTLV-III doit être considérée comme faisant partie de la présente invention, dès lors que les techniques décrites par cette littérature s'appliquent dans des conditions semblables à l'isolement du virus HIV-2 ou de virus équivalents, à l'obtention à partir de ces virus de leurs différents constituants (notamment protéines, glycoprotéines, polypeptides, acides nucléiques). On peut également faire appel aux enseignements de cette littérature technique pour ce qui est de l'application des différents constituants concernés, notamment à des opérations de diagnostic des formes correspondantes de SLA ou de SIDA.

La présente invention concerne plus particulièrement un procédé de diagnostic in vitro du SIDA, qui comprend la mise en contact d'un sérum ou d'un autre milieu biologique provenant d'un malade faisant l'objet du diagnostic avec une composition contenant l'une au moins des protéines ou glycoprotéines du HIV-2, ou encore un extrait ou lysat du virus, et la détection de la réaction immunologique. Des exemples de telles compositions ont été indiqués plus haut.

Des méthodes préférées mettent en jeu par exemple des réactions immunoenzymatiques de type ELISA ou d'immunofluorescence. Les titrages peuvent être des mesures par immunofluorescence directe ou indirecte ou des dosages immunoenzymatiques directs ou indirects.

Ainsi la présente invention est également relative à des extraits de virus (soit d'un extrait d'un ou plusieurs HIV-2 seulement, soit d'un mélange d'extraits originaires d'un ou plusieurs HIV-2, d'une part, et d'un ou plusieurs HIV-1, d'autre part), ces extraits étant marqués. On peut utiliser tout type de marqueur approprié : enzymatique, fluorescent, radioactif, etc...

De tels titrages comprennent par exemple :

— le dépôt de quantités déterminées de l'extrait ou des compositions visées conformes à la présente invention dans les puits d'une microplaque de titrage ;

— l'introduction dans ces puits de dilutions croissantes du sérum contenant essentiellement les anticorps dont la présence doit être détectée in vitro ;

— l'incubation de la microplaque ;

— le lavage soigneux de la microplaque avec un tampon approprié ;

— l'introduction dans les puits de la microplaque d'anticorps marqués spécifiques d'immunoglobulines humaines, le marquage étant réalisé par une enzyme choisie parmi celles qui sont capables d'hydrolyser un substrat de telle sorte que ce dernier subit alors une modification de son absorption des radiations, au moins dans une bande de longueurs d'ondes déterminée et

— la détection, de préférence de façon comparative par rapport à un témoin, de l'importance de l'hydrolyse du substrat en tant que mesure des risques potentiels ou de la présence effective de la maladie.

La présente invention est également relative à des nécessaires ou « kits » pour le diagnostic ci-dessus, lesquels comprennent :

— un extrait ou une fraction plus purifiée des types de virus indiqués ci-dessus, cet extrait ou fraction étant marqué, par exemple de façon radioactive, enzymatique ou immunofluorescence ;

— des anti-immunoglobulines humaines ou une protéine A (fixée de façon avantageuse sur un support insoluble dans l'eau, tel que des billes d'agarose) ;

— un extrait de lymphocytes obtenus à partir d'une personne en bonne santé ;

— des tampons et, le cas échéant, des substrats pour la visualisation du marquage.

De ce qui précède, il résulte que l'invention concerne le diagnostic du virus HIV-2 ou d'un variant grâce à la mise en œuvre des sondes décrites précédemment dans un procédé faisant appel à différentes étapes rappelées ci-après, ces étapes étant spécifiquement prévues pour faire apparaître les propriétés caractéristiques du virus HIV-2.

L'invention concerne naturellement aussi l'utilisation des cADNs ou de leurs fragments (ou de recombinants les contenant) en tant que sondes, pour le diagnostic de la présence ou non de virus HIV-2 dans des échantillons de sérums ou d'autres liquides ou tissus biologiques obtenus à partir de patients suspectés d'être porteurs du virus HIV-2. Ces sondes sont de préférence marquées également (marqueurs radio-actifs, enzymatiques, fluorescents, etc.). Des sondes particulièrement intéressantes pour la mise en œuvre du procédé de diagnostic du virus HIV-2 ou d'un variant de HIV-2 peuvent être caractérisées en ce qu'elles comprennent la totalité ou une fraction de l'ADNc complémentaire du génome du virus HIV-2 ou encore notamment les fragments contenus dans les divers clones identifiés ci-dessus. On mentionnera plus particulièrement une fraction de l'ADNc de HIV-2 contenu dans le clone E2, plus particulièrement la séquence de l'extrémité 3' (LTR) et/ou de l'extrémité 5' de la séquence HIV du clone E2 précité, ou encore l'ADNc contenant la région env, de l'ADNc du virus HIV-2.

Les sondes mises en œuvre dans ce procédé de diagnostic du virus HIV-2 et dans les kits de diagnostic, ne sont en aucune façon réduites aux sondes décrites précédemment. Elles comprennent au contraire toutes les séquences nucléotidiques issues du génome du virus HIV-2, d'un variant de HIV-2 ou d'un virus proche par sa structure, dès lors qu'elles permettent la détection dans des fluides biologiques de personnes susceptibles de développer un SIDA d'anticorps dirigés contre un HIV-2. Naturellement l'utilisation de séquences nucléotidiques issues d'un HIV-2, initialement infectieux pour l'homme, est néanmoins préférée.

La détection peut être réalisée de toutes façons en soi connues, notamment par une mise en contact de ces sondes soit avec les acides nucléiques obtenus à partir des cellules contenues dans ces sérums ou autres milieux biologiques, par exemple liquides céphalo-rachidiens, salive, etc..., soit avec ces milieux eux-mêmes dès lors que leurs acides nucléiques auront été rendus accessibles à l'hybridation avec ces sondes, et ce dans des conditions permettant l'hybridation entre ces sondes et ces acides nucléiques et par une détection de l'hybridation éventuellement produite. Le susdit diagnostic mettant en jeu des réactions d'hybridation peut également être réalisé à l'aide de mélanges de sondes respectivement originaires d'un HIV-1 et d'un HIV-2, dès lors qu'il n'est pas nécessaire de faire une différence entre le type de virus HIV recherché.

D'une façon générale, le procédé de diagnostic de la présence ou non du virus HIV-2 ou d'un variant dans des échantillons de sérums ou d'autres liquides ou tissus obtenus à partir de patients suspectés d'être porteurs du virus HIV-2 comprend les étapes suivantes :

1) la fabrication d'une sonde marquée,

2) au moins une étape d'hybridation conduite dans des conditions stringentes, par mise en contact de l'ADN de cellules de l'échantillon du patient suspect avec ladite sonde marquée sur une membrane appropriée,

3) le lavage de ladite membrane avec une solution assurant la conservation de ces conditions stringentes de l'hybridation,

4) la détection de la présence ou non du virus HIV-2 par une méthode d'immunodétection.

Dans un autre mode de réalisation préféré du procédé selon l'invention l'hybridation précitée est conduite dans des conditions non stringentes et le lavage de la membrane est réalisé dans des conditions adaptées à celles de l'hybridation.

L'invention concerne en particulier les virus HIV-2, caractérisés par le fait que leur ARN viral est en correspondance avec un ADNc dont les régions contenant les gènes gag et env comportent respectivement les séquences nucléotidiques qui suivent (GAGRODN et ENVRN). Elles résultent du séquençage des régions correspondantes de l'ADNc correspondant au génome de HIV-2 ROD. Elles sont mises en correspondance avec les acides aminés qu'elles codent.

Il est entendu que la référence quelquefois faite pour des raisons de commodités dans la description et même dans les revendications quant aux possibilités d'hybridation entre des ARNs, doit être comprise comme signifiant que ce sont les brins appropriés correspondants dérivés de cADNs eux-mêmes produits à partir de ces ARNs qui sont susceptibles d'hybridation mutuelle.

```
GAGRODN


MetGlyAlaArgAsnSerValLeuArgGlyLysLysAlaAspGlu
ATGGGCGCGAGAAACTCCGTCTTGAGAGGGAAAAAAGCAGATGAA


LeuGluArgIleArgLeuArgProGlyGlyLysLysLysTyrArg
TTAGAAAGAATCAGGTTACGGCCCGGCGGAAAGAAAAAGTACAGG
```

```
LeuLysHisIleValTrpAlaAlaAsnLysLeuAspArgPheGly
CTAAAACATATTGTGTGGGCAGCGAATAAATTGGACAGATTCGGA
            100
LeuAlaGluSerLeuLeuGluSerLysGluGlyCysGlnLysIle
TTAGCAGAGAGCCTGTTGGAGTCAAAAGAGGGTTGTCAAAAAATT

LeuThrValLeuAspProMetValProThrGlySerGluAsnLeu
CTTACAGTTTTAGATCCAATGGTACCGACAGGTTCAGAAAATTTA
            200
LysSerLeuPheAsnThrValCysValIleTrpCysIleHisAla
AAAAGTCTTTTTAATACTGTCTGCGTCATTTGGTGCATACACGCA

GluGluLysValLysAspThrGluGlyAlaLysGlnIleValArg
GAAGAGAAAGTGAAAGATACTGAAGGAGCAAAACAAATAGTGCGG
                        300
ArgHisLeuValAlaGluThrGlyThrAlaGluLysMetProSer
AGACATCTAGTGGCAGAAACAGGAACTGCAGAGAAAATGCCAAGC

ThrSerArgProThrAlaProSerSerGluLysGlyGlyAsnTyr
ACAAGTAGACCAACAGCACCATCTAGCGAGAAGGGAGGAAATTAC
                            400
ProValGlnHisValGlyGlyAsnTyrThrHisIleProLeuSer
CCAGTGCAACATGTAGGCGGCAACTACACCCATATACCGCTGAGT

ProArgThrLeuAsnAlaTrpValLysLeuValGluGluLysLys
CCCCGAACCCTAAATGCCTGGGTAAAATTAGTAGAGGAAAAAAAG

PheGlyAlaGluValValProGlyPheGlnAlaLeuSerGluGly
TTCGGGGCAGAAGTAGTGCCAGGATTTCAGGCACTCTCAGAAGGC
500
CysThrProTyrAspIleAsnGlnMetLeuAsnCysValGlyAsp
TGCACGCCCTATGATATCAACCAAATGCTTAATTGTGTGGGCGAC

HisGlnAlaAlaMetGlnIleIleArgGluIleIleAsnGluGlu
CATCAAGCAGCCATGCAGATAATCAGGGAGATTATCAATGAGGAA
        600
AlaAlaGluTrpAspValGlnHisProIleProGlyProLeuPro
GCAGCAGAATGGGATGTGCAACATCCAATACCAGGCCCCTTACCA

AlaGlyGlnLeuArgGluProArgGlySerAspIleAlaGlyThr
GCGGGGCAGCTTAGAGAGCCAAGGGGATCTGACATAGCAGGACA
                700
ThrSerThrValGluGluGlnIleGlnTrpMetPheArgProGln
ACAAGCACAGTAGAAGAACAGATCCAGTGGATGTTTAGGCCACAA

AsnProValProValGlyAsnIleTyrArgArgTrpIleGlnIle
AATCCTGTACCAGTAGGAAACATCTATAGAAGATGGATCCAGATA
                            800
GlyLeuGlnLysCysValArgMetTyrAsnProThrAsnIleLeu
GGATTGCAGAAGTGTGTCAGGATGTACAACCCGACCAACATCCTA

AspIleLysGlnGlyProLysGluProPheGlnSerTyrValAsp
GACATAAAACAGGGACCAAAGGAGCCGTTCCAAAGCTATGTAGAT
                                900
ArgPheTyrLysSerLeuArgAlaGluGlnThrAspProAlaVal
AGATTCTACAAAAGCTTGAGGGCAGAACAAACAGATCCAGCAGTG

LysAsnTrpMetThrGlnThrLeuLeuValGlnAsnAlaAsnPro
AAGAATTGGATGACCCAAACACTGCTAGTACAAAATGCCAACCCA

AspCysLysLeuValLeuLysGlyLeuGlyMetAsnProThrLeu
GACTGTAAATTAGTGCTAAAAGGACTAGGGATGAACCCTACCTTA
            1000
GluGluMetLeuThrAlaCysGlnGlyValGlyGlyProGlyGln
GAAGAGATGCTGACCGCCTGTCAGGGGGTAGGTGGGCCAGGCCAG
```

22

```
          LysAlaArgLeuMetAlaGluAlaLeuLysGluValIleGlyPro
          AAAGCTAGATTAATGGCAGAGGCCCTGAAAGAGGTCATAGGACCT
                              1100
          AlaProIleProPheAlaAlaAlaGlnGlnArgLysAlaPheLys
          GCCCCTATCCCATTCGCAGCAGCCCAGCAGAGAAAGGCATTTAAA

          CysTrpAsnCysGlyLysGluGlyHisSerAlaArgGlnCysArg
          TGCTGGAACTGTGGAAAGGAAGGGCACTCGGCAAGACAATGCCGA
                              1200
          AlaProArgArgGlnGlyCysTrpLysCysGlyLysProGlyHis
          GCACCTAGAAGGCAGGGCTGCTGGAAGTGTGGTAAGCCAGGACAC

          IleMetThrAsnCysProAspArgGlnAlaGlyPheLeuGlyLeu
          ATCATGACAAACTGCCCAGATAGACAGGCAGGTTTTTTAGGACTG
                                      1300
          GlyProTrpGlyLysLysProArgAsnPheProValAlaGlnVal
          GGCCCTTGGGGAAAGAAGCCCCGCAACTTCCCCGTGGCCCAAGTT

          ProGlnGlyLeuThrProThrAlaProProValAspProAlaVal
          CCGCAGGGGCTGACACCAACAGCACCCCCAGTGGATCCAGCAGTG

          AspLeuLeuGluLysTyrMetGlnGlnGlyLysArgGlnArgGlu
          GATCTACTGGAGAAATATATGCAGCAAGGGAAAAGACAGAGAGAG
          1400
          GlnArgGluArgProTyrLysGluValThrGluAspLeuLeuHis
          CAGAGAGAGAGACCATACAAGGAAGTGACAGAGGACTTACTGCAC

          LeuGluGlnGlyGluThrProTyrArgGluProProThrGluAsp
          CTCGAGCAGGGGGAGACACCATACAGGGAGCCACCAACAGAGGAC
                              1500
          LeuLeuHisLeuAsnSerLeuPheGlyLysAspGln
          TTGCTGCACCTCAATTCTCTCTTTGGAAAAGACCAG


ENVRN

          MetMetAsnGlnLeuLeuIleAlaIleLeuLeuAlaSerAlaCys
          ATGATGAATCAGCTGCTTATTGCCATTTTATTAGCTAGTGCTTGC

          LeuValTyrCysThrGlnTyrValThrValPheTyrGlyValPro
          TTAGTATATTGCACCCAATATGTAACTGTTTTCTATGGCGTACCC

          ThrTrpLysAsnAlaThrIleProLeuPheCysAlaThrArgAsn
          ACGTGGAAAAATGCAACCATTCCCCTCTTTTGTGCAACCAGAAAT
                              100
          ArgAspThrTrpGlyThrIleGlnCysLeuProAspAsnAspAsp
          AGGGATACTTGGGGAACCATACAGTGCTTGCCTGACAATGATGAT

          TyrGlnGluIleThrLeuAsnValThrGluAlaPheAspAlaTrp
          TATCAGGAAATAACTTTGAATGTAACAGAGGCTTTTGATGCATGG
                              200
          AsnAsnThrValThrGluGlnAlaIleGluAspValTrpHisLeu
          AATAATACAGTAACAGAACAAGCAATAGAAGATGTCTGGCATCTA

          PheGluThrSerIleLysProCysValLysLeuThrProLeuCys
          TTCGAGACATCAATAAAACCATGTGTCAAACTAACACCTTTATGT
                              300
          ValAlaMetLysCysSerSerThrGluSerSerThrGlyAsnAsn
          GTAGCAATGAAATGCAGCAGCACAGAGAGCAGCACAGGGAACAAC

          ThrThrSerLysSerThrSerThrThrThrThrThrProThrAsp
          ACAACCTCAAAGAGCACAAGCACAACCACAACCACACCCACAGAC
                                      400
          GlnGluGlnGluIleSerGluAspThrProCysAlaArgAlaAsp
          CAGGAGCAAGAGATAAGTGAGGATACTCCATGCGCACGCGCAGAC
```

23

```
AsnCysSerGlyLeuGlyGluGluGluThrIleAsnCysGlnPhe
AACTGCTCAGGATTGGGAGAGGAAGAAACGATCAATTGCCAGTTC
            •               •               •

AsnMetThrGlyLeuGluArgAspLysLysLysGlnTyrAsnGlu
AATATGACAGGATTAGAAAGAGATAAGAAAAAACAGTATAATGAA
    500            •               •           •

ThrTrpTyrSerLysAspValValCysGluThrAsnAsnSerThr
ACATGGTACTCAAAAGATGTGGTTTGTGAGACAAATAATAGCACA
       •       •   •       •           •       •

AsnGlnThrGlnCysTyrMetAsnHisCysAsnThrSerValIle
AATCAGACCCAGTGTTACATGAACCATTGCAACACATCAGTCATC
    •   •       600         •           •       •

ThrGluSerCysAspLysHisTyrTrpAspAlaIleArgPheArg
ACAGAATCATGTGACAAGCACTATTGGGATGCTATAAGGTTTAGA
                           •           •       •

TyrCysAlaProProGlyTyrAlaLeuLeuArgCysAsnAspThr
TACTGTGCACCACGGGTTATGCCCTATTAAGATGTAATGATACC
    •           700         •           •       •

AsnTyrSerGlyPheAlaProAsnCysSerLysValValAlaSer
AATTATTCAGGCTTTGCACCCAACTGTTCTAAAGTAGTAGCTTCT
    •                                           •

ThrCysThrArgMetMetGluThrGlnThrSerThrTrpPheGly
ACATGCACCAGGATGATGGAAACGCAAACTTCCACATGGTTTGGC
    •           •               •       800     •

PheAsnGlyThrArgAlaGluAsnArgThrTyrIleTyrTrpHis
TTTAATGGCACTAGAGCAGAGAATAGAACATATATCTATTGGCAT
        •               •               •

GlyArgAspAsnArgThrIleIleSerLeuAsnLysTyrTyrAsn
GGCAGAGATAATAGAACTATCATCAGCTTAAACAAATATTATAAT
    •           •               •           •
                                            900

LeuSerLeuHisCysLysArgProGlyAsnLysThrValLysGln
CTCAGTTTGCATTGTAAGAGGCCAGGGAATAAGACAGTGAAACAA

IleMetLeuMetSerGlyHisValPheHisSerHisTyrGlnPro
ATAATGCTTATGTCAGGACATGTGTTTCACTCCCACTACCAGCCG

IleAsnLysArgProArgGlnAlaTrpCysTrpPheLysGlyLys
ATCAATAAAAGACCCAGACAAGCATGGTGCTGGTTCAAAGGCAAA
            1000        •           •           •

TrpLysAspAlaMetGlnGluValLysThrLeuAlaLysHisPro
TGGAAAGACGCCATGCAGGAGGTGAAGACCCTTGCAAAACATCCC
    •               •               •       •

ArgTyrArgGlyThrAsnAspThrArgAsnIleSerPheAlaAla
AGGTATAGAGGAACCAATGACACAAGGAATATTAGCTTTGCAGCG
                •   1100        •           •

ProGlyLysGlySerAspProGluValAlaTyrMetTrpThrAsn
CCAGGAAAAGGCTCAGACCCAGAAGTAGCATACATGTGGACTAAC
    •           •       •           •           •

CysArgGlyGluPheLeuTyrCysAsnMetThrTrpPheLeuAsn
TGCAGAGGAGAGTTTCTCTACTGCAACATGACTTGGTTCCTCAAT
    •           •       1200        •

TrpIleGluAsnLysThrHisArgAsnTyrAlaProCysHisIle
TGGATAGAGAATAAGACACACCGCAATTATGCACCGTGCCATATA
    •           •               •           •

LysGlnIleIleAsnThrTrpHisLysValGlyArgAsnValTyr
AAGCAAATAATTAACACATGGCATAAGGTAGGGAGAAATGTATAT
    •           •               •   1300

LeuProProArgGluGlyGluLeuSerCysAsnSerThrValThr
TTGCCTCCCAGGGAAGGGGAGCTGTCCTGCAACTCAACAGTAACC

SerIleIleAlaAsnIleAspTrpGlnAsnAsnAsnGlnThrAsn
AGCATAATTGCTAACATTGACTGGCAAAACAATAATCAGACAAAC
            •               •           •
```

24

IleThrPheSerAlaGluValAlaGluLeuTyrArgLeuGluLeu
ATTACCTTTAGTGCAGAGGTGGCAGAACTATACAGATTGGAGTTG
1400

GlyAspTyrLysLeuValGluIleThrProIleGlyPheAlaPro
GGAGATTATAAATTGGTAGAAATAACACCAATTGGCTTCGCACCT

ThrLysGluLysArgTyrSerSerAlaHisGlyArgHisThrArg
ACAAAGAAAAAGATACTCCTCTGCTCACGGGAGACATACAAGA
1500

GlyValPheValLeuGlyPheLeuGlyPheLeuAlaThrAlaGly
GGTGTGTTCGTGCTAGGGTTCTTGGGTTTTCTCGCAACAGCAGGT

SerAlaMetGlyAlaArgAlaSerLeuThrValSerAlaGlnSer
TCTGCAATGGGCGCTCGAGCGTCCCTGACCGTGTCGGCTCAGTCC
1600

ArgThrLeuLeuAlaGlyIleValGlnGlnGlnGlnGlnLeuLeu
CGGACTTTACTGGCCGGGATAGTGCAGCAACAGCAACAGCTGTTG

AspValValLysArgGlnGlnGluLeuLeuArgLeuThrValTrp
GACGTGGTCAAGAGACAACAAGAACTGTTGCGACTGACCGTCTGG
1700

GlyThrLysAsnLeuGlnAlaArgValThrAlaIleGluLysTyr
CGAACGAAAAACCTCCAGGCAAGAGTCACTGCTATAGAGAAGTAC

LeuGlnAspGlnAlaArgLeuAsnSerTrpGlyCysAlaPheArg
CTACAGGACCAGGCGCGGCTAAATTCATGGGGATGTGCGTTTAGA
1800

GlnValCysHisThrThrValProTrpValAsnAspSerLeuAla
CAAGTCTGCCACACTACTGTACCATGGGTTAATGATTCCTTAGCA

ProAspTrpAspAsnMetThrTrpGlnGluTrpGluLysGlnVal
CCTGACTGGGACAATATGACGTGGCAGGAATGGGAAAAACAAGTC

ArgTyrLeuGluAlaAsnIleSerLysSerLeuGluGlnAlaGln
CGCTACCTGGAGGCAAATATCAGTAAAAGTTTAGAACAGGCACAA
1900

IleGlnGlnGluLysAsnMetTyrGluLeuGlnLysLeuAsnSer
ATTCAGCAAGAGAAAAATATGTATGAACTACAAAAATTAAATAGC

TrpAspIlePheGlyAsnTrpPheAspLeuThrSerTrpValLys
TGGGATATTTTTGGCAATTGGTTTGACTTAACCTCCTGGGTCAAG
2000

TyrIleGlnTyrGlyValLeuIleIleValAlaValIleAlaLeu
TATATTCAATATGGAGTGCTTATAATAGTAGCAGTAATAGCTTTA

ArgIleValIleTyrValValGlnMetLeuSerArgLeuArgLys
AGAATAGTGATATATGTAGTACAAATGTTAAGTAGGCTTAGAAAG

GlyTyrArgProValPheSerSerProProGlyTyrIleGlnGln
GGCTATAGGCCTGTTTTCTCTTCCCCCCCCGGTTATATCCAACAG

IleHisIleHisLysAspArgGlyGlnProAlaAsnGluGluThr
ATCCATATCCACAAGGACCGGGGACAGCCAGCCAACGAAGAAACA
2200

GluGluAspGlyGlySerAsnGlyGlyAspArgTyrTrpProTrp
GAAGAAGACGGTGGAAGCAACGGTGGAGACAGATACTGGCCCTGG

ProIleAlaTyrIleHisPheLeuIleArgGlnLeuIleArgLeu
CCGATAGCATATATACATTTCCTGATCCGCCAGCTGATTCGCCTC

LeuThrArgLeuTyrSerIleCysArgAspLeuLeuSerArgSer
TTGACCAGACTATACAGCATCTGCAGGGACTTACTATCCAGGAGC
2300

PheLeuThrLeuGlnLeuIleTyrGlnAsnLeuArgAspTrpLeu
TTCCTGACCCTCCAACTCATCTACCAGAATCTCAGAGACTGGCTG

25

```
ArgLeuArgThrAlaPheLeuGlnTyrGlyCysGluTrpIleGln
AGACTTAGAACAGCCTTCTTGCAATATGGGTGCGAGTGGATCCAA
                    2400
GluAlaPheGlnAlaAlaAlaArgAlaThrArgGluThrLeuAla
GAAGCATTCCAGGCCGCCGCGAGGGCTACAAGAGAGACTCTTGCG

GlyAlaCysArgGlyLeuTrpArgValLeuGluArgIleGlyArg
GGCGCGTGCAGGGGCTTGTGGAGGGTATTGGAACGAATCGGGAGG
                    2500
GlyIleLeuAlaValProArgArgIleArgGlnGlyAlaGluIle
GGAATACTCGCGGTTCCAAGAAGGATCAGACAGGGAGCAGAAATC

AlaLeuLeu
GCCCTCCTG
```

Comme déjà indiqué plus haut, l'invention concerne naturellement tous les HIV-2 dont les ARNs présentent des caractéristiques semblables, en particulier des régions _gag_ et _env_ comportant des séquences ayant des homologies de séquence nucléotidique d'au moins 50 %, de préférence 70 % et plus avantageusement encore 90 % avec les séquences _gag_ et _env_ de HIV-2 ROD.

L'invention concerne plus particulièrement les fragments d'ADNc qui codent respectivement pour les protéines p16, p26 et p12 et qui sont également inclues dans GAGRODN. En particulier elle concerne les séquences s'étendant :

— à partir du nucléotide 1 jusqu'au nucléotide 405 (codant pour p16)
— à partir du nucléotide 406 jusqu'au nucléotide 1155 (codant pour p26) et,
— à partir du nucléotide 1156 jusqu'au nucléotide 1566 (codant pour p12).

Elle concerne aussi plus particulièrement le fragment d'ADNc codant pour la gp140 inclue dans ENVRN et s'étendant à partir du nucléotide 1 jusqu'au nucléotide 2574.

Elle concerne également les séquences de nucléotides qui se distinguent des précédentes par des substitutions de nucléotides mettant à profit la dégénérescence du code génétique, dès lors que ces substitutions n'entraînent pas une modification des séquences en acides aminés que codent lesdites séquences de nucléotides.

De même l'invention concerne les protéines ou glycoprotéines dont les séquences en acides aminés correspondent à celles qui découlent des pages précédentes, ainsi que les peptides équivalents à savoir des peptides se distinguant de ceux qui découlent des pages précédentes par addition, substitution ou délétion d'acides aminés qui n'affectent pas les propriétés immunologiques globales desdits peptides.

L'invention concerne tout particulièrement la glycoprotéine d'enveloppe présentant la séquence en acides aminés qui découle de ENVRN.

L'invention concerne également une composition immunogène caractérisée en ce qu'elle est dosée en antigène, tel que la gp 140 du virus HIV-2, de façon à permettre l'administration de doses unitaires de 10 à 500, notamment du 50 à 100 mg par kg de corps.

A titre indicatif, les poids moléculaires théoriques (PM) des protéines de HIV-2 sont indiqués, en comparaison avec ceux de HIV-1.

| PM des protéines de HIV-2 en kd | | PM des protéines pour HIV-1 en kd | |
|---|---|---|---|
| _gag_ complet | 58,3 | _gag_ complet | 55,8 |
| p 16 | 15 | p 18 | 14,9 |
| p 26 | 27,6 | | |
| p 12 | 15,8 | | |
| _env_ | 98,6 | _env_ | 97,4 |
| _env_ externe | 57,4 | | |
| _env_ transmembranaire | 41,2 | | |

HIV-2 MIR et HIV-2 ROD ont également été déposés à la « National Collection of Animal Cell Cultures » (ECACC) à SALISBURY (Grande-Bretagne) le 9 janvier 1987 sous les numéros d'accès 87.01.1001 et 87.01.1002 respectivement.

EP 0 239 425 B1

De plus, les plasmides pROD35 et pROD27,5 ont été déposés à la « National Collection of Industrial Bacteria » (NCIB) à ABERDEEN (Grande-Bretagne) le 9 janvier 1987 sous les numéros d'accès respectifs 12.398 et 12.399.

Toutes les publications auxquelles il a été fait référence dans cette description doivent être considérées comme faisant partie de cette description.

## Bibliographie

1 — F. Barré-Sinoussi et al., Science 220, 868 (1983).
2 — L. Montagnier et al., In : Human Tcell leukemia or lymphoma viruses (Gallo, R. C., Essex, M. E., Gross, L., eds.) Cold Spring Harbor Laboratory, New York, 363 (1964).
3 — M. Popovic, M. G. Sarngadharan, E. Read, R. C. Gallo, Science 224, 497 (1984).
4 — J. Levy et al., Science 225, 840 (1984).
5 — P. Sonigo et al., Cell 42, 369 (1985).
7 — J. W. Curran et al., Science 229, 1352 (1985).
8 — A. Ellrodt et al., Lancet i, 1383 (1984).
9 — P. Piot et al., Lancet ii, 65 (1984).
10 — F. Brun-Vezinet et al., Science 226, 453 (1984).
11 — N. Clumeck et al., N. Engl. J. Med. 313, 182 (1985).
12 — A. B. Rabson and M. A. Martin, Cell 40, 477 (1985).
13 — S. Benn et al., Science 230, 949 (1985).
14 — M. Alizon, Manuscript in preparation.
15 — F. Brun-Verzinet, Unpublished data.
16 — M. D. Daniel et al., Science 228, 1201 (1985).
17 — P. J. Kanki et al., Science 228, 1199 (1985).
18 — N. L. Letwin et al., Science 230, 71 (1985).
19 — A. Gatzar et al., Blood 55, 409 (1980).
20 — D. Klatzmann et al., Science 225, 59 (1984).
21 — L. Montagnier et al., Virology 144, 283 (1985).
22 — J. S. Allan et al., Science 228, 1091 (1985).
23 — U. Gübler et B. J. Hoffmann, Gene 25, 263 (1983).
24 — F. diMarzo Veronese et al., Science 229, 1402 (1985).
25 — V. S. Kalyanaraman et al., Science 218, 571 (1982).
26 — I. S. Y. Chen, J. McLaughlin, J. C. Gasson, S. C. Clark and D. W. Golde, Nature 305, 502 (1983).
27 — F. Barin et al., Lancet ii, 1387 (1985).
28 — P. J. Kanki, J. alroy, M. Essex, Science 230, 951 (1985).
29 — H. Towbin et al., Proc. Natl. Acad. Sci. USA 76, 4350 (1979).
30 — S. Wain-Hobson, P. Sonigo, O. Danos, S. Cole et M. Alizon, Cell 40, 9 (1985).
31 — M. Alizon et al., Nature 312, 757 (1984).

**Revendications** (pour les Etats contractants : BE, CH, DE, FR, GB, IT, LI, LU, NL, SE)

1. Rétrovirus humain HIV-2 ayant la capacité d'infecter des lymphocytes T4 humains et susceptible de provoquer un SIDA chez l'homme, ce rétrovirus étant constitué par l'un des rétrovirus déposés à la CNCM sous les n° I-502, I-532, I-642 et I-643 et à l'ECACC sous les n° 87011001 et 87011002 (correspondant respectivement aux n° I-502 et I-532) ou un variant de l'un de ces rétrovirus comprenant tous les antigènes qui sont reconnus par les anticorps formés contre les antigènes correspondants de l'un quelconque des rétrovirus ayant fait l'objet des susdits dépôts à la CNCM ou à l'ECACC.

2. Rétrovirus humain selon la revendication 1 caractérisé en ce que son ARN génomique est susceptible de s'hybrider dans des conditions stringentes avec la chaîne complémentaire d'un cADN ou fragment de cADN dérivé de l'ARN génomique de l'un quelconque des rétrovirus déposés à la CNCM sous les n° I-502, I-532, I-642 et I-643 et à l'ECACC sous les n° 87011001 et 87011002 (correspondant respectivement aux n° I-502 et I-532).

3. Rétrovirus humain selon la revendication 1, caractérisé en ce qu'il comprend un ensemble d'antigènes formés par des protéines du noyau (core) ayant des poids moléculaires de l'ordre de ·12.000, 16.000 et 26.000 daltons respectivement, des protéines ou glycoprotéines ayant des poids moléculaires respectivement de l'ordre de 36.000 daltons et de l'ordre de 42.000-45.000 daltons et une glycoprotéine majeure d'enveloppe ayant un poids moléculaire de l'ordre de 130.000-140.000 daltons.

4. Rétrovirus humain purifié selon la revendication 1, pathogène pour l'homme et susceptible de provoquer un SIDA, caractérisé par l'ensemble des propriétés suivantes :

— la cible préférentielle du rétrovirus HIV-2 est constituée par les cellules Leu3 (ou lymphocytes T4) humaines et pour des lignées cellulaires permanentes dérivées de ces lymphocytes T4 ;

— il est susceptible d'avoir un effet cytopathogène pour les lymphocytes T4 humains qu'il infecte ;

27

— il a une activité de transcriptase inverse nécessitant la présence d'ions Mg²⁺ et présentant une forte activité pour le poly(adénylate-oligodéoxythymidylate) (poly(A)-oligo(DT) 12-18) ;

— il a une densité d'environ 1,16 dans un gradient de sucrose ;

— il a un diamètre moyen de 140 nanomètres et un noyau ayant un diamètre moyen de 41 nanomètres ;

— il peut être cultivé dans des lignées permanentes exprimant la protéine T4 ;

— il n'est pas infectieux pour les lymphocytes T8 ;

— les lysats de ce virus contiennent une protéine p26 qui ne croise pas immunologiquement avec la protéine p24 du virus HTLV-1 ou du virus HTLV-2 ;

— ces lysats contiennent en outre une protéine p16 qui ne croise pas immunologiquement avec la protéine p19 de HTLV-1 ou de HTLV-2 dans des essais de radioimmuno-précipitation ;

— ils contiennent en outre une glycoprotéine d'enveloppe ayant un poids moléculaire de l'ordre de 130.000-140.000 daltons qui ne croise pas immunologiquement avec la glycoprotéine d'enveloppe gp110 du rétrovirus HIV-1 ;

ces lysats contiennent encore une protéine ou glycoprotéine, marquable par la ³⁵S-cystéine, ayant un poids moléculaire de l'ordre de 36.000 daltons ;

— l'ARN génomique de HIV-2 n'hybride pas, dans des conditions stringentes, avec l'ARN génomique de HIV-1 et n'hybride pas, dans des conditions non stringentes, soit avec le gène env, soit avec les LTR de HIV-1 ;

— l'ARN génomique de HIV-2 hybride faiblement dans des conditions non stringentes avec les séquences de nucléotides comprenant d'une part les nucléotides 990-1070, d'autre part les nucléotides 990-1260 de la région gag du génome de HIV-1.

5. Rétrovirus selon l'une quelconque des revendications 1 à 4, caractérisé en ce qu'il ne se multiplie pas de façon chronique dans les lymphocytes de singe macaque rhésus, lorsqu'il a été injecté in vivo et dans des conditions opératoires permettant le développement du virus STLV-III$_{mac}$ telles qu'elles ont été décrites par Letvin et al., Science (1985) vol. 230, 71-75.

6. Rétrovirus selon l'une quelconque des revendications 1 à 5, caractérisé en ce que la séquence comprenant la région R et la région U3 de la séquence comprenant son ARN génomique contient une séquence nucléotidique correspondant à la séquence de nucléotides suivante :

GTGGAAGGCGAGACTGAAAGCAAGAGGAATACCATTTAGTTAAAGGACAG

GAACAGCTATACTTGGTCAGGGCAGGAAGTAACTAACAGAAACAGCTGAG

ACTGCAGGGACTTTCCAGAAGGGGCTGTAACCAAGGGAGGGACATGGGAG

GAGCTGGTGGGGAACGCCTCATATTCTCTGTATAATATACCCGCTGCTTG

CATTGTACTTCAGTCGCTCTGCGGAGAGGCTGGCAGATTGAGCCCTGGAG

GATCTCTCCAGCACTAGACGGATGAGCCTGGGTGCCCTGCTAGACTCTCA

CCAGCACTTGGCCGGTGCTGGCAGACGGCCCCACGCTTGCCTGCTTAAAA

ACCTTCCTTAATAAAGCTGCAGTAGAAGCA

7. Rétrovirus selon l'une quelconque des revendications 1 à 5, caractérisé en ce que son ARN génomique contient une séquence gag correspondant à la séquence de nucléotides :

GAGAGAGATG .

ATGGGCGCGAGAAACTCCGTCTTGACAGGGAAAAAAGCAGATGAA

TTAGAAAGAATCAGGTTACGGCCCGGCGGAAAGAAAAAGTACAGG

CTAAAACATATTGTGTGGGCAGCGAATAAATTGGACAGATTCGGA
100

TTAGCAGAGAGCCTGTTGGAGTCAAAAGAGGGTTGTCAAAAAATT

CTTACAGTTTTAGATCCAATGGTACCGACAGGTTCAGAAAATTTA
200

28

AAAAGTCTTTTTAATACTGTCTGCGTCATTTGGTGCATACACGCA

GAAGAGAAAGTGAAAGATACTGAAGGAGCAAAACAAATAGTGCGG
300

AGACATCTAGTGGCAGAAACAGGAACTGCAGAGAAAATGCCAAGC

ACAAGTAGACCAACAGCACCATCTAGCGAGAAGGGAGGAAATTAC
400

CCAGTGCAACATGTAGGCGGCAACTACACCCATATACCGCTGAGT

CCCCGAACCCTAAATGCCTGCGTAAAATTAGTAGAGGAAAAAAAG

TTCGGGGCAGAAGTAGTGCCAGGATTTCAGGCACTCTCAGAAGGC
500

TGCACGCCCTATGATATCAACCAAATGCTTAATTGTGTGGGCGAC

CATCAAGCAGCCATGCAGATAATCAGGGAGATTATCAATGAGGAA
600

GCAGCAGAATGGGATGTGCAACATCCAATACCAGGCCCCTTACCA

GCGGGGCAGCTTAGAGAGCCAAGGGGATCTGACATAGCAGGGACA
700

ACAAGCACAGTACAAGAACAGATCCAGTGGATGTTTAGGCCACAA

AATCCTGTACCAGTAGGAAACATCTATAGAAGATGGATCCAGATA
800

GGATTGCAGAAGTGTGTCAGGATGTACAACCCGACCAACATCCTA

GACATAAAACAGGGACCAAAGGAGCCGTTCCAAAGCTATGTAGAT
900

AGATTCTACAAAAGCTTGAGGGCAGAACAAACAGATCCAGCAGTG

AAGAATTGGATGACCCAAACACTGCTAGTACAAAATGCCAACCCA

GACTGTAAATTAGTGCTAAAAGGACTAGGGATGAACCCTACCTTA
1000

GAAGAGATGCTGACCGCCTGTCAGGGGGTAGGTGGGCCAGGCCAG

AAAGCTAGATTAATGGCAGAGGCCCTGAAAGAGGTCATAGGACCT
1100

GCCCCTATCCCATTCGCAGCAGCCCAGCAGAGAAAGGCATTTAAA

TGCTGGAACTGTGGAAAGGAAGGGCACTCGTCAAGACAATGCCGA
1200

29

GCACCTAGAAGGCAGGGCTGCTGGAAGTGTGGTAAGCCAGGACAC

ATCATGACAAACTGCCCAGATAGACAGGCAGGTTTTTTAGGACTG
1300

CGCCCTTGGGGAAAGAAGCCCCGCAACTTCCCCGTGGCCCAAGTT

CCGCAGGGGCTGACACCAACAGCACCCCCAGTGGATCCAGCAGTG

GATCTACTGGAGAAATATATGCAGCAAGGGAAAAGACAGAGAGAG
1400

CAGAGAGAGAGACCATACAAGGAAGTGACAGAGGACTTACTGCAC.

CTCGAGCAGGGGGAGACACCATACAGGGAGCCACCAACAGAGGAC
1500

TTGCTGCACCTCAATTCTCTCTTTGGAAAAGACCAG

8. Rétrovirus selon l'une quelconque des revendications 1 à 6, caractérisé en ce que son ARN génomique contient une séquence _env_ correspondant à la séquence de nucléotides :

ENVEN

ATGATGAATCAGCTGCTTATTGCCATTTTATTAGCTAGTGCTTGC

TTAGTATATTGCACCCAATATGTAACTGTTTTCTATGGCGTACCC

ACGTGGAAAAATGCAACCATTCCCCTCTTTTGTGCAACCAGAAAT
100

AGGGATACTTGGGGAACCATACAGTGCTTGCCTGACAATGATGAT

TATCAGGAAATAACTTTGAATGTAACAGAGGCTTTTGATGCATGG
200

AATAATACAGTAACAGAACAAGCAATAGAAGATGTCTGGCATCTA

TTCGAGACATCAATAAAACCATGTGTCAAACTAACACCTTTATGT
300

CTAGCAATGAAATGCAGCAGCACAGAGAGCAGCACAGGGAACAAC

ACAACCTCAAAGAGCACAAGCACAACCACAACCACACCCACAGAC
400

CAGGAGCAAGAGATAAGTGAGGATACTCCATGCGCACGCGCAGAC

AACTGCTCAGGATTGGGAGAGGAAGAAACGATCAATTGCCAGTTC

30

AATATGACAGGATTAGAAAGAGATAAGAAAAACAGTATAATGAA
500

ACATGGTACTCAAAAGATGTGGTTTGTGAGACAAATAATAGCACA

AATCAGACCCAGTGTTACATGAACCATTGCAACACATCAGTCATC
600

ACAGAATCATGTGACAAGCACTATTGGGATGCTATAAGGTTTAGA

TACTGTGCACCACCGGGTTATGCCCTATTAAGATGTAATGATACC
700

AATTATTCAGGCTTTCCACCCAACTGTTCTAAAGTAGTAGCTTCT

ACATGCACCAGGATGATGGAAACGCAAACTTCCACATGGTTTGGC
800

TTTAATGGCACTAGAGCAGAGAATAGAACATATATCTATTGGCAT

GGCAGAGATAATAGAACTATCATCAGCTTAAACAAATATTATAAT
900

CTCAGTTTGCATTGTAAGAGGCCAGGGAATAAGACAGTGAAACAA

ATAATGCTTATGTCAGGACATGTGTTTCACTCCCACTACCAGCCG

ATCAATAAAAGACCCAGACAAGCATGGTGCTGGTTCAAAGGCAAA
1000

TGGAAAGACGCCATGCAGGAGGTGAAGACCCTTGCAAAACATCCC

AGGTATAGAGGAACCAATGACACAAGGAATATTAGCTTTGCAGCG
1100

CCAGGAAAAGGCTCAGACCCAGAAGTAGCATACATGTGGACTAAC

TGCAGAGGAGAGTTTCTCTACTGCAACATGACTTGGTTCCTCAAT
1200

TGGATAGAGAATAAGACACACCGCAATTATGCACCGTGCCATATA

AAGCAAATAATTAACACATGGCATAAGGTAGGGAGAAATGTATAT
1300

TTGCCTCCCAGGGAAGGGGAGCTGTCCTGCAACTCAACAGTAACC

AGCATAATTGCTAACATTGACTGGCAAAACAATAATCAGACAAAC

ATTACCTTTAGTGCAGAGGTGGCAGAACTATACAGATTGGAGTTG
1400

GCAGATTATAAATTGGTAGAAATAACACCAATTGGCTTCGCACCT

ACAAAAGAAAAAGATACTCCTCTGCTCACGGGAGACATACAAGA
      1500

GGTGTGTTCGTGCTAGGGTTCTTGGGTTTTCTCGCAACAGCACGT

TCTGCAATGGGCGCTCGAGCGTCCCTGACCGTGTCGGCTCAGTCC
                              1600

CGGACTTTACTGGCCGGGATAGTGCAGCAACAGCAACAGCTGTTG

GACGTGGTCAAGAGACAACAAGAACTGTTGCGACTGACCGTCTGG
                              1700

GGAACGAAAAACCTCCAGGCAAGAGTCACTGCTATAGAGAAGTAC

CTACAGGACCAGCCGCGGCTAAATTCATGGGGATGTGCGTTTAGA
                              1800

CAAGTCTGCCACACTACTGTACCATGGGTTAATGATTCCTTAGCA

CCTGACTGGGACAATATGACGTGGCAGGAATGGGAAAAACAAGTC

CGCTACCTGGAGGCAAATATCAGTAAAGTTTAGAACAGGCACAA
      1900

ATTCAGCAAGAGAAAAATATGTATGAACTACAAAAATTAAATAGC

TGGGATATTTTTGGCAATTGGTTTGACTTAACCTCCTGGGTCAAG
                  2000

TATATTCAATATGGAGTGCTTATAATAGTAGCAGTAATAGCTTTA

AGAATAGTGATATATGTAGTACAAATGTTAAGTAGGCTTAGAAAG
                              2100

GGCTATAGGCCTGTTTTCTCTTCCCCCCCCGGTTATATCCAACAG

ATCCATATCCACAAGGACCGGGGACAGCCAGCCAACGAAGAAACA
                              2200

GAAGAAGACGGTGGAAGCAACGGTGGAGACAGATACTGGCCCTGG

CCGATAGCATATATACATTTCCTGATCCGCCAGCTGATTCGCCTC

TTGACCAGACTATACAGCATCTGCAGGGACTTACTATCCAGGAGC
2300

TTCCTGACCCTCCAACTCATCTACCAGAATCTCAGAGACTGGCTG

AGACTTAGAACAGCCTTCTTGCAATATGGGTGCGAGTGGATCCAA
                  2400

GAAGCATTCCAGGCCGCCGCGAGGGCTACAAGAGAGACTCTTGCG

GGCGCGTGCAGGGGCTTGTCGAGGGTATTGGAACGAATCGGGAGG

2500

GGAATACTCGCGGTTCCAAGAAGGATCAGACAGGGAGCAGAAATC

GCCCTCCTG

9. Rétrovirus selon l'une quelconque des revendications 1 à 8, caractérisé en ce que son ARN n'hybride pas dans des conditions stringentes avec l'ARN génomique de HIV-1 et n'hybride pas, dans des conditions non stringentes, avec le gène env et le LTR qui le jouxte, plus particulièrement avec la séquence de nucléotides 5290-9130, de HIV-1, et n'hybride pas, dans des conditions non stringentes, avec des séquences de la région pol du génome de HIV-1, en particulier avec la séquence de nucléotides 2170-2240.

10. Antigène purifié du rétrovirus HIV-2 ayant les caractéristiques immunologiques de la protéine p12 de HIV-2, reconnu immunologiquement par des anticorps formés contre un rétrovirus selon l'une quelconque des revendications 1 à 9, et ayant un poids moléculaire de l'ordre de 12.000 daltons.

11. Antigène purifié du rétrovirus HIV-2 ayant les caractéristiques immunologiques de la protéine p16 de HIV-2, reconnu immunologiquement par des anticorps formés contre un rétrovirus selon l'une quelconque des revendications 1 à 9 et ayant un poids moléculaire de l'ordre de 16.000 daltons.

12. Antigène purifié du rétrovirus HIV-2, ayant les caractéristiques immunologiques de la protéine p26 de HIV-2, reconnu immunologiquement par des anticorps formés contre un rétrovirus selon l'une quelconque des revendications 1 à 9 et ayant un poids moléculaire de l'ordre de 26.000 daltons.

13. Antigène purifié, du rétrovirus HIV-2 ayant les caractéristiques immunologiques de la glyprotéine gp36 de HIV-2, reconnu immunologiquement par des anticorps formé contre un rétrovirus selon l'une quelconque des revendications 1 à 9 et ayant un poids moléculaire de l'ordre de 36.000 daltons.

14. Antigène purifié, du rétrovirus HIV-2 ayant les caractéristiques immunologiques de la protéine p42 de HIV-2, reconnu immunologiquement par des anticorps formés contre un rétrovirus selon l'une quelconque des revendications 1 à 9 et ayant un poids moléculaire de l'ordre de 42.000-45.000 daltons.

15. Antigène purifié, du rétrovirus HIV-2 ayant les caractéristiques immunologiques de la glycoprotéine gp140 de HIV-2, reconnu immunologiquement par des anticorps formés contre un rétrovirus selon l'une quelconque des revendications 1 à 9 et ayant un poids moléculaire de l'ordre de 130.000-140.000 daltons.

16. Antigène ayant la séquence d'aminoacides ci-après ou partie de cette séquence dès lors qu'elle donne lieu à une réaction immunologique spécifique avec des anticorps contre un rétrovirus HIV-2, selon l'une quelconque des revendications 1 à 9, notamment lorsque cet antigène est mis en contact avec un sérum d'un patient infecté avec HIV-2 :

ArgLysAlaPheLys

CysTrpAsnCysGlyLysGluGlyHisSerAlaArgGlnCysArg

1200
AlaProArgArgGlnGlyCysTrpLysCysGlyLysProGlyHis

IleMetThrAsnCysProAspArgGlnAlaGlyPheLeuGlyLeu

1300
GlyProTrpGlyLysLysProArgAsnPheProValAlaGlnVal

ProGlnGlyLeuThrProThrAlaProProValAspProAlaVal

AspLeuLeuGluLysTyrMetGlnGlnGlyLysArgGlnArgGln

1400
GlnArgGlnArgProTyrLysGluValThrGluAspLeuLeuHis

LeuGluGlnGlyGlnThrProTyrArgGluProProThrGluAsp

1500
LeuLeuHisLeuAsnSerLeuPheGlyLysAspGln

33

17. Antigène ayant la séquence d'aminoacides ci-après ou une partie de cette séquence, dès lors qu'elle donne lieu à une réaction immunologique spécifique avec des anticorps contre un rétrovirus HIV-2, selon l'une quelconque des revendications 1 à 9, notamment lorsque cet antigène est mis en contact avec un sérum d'un patient infecté avec HIV-2 :

MetGlyAlaArgAsnSerValLeuArgGlyLysLysAlaAspGlu

LeuGluArgIleArgLeuArgProGlyGlyLysLysLysTyrArg

LeuLysHisIleValTrpAlaAlaAsnLysLeuAspArgPheGly

100
LeuAlaGluSerLeuLeuGluSerLysGluGlyCysGlnLysIle

LeuThrValLeuAspProMetValProThrGlySerGluAsnLeu

200
LysSerLeuPheAsnThrValCysValIleTrpCysIleHisAla

GluGlnLysValLysAspThrGluGlyAlaLysGlnIleValArg

300
ArgHisLeuValAlaGluThrGlyThrAlaGluLysMetProSer

ThrSerArgProThrAlaProSerSerGluLysGlyGlyAsnTyr

400

18. Antigène ayant la séquence d'aminoacides ci-après ou une partie de cette séquence, dès lors qu'elle donne lieu à une réaction immunologique spécifique avec des anticorps contre un rétrovirus HIV-2, selon l'une quelconque des revendications 1 à 9, notamment lorsque cet antigène est mis en contact avec un sérum d'un patient infecté avec HIV-2 :

ProValGlnHisValGlyGlyAsnTyrThrHisIleProLeuSer

ProArgThrLeuAsnAlaTrpValLysLeuValGluGluLysLys

PheGlyAlaGluValValProGlyPheGlnAlaLeuSerGluGly

500
CysThrProTyrAspIleAsnGlnMetLeuAsnCysValGlyAsp

HisGlnAlaAlaMetGlnIleIleArgGlnIleIleAsnGluGln

600
AlaAlaGluTrpAspValGlnHisProIleProGlyProLeuPro

AlaGlyGlnLeuArgGluProArgGlySerAspIleAlaGlyThr

700
ThrSerThrValGlnGluGlnIleGlnTrpMetPheArgProGln

AsnProValProValGlyAsnIleTyrArgArgTrpIleGlnIle

GlyLeuGlnLysCysValArgMetTyrAsnProThrAsnIleLeu 800

AspIleLysGlnGlyProLysGluProPheGlnSerTyrValAsp

ArgPheTyrLysSerLeuArgAlaGluGlnThrAspProAlaVal 900

LysAsnTrpMetThrGlnThrLeuLeuValGlnAsnAlaAsnPro

AspCysLysLeuValLeuLysGlyLeuGlyMetAsnProThrLeu

GluGluMetLeuThrAlaCysGlnGlyValGlyGlyProGlyGln 1000

LysAlaArgLeuMetAlaGluAlaLeuLysGluValIleGlyPro

AlaProIleProPheAlaAlaAlaGlnGln 1100

19. Antigène ayant la séquence d'aminoacides ci-après, ou partie de cette séquence, dès lors qu'elle donne lieu à une réaction immunologique spécifique avec des anticorps contre un rétrovirus HIV-2, selon l'une quelconque des revendications 1 à 9, notamment lorsque cet antigène est mis en contact avec un sérum d'un patient infecté avec HIV-2 :

ENVRN

MetMetAsnGlnLeuLeuIleAlaIleLeuLeuAlaSerAlaCys

LeuValTyrCysThrGlnTyrValThrValPheTyrGlyValPro

ThrTrpLysAsnAlaThrIleProLeuPheCysAlaThrArgAsn

ArgAspThrTrpGlyThrIleGlnCysLeuProAspAsnAspAsp 100

TyrGlnGluIleThrLeuAsnValThrGluAlaPheAspAlaTrp

AsnAsnThrValThrGluGlnAlaIleGluAspValTrpHisLeu 200

PheGluThrSerIleLysProCysValLysLeuThrProLeuCys

ValAlaMetLysCysSerSerThrGluSerSerThrGlyAsnAsn 300

ThrThrSerLysSerThrSerThrThrThrThrThrProThrAsp 400

35

GlnGluGlnGluIleSerGluAspThrProCysAlaArgAlaAsp

AsnCysSerGlyLeuGlyGluGluGluThrIleAsnCysGlnPhe

AsnMetThrGlyLeuGluArgAspLysLysLysGlnTyrAsnGlu

500
ThrTrpTyrSerLysAspValValCysGluThrAsnAsnSerThr

AsnGlnThrGlnCysTyrMetAsnHisCysAsnThrSerValIle

600
ThrGluSerCysAspLysHisTyrTrpAspAlaIleArgPheArg

TyrCysAlaProProGlyTyrAlaLeuLeuArgCysAsnAspThr

700
AsnTyrSerGlyPheAlaProAsnCysSerLysValValAlaSer

ThrCysThrArgMetMetGluThrGlnThrSerThrTrpPheGly

800
PheAsnGlyThrArgAlaGluAsnArgThrTyrIleTyrTrpHis

GlyArgAspAsnArgThrIleIleSerLeuAsnLysTyrTyrAsn

900
LeuSerLeuHisCysLysArgProGlyAsnLysThrValLysGln

IleMetLeuMetSerGlyHisValPheHisSerHisTyrGlnPro

IleAsnLysArgProArgGlnAlaTrpCysTrpPheLysGlyLys

1000
TrpLysAspAlaMetGlnGluValLysThrLeuAlaLysHisPro

ArgTyrArgGlyThrAsnAspThrArgAsnIleSerPheAlaAla

1100
ProGlyLysGlySerAspProGluValAlaTyrMetTrpThrAsn

CysArgGlyGluPheLeuTyrCysAsnMetThrTrpPheLeuAsn

1200
TrpIleGluAsnLysThrHisArgAsnTyrAlaProCysHisIle

LysGlnIleIleAsnThrTrpHisLysValGlyArgAsnValTyr

1300
LeuProProArgGluGlyGluLeuSerCysAsnSerThrValThr

36

SerIleIleAlaAsnIleAspTrpGlnAsnAsnAsnGlnThrAsn

IleThrPheSerAlaGluValAlaGluLeuTyrArgLeuGluLeu

1400
GlyAspTyrLysLeuValGluIleThrProIleGlyPheAlaPro

ThrLysGluLysArgTyrSerSerAlaHisGlyArgHisThrArg

1500
GlyValPheValLeuGlyPheLeuGlyPheLeuAlaThrAlaGly

SerAlaMetGlyAlaArgAlaSerLeuThrValSerAlaGlnSer

1600
ArgThrLeuLeuAlaGlyIleValGlnGlnGlnGlnGlnLeuLeu

AspValValLysArgGlnGlnGluLeuLeuArgLeuThrValTrp

1700
GlyThrLysAsnLeuGlnAlaArgValThrAlaIleGluLysTyr

LeuGlnAspGlnAlaArgLeuAsnSerTrpGlyCysAlaPheArg

1800
GlnValCysHisThrThrValProTrpValAsnAspSerLeuAla

ProAspTrpAspAsnMetThrTrpGlnGluTrpGluLysGlnVal

ArgTyrLeuGluAlaAsnIleSerLysSerLeuGluGlnAlaGln

1900
IleGlnGlnGluLysAsnMetTyrGluLeuGlnLysLeuAsnSer

TrpAspIlePheGlyAsnTrpPheAspLeuThrSerTrpValLys

2000
TyrIleGlnTyrGlyValLeuIleIleValAlaValIleAlaLeu

ArgIleValIleTyrValValGlnMetLeuSerArgLeuArgLys

2100
GlyTyrArgProValPheSerSerProProGlyTyrIleGlnGln

IleHisIleHisLysAspArgGlyGlnProThrAsnGluGluThr

2200
GluGluAspGlyGlySerAsnGlyGlyAspArgTyrTrpProTrp

ProIleAlaTyrIleHisPheLeuIleArgGlnLeuIleArgLeu

LeuThrArgLeuTyrSerIleCysArgAspLeuLeuSerArgSer

2300

37

EP 0 239 425 B1

PheLeuThrLeuGlnLeuIleTyrGlnAsnLeuArgAspTrpLeu

ArgLeuArgThrAlaPheLeuGlnTyrGlyCysGlnTrpIleGln

2400
GluAlaPheGlnAlaAlaAlaArgAlaThrArgGlnThrLeuAla

GlyAlaCysArgGlyLeuTrpArgValLeuGlnArgIleGlyArg

2500
GlyIleLeuAlaValProArgArgIleArgGlnGlyAlaGluIle

AlaLeuLeu

20. Composition pour le diagnostic in vitro de la présence dans un prélèvement biologique humain, d'anticorps contre un virus HIV-2 humain susceptible d'avoir un effet cytopathogène pour les lymphocytes T4, caractérisée en ce qu'elle contient au moins un antigène, notamment une protéine ou une glycoprotéine du rétrovirus HIV-2 selon l'une quelconque des revendications 1 à 9, ou au moins un antigène selon l'une quelconque des revendications 10 à 19.

21. Composition selon la revendication 20, caractérisée en ce qu'elle consiste en un extrait total ou en un lysat du susdit rétrovirus.

22. Composition selon la revendication 20, caractérisée en ce que l'antigène consiste en au moins l'une des protéines internes du noyau (core) du susdit virus, choisies parmi les protéines ou glycoprotéines p12, p16 et p26, ayant respectivement des poids moléculaires de l'ordre de 12.000, 16.000 et 26.000 daltons.

23. Composition selon la revendication 20 ou la revendication 21, caractérisée en ce qu'elle contient une glycoprotéine gp140 ayant un poids moléculaire de l'ordre de 130.000 à 140.000 daltons.

24. Composition selon la revendication 20 ou la revendication 21, caractérisée en ce qu'elle contient une glycoprotéine gp36 ayant un poids moléculaire de l'ordre de 36.000 daltons.

25. Composition pour le diagnostic in vitro dans un prélèvement biologique humain, d'anticorps contre un rétrovirus humain susceptible d'avoir un effet cytopathogène pour les lymphocytes T4 humains, comprenant au moins un antigène de HIV-1, caractérisée en ce qu'elle comprend également au moins un antigène de HIV-2, selon l'une quelconque des revendications 1 à 9, notamment une protéine ou une glycoprotéine de ce rétrovirus HIV-2 ou au moins un antigène selon l'une quelconque des revendications 10 à 19.

26. Composition selon la revendication 25, caractérisée en ce qu'elle contient des protéines du noyau (core) de HIV-1 et de HIV-2.

27. Composition selon la revendication 26, caractérisée en ce qu'elle contient les p25 d'un HIV-1 et p26 d'un HIV-2.

28. Composition selon la revendication 26, ou la revendication 26, caractérisée en ce qu'elle contient la p18 d'un HIV-1 et la p16 d'un HIV-2.

29. Composition selon la revendication 25, caractérisée en ce qu'elle contient des glycoprotéines d'enveloppe d'un HIV-1 et des glycoprotéines d'enveloppe d'un HIV-2.

30. Composition selon la revendication 29, caractérisée en ce qu'elle contient la gp110 de HIV-1 et la gp140 de HIV-2.

31. Composition selon la revendication 29 ou la revendication 30, caractérisée en ce qu'elle contient la gp42 de HIV-1 et la p36 de HIV-2.

32. Composition selon la revendication 26, caractérisée en ce qu'elle contient des mélanges d'une part, d'antigènes de protéines ou de glycoprotéines de HIV-1 ou de l'ensemble, d'autre part, des mélanges de protéines ou de glycoprotéines de HIV-2 ou des deux à la fois.

33. Méthode pour la détection in vitro de la présence d'anticorps induits chez l'homme infecté par un rétrovirus humain HIV-2 dans un échantillon biologique humain, tel qu'un sérum et, plus particulièrement pour le diagnostic in vitro d'un SLA ou SIDA potentiel ou existant dû à un tel rétrovirus et provenant de la personne faisant l'objet du diagnostic, caractérisée en ce que l'on met en contact cet échantillon biologique avec un antigène reconnu par un anticorps induit chez l'homme infecté par un rétrovirus humain HIV-2 tel que défini dans l'une quelconque des revendications 1 à 9, dans des conditions autorisant la formation d'un composé immunologique entre cet antigène et ledit anticorps et en ce que l'on détecte le conjugué immunologique éventuellement formé entre cet anticorps et l'antigène mis en œuvre.

34. Méthode selon la revendication 33, caractérisée en ce que l'échantillon biologique est mis en

38

contact avec un antigène selon l'une quelconque des revendications 10 à 19 ou une composition selon l'une quelconque des revendications 20 à 24 dans des conditions autorisant la formation d'un composé immunologique entre cet antigène et lesdits anticorps et en ce que l'on détecte le conjugué immunologique éventuellement formé entre ces anticorps anti-HIV-2 et l'antigène mis en œuvre.

35. Méthode selon la revendication 34, caractérisée en ce que l'on réalise la détection dudit conjugué immunologique en faisant réagir le conjugué immunologique éventuellement formé avec un réactif marqué formé soit par des anticorps anti-immunoglobulines humaines, soit par une protéine A bactérienne et en ce que l'on détecte le complexe formé entre le réactif et le susdit conjugué immunologique.

36. Méthode pour la détection in vitro de la présence d'anticorps reconnaissant des antigènes d'un rétrovirus humain susceptible d'avoir un effet cytopathogène pour les lymphocytes T4 humains, dans un échantillon biologique humain, tel qu'un sérum, et plus particulièrement pour le diagnostic in vitro d'un SLA ou SIDA potentiel ou existant dû à un rétrovirus humain caractérisée en ce que l'on met en contact cet échantillon biologique provenant de la personne faisant l'objet du diagnostic avec un mélange d'antigènes capables de donner une réaction immunologique spécifique avec les anticorps contre d'une part un rétrovirus humain HIV-2 susceptible d'avoir un effet cytopathogène tel que défini dans l'une quelconque des revendications 1 à 9, d'autre part un rétrovirus humain HIV-1 et, en ce que l'on détecte le conjugué immunologique éventuellement formé entre l'un au moins de ces antigènes et lesdits anticorps.

37. Méthode selon la revendication 36, caractérisée en ce que l'échantillon biologique provenant de la personne faisant l'objet du diagnostic est mis en contact avec un mélange d'antigènes issus à la fois de HIV-1 et d'un HIV-2, selon l'une quelconque des revendications 1 à 9, notamment avec une composition selon l'une quelconque des revendications 26 à 32 et en ce que l'on détecte le conjugué immunologique éventuellement formé entre l'un au moins de ces antigènes et lesdits anticorps.

38. Nécessaire ou kit pour la détection d'anticorps contre un rétrovirus susceptible d'avoir un effet cytopathogène pour l'homme, du type HIV-2 dans un échantillon biologique, notamment d'un porteur éventuel de ces anticorps, caractérisé en ce qu'il comprend :
— au moins un antigène selon l'une quelconque des revendications 10 à 19 ou une composition telle que définie dans l'une quelconque des revendications 20 à 32, et 34
— des moyens pour détecter le conjugué immunologique résultant de la réaction immunologique entre l'antigène et ledit échantillon biologique.

39. Nécessaire ou kit selon la revendication 38, caractérisé en ce que les moyens pour détecter le conjugué immunologique formé comprennent des anti-immunoglobulines humaines ou une protéine A et des moyens pour détecter le complexe formé entre les anticorps anti-HIV-2 contenus dans le conjugué immunologique détecté.

40. Nécessaire ou kit pour le diagnostic in vitro de la présence d'anticorps contre un rétrovirus cytopathogène pour des lymphocytes T4 humains, notamment d'un porteur éventuel de ces anticorps, caractérisé en ce qu'il comprend :
— un mélange d'au moins un antigène de HIV-1 et d'au moins un antigène de HIV-2, notamment une composition telle que définie dans l'une quelconque des revendications 25 à 32,
— des moyens pour détecter le conjugué immunologique résultant de la réaction immunologique entre les antigènes et les anticorps du fluide biologique.

41. Composition immunogène contenant une glycoprotéine d'enveloppe du rétrovirus humain HIV-2, tel que défini dans l'une quelconque des revendications 1 à 9, telle que la gp140 de ce rétrovirus, ou une partie de cette glycoprotéine capable d'induire des anticorps contre HIV-2 en association avec un véhicule pharmaceutiquement acceptable, approprié à la constitution de vaccins contre HIV-2.

42. Composition selon la revendication 41, caractérisée en ce qu'elle contient au moins une partie immunogène d'une glycoprotéine contenant une ossature protéique ayant la séquence suivante, cette partie immunogène étant capable d'induire des anticorps contre un rétrovirus humain HIV-2 tel que défini dans l'une quelconque des revendications 1 à 9 :

ENVRX

MetMetAsnGlnLeuLeuIleAlaIleLeuLeuAlaSerAlaCys

LeuValTyrCysThrGlnTyrValThrValPheTyrGlyValPro

ThrTrpLysAsnAlaThrIleProLeuPheCysAlaThrArgAsn

100
ArgAspThrTrpGlyThrIleGlnCysLeuProAspAsnAspAsp

39

TyrGlnGluIleThrLeuAsnValThrGluAlaPheAspAlaTrp

200
AsnAsnThrValThrGluGlnAlaIleGluAspValTrpHisLeu

PheGluThrSerIleLysProCysValLysLeuThrProLeuCys

300
ValAlaMetLysCysSerSerThrGluSerSerThrGlyAsnAsn

ThrThrSerLysSerThrSerThrThrThrThrThrProThrAsp

400
GlnGluGlnGluIleSerGluAspThrProCysAlaArgAlaAsp

AsnCysSerGlyLeuGlyGluGluGluThrIleAsnCysGlnPhe

AsnMetThrGlyLeuGluArgAspLysLysLysGlnTyrAsnGlu

500
ThrTrpTyrSerLysAspValValCysGluThrAsnAsnSerThr

AsnGlnThrGlnCysTyrMetAsnHisCysAsnThrSerValIle

600
ThrGluSerCysAspLysHisTyrTrpAspAlaIleArgPheArg

TyrCysAlaProProGlyTyrAlaLeuLeuArgCysAsnAspThr

700
AsnTyrSerGlyPheAlaProAsnCysSerLysValValAlaSer

ThrCysThrArgMetMetGluThrGlnThrSerThrTrpPheGly

800
PheAsnGlyThrArgAlaGluAsnArgThrTyrIleTyrTrpHis

GlyArgAspAsnArgThrIleIleSerLeuAsnLysTyrTyrAsn

900
LeuSerLeuHisCysLysArgProGlyAsnLysThrValLysGln

IleMetLeuMetSerGlyHisValPheHisSerHisTyrGlnPro

IleAsnLysArgProArgGlnAlaTrpCysTrpPheLysGlyLys

1000
TrpLysAspAlaMetGlnGluValLysThrLeuAlaLysHisPro

ArgTyrArgGlyThrAsnAspThrArgAsnIleSerPheAlaAla

1100

40

ProGlyLysGlySerAspProGluValAlaTyrMetTrpThrAsn

CysArgGlyGluPheLeuTyrCysAsnMetThrTrpPheLeuAsn

1200
TrpIleGluAsnLysThrHisArgAsnTyrAlaProCysHisIle

LysGlnIleIleAsnThrTrpHisLysValGlyArgAsnValTyr

1300
LeuProProArgGluGlyGluLeuSerCysAsnSerThrValThr

SerIleIleAlaAsnIleAspTrpGlnAsnAsnAsnGlnThrAsn

IleThrPheSerAlaGluValAlaGluLeuTyrArgLeuGluLeu

1400
GlyAspTyrLysLeuValGluIleThrProIleGlyPheAlaPro

ThrLysGluLysArgTyrSerSerAlaHisGlyArgHisThrArg

1500
GlyValPheValLeuGlyPheLeuGlyPheLeuAlaThrAlaGly

SerAlaMetGlyAlaArgAlaSerLeuThrValSerAlaGlnSer

1600
ArgThrLeuLeuAlaGlyIleValGlnGlnGlnGlnGlnLeuLeu

AspValValLysArgGlnGlnGluLeuLeuArgLeuThrValTrp

1700
GlyThrLysAsnLeuGlnAlaArgValThrAlaIleGluLysTyr

LeuGlnAspGlnAlaArgLeuAsnSerTrpGlyCysAlaPheArg

1800
GlnValCysHisThrThrValProTrpValAsnAspSerLeuAla

ProAspTrpAspAsnMetThrTrpGlnGluTrpGluLysGlnVal

ArgTyrLeuGluAlaAsnIleSerLysSerLeuGluGlnAlaGln

1900
IleGlnGlnGluLysAsnMetTyrGluLeuGlnLysLeuAsnSer

TrpAspIlePheGlyAsnTrpPheAspLeuThrSerTrpValLys

2000
TyrIleGlnTyrGlyValLeuIleIleValAlaValIleAlaLeu

41

```
ArgIleValIleTyrValValGlnMetLeuSerArgLeuArgLys

                                  2100
GlyTyrArgProValPheSerSerProProGlyTyrIleGlnGln

IleHisIleHisLysAspArgGlyGlnProAlaAsnGluGluThr

                                  2200
GluGluAspGlyGlySerAsnGlyGlyAspArgTyrTrpProTrp

ProIleAlaTyrIleHisPheLeuIleArgGlnLeuIleArgLeu

LeuThrArgLeuTyrSerIleCysArgAspLeuLeuSerArgSer

2300
PheLeuThrLeuGlnLeuIleTyrGlnAsnLeuArgAspTrpLeu

ArgLeuArgThrAlaPheLeuGlnTyrGlyCysGlnTrpIleGln

               2400
GluAlaPheGlnAlaAlaAlaArgAlaThrArgGluThrLeuAla

GlyAlaCysArgGlyLeuTrpArgValLeuGluArgIleGlyArg

               2500
GlyIleLeuAlaValProArgArgIleArgGlnGlyAlaGluIle


AlaLeuLeu
```

43. Composition immunogène selon la revendication 41 ou la revendication 42, caractérisée en ce qu'elle est dosée en antigène de façon à permettre l'administration d'une dose unitaire de 10 à 500, notamment de 50 à 100 microgrammes par kilogramme de corps.

44. Anticorps reconnaissant spécifiquement un antigène d'un rétrovirus HIV-2, selon l'une quelconque des revendications 1 à 9.

45. Anticorps selon la revendication 44, caractérisé en ce qu'il est monoclonal.

46. Acide nucléique, le cas échéant marqué, dérivé d'une partie au moins de l'ARN du virus HIV-2 ou de l'un de ses variants, tel que défini dans l'une quelconque des revendications 1 à 9, caractérisé par sa capacité d'hybridation avec l'ARN dudit virus HIV-2 et par l'absence d'hybridation dans les conditions stringentes avec les sondes 1 à 4 comprenant respectivement les nucléotides 990-1070, 990-1260, 2170-2240, 3370-3640 de l'ADN dérivé du génome de HIV-1.

47. Acide nucléique selon la revendication 46, contenant une partie au moins d'une séquence d'ADNc capable de s'hybrider avec l'ARN génomique entier d'un rétrovirus HIV-2 selon une quelconque des revendications 1 à 9, et ne s'hybridant pas dans les conditions stringentes avec les sondes dont les fragments d'ADN dérivés du génome de HIV-I respectivement constitués de fragments délimités par les nucléotides 990-1070, 990-1260, 2170-2240, 3370-3640 de l'ADN dérivé du génome de HIV-1.

48. Acide nucléique selon la revendication 46, caractérisé en ce qu'il contient la séquence de nucléotides :

```
GTGGAAGGCGAGACTGAAAGCAAGAGGAATACCATTTAGTTAAAGGACAG

GAACAGCTATACTTGGTCAGGGCAGGAAGTAACTAACAGAAACAGCTGAG

ACTGCAGGGACTTTCCAGAAGGGGCTGTAACCAAGGGAGGGACATGGGAG

GAGCTGGTGGGGAACGCCTCATATTCTCTGTATAATATACCCGCTGCTTG

CATTGTACTTCAGTCGCTCTGCGGAGAGGCTGGCAGATTGAGCCCTGGAG

GATCTCTCCAGCACTAGACGGATGAGCCTGGGTGCCCTGCTAGACTCTCA

CCAGCACTTGGCCGGTGCTGGCAGACGGCCCCACGCTTGCCTGCTTAAAA

ACCTTCCTTAATAAAGCTGCAGTAGAAGCA
```

42

49. Acide nucléique selon la revendication 46, caractérisé en ce qu'il contient une séquence nucléotidique codant pour tout ou partie de la séquence d'aminoacides indiquée ci-après, cette séquence ou partie de séquence d'aminoacides ayant la capacité de donner une réaction immunologique spécifique avec des anticorps contre un rétrovirus HIV-2 tel que défini dans l'une quelconque des revendications 1 à 9, lorsque cette séquence d'aminoacides est mise en contact avec un sérum d'un patient infecté avec un rétrovirus HIV-2 :

GAGRODI                    :

MetGlyAlaArgAsnSerValLeuArgGlyLysLysAlaAspGlu

LeuGluArgIleArgLeuArgProGlyGlyLysLysLysTyrArg

LeuLysHisIleValTrpAlaAlaAsnLysLeuAspArgPheGly

LeuAlaGlySerLeuLeuGluSerLysGluGlyCysGlnLysIle
100

LeuThrValLeuAspProMetValProThrGlySerGluAsnLeu

LysSerLeuPheAsnThrValCysValIleTrpCysIleHisAla
200

GluGluLysValLysAspThrGluGlyAlaLysGlnIleValArg

ArgHisLeuValAlaGluThrGlyThrAlaGluLysMetProSer
300

ThrSerArgProThrAlaProSerSerGlnLysGlyGlyAsnTyr

ProValGlnHisValGlyGlyAsnTyrThrHisIleProLeuSer
400

ProArgThrLeuAsnAlaTrpValLysLeuValGluGluLysLys

PheGlyAlaGluValValProGlyPheGlnAlaLeuSerGluGly

CysThrProTyrAspIleAsnGlnMetLeuAsnCysValGlyAsp
500

HisGlnAlaAlaMetGlnIleIleArgGluIleIleAsnGluGlu

AlaAlaGlyTrpAspValGlnHisProIleProGlyProLeuPro
600

AlaGlyGlyLeuArgGluProArgGlySerAspIleAlaGlyThr

ThrSerThrValGluGluGlnIleGlnTrpMetPheArgProGln
700

AsnProValProValGlyAsnIleTyrArgArgTrpIleGlnIle
800

```
GlyLeuGlnLysCysValArgMetTyrAsnProThrAsnIleLeu

AspIleLysGlnGlyProLysGluProPheGlnSerTyrValAsp
                                        900
ArgPheTyrLysSerLeuArgAlaGluGlnThrAspProAlaVal

LysAsnTrpMetThrGlnThrLeuLeuValGlnAsnAlaAsnPro

AspCysLysLeuValLeuLysGlyLeuGlyMetAsnProThrLeu
        1000
GluGluMetLeuThrAlaCysGlnGlyValGlyGlyProGlyGln

LysAlaArgLeuMetAlaGluAlaLeuLysGluValIleGlyPro
            1100
AlaProIleProPheAlaAlaAlaGlnGlnArgLysAlaPheLys

CysTrpAsnCysGlyLysGluGlyHisSerAlaArgGlnCysArg
                        1200
AlaProArgArgGlnGlyCysTrpLysCysGlyLysProGlyHis

IleMetThrAsnCysProAspArgGlnAlaGlyPheLeuGlyLeu
                            1300
GlyProTrpGlyLysLysProArgAsnPheProValAlaGlnVal

ProGlnGlyLeuThrProThrAlaProProValAspProAlaVal

AspLeuLeuGluLysTyrMetGlnGlnGlyLysArgGlnArgGlu
1400
GlnArgGluArgProTyrLysGluValThrGluAspLeuLeuHis

LeuGluGlnGlyGluThrProTyrArgGluProProThrGluAsp
        1500
LeuLeuHisLeuAsnSerLeuPheGlyLysAspGln
```

50. Acide nucléique selon la revendication 46, caractérisé en ce qu'il contient une séquence nucléotidique codant pour une partie au moins de la séquence d'aminoacides indiquée ci-après, cette séquence d'aminoacides étant capable de donner une réaction immunologique spécifique avec des anticorps contre un rétrovirus HIV-2 tel que défini dans l'une quelconque des revendications 1 à 9, lorsque cette séquence d'aminoacides est mise en contact avec un sérum d'un patient infecté avec un rétrovirus HIV-2.

```
                                        ArgLysAlaPheLys

CysTrpAsnCysGlyLysGluGlyHisSerAlaArgGlnCysArg
                        1200
```

```
AlaProArgArgGlnGlyCysTrpLysCysGlyLysProGlyHis

IleMetThrAsnCysProAspArgGlnAlaGlyPheLeuGlyLeu
                                        1300
GlyProTrpGlyLysLysProArgAsnPheProValAlaGlnVal

ProGlnGlyLeuThrProThrAlaProProValAspProAlaVal

AspLeuLeuGluLysTyrMetGlnGlnGlyLysArgGlnArgGln
 1400
GlnArgGluArgProTyrLysGluValThrGluAspLeuLeuHis

LeuGluGlnGlyGluThrProTyrArgGluProProThrGluAsp
            1500
LeuLeuHisLeuAsnSerLeuPheGlyLysAspGln
```

51. Acide nucléique selon la revendication 46, caractérisé en ce qu'il contient une séquence nucléotidique codant pour tout ou partie de la séquence d'aminoacides indiquée ci-après, la séquence codée d'aminoacides ayant la capacité de donner· une réaction immunologique spécifique avec des anticorps contre un rétrovirus HIV-2 tel que défini dans l'une quelconque des revendications 1 à 9, lorsqu'elle est mise en contact avec un sérum d'un patient infecté avec un rétrovirus HIV-2 :

```
MetGlyAlaArgAsnSerValLeuArgGlyLysLysAlaAspGlu

LeuGluArgIleArgLeuArgProGlyGlyLysLysLysTyrArg

LeuLysHisIleValTrpAlaAlaAsnLysLeuAspArgPheGly
        100
LeuAlaGluSerLeuLeuGluSerLysGlnGlyCysGlnLysIle

LeuThrValLeuAspProMetValProThrGlySerGluAsnLeu
        200
LysSerLeuPheAsnThrValCysValIleTrpCysIleHisAla

GluGlnLysValLysAspThrGluGlyAlaLysGlnIleValArg
                300
ArgHisLeuValAlaGluThrGlyThrAlaGluLysMetProSer

ThrSerArgProThrAlaProSerSerGluLysGlyGlyAsnTyr
                        400
```

52. Acide nucléique selon la revendication 46, caractérisé en ce qu'il contient une séquence nucléotidique codant pour tout ou partie de la séquence d'aminoacides indiquée ci-après, la séquence codée d'aminoacides ayant la capacité de donner une réaction immunologique spécifique avec des anticorps contre un rétrovirus HIV-2 tel que défini dans l'une quelconque des revendications 1 à 9, lorsqu'elle est mise en contact avec un sérum d'un patient infecté spécifiquement avec un rétrovirus HIV-2 :

45

```
ProValGlnHisValGlyGlyAsnTyrThrHisIleProLeuSer

ProArgThrLeuAsnAlaTrpValLysLeuValGluGluLysLys

PheGlyAlaGluValValProGlyPheGlnAlaLeuSerGluGly
          500
CysThrProTyrAspIleAsnGlnMetLeuAsnCysValGlyAsp

HisGlnAlaAlaMetGlnIleIleArgGluIleIleAsnGluGlu
          600
AlaAlaGluTrpAspValGlnHisProIleProGlyProLeuPro

AlaGlyGlnLeuArgGluProArgGlySerAspIleAlaGlyThr
                    700
ThrSerThrValGluGluGlnIleGlnTrpMetPheArgProGln

AsnProValProValGlyAsnIleTyrArgArgTrpIleGlnIle
                         800
GlyLeuGlnLysCysValArgMetTyrAsnProThrAsnIleLeu

AspIleLysGlnGlyProLysGluProPheGlnSerTyrValAsp
                              900
ArgPheTyrLysSerLeuArgAlaGluGlnThrAspProAlaVal

LysAsnTrpMetThrGlnThrLeuLeuValGlnAsnAlaAsnPro

AspCysLysLeuValLeuLysGlyLeuGlyMetAsnProThrLeu
     1000
GluGlnMetLeuThrAlaCysGlnGlyValGlyGlyProGlyGln

LysAlaArgLeuMetAlaGluAlaLeuLysGluValIleGlyPro
          1100
AlaProIleProPheAlaAlaAlaGlnGln
```

53. Acide nucléique selon la revendication 46, caractérisé en ce qu'il contient une séquence nucléotidique codant pour tout ou partie de la séquence d'aminoacides indiquée ci-après, la séquence codée d'acide aminée ayant la capacité de donner une réaction immunologique spécifique avec des anticorps contre un rétrovirus HIV-2 tel que défini dans l'une quelconque des revendications 1 à 9, lorsqu'elle est mise en contact avec un sérum d'un patient infecté avec un rétrovirus HIV-2 :

**ENV**

```
MetMetAsnGlnLeuLeuIleAlaIleLeuLeuAlaSerAlaCys

LeuValTyrCysThrGlnTyrValThrValPheTyrGlyValPro
```

ThrTrpLysAsnAlaThrIleProLeuPheCysAlaThrArgAsn

100

ArgAspThrTrpGlyThrIleGlnCysLeuProAspAsnAspAsp

TyrGlnGluIleThrLeuAsnValThrGluAlaPheAspAlaTrp

200

AsnAsnThrValThrGluGlnAlaIleGluAspValTrpHisLeu

PheGluThrSerIleLysProCysValLysLeuThrProLeuCys

300

ValAlaMetLysCysSerSerThrGluSerSerThrGlyAsnAsn

ThrThrSerLysSerThrSerThrThrThrThrThrProThrAsp

400

GlnGluGlnGluIleSerGluAspThrProCysAlaArgAlaAsp

AsnCysSerGlyLeuGlyGluGluGluThrIleAsnCysGlnPhe

AsnMetThrGlyLeuGluArgAspLysLysLysGlnTyrAsnGlu

500

ThrTrpTyrSerLysAspValValCysGluThrAsnAsnSerThr

AsnGlnThrGlnCysTyrMetAsnHisCysAsnThrSerValIle

600

ThrGluSerCysAspLysHisTyrTrpAspAlaIleArgPheArg

TyrCysAlaProProGlyTyrAlaLeuLeuArgCysAsnAspThr

700

AsnTyrSerGlyPheAlaProAsnCysSerLysValValAlaSer

ThrCysThrArgMetMetGluThrGlnThrSerThrTrpPheGly

800

PheAsnGlyThrArgAlaGluAsnArgThrTyrIleTyrTrpHis

GlyArgAspAsnArgThrIleIleSerLeuAsnLysTyrTyrAsn

900

LeuSerLeuHisCysLysArgProGlyAsnLysThrValLysGln

IleMetLeuMetSerGlyHisValPheHisSerHisTyrGlnPro

IleAsnLysArgProArgGlnAlaTrpCysTrpPheLysGlyLys

1000

TrpLysAspAlaMetGlnGluValLysThrLeuAlaLysHisPro

ArgTyrArgGlyThrAsnAspThrArgAsnIleSerPheAlaAla

1100
ProGlyLysGlySerAspProGluValAlaTyrMetTrpThrAsn

CysArgGlyGluPheLeuTyrCysAsnMetThrTrpPheLeuAsn

1200
TrpIleGluAsnLysThrHisArgAsnTyrAlaProCysHisIle

LysGlnIleIleAsnThrTrpHisLysValGlyArgAsnValTyr

1300
LeuProProArgGluGlyGluLeuSerCysAsnSerThrValThr

SerIleIleAlaAsnIleAspTrpGlnAsnAsnAsnGlnThrAsn

IleThrPheSerAlaGluValAlaGluLeuTyrArgLeuGluLeu

1400
GlyAspTyrLysLeuValGluIleThrProIleGlyPheAlaPro

ThrLysGluLysArgTyrSerSerAlaHisGlyArgHisThrArg

1500
GlyValPheValLeuGlyPheLeuGlyPheLeuAlaThrAlaGly

SerAlaMetGlyAlaArgAlaSerLeuThrValSerAlaGlnSer

1600
ArgThrLeuLeuAlaGlyIleValGlnGlnGlnGlnGlnLeuLeu

AspValValLysArgGlnGlnGluLeuLeuArgLeuThrValTrp

1700
GlyThrLysAsnLeuGlnAlaArgValThrAlaIleGluLysTyr

LeuGlnAspGlnAlaArgLeuAsnSerTrpGlyCysAlaPheArg

1800
GlnValCysHisThrThrValProTrpValAsnAspSerLeuAla

ProAspTrpAspAsnMetThrTrpGlnGluTrpGluLysGlnVal

ArgTyrLeuGluAlaAsnIleSerLysSerLeuGluGlnAlaGln

1900
IleGlnGlnGluLysAsnMetTyrGluLeuGlnLysLeuAsnSer

```
TrpAspIlePheGlyAsnTrpPheAspLeuThrSerTrpValLys
                      2000
TyrIleGlnTyrGlyValLeuIleIleValAlaValIleAlaLeu

ArgIleValIleTyrValValGlnMetLeuSerArgLeuArgLys
                      2100
GlyTyrArgProValPheSerSerProProGlyTyrIleGlnGln

IleHisIleHisLysAspArgGlyGlnProAlaAsnGluGluThr
                           2200
GluGluAspGlyGlySerAsnGlyGlyAspArgTyrTrpProTrp

ProIleAlaTyrIleHisPheLeuIleArgGlnLeuIleArgLeu

LeuThrArgLeuTyrSerIleCysArgAspLeuLeuSerArgSer
2300
PheLeuThrLeuGlnLeuIleTyrGlnAsnLeuArgAspTrpLeu

ArgLeuArgThrAlaPheLeuGlnTyrGlyCysGluTrpIleGln
              2400
GluAlaPheGlnAlaAlaAlaArgAlaThrArgGluThrLeuAla

GlyAlaCysArgGlyLeuTrpArgValLeuGluArgIleGlyArg
                    2500
GlyIleLeuAlaValProArgArgIleArgGlnGlyAlaGluIle

AlaLeuLeu
```

54. Acide nucléique recombinant comprenant un ADNc ou une partie de cet ADNc selon l'une quelconque des revendications 46 à 53, et/ou un ARN ou une partie d'un ARN correspondant à cet ADNc, inséré dans un acide nucléique provenant d'un vecteur.

55. Acide nucléique recombinant selon la revendication 52, caractérisé en ce qu'il est marqué.

56. Procédé de détection d'un rétrovirus susceptible d'avoir un effet cytopathogène pour l'homme du type HIV-2 ou de son ARN dans un liquide ou tissu biologique, notamment en vue du diagnostic in vitro chez l'homme de la potentialité ou de l'existence d'un SLA ou d'un SIDA, caractérisé par la mise en contact des acides nucléiques contenus dans ce liquide ou tissu biologique avec une sonde contenant un acide nucléique selon l'une quelconque des revendications 49 à 53 dans des conditions d'hybridation stringentes, ou contenant un ADN présentant une homologie de séquence au moins égale à 70 %, de préférence au moins égale à 90 % avec l'ARN d'un rétrovirus selon l'une quelconque des revendications 1 à 9 et capable de s'hybrider avec celui-ci, dans des conditions non stringentes, dans la technique du « spot blot » la mise en contact ci-dessus étant réalisée pendant le temps nécessaire à la réalisation de cette hybridation et étant suivie du lavage de l'hybride formé, avec une solution assurant la conservation de ces conditions stringentes, et la détection de l'hybride formé.

57. Procédé de production d'un rétrovirus susceptible d'avoir un effet cytopathogène pour l'homme du type HIV-2, caractérisé par la culture dans des lymphocytes T4 humains ou dans des lignées cellulaires permanentes dérivées de lymphocytes T4 et portant le phénotype T4, ces lymphocytes ou ces lignées ayant préalablement été infectés avec un isolat de virus HIV-2, tel que défini dans l'une quelconque des revendications 1 à 9, et notamment lorsque le niveau d'activité de la transcriptase inverse (ou reverse-transcriptase) a atteint un seuil déterminé, par la récupération et la purification de quantités de virus libérées dans le milieu de culture de ces lymphocytes ou lignées, notamment par centrifugation différentielle dans un gradient de sucrose ou de métrizamide.

58. Procédé pour la production d'antigène spécifiques du rétrovirus HIV-2, caractérisé par la lyse, notamment à l'aide de détergents, tels que le Sodium dodécylsulfate (par exemple SDS à 0,1 % dans un tampon RIPA), du virus purifié obtenu au terme du procédé selon la revendication 57, et la récupération du lysat contenant lesdits antigènes.

59. Procédé de production d'une sonde d'hybridation pour la détection d'un ARN de rétrovirus HIV-2, caractérisé par l'insertion d'une séquence l'ADN, notamment selon l'une quelconque des revendications 46 à 53 dans un vecteur de clonage, par recombinaison in vitro, par le clonage du vecteur modifié obtenu dans un hôte cellulaire compétent et par la récupération des ADN-recombinants obtenus.

60. Les hybridomes secréteurs de l'anticorps monoclonal selon la revendication 45.

61. Application des anticorps polyclonaux selon la revendication 44, ou monoclonaux selon la revendication 45, à la détection du rétrovirus HIV-2 tel que défini dans l'une quelconque des revendications 1 à 9 dans une préparation biologique.

62. Application des anticorps polyclonaux selon la revendication 44 ou des anticorps monoclonaux selon la revendication 45 à la détection d'antigènes du rétrovirus HIV-2 tel que défini dans l'une quelconque des revendications 1 à 9 dans une préparation biologique.

63. Application des anticorps polyclonaux selon la revendication 44, ou monoclonaux selon la revendication 45, à la purification d'antigènes viraux d'un rétrovirus HIV-2 tel que défini dans l'une quelconque des revendications 1 à 9.

**Revendications** (pour les Etats contractants : AT, ES, GR)

1. Rétrovirus humain HIV-2 ayant la capacité d'infecter des lymphocytes T4 humains et susceptible de provoquer un SIDA chez l'homme, ce rétrovirus étant constitué par l'un des rétrovirus déposés à la CNCM sous les n° I-502, I-532, I-642 et I-643 et à l'ECACC sous les n° 87011001 et 87011002 (correspondant respectivement aux n° I-502 et I-532) ou un variant de l'un de ces rétrovirus comprenant tous les antigènes qui sont reconnus par les anticorps formés contre les antigènes correspondants de l'un quelconque des rétrovirus ayant fait l'objet des susdits dépôts à la CNCM ou à l'ECACC.

2. Rétrovirus humain selon la revendication 1 caractérisé en ce que son ARN génomique est susceptible de s'hybrider dans des conditions stringentes avec la chaîne complémentaire d'un cADN ou fragment de cADN dérivé de l'ARN génomique de l'un quelconque des rétrovirus déposés à la CNCM sous les n° I-502, I-532, I-642 et I-643 et à l'ECACC sous les n° 87011001 et 87011002 (correspondant respectivement aux n° I-502 et I-532).

3. Rétrovirus humain selon la revendication 1, caractérisé en ce qu'il comprend un ensemble d'antigènes formés par des protéines du noyau (core) ayant des poids moléculaires de l'ordre de 12.000, 16.000 et 26.000 daltons respectivement, des protéines ou glycoprotéines ayant des poids moléculaires respectivement de l'ordre de 36.000 daltons et de l'ordre de 42.000-45.000 daltons et une glycoprotéine majeure d'enveloppe ayant un poids moléculaire de l'ordre de 130.000-140.000 daltons.

4. Rétrovirus humain purifié selon la revendication 1, pathogène pour l'homme et susceptible de provoquer un SIDA, caractérisé par l'ensemble des propriétés suivantes :

— la cible préférentielle du rétrovirus HIV-2 est constituée par les cellules Leu3 (ou lymphocytes T4) humaines et pour des lignées cellulaires permanentes dérivées de ces lymphocytes T4 ;

— il est susceptible d'avoir un effet cytopathogène pour les lymphocytes T4 humains qu'il infecte ;

— il a une activité de transcriptase inverse nécessitant la présence d'ions $Mg^{2+}$ et présentant une forte activité pour le poly(adénylate-oligodéoxythymidylate) (poly(A)- oligo(DT) 12-18) ;

— il a une densité d'environ 1,16 dans un gradient de sucrose ;

— il a un diamètre moyen de 140 nanomètres et un noyau ayant un diamètre moyen de 41 nanomètres ;

— il peut être cultivé dans des lignées permanentes exprimant la protéine T4 ;

— il n'est pas infectieux pour les lymphocytes T8 ;

— les lysats de ce virus contiennent une protéine p26 qui ne croise pas immunologiquement avec la protéine p24 du virus HTLV-1 ou du virus HTLV-2 ;

— ces lysats contiennent en outre une protéine p16 qui ne croise pas immunologiquement avec la protéine p19 de HTLV-1 ou de HTLV-2 dans des essais de radioimmunoprécipitation ;

— ils contiennent en outre une glycoprotéine d'enveloppe ayant un poids moléculaire de l'ordre de 130.000-140.000 daltons qui ne croise pas immunologiquement avec la gp110 du rétrovirus HIV-1 ;

— ces lysats contiennent encore une protéine ou glycoprotéine, marquable par la $^{35}$S-cystéine, ayant un poids moléculaire de l'ordre de 36.000 daltons ;

— l'ARN génomique de HIV-2 n'hybride pas, dans des conditions stringentes, avec l'ARN génomique de HIV-1 et n'hybride pas, dans des conditions non stringentes, soit avec le gène env, soit avec les LTR de HIV-1 ;

— l'ARN génomique de HIV-2 hybride faiblement dans des conditions non stringentes avec les séquences de nucléotides comprenant d'une part les nucléotides 990-1070, d'autre part les nucléotides 990-1260 de la région gag du génome de HIV-1.

5. Rétrovirus selon l'une quelconque des revendications 1 à 4, caractérisé en ce qu'il ne se multiplie

50

pas de façon chronique dans les lymphocytes de singe macaque rhésus, lcrsqu'il a été injecté in vivo et dans des conditions opératoires permettant le développement du virus STLV-III$_{mac}$ telles qu'elles ont été décrites par Letvin et al., Science (1985) vol. 230, 71-75.

6. Rétrovirus selon l'une quelconque des revendications 1 à 5, caractérisé en ce que la séquence comprenant la région R et la région U3 de la séquence comprenant son ARN génomique contient une séquence nucléotidique correspondant à la séquence de nucléotides suivante :

GTGGAACGCGAGACTGAAAGCAAGAGGAATACCATTTAGTTAAAGGACAG
GAACAGCTATACTTGGTCAGGGCAGGAAGTAACTAACAGAAACAGCTGAG
ACTGCAGGGACTTTCCAGAAGGGGCTGTAACCAAGGGAGGGACATGGGAG
GAGCTGGTGGGGAACGCCTCATATTCTCTGTATAATATACCCGCTGCTTG
CATTGTACTTCAGTCGCTCTGCGGAGAGGCTGGCAGATTGAGCCCTGGAG
GATCTCTCCAGCACTAGACGGATGAGCCTGGGTGCCCTGCTAGACTCTCA
CCAGCACTTGGCCGGTGCTGGCAGACGGCCCCACGCTTGCCTGCTTAAAA
ACCTTCCTTAATAAAGCTGCAGTAGAAGCA

7. Rétrovirus selon l'une quelconque des revendications 1 à 5, caractérisé en ce que son ARN génomique contient une séquence gag correspondant à la séquence de nucléotide :

CAGRODE .

ATGGGCGCGAGAAACTCCGTCTTGAGAGGGAAAAAAGCAGATGAA

TTAGAAAGAATCAGGTTACGGCCCCGGCGGAAAGAAAAAGTACAGG

CTAAAACATATTGTGTGGGCAGCGAATAAATTGGACAGATTCGGA
100

TTAGCAGAGAGCCTGTTGGAGTCAAAAGAGGGTTGTCAAAAAATT

CTTACAGTTTTAGATCCAATGGTACCGACAGGTTCAGAAAATTTA
200

AAAAGTCTTTTTAATACTGTCTGCGTCATTTGGTGCATACACGCA

GAAGAGAAAGTGAAAGATACTGAAGCAGCAAAACAAATAGTGCGG
300

AGACATCTAGTGGCAGAAACAGGAACTGCAGAGAAAATGCCAAGC

ACAAGTAGACCAACAGCACCATCTAGCGAGAAGGGAGGAAATTAC
400

CCAGTGCAACATGTAGGCGGCAACTACACCCATATACCGCTGAGT

CCCCGAACCCTAAATGCCTGGGTAAAATTAGTAGAGGAAAAAAAG

TTCGGGGCAGAAGTAGTGCCAGGATTTCAGGCACTCTCAGAAGGC
500

TGCACGCCCTATGATATCAACCAAATGCTTAATTGTGTGGGCGAC

```
CATCAAGCAGCCATGCAGATAATCAGGGAGATTATCAATGAGGAA
            600

GCAGCAGAATGGGATGTGCAACATCCAATACCAGGCCCCTTACCA

GCCGGGCAGCTTAGAGAGCCAAGGGGATCTGACATAGCAGGGACA
            700

ACAAGCACAGTACAACAACACATCCACTGGATGTTTAGGCCACAA

AATCCTGTACCAGTAGGAAACATCTATAGAAGATGGATCCAGATA
                                        800

GGATTCCAGAAGTGTGTCAGGATGTACAACCCGACCAACATCCTA

GACATAAACAGGGACCAAAGGAGCCGTTCCAAAGCTATGTAGAT
                                          900

AGATTCTACAAAAGCTTGAGGGCAGAACAAACAGATCCAGCAGTG

AAGAATTGGATGACCCAAACACTGCTAGTACAAAATGCCAACCCA

GACTGTAAATTAGTGCTAAAAGGACTAGGGATGAACCCTACCTTA
1000

GAAGAGATGCTGACCGCCTGTCAGGGGGTAGGTCGGCCAGGCCAG

AAAGCTAGATTAATGGCAGAGGCCCTGAAAGAGGTCATAGGACCT
            1100

GCCCCTATCCCATTCGCAGCAGCCCAGCAGAGAAAGGCATTTAAA

TGCTGGAACTGTGGAAAGGAAGGGCACTCGGCAAGACAATGCCGA
                            1200

GCACCTAGAAGGCAGGGCTGCTGGAAGTGTGGTAAGCCAGGACAC

ATCATGACAAACTGCCCAGATAGACAGGCAGGTTTTTTAGGACTG
                                        1300

CGCCCTTGGGGAAAGAAGCCCCGCAACTTCCCCGTGGCCCAAGTT

CCGCAGGGGCTGACACCAACAGCACCCCCAGTGGATCCAGCAGTG

GATCTACTGGAGAAATATATGCAGCAAGGGAAAAGACAGAGAGAG
1400

CAGAGAGAGAGACCATACAAGGAAGTGACACAGGACTTACTGCAC

CTCGAGCAGGGGGAGACACCATACAGGGAGCCACCAACAGAGGAC
            1500

TTGCTGCACCTCAATTCTCTCTTTGGAAAAGACCAG
```

EP 0 239 425 B1

8. Rétrovirus selon l'une quelconque des revendications 1 à 6, caractérisé en ce que son ARN génomique contient une séquence _env_ correspondant à la séquence de nucléotides :

```
ENVRN

ATGATGAATCAGCTGCTTATTGCCATTTTATTAGCTAGTGCTTGC

TTAGTATATTGCACCCAATATGTAACTGTTTTCTATGGCGTACCC

ACGTGGAAAAATGCAACCATTCCCCTCTTTTGTGCAACCAGAAAT
            100

ACGGATACTTGGGGAACCATACAGTGCTTGCCTGACAATGATGAT

TATCAGGAAATAACTTTGAATGTAACAGAGGCTTTTGATGCATGG
            200

AATAATACAGTAACAGAACAAGCAATAGAAGATGTCTGGCATCTA

TTCGAGACATCAATAAAACCATGTGTCAAACTAACACCTTTATGT
                      300

GTAGCAATGAAATGCAGCAGCACAGAGAGCAGCACAGGGAACAAC

ACAACCTCAAAGAGCACAAGCACAACCACAACCACACCCACAGAC
                              400

CAGGAGCAAGAGATAAGTGAGGATACTCCATGCGCACGCGCAGAC

AACTGCTCAGGATTGGGAGAGGAAGAAACGATCAATTGCCAGTTC

AATATGACAGGATTAGAAAGAGATAAGAAAAAACAGTATAATGAA
500

ACATGGTACTCAAAAGATGTGGTTTGTGAGACAAATAATAGCACA

AATCAGACCCAGTGTTACATGAACCATTGCAACACATCAGTCATC
            600

ACAGAATCATGTGACAAGCACTATTGGGATGCTATAAGGTTTAGA

TACTGTGCACCACCGGGTTATGCCCTATTAAGATGTAATGATACC
                      700

AATTATTCAGGCTTTGCACCCAACTGTTCTAAAGTAGTAGCTTCT

ACATGCACCAGGATGATGGAAACGCAAACTTCCACATGGTTTGGC
                              800

TTTAATGGCACTAGAGCAGAGAATAGAACATATATCTATTGGCAT

GGCAGAGATAATAGAACTATCATCAGCTTAAACAAATATTATAAT
                              900
```

53

CTCAGTTTGCATTGTAAGAGGCCAGGGAATAAGACAGTGAAACAA

ATAATGCTTATGTCAGGACATGTGTTTCACTCCCACTACCAGCCG

ATCAATAAAAGACCCAGACAAGCATGGTGCTGGTTCAAAGGCAAA
1000

TGGAAAGACGCCATGCAGGAGGTGAAGACCCTTGCAAAACATCCC

AGGTATAGAGGAACCAATGACACAAGGAATATTAGCTTTGCAGCG
1100

CCAGGAAAAGGCTCAGACCCAGAAGTAGCATACATGTGGACTAAC

TGCAGAGGAGAGTTTCTCTACTGCAACATGACTTGGTTCCTCAAT
1200

TGGATAGAGAATAAGACACACCGCAATTATGCACCGTGCCATATA

AAGCAAATAATTAACACATGGCATAAGGTAGGGAGAAATGTATAT
1300

TTGCCTCCCAGGGAAGGGGAGCTGTCCTGCAACTCAACAGTAACC

AGCATAATTGCTAACATTGACTGGCAAAACAATAATCAGACAAAC

ATTACCTTTAGTGCAGAGGTGGCAGAACTATACAGATTGGAGTTG
1400

GGAGATTATAAATTGGTAGAAATAACACCAATTGGCTTCGCACCT

ACAAAAGAAAAAAGATACTCCTCTGCTCACGGGAGACATACAAGA
1500

GGTGTGTTCGTGCTAGGGTTCTTGGGTTTTCTCGCAACAGCAGGT

TCTGCAATGGGCGCTCGAGCGTCCCTGACCGTGTCGGCTCAGTCC
1600

CGGACTTTACTGGCCGGGATAGTGCAGCAACAGCAACAGCTGTTG

GACGTGGTCAAGAGACAACAAGAACTGTTGCGACTGACCGTCTGG
1700

GGAACGAAAAACCTCCAGGCAAGAGTCACTGCTATAGAGAAGTAC

CTACAGGACCAGGCGCGGCTAAATTCATGGGGATGTGCGTTTAGA
1800

CAAGTCTGCCACACTACTGTACCATGGGTTAATGATTCCTTAGCA

CCTGACTGGGACAATATGACGTGGCAGGAATGGGAAAAACAAGTC

CGCTACCTGGAGGCAAATATCAGTAAAAGTTTAGAACAGGCACAA
1900

ATTCAGCAAGAGAAAAATATGTATGAACTACAAAAATTAAATAGC

TGGGATATTTTTGGCAATTGGTTTGACTTAACCTCCTGGGTCAAG
2000

TATATTCAATATGGAGTGCTTATAATAGTAGCAGTAATAGCTTTA

AGAATAGTGATATATGTAGTACAAATGTTAAGTAGGCTTAGAAAG
2100

GGCTATAGGCCTGTTTTCTCTTCCCCCCCCGGTTATATCCAACAG

ATCCATATCCACAAGGACCGGGGACAGCCAGCCAACGAAGAAACA
2200

GAAGAAGACGGTGGAAGCAACGGTGGAGACAGATACTGGCCCTGG

CCGATAGCATATATACATTTCCTGATCCGCCAGCTGATTCGCCTC

TTGACCAGACTATACAGCATCTGCAGGGACTTACTATCCAGGAGC
2300

TTCCTGAGGGTCCAACTCATCTACCAGAATCTCAGAGACTGGCTG

AGACTTAGAACAGCCTTCTTGCAATATCGGTGCGAGTGGATCCAA
2400

GAAGCATTCCAGGCCGCCGCGAGGGCTACAAGAGAGACTCTTGCG

GGCGCGTGCAGGGGCTTGTGGAGGGTATTGGAACGAATCGGGAGG
2500

GGAATACTCGCGGTTCCAAGAAGGATCAGACAGGGAGCAGAAATC

GCCCTCCTG

9. Rétrovirus selon l'une quelconque des revendications 1 à 8, caractérisé en ce que son ARN n'hybride pas dans des conditions stringentes avec l'ARN génomique de HIV-1 et n'hybride pas, dans des conditions non stringentes, avec le gène env et le LTR qui le jouxte, plus particulièrement avec la séquence de nucléotides 5290-9130, de HIV-1, et n'hybride pas, dans des conditions non stringentes, avec des séquences de la région pol du génome de HIV-1, en particulier avec la séquence de nucléotides 2170-2240.

10. Procédé pour la production d'antigènes spécifiques d'un rétrovirus HIV-2, caractérisé par :
— la lyse, notamment à l'aide de détergents, tels que le Sodium dodécylsulfate (par exemple SDS à 0,1 % dans un tampon RIPA), du rétrovirus HIV-2 selon l'une quelconque des revendications 1 à 9,
— la récupération du lysat contenant lesdits antigènes.

11. Procédé selon la revendication 10, comprenant en outre :
— la séparation à partir du lysat et la purification de l'un des antigènes consistant en l'une des protéines ou glycoprotéines p12, p16, p26, gp36, p42, gp140.

12. Procédé selon la revendication 11, caractérisé en ce que l'on sépare et purifie l'antigène ayant les caractéristiques immunologiques de la protéine p12 de HIV-2, reconnu immunologiquement par des anticorps formés contre un rétrovirus selon l'une quelconque des revendications 1 à 9, cet antigène ayant un poids moléculaire de l'ordre de 12.000 daltons.

13. Procédé selon la revendication 11, caractérisé en ce que l'on sépare et purifie l'antigène ayant les caractéristiques immunologiques de la protéine p16 de HIV-2, reconnu immunologiquement par des anticorps formés contre un rétrovirus selon l'une quelconque des revendications 1 à 9 cet antigène ayant un poids moléculaire de l'ordre de 16.000 daltons.

14. Procédé selon la revendication 11, caractérisé en ce que l'on sépare et purifie l'antigène ayant les caractéristiques immunologiques de la protéine p26 de HIV-2, reconnu immunologiquement par des anticorps formés contre un rétrovirus selon l'une quelconque des revendications 1 à 9, cet antigène ayant un poids moléculaire de l'ordre de 26.000 daltons.

15. Procédé selon la revendication 11, caractérisé en ce que l'on sépare et purifie l'antigène ayant les caractéristiques immunologiques de la glycoprotéine gp36 de HIV-2, reconnu immunologiquement par des anticorps formés contre un rétrovirus selon l'une quelconque des revendications 1 à 9, et antigène ayant un poids moléculaire de l'ordre de 36.000 daltons.

16. Procédé selon la revendication 11, caractérisé en ce que l'on sépare et purifie l'antigène ayant les caractéristiques immunologiques de la protéine p42 de HIV-2, reconnu immunologiquement par des anticorps formés contre un rétrovirus selon l'une quelconque des revendications 1 à 9, cet antigène ayant un poids moléculaire de l'ordre de 42.000-45.000 daltons.

17. Procédé selon la revendication 11, caractérisé en ce que l'on sépare et purifie l'antigène ayant les caractéristiques immunologiques de la glygoprotéine gp140 de HIV-2, reconnu immunologiquement par des anticorps formés contre un rétrovirus selon l'une quelconque des revendications 1 à 9, cet antigène ayant un poids moléculaire de l'ordre de 130.000-140.000 daltons.

18. Procédé selon la revendication 11, caractérisé en ce que l'on récupère et l'on purifie l'antigène ayant la séquence d'aminoacides ci-après ou partie de cette séquence, dès lors qu'elle donne lieu à une réaction immunologique spécifique avec des anticorps contre un rétrovirus HIV-2 selon l'une quelconque des revendications 1 à 9, notamment lorsque cet antigène est mis en contact avec un sérum d'un patient infecté avec HIV-2 :

ArgLysAlaPheLys

CysTrpAsnCysGlyLysGluGlyHisSerAlaArgGlnCysArg

1200

AlaProArgArgGlnGlyCysTrpLysCysGlyLysProGlyHis

IleMetThrAsnCysProAspArgGlnAlaGlyPheLeuGlyLeu

1300

GlyProTrpGlyLysLysProArgAsnPheProValAlaGlnVal

ProGlnGlyLeuThrProThrAlaProProValAspProAlaVal

AspLeuLeuGluLysTyrMetGlnGlnGlyLysArgGlnArgGln

1400

GlnArgGlnArgProTyrLysGluValThrGluAspLeuLeuHis

LeuGluGlnGlyGluThrProTyrArgGluProProThrGluAsp

1500

LeuLeuHisLeuAsnSerLeuPheGlyLysAspGln

19. Procédé selon la revendication 11, caractérisé en ce que l'on récupère et l'on purifie l'antigène ayant la séquence d'aminoacides ci-après ou une partie de cette séquence, dès lors qu'elle donne lieu à une réaction immunologique spécifique avec des anticorps contre un rétrovirus HIV-2 selon l'une quelconque des revendications 1 à 9, notamment lorsque cet antigène est mis en contact avec un sérum d'un patient infecté avec HIV-2 :

MetGlyAlaArgAsnSerValLeuArgGlyLysLysAlaAspGlu

LeuGluArgIleArgLeuArgProGlyGlyLysLysLysTyrArg

LeuLysHisIleValTrpAlaAlaAsnLysLeuAspArgPheGly

100
LeuAlaGluSerLeuLeuGluSerLysGluGlyCysGlnLysIle

LeuThrValLeuAspProMetValProThrGlySerGluAsnLeu

200
LysSerLeuPheAsnThrValCysValIleTrpCysIleHisAla

GluGlnLysValLysAspThrGluGlyAlaLysGlnIleValArg

300
ArgHisLeuValAlaGluThrGlyThrAlaGluLysMetProSer

ThrSerArgProThrAlaProSerSerGluLysGlyGlyAsnTyr

400

20. Procédé selon la revendication 11, caractérisé en ce que l'on récupère et l'on purifie l'antigène ayant la séquence d'aminoacides ci-après ou une partie de cette séquence, dès lors qu'elle donne lieu à une réaction immunologique spécifique avec des anticorps contre un rétrovirus HIV-2 selon l'une quelconque des revendications 1 à 9, notamment lorsque cet antigène est mis en contact avec un sérum d'un patient infecté avec HIV-2 :

ProValGlnHisValGlyGlyAsnTyrThrHisIleProLeuSer

ProArgThrLeuAsnAlaTrpValLysLeuValGluGluLysLys

PheGlyAlaGluValValProGlyPheGlnAlaLeuSerGluGly

500
CysThrProTyrAspIleAsnGlnMetLeuAsnCysValGlyAsp

HisGlnAlaAlaMetGlnIleIleArgGluIleIleAsnGluGlu

600
AlaAlaGluTrpAspValGlnHisProIleProGlyProLeuPro

AlaGlyGlnLeuArgGluProArgGlySerAspIleAlaGlyThr

700
ThrSerThrValGluGluGlnIleGlnTrpMetPheArgProGln

AsnProValProValGlyAsnIleTyrArgArgTrpIleGlnIle

800

57

GlyLeuGlnLysCysValArgMetTyrAsnProThrAsnIleLeu

AspIleLysGlnGlyProLysGluProPheGlnSerTyrValAsp

900
ArgPheTyrLysSerLeuArgAlaGluGlnThrAspProAlaVal

LysAsnTrpMetThrGlnThrLeuLeuValGlnAsnAlaAsnPro

AspCysLysLeuValLeuLysGlyLeuGlyMetAsnProThrLeu

1000
GluGluMetLeuThrAlaCysGlnGlyValGlyGlyProGlyGln

LysAlaArgLeuMetAlaGluAlaLeuLysGluValIleGlyPro

1100
AlaProIleProPheAlaAlaAlaGlnGln

21. Procédé selon la revendication 11, caractérisé en ce que l'on récupère et l'on purifie l'antigène ayant la séquence d'aminoacides ci-après, ou partie de cette séquence, dès lors qu'elle donne lieu à une réaction immunologique spécifique avec des anticorps contre un rétrovirus HIV-2 selon l'une quelconque des revendications 1 à 9, notamment lorsque cet antigène est mis en contact avec un sérum d'un patient infecté avec HIV-2 :

ENVRE

MetMetAsnGlnLeuLeuIleAlaIleLeuLeuAlaSerAlaCys

LeuValTyrCysThrGlnTyrValThrValPheTyrGlyValPro

ThrTrpLysAsnAlaThrIleProLeuPheCysAlaThrArgAsn

100
ArgAspThrTrpGlyThrIleGlnCysLeuProAspAsnAspAsp

TyrGlnGluIleThrLeuAsnValThrGluAlaPheAspAlaTrp

200
AsnAsnThrValThrGluGlnAlaIleGluAspValTrpHisLeu

PheGluThrSerIleLysProCysValLysLeuThrProLeuCys

300
ValAlaMetLysCysSerSerThrGluSerSerThrGlyAsnAsn

ThrThrSerLysSerThrSerThrThrThrThrThrProThrAsp

400
GlnGluGlnGluIleSerGluAspThrProCysAlaArgAlaAsp

58

AsnCysSerGlyLeuGlyGluGluGluThrIleAsnCysGlnPhe

AsnMetThrGlyLeuGluArgAspLysLysLysGlnTyrAsnGlu

500
ThrTrpTyrSerLysAspValValCysGluThrAsnAsnSerThr

AsnGlnThrGlnCysTyrMetAsnHisCysAsnThrSerValIle

600
ThrGluSerCysAspLysHisTyrTrpAspAlaIleArgPheArg

TyrCysAlaProProGlyTyrAlaLeuLeuArgCysAsnAspThr

700
AsnTyrSerGlyPheAlaProAsnCysSerLysValValAlaSer

ThrCysThrArgMetMetGluThrGlnThrSerThrTrpPheGly

800
PheAsnGlyThrArgAlaGluAsnArgThrTyrIleTyrTrpHis

GlyArgAspAsnArgThrIleIleSerLeuAsnLysTyrTyrAsn

900
LeuSerLeuHisCysLysArgProGlyAsnLysThrValLysGln

IleMetLeuMetSerGlyHisValPheHisSerHisTyrGlnPro

IleAsnLysArgProArgGlnAlaTrpCysTrpPheLysGlyLys

1000
TrpLysAspAlaMetGlnGluValLysThrLeuAlaLysHisPro

ArgTyrArgGlyThrAsnAspThrArgAsnIleSerPheAlaAla

1100
ProGlyLysGlySerAspProGluValAlaTyrMetTrpThrAsn

CysArgGlyGluPheLeuTyrCysAsnMetThrTrpPheLeuAsn

1200
TrpIleGluAsnLysThrHisArgAsnTyrAlaProCysHisIle

LysGlnIleIleAsnThrTrpHisLysValGlyArgAsnValTyr

1300
LeuProProArgGluGlyGluLeuSerCysAsnSerThrValThr

SerIleIleAlaAsnIleAspTrpGlnAsnAsnAsnGlnThrAsn

IleThrPheSerAlaGluValAlaGluLeuTyrArgLeuGluLeu

1400
GlyAspTyrLysLeuValGluIleThrProIleGlyPheAlaPro

59

ThrLysGluLysArgTyrSerSerAlaHisGlyArgHisThrArg

GlyValPheValLeuGlyPheLeuGlyPheLeuAlaThrAlaGly

SerAlaMetGlyAlaArgAlaSerLeuThrValSerAlaGlnSer

ArgThrLeuLeuAlaGlyIleValGlnGlnGlnGlnGlnLeuLeu

AspValValLysArgGlnGlnGluLeuLeuArgLeuThrValTrp

GlyThrLysAsnLeuGlnAlaArgValThrAlaIleGluLysTyr

LeuGlnAspGlnAlaArgLeuAsnSerTrpGlyCysAlaPheArg

GlnValCysHisThrThrValProTrpValAsnAspSerLeuAla

ProAspTrpAspAsnMetThrTrpGlnGluTrpGluLysGlnVal

ArgTyrLeuGluAlaAsnIleSerLysSerLeuGluGlnAlaGln

IleGlnGlnGluLysAsnMetTyrGluLeuGlnLysLeuAsnSer

TrpAspIlePheGlyAsnTrpPheAspLeuThrSerTrpValLys

TyrIleGlnTyrGlyValLeuIleIleValAlaValIleAlaLeu

ArgIleValIleTyrValValGlnMetLeuSerArgLeuArgLys

GlyTyrArgProValPheSerSerProProGlyTyrIleGlnGln

IleHisIleHisLysAspArgGlyGlnProAlaAsnGluGluThr

GluGlnAspGlyGlySerAsnGlyGlyAspArgTyrTrpProTrp

ProIleAlaTyrIleHisPheLeuIleArgGlnLeuIleArgLeu

LeuThrArgLeuTyrSerIleCysArgAspLeuLeuSerArgSer

PheLeuThrLeuGlnLeuIleTyrGlnAsnLeuArgAspTrpLeu

ArgLeuArgThrAlaPheLeuGlnTyrGlyCysGluTrpIleGln

EP 0 239 425 B1

GluAlaPheGlnAlaAlaAlaArgAlaThrArgGluThrLeuAla

GlyAlaCysArgGlyLeuTrpArgValLeuGluArgIleGlyArg

2500

GlyIleLeuAlaValProArgArgIleArgGlnGlyAlaGluIle

AlaLeuLeu

22. Méthode pour la détection in vitro de la présence d'anticorps induits chez l'homme infecté par un rétrovirus humain HIV-2 dans un échantillon biologique humain, tel qu'un sérum et, plus particulièrement pour le diagnostic in vitro d'un SLA ou SIDA potentiel ou existant dû à un tel rétrovirus et provenant de la personne faisant l'objet du diagnostic, caractérisée en ce que l'on met en contact cet échantillon biologique avec un antigène reconnu par un anticorps induit chez l'homme infecté par un rétrovirus humain HIV-2 tel que défini dans l'une quelconque des revendications 1 à 9, dans des conditions autorisant la formation d'un composé immunologique entre cet antigène et ledit anticorps et en ce que l'on détecte le conjugué immunologique éventuellement formé entre cet anticorps et l'antigène mis en œuvre.

23. Méthode selon la revendication 22, caractérisée en ce que l'échantillon biologique est mis en contact avec un antigène obtenu par le procédé selon l'une quelconque des revendications 12 à 21 ou un mélange de ces antigènes, dans des conditions autorisant la formation d'un composé immunologique entre cet antigène et lesdits anticorps et en ce que l'on détecte le conjugué immunologique éventuellement formé entre ces anticorps anti-HIV-2 et l'antigène mis en œuvre.

24. Méthode selon la revendication 23, caractérisée en ce que l'on réalise la détection dudit conjugué immunologique en faisant réagir le conjugué immunologique éventuellement formé avec un réactif marqué formé soit par des anticorps anti-immunoglobulines humaines, soit par une protéine A bactérienne et en ce que l'on détecte le complexe formé entre le réactif et le susdit conjugué immunologique.

25. Méthode pour la détection in vitro de la présence d'anticorps reconnaissant des antigènes d'un rétrovirus humain susceptible d'avoir un effet cytopathogène pour les lymphocytes T4 humains, dans un échantillon biologique humain, tel qu'un sérum, et plus particulièrement pour le diagnostic in vitro d'un SLA ou SIDA potentiel ou existant dû à un rétrovirus humain caractérisée en ce que l'on met en contact cet échantillon biologique provenant de la personne faisant l'objet du diagnostic avec un mélange d'antigènes capables de donner une réaction immunologique spécifique avec les anticorps contre d'une part un rétrovirus humain HIV-2 susceptible d'avoir un effet cytopathogène tel que défini dans l'une quelconque des revendications 1 à 9, d'autre part un rétrovirus humain HIV-1 et, en ce que l'on détecte le conjugué immunologique éventuellement formé entre l'un au moins de ces antigènes et lesdits anticorps.

26. Méthode selon la revendication 25, caractérisée en ce que l'échantillon biologique provenant de la personne faisant l'objet du diagnostic est mis en contact avec un mélange d'antigènes issus à la fois de HIV-1 et d'un HIV-2 selon l'une quelconque des revendications 1 à 9, notamment avec des antigènes obtenus par le procédé selon l'une quelconque des revendications 11 à 21 et des antigènes de HIV-1 obtenu par le même procédé, si ce n'est qu'il est appliqué à un rétrovirus HIV-1, et en ce que l'on détecte le conjugué immunologique éventuellement formé entre l'un au moins de ces antigènes et lesdits anticorps.

27. Procédé pour l'obtention d'anticorps reconnaissant spécifiquement un antigène un rétrovirus HIV-2, comprenant :
— l'immunisation d'un animal avec un rétrovirus HIV-2, selon l'une quelconque des revendications 1 à 9,
— la récupération des anticorps formés.

28. Procédé de production d'un acide nucléique ou d'un fragment d'acide nucléique dérivé de l'ARN d'un rétrovirus HIV-2 selon l'une quelconque des revendications 1 à 9 comprenant :
— la ultracentrifugation du surnageant d'une culture de cellules infectées par un isolat du rétrovirus HIV-2 selon l'une quelconque des revendications 1 à 9, et récupération du culot de centrifugation,
— le cas échéant la centrifugation du culot récupéré sur un gradient de sucrose par la méthode décrite dans EP-A-0138667 et la récupération du culot sédimenté contenant l'ARN génomique,
— la synthèse d'un brin d'ADNc initiateur au contact de l'ARN du virus purifié sédimenté, en présence d'un initiateur oligo (dT) lors d'une réaction endogène activée par un détergent, selon la technique décrite dans Nature 312 : 757-760 (1984),
— le cas échéant la purification des hybrides ARN-ADNc par extraction par un mélange phénolchloroforme et précipitation à l'éthanol,
— la fabrication d'un second brin d'ADNc en présence d'ADN polymérase I, de RNaseH et des

61

4 désoxynucléotides selon la méthode décrite dans Gene, 25 : 263-269 (1983), si besoin après extraction des protéines présentes dans le milieu de réaction,

— la récupération de l'acide nucléique ainsi obtenu.

29. Procédé selon la revendication 28, caractérisé en ce que l'on récupère un acide nucléique, le cas échéant marqué, dérivé d'une partie au moins de l'ARN du virus HIV-2 tel que défini dans l'une quelconque des revendications 1 à 9, qui hybride avec l'ARN dudit virus HIV-2 dans les conditions stringentes, et qui n'hybride pas, dans ces conditions stringentes, avec les sondes 1 à 4 comprenant respectivement les nucléotides 990-1070, 990-1260, 2170-2240, 3370-3640 de l'ADN dérivé du génome de HIV-1.

30. Procédé selon la revendication 29, caractérisé en ce que l'on récupère un acide nucléique contenant une partie au moins d'une séquence d'ADNc capable de s'hybrider avec l'ARN génomique entier d'un rétrovirus HIV-2 selon une quelconque des revendications 1 à 9, et ne s'hybridant pas dans les conditions stringentes avec les sondes dont les fragments d'ADN dérivés du génome de HIV-1 respectivement constitués de fragments délimités par les nucléotides 990-1070, 990-1260, 2170-2240, 3370-3640 de l'ADN dérivé du génome de HIV-1.

31. Procédé selon la revendication 29, caractérisé en ce que l'on récupère un acide nucléique contenant la séquence de nucléotides :

GTGGAAGGCGAGACTGAAAGCAAGAGGAATACCATTTAGTTAAAGGACAG

GAACAGCTATACTTGGTCAGGGCAGGAAGTAACTAACAGAAACAGCTGAG

ACTGCAGGGACTTTCCAGAAGGGGCTGTAACCAAGGGAGGGACATGGGAG

GAGCTGGTGGGGAACGCCTCATATTCTCTGTATAATATACCCGCTGCTTG

CATTGTACTTCAGTCGCTCTGCGGAGAGGCTGGCAGATTGAGCCCTGGAG

GATCTCTCCAGCACTAGACGGATGAGCCTGGGTGCCCTGCTAGACTCTCA

CCAGCACTTGGCCGGTGCTGGCAGACGGCCCCACGCTTGCCTGCTTAAAA

ACCTTCCTTAATAAAGCTGCAGTAGAAGCA

32. Procédé selon la revendication 29, caractérisé en ce que l'on récupère un acide nucléique contenant une séquence nucléotidique codant pour tout ou partie de la séquence d'aminoacides indiquée ci-après, cette séquence ou partie de séquence d'aminoacides ayant la capacité de donner une réaction immunologique spécifique avec des anticorps contre un rétrovirus HIV-2 tel que défini dans l'une quelconque des revendications 1 à 9, lorsque cette séquence d'aminoacides est mise en contact avec un sérum d'un patient infecté avec un rétrovirus HIV-2 :

GAGRODH                          :

MetGlyAlaArgAsnSerValLeuArgGlyLysLysAlaAspGlu

LeuGluArgIleArgLeuArgProGlyGlyLysLysLysTyrArg

LeuLysHisIleValTrpAlaAlaAsnLysLeuAspArgPheGly

100
LeuAlaGlySerLeuLeuGluSerLysGluGlyCysGlnLysIle

LeuThrValLeuAspProMetValProThrGlySerGluAsnLeu

200
LysSerLeuPheAsnThrValCysValIleTrpCysIleHisAla

GluGluLysValLysAspThrGluGlyAlaLysGlnIleValArg

300

ArgHisLeuValAlaGluThrGlyThrAlaGluLysMetProSer

ThrSerArgProThrAlaProSerSerGluLysGlyGlyAsnTyr

400

ProValGlnHisValGlyGlyAsnTyrThrHisIleProLeuSer

ProArgThrLeuAsnAlaTrpValLysLeuValGluGluLysLys

PheGlyAlaGluValValProGlyPheGlnAlaLeuSerGluGly

500

CysThrProTyrAspIleAsnGlnMetLeuAsnCysValGlyAsp

HisGlnAlaAlaMetGlnIleIleArgGluIleIleAsnGluGln

600

AlaAlaGlyTrpAspValGlnHisProIleProGlyProLeuPro

AlaGlyGlyLeuArgGluProArgGlySerAspIleAlaGlyThr

700

ThrSerThrValGluGluGlnIleGlnTrpMetPheArgProGln

AsnProValProValGlyAsnIleTyrArgArgTrpIleGlnIle

800

GlyLeuGlnLysCysValArgMetTyrAsnProThrAsnIleLeu

AspIleLysGlnGlyProLysGluProPheGlnSerTyrValAsp

900

ArgPheTyrLysSerLeuArgAlaGluGlnThrAspProAlaVal

LysAsnTrpMetThrGlnThrLeuLeuValGlnAsnAlaAsnPro

AspCysLysLeuValLeuLysGlyLeuGlyMetAsnProThrLeu

1000

GluGluMetLeuThrAlaCysGlnGlyValGlyGlyProGlyGln

LysAlaArgLeuMetAlaGluAlaLeuLysGluValIleGlyPro

1100

AlaProIleProPheAlaAlaAlaGlnGlnArgLysAlaPheLys

CysTrpAsnCysGlyLysGluGlyHisSerAlaArgGlnCysArg

1200

AlaProArgArgGlnGlyCysTrpLysCysGlyLysProGlyHis

IleMetThrAsnCysProAspArgGlnAlaGlyPheLeuGlyLeu

1300

63

GlyProTrpGlyLysLysProArgAsnPheProValAlaGlnVal

ProGlnGlyLeuThrProThrAlaProProValAspProAlaVal

AspLeuLeuGluLysTyrMetGlnGlnGlyLysArgGlnArgGlu

1400
GlnArgGluArgProTyrLysGluValThrGluAspLeuLeuHis

LeuGluGlnGlyGluThrProTyrArgGluProProThrGluAsp

1500
LeuLeuHisLeuAsnSerLeuPheGlyLysAspGln

33. Procédé selon la revendication 29, caractérisé en ce que l'on récupère un acide nucléique contenant une séquence nucléotidique codant pour une partie au moins de la séquence d'aminoacides indiquée ci-après, cette séquence d'aminoacides étant capable de donner une réaction immunologique spécifique avec des anticorps contre un rétrovirus HIV-2 tel que défini dans l'une quelconque des revendications 1 à 9, lorsque cette séquence d'aminoacides est mise en contact avec un sérum d'un patient infecté avec un rétrovirus HIV-2 :

ArgLysAlaPheLys

CysTrpAsnCysGlyLysGluGlyHisSerAlaArgGlnCysArg

1200
AlaProArgArgGlnGlyCysTrpLysCysGlyLysProGlyHis

IleMetThrAsnCysProAspArgGlnAlaGlyPheLeuGlyLeu

1300
GlyProTrpGlyLysLysProArgAsnPheProValAlaGlnVal

ProGlnGlyLeuThrProThrAlaProProValAspProAlaVal

AspLeuLeuGluLysTyrMetGlnGlnGlyLysArgGlnArgGlu

1400
GlnArgGluArgProTyrLysGluValThrGluAspLeuLeuHis

LeuGluGlnGlyGluThrProTyrArgGlnProProThrGluAsp

1500
LeuLeuHisLeuAsnSerLeuPheGlyLysAspGln

34. Procédé selon la revendication 29, caractérisé en ce que l'on récupère un acide nucléique contenant une séquence nucléotidique codant pour tout ou partie de la séquence d'aminoacides indiquée ci-après, la séquence codée d'aminoacides ayant la capacité de donner une réaction immunologique spécifique avec des anticorps contre un rétrovirus HIV-2 tel que défini dans l'une quelconque des revendications 1 à 9, lorsqu'elle est mise en contact avec un sérum d'un patient infecté avec un rétrovirus HIV-2 :

MetGlyAlaArgAsnSerValLeuArgGlyLysLysAlaAspGlu

LeuGluArgIleArgLeuArgProGlyGlyLysLysLysTyrArg

LeuLysHisIleValTrpAlaAlaAsnLysLeuAspArgPheGly

100
LeuAlaGluSerLeuLeuGluSerLysGlnGlyCysGlnLysIle

LeuThrValLeuAspProMetValProThrGlySerGluAsnLeu

200
LysSerLeuPheAsnThrValCysValIleTrpCysIleHisAla

GluGluLysValLysAspThrGluGlyAlaLysGlnIleValArg

300
ArgHisLeuValAlaGluThrGlyThrAlaGluLysMetProSer

ThrSerArgProThrAlaProSerSerGluLysGlyGlyAsnTyr

400

35. Procédé selon la revendication 29, caractérisé en ce que l'on récupère un acide nucléique contenant une séquence nucléotidique codant pour tout ou partie de la séquence d'aminoacides indiquée ci-après, la séquence codée d'aminoacides ayant la capacité de donner une réaction immunologique spécifique avec des anticorps contre un rétrovirus HIV-2 tel que défini dans l'une quelconque des revendications 1 à 9, lorsqu'elle est mise en contact avec un sérum d'un patient infecté spécifiquement avec un rétrovirus HIV-2 :

ProValGlnHisValGlyGlyAsnTyrThrHisIleProLeuSer

ProArgThrLeuAsnAlaTrpValLysLeuValGluGluLysLys

PheGlyAlaGluValValProGlyPheGlnAlaLeuSerGluGly

500
CysThrProTyrAspIleAsnGlnMetLeuAsnCysValGlyAsp

HisGlnAlaAlaMetGlnIleIleArgGlnIleIleAsnGluGln

600
AlaAlaGluTrpAspValGlnHisProIleProGlyProLeuPro

AlaGlyGlnLeuArgGluProArgGlySerAspIleAlaGlyThr

700
ThrSerThrValGluGluGlnIleGlnTrpMetPheArgProGln

AsnProValProValGlyAsnIleTyrArgArgTrpIleGlnIle

800

```
GlyLeuGlnLysCysValArgMetTyrAsnProThrAsnIleLeu

AspIleLysGlnGlyProLysGluProPheGlnSerTyrValAsp
                                                    900
ArgPheTyrLysSerLeuArgAlaGluGlnThrAspProAlaVal

LysAsnTrpMetThrGlnThrLeuLeuValGlnAsnAlaAsnPro

AspCysLysLeuValLeuLysGlyLeuGlyMetAsnProThrLeu
        1000
GluGlnMetLeuThrAlaCysGlnGlyValGlyGlyProGlyGln

LysAlaArgLeuMetAlaGluAlaLeuLysGluValIleGlyPro
                1100
AlaProIleProPheAlaAlaAlaGlnGln
```

36. Procédé selon la revendication 29, caractérisé en ce que l'on récupère un acide nucléique contenant une séquence nucléotidique codant pour tout ou partie de la séquence d'aminoacides indiquée ci-après, la séquence codée d'acide aminée ayant la capacité de donner une réaction immunologique spécifique avec des anticorps contre un rétrovirus HIV-2 tel que défini dans l'une quelconque des revendications 1 à 9, lorsqu'elle est mise en contact avec un sérum d'un patient infecté avec un rétrovirus HIV-2 :

```
ENVII

MetMetAsnGlnLeuLeuIleAlaIleLeuLeuAlaSerAlaCys

LeuValTyrCysThrGlnTyrValThrValPheTyrGlyValPro

ThrTrpLysAsnAlaThrIleProLeuPheCysAlaThrArgAsn
        100
ArgAspThrTrpGlyThrIleGlnCysLeuProAspAsnAspAsp

TyrGlnGluIleThrLeuAsnValThrGluAlaPheAspAlaTrp
                200
AsnAsnThrValThrGluGlnAlaIleGluAspValTrpHisLeu

PheGluThrSerIleLysProCysValLysLeuThrProLeuCys
                300
ValAlaMetLysCysSerSerThrGluSerSerThrGlyAsnAsn

ThrThrSerLysSerThrSerThrThrThrThrThrProThrAsp
                        400
```

GlnGluGlnGluIleSerGluAspThrProCysAlaArgAlaAsp

AsnCysSerGlyLeuGlyGluGluGluThrIleAsnCysGlnPhe

AsnMetThrGlyLeuGluArgAspLysLysLysGlnTyrAsnGlu

500
ThrTrpTyrSerLysAspValValCysGluThrAsnAsnSerThr

AsnGlnThrGlnCysTyrMetAsnHisCysAsnThrSerValIle

600
ThrGluSerCysAspLysHisTyrTrpAspAlaIleArgPheArg

TyrCysAlaProProGlyTyrAlaLeuLeuArgCysAsnAspThr

700
AsnTyrSerGlyPheAlaProAsnCysSerLysValValAlaSer

ThrCysThrArgMetMetGluThrGlnThrSerThrTrpPheGly

800
PheAsnGlyThrArgAlaGluAsnArgThrTyrIleTyrTrpHis

GlyArgAspAsnArgThrIleIleSerLeuAsnLysTyrTyrAsn

900
LeuSerLeuHisCysLysArgProGlyAsnLysThrValLysGln

IleMetLeuMetSerGlyHisValPheHisSerHisTyrGlnPro

IleAsnLysArgProArgGlnAlaTrpCysTrpPheLysGlyLys

1000
TrpLysAspAlaMetGlnGluValLysThrLeuAlaLysHisPro

ArgTyrArgGlyThrAsnAspThrArgAsnIleSerPheAlaAla

1100
ProGlyLysGlySerAspProGluValAlaTyrMetTrpThrAsn

CysArgGlyGluPheLeuTyrCysAsnMetThrTrpPheLeuAsn

1200
TrpIleGluAsnLysThrHisArgAsnTyrAlaProCysHisIle

LysGlnIleIleAsnThrTrpHisLysValGlyArgAsnValTyr

1300
LeuProProArgGluGlyGluLeuSerCysAsnSerThrValThr

SerIleIleAlaAsnIleAspTrpGlnAsnAsnAsnGlnThrAsn

67

IleThrPheSerAlaGluValAlaGluLeuTyrArgLeuGluLeu

1400

GlyAspTyrLysLeuValGluIleThrProIleGlyPheAlaPro

ThrLysGluLysArgTyrSerSerAlaHisGlyArgHisThrArg

1500

GlyValPheValLeuGlyPheLeuGlyPheLeuAlaThrAlaGly

SerAlaMetGlyAlaArgAlaSerLeuThrValSerAlaGlnSer

1600

ArgThrLeuLeuAlaGlyIleValGlnGlnGlnGlnGlnLeuLeu

AspValValLysArgGlnGlnGluLeuLeuArgLeuThrValTrp

1700

GlyThrLysAsnLeuGlnAlaArgValThrAlaIleGluLysTyr

LeuGlnAspGlnAlaArgLeuAsnSerTrpGlyCysAlaPheArg

1800

GlnValCysHisThrThrValProTrpValAsnAspSerLeuAla

ProAspTrpAspAsnMetThrTrpGlnGluTrpGluLysGlnVal

ArgTyrLeuGluAlaAsnIleSerLysSerLeuGluGlnAlaGln

1900

IleGlnGlnGluLysAsnMetTyrGluLeuGlnLysLeuAsnSer

TrpAspIlePheGlyAsnTrpPheAspLeuThrSerTrpValLys

2000

TyrIleGlnTyrGlyValLeuIleIleValAlaValIleAlaLeu

ArgIleValIleTyrValValGlnMetLeuSerArgLeuArgLys

2100

GlyTyrArgProValPheSerSerProProGlyTyrIleGlnGln

IleHisIleHisLysAspArgGlyGlnProAlaAsnGluGluThr

2200

GluGluAspGlyGlySerAsnGlyGlyAspArgTyrTrpProTrp

ProIleAlaTyrIleHisPheLeuIleArgGlnLeuIleArgLeu

LeuThrArgLeuTyrSerIleCysArgAspLeuLeuSerArgSer

2300

PheLeuThrLeuGlnLeuIleTyrGlnAsnLeuArgAspTrpLeu

```
ArgLeuArgThrAlaPheLeuGlnTyrGlyCysGluTrpIleGln
                    2400
GluAlaPheGlnAlaAlaAlaArgAlaThrArgGluThrLeuAla

GlyAlaCysArgGlyLeuTrpArgValLeuGlnArgIleGlyArg
                    2500
GlyIleLeuAlaValProArgArgIleArgGlnGlyAlaGluIle

AlaLeuLeu
```

37. Procédé selon la revendication 28, caractérisé en ce qu'il comprend en outre les étapes suivantes :

— l'action de la T4 ADN polymérase jusqu'à obtention d'extrémités franches sur la double chaîne d'ADNc,

— l'insertion de l'ADNc muni d'adaptateurs et digéré par les enzymes adéquates dans l'acide nucléique d'un vecteur,

— la récupération d'un acide nucléique recombinant comprenant un ADNc ou une partie de cet ADNc obtenu par un procédé selon l'une quelconque des revendications 28 à 36, et/ou un ARN ou une partie d'un ARN correspondant à cet ADNc, inséré dans un article nucléique provenant dudit vecteur.

38. Procédé selon la revendication 37, caractérisé en ce que l'on marque l'acide nucléique recombinant récupéré.

39. Procédé de détection d'un rétrovirus susceptible d'avoir un effet cytopathogène pour l'homme, du type HIV-2 ou de son ARN dans un liquide ou tissu biologique, notamment en vue du diagnostic in vitro chez l'homme de la potentialité ou de l'existence d'un SLA ou d'un SIDA, caractérisé par la mise en contact des acides nucléiques contenus dans ce liquide ou tissu biologique avec une sonde contenant un acide nucléique obtenu par un procédé selon l'une quelconque des revendications 32 à 36 dans des conditions d'hybridation stringentes, ou contenant un ADN présentant une homologie de séquence au moins égale à 70 %, de préférence au moins égale 90 % avec l'ARN d'un rétrovirus selon l'une quelconque des revendications 1 à 9 et capable de s'hybrider avec celui-ci, dans des conditions non stringentes, dans la technique du « spot blot » la mise en contact ci-dessus étant réalisée pendant le temps nécessaire à la réalisation de cette hybridation et étant suivie du lavage de l'hybride formé, avec une solution assurant la conservation de ces conditions stringentes, et la détection de l'hybride formé.

40. Procédé de production d'un rétrovirus susceptible d'avoir un effet cytopathogène pour l'homme du type HIV-2, caractérisé par la culture dans des lymphocytes T4 humains ou dans des lignées cellulaires permanentes dérivées de lymphocytes T4 et portant le phénotype T4, ces lymphocytes ou ces lignées ayant préalablement été infectés avec un isolat de virus HIV-2, tel que défini dans l'une quelconque des revendications 1 à 9, et notamment lorsque le niveau d'activité de la transcriptase inverse (ou reverse-transcriptase) a atteint un seuil déterminé, par la récupération et la purification de quantités de virus libérées dans le milieu de culture de ces lymphocytes ou lignées, notamment par centrifugation différentielle dans un gradient de sucrose ou de métrizamide.

41. Procédé de production d'une sonde d'hybridation pour la détection d'un ARN de rétrovirus HIV-2, caractérisé par l'insertion d'une séquence d'ADN, notamment une sonde obtenue par un procédé selon l'une quelconque des revendications 28 à 36 dans un vecteur de clonage, par recombinaison in vitro, par le clonage du vecteur modifié obtenu dans un hôte cellulaire compétent et par la récupération des ADN-recombinants obtenus.

42. Les hybridomes secréteurs d'un anticorps monoclonal reconnaissant spécifiquement un antigène d'un rétrovirus HIV-2 selon l'une quelconque des revendications 1 à 9.

43. Application des anticorps polyclonaux obtenus par un procédé selon la revendication 27, à la détection du rétrovirus HIV-2 tel que défini dans l'une quelconque des revendications 1 à 9 dans une préparation biologique.

44. Application des anticorps polyclonaux obtenus par un procédé selon la revendication 27 à la détection d'antigènes du rétrovirus HIV-2 tel que défini dans l'une quelconque des revendications 1 à 9 dans une préparation biologique.

45. Application des anticorps polyclonaux obtenus par un procédé selon la revendication 27, à la purification d'antigènes viraux d'un rétrovirus HIV-2 tel que défini dans l'une quelconque des revendications 1 à 9.

**Claims** (for the Contracting States : BE, CH, DE, FR, GB, IT, LI, LU, NL, SE)

1. HIV-2 human retrovirus, having the capacity of infecting human T4 lymphocytes and being capable of causing AIDS in man, this retrovirus being constituted of one of the retroviruses deposited with the CNCM under n° I-502, I-532, I-642 and I-643 and with the ECACC under n° 87.011001 and 87.011002 (corresponding to n° I-502 and I-532 respectively), or a variant of one of these retroviruses comprising all the antigens which are recognized by the antibodies raised against the corresponding antigens of anyone of the retroviruses having been deposited with the CNCM or with the ECACC as said above.

2. Human retrovirus according to claim 1, characterised in that its genomic RNA is capable of hybridizating under stringent conditions with the complementary chain of a cDNA or cDNA fragment derived from the genomic RNA of anyone of the retroviruses deposited with the CNCM under n° I-502, I-532, I-642 and I-643 and with the ECACC under n° 87.011001 and 87.011002 (corresponding to n° I-502 and I-532 respectively).

3. Human retrovirus according to claim 1, characterised in that it comprises a set of antigens formed by core proteins having molecular weights of the order or 12000, 16000 and 26000 daltons respectively, proteins or glycoproteins having molecular weights respectively of the order of 36000 daltons and of the order of 42000-45000 daltons and a major envelope glycoprotein having a molecular weight of the order of 13000-14000 daltons.

4. Human purified retrovirus according to claim 1, pathogenic for man and. capable of causing AIDS, characterised by the whole following properties :

— the preferred target for the HIV-2 retrovirus consists of human Leu 3 cells (or T4 lymphocytes) and for permanent cell lines derived of said T4 lymphocytes ;

— it is capable of having a cytopathogenic effect for the human T4 lymphocytes which it infects ;

— it has a reverse transcriptase activity which requires the presence of $Mg^{2+}$ ions and has a strong affinity for polyadenylate oligodeoxythymidylate (Lpoly(A)-oligo(dT)12-18) ;

— it has a density of approximately 1.16 in a sucrose gradient ;

— it has a mean diameter of 140 nanometres and a core having mean diameter of 41 nanometres ;

— it can be cultivated in permanent cell lines expressing the T4 protein ;

— it is not infectious in T8 lymphocytes ;

— the lysates of this virus contain p26 protein which does not crossreact immunologically with p24 protein of the HTLV-1 virus or of the HTLV-2 ;

— said lysates further contain p16 protein which is not recognized immunologically by p19 protein of HTLV-1 or of HTLV-2 in radioimmunoprecipitation assays ;

— said lysates further contain an envelope glycoprotein having a molecular weight of the order of 130,000-140,000 daltons which does not crossreact immunologically with gp110 of HTLV-1 retrovirus ;

— said lysates further contain a protein or a glycoprotein which can be labelled by [35]-S-cystein, having a molecular weight of the order of 36,000 daltons ;

— the genomic RNA of HIV-2 does not hybridize under stringent conditions with the genomic RNA of HIV-1 and does not hybridize under non-stringent conditions either with the _env_ gene, or with the LTRs of HIV-1 ;

— the genomic RNA of HIV-2 hybridizes weakly under non-stringent conditions with nucleotide sequences comprising on the one hand nucleotides 990-1070 and on the other hand nucleotides 990-1260 of the _GAG_ region of the HIV-1 genome.

5. Retrovirus according to anyone of claims 1 to 4, characterised in that it does not multiply in chronic fashion in the lymphocytes of macaques rhesus monkeys, when it has been injected in vivo and in working conditions which permit the development of the STLVIII_mac virus, as have been described by Letvin et al., Science (1985) vol. 230, 71-75.

6. The retrovirus of any of claims 1 to 5, characterised in that the sequence comprising the R region and the U3 region of the sequence comprising its genomic RNA also contains a nucleotidic sequence which corresponds to the following nucleotide sequence.

```
GTGGAAGGCGAGACTGAAAGCAAGAGGAATACCATTTAGTTAAAGGACAG
GAACAGCTATACTTGGTCAGGGCAGGAAGTAACTAACAGAAACAGCTGAG
ACTGCAGGGACTTTCCAGAAGGGGCTGTAACCAAGGGAGGGACATGGGAG
GAGCTGGTGGGGAACGCCTCATATTCTCTGTATAATATACCCGCTGCTTG
CATTGTACTTCAGTCGCTCTGCGGAGAGGCTGGCAGATTGAGCCCTGGAG
GATCTCTCCAGCACTAGACGGATGAGCCTGGGTGCCCTGCTAGACTCTCA
CCAGCACTTGGCCCGGTGCTGGCAGACCGGCCCCACGCTTGCCTGCTTAAA
ACCTTCCTTAATAAAGCTGCAGTAGAAGCA
```

7. Retrovirus according to anyone of claims 1 to 5 characterised in that its genomic RNA contains a gag sequence corresponding to the following nucleotide sequence :

GAGRODE .

ATCGGCGCCAGAAACTCCGTCTTGACAGGGAAAAAGCACATGAA

TTAGAAAGAATCAGGTTACCGCCCCGCCCAAAGAAAAGTACACG

CTAAAACATATTGTGTGGGCAGCCAATAAAITGGACAGATTCGGA
100

TTAGCAGAGAGCCTGTTGGAGTCAAAAGACGGTTCTCAAAAAATT

CTTACAGTTTTAGATCCAATGGTACCGACAGGTTCAGAAAATTTA
200

AAAGTCTTTTTAATACTGTCTGCGTCATTTGGTGCATACACGGGA

GAAGAGAAAGTGAAAGATACTGAAGGAGCCAAACAAATAGTGCGG
300

AGACATCTAGTGGCAGAAACAGGAACTGCAGAGAAATGCCAAGG

ACAAGTAGACCAACAGCACCATCTAGCCGAGAACGGACGAAATTAG
400

GCAGTGCAACATGTAGGCGGGAAACTACACCCATATACCGCTGAGT

GCCCGAACCCTAAATGCCTGGGTAAAATTAGTAGAGGAAAAAAG

TTCGGGGCAGAAGTAGTGCCAGGATTTCAGGCACTCTCAGAAGGC
500

TGCACGCCCTATGATATCAACCAAATGCTTAATTGTGTGGGCCAG

CATCAAGCAGCCATGCAGATAATCAGGGAGATTATCAATGAGGAA
600

GCAGCAGAATGGGATGTGCAACATCCAATACCAGGCCCCTTACCA

GCGGGGCAGCTTAGAGAGCCAAGGGGATCTGACATACCAGGGACA
700

ACAAGCACAGTAGAAGAACAGATCCACTGCATGTTTACGCCACAA

AATCCTGTACCAGTAGGAAACATCTATAGAAGATGGATCCAGATA
800

GGATTGCAGAAGTGTCTCAGGATGTACAACCCGACCAACATCCTA

GACATAAAACAGGGACCAAAGGAGCCGTTCCAAGGCTATGTAGAT
900

AGATTCTACAAAAGCTTGAGGGCCACAACAAACAGATCCAGCAGTG

AAGAATTCGATGACCCAAACACTGCTACTACAAAATGCCAACCCA

GACTGTAAATTAGTGCTAAAAGGACTACGGATGAACCCTACCTTA
1000

GAAGAGATGCTGACCGCCTGTCAGGGGGTAGGTGGGCCAGGCCAG

AAAGCTAGATTAATGGCAGAGGCCCTGAAAGAGGTCATAGGCACCT
1100

GCCCCTATCCCATTCGCAGCAGCCCAGCAGAGAAAGGCATTTAAA

TGCTGGAACTGTGGAAAGGAAGGGCACTCGUAAGACAATGCCCA
1200

GCACCTAGAAGGCAGGGCTGCTGGAAGTGTGGTAAGCCAGGACAG

ATCATGACAAACTGCCCAGATAGACAGGCAGGTTTTTTAGGACTG
1300

GGCCCTGGGGAAAGAAGCCCCGCAACTTCCCCGTGGCCCAAGTT

CCGCAGCGGCTGACACCAACAGCACCCCCAGTGCATCCAGCAGTG

GATCTACTGGACAAATATATGCAGCAAGGGAAAAGACAGAGAGAG
1400

GAGAGAGAGAGACCATACAAGGAAGTGACACAGGACTTACTGCAC.

CTCGAGCAGGGGGAGACACCATACAGGGAGCCACCAACAGAGGAG
1500

TTGCTGCCACCTCAATTCTCTCTTTGGAAAAGACCAG

8. Retrovirus according to anyone of claims 1 to 6 characterised in that its genomic RNA contains a env sequence corresponding to the following nucleotide sequence :

ENV

ATGATGAATCAGCTGCTTATTGCCATTTTATTAGCTAGTGCTTGC

TTAGTATATTGCACCCAATATGTAACTGTTTTCTATGGCGTACCC

ACGTCGAAAAATGCAACCATTCCCCTCTTTTGTGCAACCAGAAAT
100

ACGGATACTTGGGGAACCATACAGTCCTTGCCTCACAATCATGAT

TATCAGGAAATAACTTTGAATGTAACACACGGCTTTTCATGCATGG
200

AATAATACAGTAACAGAACAAGCAATAGAAGATCTCTGGCATCTA

TTCCAGACATCAATAAAACCATGTGTCAAACTAACACCTTTATGT
300

CTAGCAATGAAATGCACCAGCACACACAGCCAGCACACGGAACAAG

ACAACCTCAAAGAGCACAAGCACAACCACAACCACACCCACAGAC
400

CAGGAGCAAGAGATAAGTGAGGATACTCCATGCGCACGCGGCAGAC

AACTGCTCAGGATTGGCACAGGAAGAAACGATCAATTGCCAGTTC

AATATGACAGGATTAGAAAGAGATAAGAAAAACACTATAATGAA
500

ACATCGTACTCAAAAGATGTGGTTTGTCAGACAAATAATAGCACA

AATCAGACCCAGTGTTACATGAACCATTGCAACACATCAGTCATC
600

ACAGAATCATGTGACAAGCACTATTGGCATGCTATAAGGTTTACA

TACTGTGCACCACCGGGTTATGCCCTATTAAGATGTAATGATACC
700

AATTATTCAGGCTTTGCACCCAACTGTTCTAAAGTAGTACCTTCT

ACATGCACCAGGATGATGGAAACGCAAACTTCCACATGGTTTGGC
800

TTTAATGGCACTAGAGCCAGAGAATAGAACATATATCTATTGGCAT

GGCACAGATAATACAACTATCATCAGCTTAAACAAATATTATAAT
900

CTCACTTTGCATTGTAAGACGCCAGCGAATAAGACACAGTGAAACAA

ATAATGCTTATGTCAGGACATGTGTTTCACTCCCACTACCAGCCG

ATCAATAAAAGACCCAGACAAGCATGGTGCTGGTTCAAAGGCAAA
1000

TGGAAAGACGCCATGCAGGAGGTGAAGACCCCTTGCAAAACATCCC

73

AGGTATAGAGGAACCAATGACACAAGGAATATTAGCTTTGCAGCG
1100

CCAGGAAAAGGCTCAGACCCAGAAGTAGCATACATGTGGACTAAG

TGCAGAGGAGAGTTTCTCTACTGCAACATGACTTGGTTCCTCAAT
1200

TGGATAGAGAATAAGACACACCGCAATTATGCACCGTGCCATATA

AAGCAAATAATTAACACATGGCATAAGGTAGGCAGAAATGTATAT
1300

TTGCCTCCCACGGAAGGGGAGCTGTCCTGCAACTCAACAGTAACC

AGCATAATTGCTAACATTGACTGGCAAAACAATAATCAGACAAAG

ATTACCTTTAGTGCAGAGGTGGCAGAACTATACAGATTGGAGTTG
1400

GGACATTATAAATTGGTACAAATAACACCAATTGGCTTCGCACCT

ACAAAAGAAAAAAGATACTCCTCTGCTCACGGGAGACATACAAGA
1500

GGTGTGTTCGTGCTAGGGTTCTTGGGTTTTCTCGCAACAGCAGGT

TCTGCAATGGGCGCTCGAGCGTCCCTGACCGTGTCGGCTCAGTCG
1600

CGGACTTTACTGGCCGGGATAGTGCAGCAACAGCAACAGCTGTTG

GACGTGGTCAAGAGACAACAAGAACTGTTGCCACTGACCGTCTGG
1700

CGAACGAAAAACCTCCAGGCAAGAGTCACTGCTATAGAGAAGTAG

CTACAGGACCAGGCGCGGCTAAATTCATGGGATGTGCCGTTTAGA
1800

CAAGTCTGCCACACTACTGTACCATGGGTTAATGATTCCTTAGCA

CCTGACTGGGACAAATATGACGTGGCAGGAATGGGAAAAACAAGTC

CGGTACCTCGAGGCAAATATCAGTAAAAGTTTAGAACAGGCACAA
1900

ATTCAGCAAGAGAAAAATATGTATGAACTACAAAAATTAAATAGC

```
TGGGATATTTTTGGCAATTGGTTTGACTTAACCTCCTGGGTCAAG
                2000
TATATTCAATATGGAGTGCTTATAATAGTAGCAGTAATAGCTTTA
AGAATAGTGATATATGTAGTACAAATGTTAAGTAGGCTTAGAAAG
                        2100
CGCTATAGGCCTGTTTTCTCTTCCCCCCCCGGTTATATCCAACAG
ATCCATATCCACAAGGACCGCGCACAGCCAGCCAACGAAGAAACA
                            2200
CAAGAAGACGGTGGAAGCAACGGTGGCACACATACTCGCCCTGG
GCGATAGCATATATACATTTCCTGATCCGCCAGCTCATTCGCCTC
TTGACCAGACTATACAGCATCTGCACGGACTTACTATCCAGGAGC
2300
TTCCTGAGGGTCCAACTCATCTACCAGAATCTCAGAGACTGGCTG
AGACTTAGAACAGCCTTCTTGCAATATGGGTCCGAGTGGATCGAA
        2400
GAAGCATTCCAGGCCGCCGCCAGCGGCTACAAGAGAGACTCTTGCG
GGCGCGTGCAGGGGCTTGTGGAGGGTATTCGAACGAATCGCCACG
                2500
GGAATACTCGCGGTTCCAAGAAGGATCACACAGGCAGCAGAAATC

GCCCTCCTG
```

9. Retrovirus according to anyone of claims 1 to 8, characterised in that its RNA does not hybridize under stringent conditions with the genomic RNA of HIV-1 and does not hybridize under non-stringent conditions, with the env gene and the LTR close to it, more particularly with the nucleotidic sequence 5290-9130 of HIV-1 and does not hybridize under non-stringent conditions with sequences of the pol region of the HIV-1 genome, particularly with the nucleotide sequence 2170-2240.

10. Purified antigen of the retrovirus HIV-2 having the immunological characteristics of the HIV-2 p12 protein, immunologically recognized by antibodies raised against a retrovirus according to anyone of claims 1 to 9 and having a molecular weight of the order of 12000 daltons.

11. Purified antigen of the retrovirus HIV-2 having the immunological characteristics of the HIV-2 p16 protein, immunologically recognized by antibodies raised against a retrovirus according to anyone of claims 1 to 9 and having a molecular weight of the order of 16000 daltons.

12. Purified antigen of the retrovirus HIV-2 having the immunological characteristics of the HIV-2 p26 protein, immunologically recognized by antibodies raised against a retrovirus according to anyone of claims 1 to 9 and having a molecular weight of the order of 26000 daltons.

13. Purified antigen of the retrovirus HIV-2 having the immunological characteristics of the HIV-2 p36 protein, immunologically recognized by antibodies raised against a retrovirus according to anyone of claims 1 to 9 and having a molecular weight of the order of 36000 daltons.

14. Purified antigen of the retrovirus HIV-2 having the immunological characteristics of the HIV-2 p42 protein, immunologically recognized by antibodies raised against a retrovirus according to anyone of claims 1 to 9 and having a molecular weight of the order of 42000-45000 daltons.

15. Purified antigen of the retrovirus HIV-2 having the immunological characteristics of the HIV-2 glycoprotein gp140, immunologically recognized by antibodies raised against a retrovirus according to anyone of claims 1 to 9 and having a molecular weight of the order of 130000-140000.

16. Antigen having the following aminoacid sequence or a part of said sequence, providing that it raises a specific immunological reaction with the antibodies against a HIV-2 retrovirus, according to anyone of claims 1 to 9, especially when this antigen is contacted with the serum of a patient infected with HIV-2.

ArgLysAlaPheLys

CysTrpAsnCysGlyLysGluGlyHisSerAlaArgGlnCysArg

1200
AlaProArgArgGlnGlyCysTrpLysCysGlyLysProGlyHis

IleHisThrAsnCysProAspArgGlnAlaGlyPheLeuGlyLeu

1300
GlyProTrpGlyLysLysProArgAsnPheProValAlaGlnVal

ProGlnGlyLeuThrProThrAlaProProValAspProAlaVal

AspLeuLeuGluLysTyrMetGlnGlnGlyLysArgGlnArgGlu

1400
GlnArgGluArgProTyrLysGluValThrGluAspLeuLeuHis

LeuGluGlnGlyGluThrProTyrArgGluProProThrGluAsp

1500
LeuLeuHisLeuAsnSerLeuPheGlyLysAspGln

17. Antigen having the following aminoacid sequence or a part of said sequence, providing that it raises a specific immunological reaction with antibodies against a HIV-2 retrovirus, according to anyone of claims 1 to 9, especially when this antigen is contacted with the serum of a patient infected with HIV-2.

MetGlyAlaArgAsnSerValLeuArgGlyLysLysAlaAspGlu

LeuGluArgIleArgLeuArgProGlyGlyLysLysLysTyrArg

LeuLysHisIleValTrpAlaAlaAsnLysLeuAspArgPheGly

100
LeuAlaGluSerLeuLeuGluSerLysGluGlyCysGlnLysIle

LeuThrValLeuAspProMetValProThrGlySerGluAsnLeu

200
LysSerLeuPheAsnThrValCysValIleTrpCysIleHisAla

GluGlnLysValLysAspThrGluGlyAlaLysGlnIleValArg

300

ArgHisLeuValAlaGlnThrGlyThrAlaGlnLysMetProSer

ThrSerArgProThrAlaProSerSerGluLysGlyGlyAsnTyr

400

18. Antigen having the following aminoacid sequence or a part of said sequence, providing that it raises a specific immunological reaction with antibodies against a HIV-2 retrovirus, according to anyone of claims 1 to 9, especially when this antigen is contacted with the serum of a patient infected with HIV-2.

ProValGlnIleValGlyGlyAsnTyrThrHisIleIleProLeuSer

ProArgThrLeuAsnAlaTrpValLysLeuValGluGluLysLys

PheGlyAlaGluValValProGlyPheGlnAlaLeuSerGluGly

500
CysThrProTyrAspIleAsnGlnMetLeuAsnCysValGlyAsp

IleGlnAlaAlaMetGlnIleIleArgGluIleIleAsnGluGlu

600
AlaAlaGluTrpAspValGlnHisProIleProGlyProLeuPro

AlaGlyGlnLeuArgGluProArgGlySerAspIleAlaGlyThr

700
ThrSerThrValGluGluGluIleGlnTrpMetPheArgProGln

AsnProValProValGlyAsnIleTyrArgArgTrpIleGlnIle

800
GlyLeuGlnLysCysValArgMetTyrAsnProThrAsnIleLeu

AspIleLysGlnGlyProLysGluProPheGlnSerTyrValAsp

900
ArgPheTyrLysSerLeuArgAlaGluGlnThrAspProAlaVal

LysAsnTrpMetThrGluThrLeuLeuValGlnAsnAlaAsnPro

AspCysLysLeuValLeuLysGlyLeuGlyMetAsnProThrLeu

1000
GluGluMetLeuThrAlaCysGlnGlyValGlyGlyProGlyGln

LysAlaArgLeuMetAlaGluAlaLeuLysGluValIleGlyPro

1100
AlaProIleProPheAlaAlaAlaGlnGln

77

**EP 0 239 425 B1**

19. Antigen having the following aminoacid sequence of a part of said sequence, providing that it raises a specific immunological reaction with the antibodies against a HIV-2 retrovirus, according to anyone of claims 1 to 9, especially when this antigen is contacted with the serum of a patient infected with HIV-2.

**IIVII**

MetMetAsnGlnLeuLeuIleAlaIleLeuLeuAlaSerAlaCys

LeuValTyrCysThrGlnTyrValThrValPheTyrGlyValPro

ThrTrpLysAsnAlaThrIleProLeuPheCysAlaThrArgAsn

100
ArgAspThrTrpGlyThrIleGlnCysLeuProAspAsnAspAsp

TyrGlnGluIleThrLeuAsnValThrGluAlaPheAspAlaTrp

200
AsnAsnThrValThrGluGlnAlaIleGluAspValTrpHisLeu

PheGluThrSerIleLysProCysValLysLeuThrProLeuCys

300
ValAlaMetLysCysSerSerThrGluSerSerThrGlyAsnAsn

ThrThrSerLysSerThrSerThrThrThrThrThrProThrAsp

400
GlnGluGlnGluIleSerGluAspThrProCysAlaArgAlaAsp

AsnCysSerGlyLeuGlyGluGluGlnThrIleAsnCysGlnPhe

AsnMetThrGlyLeuGluArgAspLysLysLysGlnTyrAsnGlu

500
ThrTrpTyrSerLysAspValValCysGluThrAsnAsnSerThr

AsnGlnThrGlnCysTyrMetAsnHisCysAsnThrSerValIle

600
ThrGluSerCysAspLysHisTyrTrpAspAlaIleArgPheArg

TyrCysAlaProProGlyTyrAlaLeuLeuArgCysAsnAspThr

700
AsnTyrSerGlyPheAlaProAsnCysSerLysValValAlaSer

78

ThrCysThrArgMetMetGluThrGlnThrSerThrTrpPheGly

PheAsnGlyThrArgAlaGluAsnArgThrTyrIleTyrTrpHis
800

GlyArgAspAsnArgThrIleIleSerLeuAsnLysTyrTyrAsn

LeuSerLeuHisCysLysArgProGlyAsnLysThrValLysGln
900

IleMetLeuMetSerGlyHisValPheHisSerHisTyrGlnPro

IleAsnLysArgProArgGlnAlaTrpCysTrpPheLysGlyLys

TrpLysAspAlaMetGlnGluValLysThrLeuAlaLysHisPro
1000

ArgTyrArgGlyThrAsnAspThrArgAsnIleSerPheAlaAla

ProGlyLysGlySerAspProGluValAlaTyrMetTrpThrAsn
1100

CysArgGlyGluPheLeuTyrCysAsnMetThrTrpPheLeuAsn

TrpIleGluAsnLysThrHisArgAsnTyrAlaProCysHisIle
1200

LysGlnIleIleAsnThrTrpHisLysValGlyArgAsnValTyr

LeuProProArgGluGlyGluLeuSerCysAsnSerThrValThr
1300

SerIleIleAlaAsnIleAspTrpGlnAsnAsnAsnGlnThrAsn

IleThrPheSerAlaGluValAlaGluLeuTyrArgLeuGluLeu

GlyAspTyrLysLeuValGluIleThrProIleGlyPheAlaPro
1400

ThrLysGluLysArgTyrSerSerAlaHisGlyArgHisThrArg

GlyValPheValLeuGlyPheLeuGlyPheLeuAlaThrAlaGly
1500

SerAlaMetGlyAlaArgAlaSerLeuThrValSerAlaGlnSer

ArgThrLeuLeuAlaGlyIleValGlnGlnGlnGlnGlnLeuLeu
1600

AspValValLysArgGlnGlnGluLeuLeuArgLeuThrValTrp
1700

79

GlyThrLysAsnLeuGlnAlaArgValThrAlaIleGluLysTyr

LeuGlnAspGlnAlaArgLeuAsnSerTrpGlyCysAlaPheArg

1800
GlnValCysHisThrThrValProTrpValAsnAspSerLeuAla

ProAspTrpAspAsnMetThrTrpGlnGluTrpGluLysGlnVal

ArgTyrLeuGluAlaAsnIleSerLysSerLeuGluGlnAlaGln

1900
IleGlnGlnGluLysAsnMetTyrGluLeuGlnLysLeuAsnSer

TrpAspIlePheGlyAsnTrpPheAspLeuThrSerTrpValLys

2000
TyrIleGlnTyrGlyValLeuIleIleValAlaValIleAlaLeu

ArgIleValIleTyrValValGlnMetLeuSerArgLeuArgLys

2100
GlyTyrArgProValPheSerSerProProGlyTyrIleGlnGln

IleHisIleHisLysAspArgGlyGlnProAlaAsnGluGluThr

2200
GluGluAspGlyGlySerAsnGlyGlyAspArgTyrTrpProTrp

ProIleAlaTyrIleHisPheLeuIleArgGlnLeuIleArgLeu

LeuThrArgLeuTyrSerIleCysArgAspLeuLeuSerArgSer

2300
PheLeuThrLeuGlnLeuIleTyrGlnAsnLeuArgAspTrpLeu

ArgLeuArgThrAlaPheLeuGlnTyrGlyCysGluTrpIleGln

2400
GluAlaPheGlnAlaAlaAlaArgAlaThrArgGluThrLeuAla

GlyAlaCysArgGlyLeuTrpArgValLeuGluArgIleGlyArg

2500
GlyIleLeuAlaValProArgArgIleArgGlnGlyAlaGluIle

AlaLeuLeu

20. Composition for the in vitro diagnosis of the presence in a biological human sample of antibodies against a human HIV-2 virus capable of having a cytopathogenic effect for the T4 lymphocytes, characterised in that it contains at least one antigen, especially a protein or a glycoprotein of the HIV-2 retrovirus according to anyone of claims 1 to 9, or at least one antigen according to anyone of claims 10 to 19.

21. Composition according to claim 20, characterised in that it consists of a total extract or of a lysate of the abovesaid retrovirus.

22. Composition according to claim 20, characterised in that the antigen consists of at least one of the internal core protein of the abovesaid virus, chosen among proteins or glycoproteins p12, p16 and p26, having respectively molecular weights of the order of 12000, 16000 and 26000 daltons.

23. Composition according to claim 20 or claim 21, characterised in that it contains a glycoprotein gp140, having a molecular weight of the order of 130000 to 140000 daltons.

24. Composition according to claim 20 or claim 21, characterised in that it contains a glycoprotein gp36, having a molecular weight of the order of 36000 daltons.

25. Composition for the in vitro diagnosis in a biological human sample, of antibodies against a human retrovirus capable of having a cytopathogenic effect for the T4 human lymphocytes, comprising at least one HIV-1 antigen, characterised in that it further comprises at least one HIV-2 antigen according to anyone of claims 1 to 9, especially a protein or a glycoprotein of this HIV-2 retrovirus or at least one antigen according to anyone of claims 10 to 19.

26. Composition according to claim 25, characterised in that it contains core proteins of HIV-1 and of HIV-2.

27. Composition according to claim 26, characterised in that it contains the p25 of a HIV-1 and p26 of a HIV-2.

28. Composition according to claim 26, characterised in that it contains the p18 of a HIV-1 and the p16 of a HIV-2.

29. Composition according to claim 25, characterised in that it contains envelope glycoproteins of a HIV-1 and envelope glycoproteins of a HIV-2.

30. Composition according to claim 29, characterised in that it contains the gp110 of HIV-1 and gp140 of HIV-2.

31. Composition according to claim 29, or claim 30, characterised in that it contains the gp42 of HIV-1 and the p36 of HIV-2.

32. Composition according to claim 26, characterised in that it contains mixtures on the one hand of antigens proteins or glycoproteins of HIV-1 or of the whole, on the other hand, mixtures of proteins or glycoproteins of HIV-2 or both together.

33. Process for the in vitro detection of the presence of antibodies, induced in man infected with a human HIV-2 retrovirus, in a human biological sample such as a serum and more particularly for the in vitro diagnosis of a potential or existing LAS or AIDS due to such a retrovirus and obtained from the person being diagnosed, characterised in that this biological sample is contacted with an antigen recognized by an antibody induced in the infected man, by a human HIV-2 retrovirus as defined in claims 1 to 9 in conditions authorizing the formation of an immunological complex between this antigen and said antibody and in that the possibly immunological conjugate formed between this antibody and the antigen used is detected.

34. Method according to claim 33, characterised in that the biological sample is contacted with an antigen according to anyone of claims 10 to 19 or a composition according to anyone of claims 20 to 24 in conditions authorizing the formation of an immunological complex between this antigen and said antibodies and in that the immunological conjugate possibly formed between these anti-HIV-2 antibodies and the antigen used is detected.

35. Method according to claim 34, characterised in that said immunological conjugate is detected by a reaction between the possibly formed immunological conjugate and a labelled reagent formed either by anti-human immunoglobulin antibodies either by bacterial A protein and in that the complex formed between the reagent and the above immunological conjugate is detected.

36. Method for the in vitro detection of the presence of antibodies recognizing antigens of a human retrovirus capable of having a cytopathogenic effect for the T4 human lymphocytes in a human biological sample like a serum and more particularly for the in vitro diagnosis of a potential or existing LAS or AIDS caused by a human retrovirus, characterised in that this biological sample obtained from the person being diagnosed, is contacted with a mixture of antigens capable of giving a specific immunological reaction with the antibodies against, on the one hand a human HIV-2 retrovirus capable of having a cytopathogenic effect, as defined in anyone of claims 1 to 9 on the other hand a human HIV-1 retrovirus and in that the immunological conjugate possibly formed between at least one of these antigens and said antibodies is detected.

37. Method according to claim 36, characterised in that the biological sample obtained from the person being diagnosed is contacted with a mixture of antigens obtained both from both a HIV-1 and HIV-2 retrovirus according to any one of claims 1 to 9, especially with a composition according to anyone of claims 26 to 32 and in that the immunological complex possibly formed between at least one of these antigens and said antibodies is detected.

38. Kit for the detection of antibodies against a retrovirus capable of having a cytopathogenic effect for man, of the HIV-2 type, in a biological sample, particularly of a person possibly carrying such antibodies, characterised in that it comprises :

— at least an antigen such as defined in any of claims 10 to 19, or a composition these as defined in anyone of claims 20 to 32 and 34 and

— means for detecting the immunological conjugate resulting from the immunological reaction between the antigen and said biological sample.

39. Kit according to claim 38, characterised in that means for detecting the immunological conjugate formed comprises human anti-immunoglobulins or an A protein and means for detecting the complex formed between the anti-HIV-2 antibodies contained in the detected immunological conjugate.

40. Kit for the in vitro diagnosis of the presence of antibodies against a retrovirus which is cytopathogenic for human T4 lymphocytes, particularly of a person possibly carrying these antibodies, characterised in that it comprises :

— a mixture of at least one HIV-1 antigen and at least one HIV-2 antigen, particularly a composition as defined in anyone of claims 25 to 32,

— means for detecting an immunological conjugate resulting from the immunological reaction between the antigens and the antibodies of the biological fluid.

41. Immunogenic composition containing an envelope glycoprotein of the HIV-2 human retrovirus as defined in anyone of claims 1 to 9 such as the gp140 of this retrovirus, or part of this glycoprotein capable of inducing antibodies against HIV-2 in association with a pharmaceutically acceptable vehicle, appropriate for the constitution of vaccines effective against HIV-2.

42. Composition according to claim 41 characterised in that it contains at least an immunogenic part of a glycoprotein containing a proteic backbone having the following sequence this immunogenic part being capable of inducing antibodies against a human HIV-2 retrovirus as defined in anyone of claims 1 to 9.


ENVRX


MetMetAsnGlnLeuLeuIleAlaIleLeuLeuAlaSerAlaCys


LeuValTyrCysThrGlnTyrValThrValPheTyrGlyValPro


ThrTrpLysAsnAlaThrIleProLeuPheCysAlaThrArgAsn


100
ArgAspThrTrpGlyThrIleGlnCysLeuProAspAsnAspAsp


TyrGlnGluIleThrLeuAsnValThrGluAlaPheAspAlaTrp


200
AsnAsnThrValThrGluGlnAlaIleGluAspValTrpHisLeu


PheGluThrSerIleLysProCysValLysLeuThrProLeuCys


300
ValAlaMetLysCysSerSerThrGluSerSerThrGlyAsnAsn


ThrThrSerLysSerThrSerThrThrThrThrThrProThrAsp


400
GlnGluGlnGluIleSerGluAspThrProCysAlaArgAlaAsp


AsnCysSerGlyLeuGlyGluGluGluThrIleAsnCysGlnPhe


82

AsnMetThrGlyLeuGluArgAspLysLysLysGlnTyrAsnGlu

500
ThrTrpTyrSerLysAspValValCysGluThrAsnAsnSerThr

AsnGlnThrGlnCysTyrMetAsnHisCysAsnThrSerValIle

600
ThrGluSerCysAspLysHisTyrTrpAspAlaIleArgPheArg

TyrCysAlaProProGlyTyrAlaLeuLeuArgCysAsnAspThr

700
AsnTyrSerGlyPheAlaProAsnCysSerLysValValAlaSer

ThrCysThrArgMetMetGluThrGlnThrSerThrTrpPheGly

800
PheAsnGlyThrArgAlaGluAsnArgThrTyrIleTyrTrpHis

GlyArgAspAsnArgThrIleIleSerLeuAsnLysTyrTyrAsn

900
LeuSerLeuHisCysLysArgProGlyAsnLysThrValLysGln

IleMetLeuMetSerGlyHisValPheHisSerHisTyrGlnPro

IleAsnLysArgProArgGlnAlaTrpCysTrpPheLysGlyLys

1000
TrpLysAspAlaMetGlnGluValLysThrLeuAlaLysHisPro

ArgTyrArgGlyThrAsnAspThrArgAsnIleSerPheAlaAla

1100
ProGlyLysGlySerAspProGluValAlaTyrMetTrpThrAsn

CysArgGlyGluPheLeuTyrCysAsnMetThrTrpPheLeuAsn

1200
TrpIleGluAsnLysThrHisArgAsnTyrAlaProCysHisIle

LysGlnIleIleAsnThrTrpHisLysValGlyArgAsnValTyr

1300
LeuProProArgGluGlyGluLeuSerCysAsnSerThrValThr

SerIleIleAlaAsnIleAspTrpGlnAsnAsnAsnGlnThrAsn

IleThrPheSerAlaGluValAlaGluLeuTyrArgLeuGluLeu

1400
GlyAspTyrLysLeuValGluIleThrProIleGlyPheAlaPro

83

ThrLysGluLysArgTyrSerSerAlaHisGlyArgHisThrArg

1500
GlyValPheValLeuGlyPheLeuGlyPheLeuAlaThrAlaGly

SerAlaMetGlyAlaArgAlaSerLeuThrValSerAlaGlnSer

1600
ArgThrLeuLeuAlaGlyIleValGlnGlnGlnGlnGlnLeuLeu

AspValValLysArgGlnGlnGluLeuLeuArgLeuThrValTrp

1700
GlyThrLysAsnLeuGlnAlaArgValThrAlaIleGluLysTyr

LeuGlnAspGlnAlaArgLeuAsnSerTrpGlyCysAlaPheArg

1800
GlnValCysHisThrThrValProTrpValAsnAspSerLeuAla

ProAspTrpAspAsnMetThrTrpGlnGluTrpGluLysGlnVal

ArgTyrLeuGluAlaAsnIleSerLysSerLeuGluGlnAlaGln

1900
IleGlnGlnGluLysAsnMetTyrGluLeuGlnLysLeuAsnSer

TrpAspIlePheGlyAsnTrpPheAspLeuThrSerTrpValLys

2000
TyrIleGlnTyrGlyValLeuIleIleValAlaValIleAlaLeu

ArgIleValIleTyrValValGlnMetLeuSerArgLeuArgLys

2100
GlyTyrArgProValPheSerSerProProGlyTyrIleGlnGln

IleHisIleHisLysAspArgGlyGlnProAlaAsnGluGluThr

2200
GluGluAspGlyGlySerAsnGlyGlyAspArgTyrTrpProTrp

ProIleAlaTyrIleHisPheLeuIleArgGlnLeuIleArgLeu

LeuThrArgLeuTyrSerIleCysArgAspLeuLeuSerArgSer

2300
PheLeuThrLeuGlnLeuIleTyrGlnAsnLeuArgAspTrpLeu

84

ArgLeuArgThrAlaPheLeuGlnTyrGlyCysGlnTrpIleGln

2400

GluAlaPheGlnAlaAlaAlaArgAlaThrArgGluThrLeuAla

GlyAlaCysArgGlyLeuTrpArgValLeuGlnArgIleGlyArg

2500

GlyIleLeuAlaValProArgArgIleArgGlnGlyAlaGluIle

AlaLeuLeu

43. Immunogenic composition according to claim 41 or claim 42 characterised in that it is dosed in antigen in order to enable the administration of a dosage-unit of 10 to 500, particularly from 50 to 100 µg/kg of bodyweight.

44. Antibody specifically recognizing an antigen of a HIV-2 retrovirus, according to anyone of claims 1 to 9.

45. Antibody according to claim 44, characterised in that it is monoclonal.

46. Nucleic acid, optionally labelled, derived of part at least of RNA of the HIV-2 virus or of one of its variants, as defined in anyone of claims 1 to 9, characterised by its hybridization capacity with the RNA of said HIV-2 virus and by the absence of hybridization in stringent conditions with probes 1 to 4 respectively comprising the nucleotides 990-1070, 990-1260, 2170-2240, 3370-3640 of the DNA derived from the HIV-1 genome.

47. Nucleic acid according to claim 46 containing at least part of a cDNA sequence capable of hybridizing with the whole genomic RNA of a HIV-2 retrovirus according to anyone of claims 1 to 9 and not hybridizing in stringent conditions with the probes which DNA fragments derived from the HIV-1 genome are respectively constituted by fragments delimited by the nucleotides 990-1070, 990-1260, 2170-2240, 3370-3640 of the DNA derived from the HIV-1 genome.

48. Nucleic acid according to claim 46, characterised in that it contains the nucleotidic sequence :

GTGGAAGGCGAGACTGAAAGCAAGAGGAATACCATTTAGTTAAAGGACAG

GAACAGCTATACTTGGTCAGGGCAGGAAGTAACTAACAGAAACAGCTGAG

ACTGCAGGGACTTTCCAGAAGGGGCTGTAACCAAGGGAGGGACATGGGAG

GAGCTGGTGGGGAACGCCTCATATTCTCTGTATAATATACCCGCTGCTTG

CATTGTACTTCAGTCGCTCTGCGGAGAGGCTGGCAGATTGAGCCCTGGAG

GATCTCTCCAGCACTAGACGGATGAGCCTGGGTGCCCTGCTAGACTCTCA

CCAGCACTTGGCCGGTGCTGGCAGACGGCCCCACGCTTGCCTGCTTAAAA

ACCTTCCTTAATAAAGCTGCAGTAGAAGCT

49. Nucleic acid according to claim 46, characterised in that it contains a nucleotidic sequence coding for all or part of the aminoacid sequence indicated hereafter, this sequence or part of aminoacid sequence having the capacity of giving a specific immunological reaction with antibodies against a HIV-2 retrovirus as defined in anyone of claims 1 to 9 when this aminoacid sequence is contacted with a serum of a patient infected with a HIV-2 retrovirus :

CACRODH

MetGlyAlaArgAsnSerValLeuArgGlyLysLysAlaAspGln

LeuGluArgIleArgLeuArgProGlyGlyLysLysLysTyrArg

LeuLysHisIleValTrpAlaAlaAsnLysLeuAspArgPheGly

100
LeuAlaGluSerLeuLeuGluSerLysGlnGlyCysGlnLysIle

LeuThrValLeuAspProMetValProThrGlySerGluAsnLeu

200
LysSerLeuPheAsnThrValCysValIleTrpCysIleHisAla

GluGluLysValLysAspThrGluGlyAlaLysGlnIleValArg

300
ArgHisLeuValAlaGluThrGlyThrAlaGluLysMetProSer

ThrSerArgProThrAlaProSerSerGluLysGlyGlyAsnTyr

400
ProValGlnHisValGlyGlyAsnTyrThrHisIleProLeuSer

ProArgThrLeuAsnAlaTrpValLysLeuValGluGluLysLys

PheGlyAlaGluValValProGlyPheGlnAlaLeuSerGluGly

500
CysThrProTyrAspIleAsnGlnMetLeuAsnCysValGlyAsp

HisGlnAlaAlaMetGlnIleIleArgGluIleIleAsnGluGlu

600
AlaAlaGluTrpAspValGlnHisProIleProGlyProLeuPro

AlaGlyGluLeuArgGluProArgGlySerAspIleAlaGlyThr

700
ThrSerThrValGluGluGlnIleGlyTrpMetPheArgProGln

AsnProValProValGlyAsnIleTyrArgArgTrpIleGlnIle

800
GlyLeuGlnLysCysValArgMetTyrAsnProThrAsnIleLeu

AspIleLysGlnGlyProLysGluProPheGlnSerTyrValAsp

900
ArgPheTyrLysSerLeuArgAlaGluGlnThrAspProAlaVal

LysAsnTrpMetThrGlnThrLeuLeuValGlnAsnAlaAsnPro

AspCysLysLeuValLeuLysGlyLeuGlyMetAsnProThrLeu

1000

GluGluMetLeuThrAlaCysGlnGlyValGlyGlyProGlyGln

LysAlaArgLeuMetAlaGluAlaLeuLysGlnValIleGlyPro

1100

AlaProIleProPheAlaAlaAlaGlnGlnArgLysAlaPheLys

CysTrpAsnCysGlyLysGluGlyHisSerAlaArgGlnCysArg

1200

AlaProArgArgGlnGlyCysTrpLysCysGlyLysProGlyHis

IleMetThrAsnCysProAspArgGlnAlaGlyPheLeuGlyLeu

1300

GlyProTrpGlyLysLysProArgAsnPheProValAlaGlnVal

ProGlnGlyLeuThrProThrAlaProProValAspProAlaVal

AspLeuLeuGluLysTyrMetGlnGlnGlyLysArgGlnArgGlu

1400

GlnArgGluArgProTyrLysGluValThrGluAspLeuLeuHis

LeuGluGlnGlyGluThrProTyrArgGluProProThrGluAsp

1500

LeuLeuHisLeuAsnSerLeuPheGlyLysAspGln

50. Nucleic acid according to claim 46, characterised in that it contains a nucleotidic sequence coding for part at least of the aminoacid sequence indicated hereafter, this aminoacid sequence being capable of giving a specific immunological reaction with antibodies against a HIV-2 retrovirus as defined in anyone of claims 1 to 9 when this aminoacid sequence is contacted with a serum of a patient infected with a HIV-2 retrovirus :

ArgLysAlaPheLys

CysTrpAsnCysGlyLysGluGlyHisSerAlaArgGlnCysArg

1200

AlaProArgArgGlnGlyCysTrpLysCysGlyLysProGlyHis

IleMetThrAsnCysProAspArgGlnAlaGlyPheLeuGlyLeu

1300

GlyProTrpGlyLysLysProArgAsnPheProValAlaGlnVal

ProGlnGlyLeuThrProThrAlaProProValAspProAlaVal

AspLeuLeuGluLysTyrMetGlnGlnGlyLysArgGlnArgGln

1400

GlnArgGlnArgProTyrLysGluValThrGluAspLeuLeuHis

LeuGluGlnGlyGluThrProTyrArgGlnProProThrGluAsp

1500

LeuLeuHisLeuAsnSerLeuPheGlyLysAspGln

51. Nucleic acid according to claim 46, characterised in that it contains a nucleotidic sequence coding for the totality or part of the aminoacid sequence indicated hereafter, the encoded aminoacid sequence having the capacity of giving a specific immunological reaction with antibodies against HIV-2 retrovirus as defined in anyone of claims 1 to 9 when it is contacted with a serum of a patient infected with a HIV-2 retrovirus :

MetGlyAlaArgAsnSerValLeuArgGlyLysLysAlaAspGln

LeuGluArgIleArgLeuArgProGlyGlyLysLysLysTyrArg

LeuLysHisIleValTrpAlaAlaAsnLysLeuAspArgPheGly

100

LeuAlaGluSerLeuLeuGluSerLysGluGlyCysGlnLysIle

LeuThrValLeuAspProMetValProThrGlySerGluAsnLeu

200

LysSerLeuPheAsnThrValCysValIleTrpCysIleHisAla

GluGlnLysValLysAsnThrGluGlyAlaLysGlnIleValArg

300

ArgHisLeuValAlaGluThrGlyThrAlaGluLysMetProSer

ThrSerArgProThrAlaProSerSerGluLysGlyGlyAsnTyr

400

52. Nucleic acid according to claim 46, characterised in that it contains a nucleotidic sequence coding for the totality or part of the aminoacid sequence indicated hereafter, the encoded aminoacid sequence having the capacity of giving a specific immunological reaction with antibodies against a HIV-2 retrovirus as defined in anyone of claims 1 to 9 when it is contacted with a serum of a patient infected specifically with a HIV-2 retrovirus.

ProValGlnHisValGlyGlyAsnTyrThrHisIleProLeuSer

ProArgThrLeuAsnAlaTrpValLysLeuValGluGluLysLys

PheGlyAlaGluValValProGlyPheGlnAlaLeuSerGluGly

500

CysThrProTyrAspIleAsnGlnMetLeuAsnCysValGlyAsp

IleGlnAlaAlaMetGlnIleIleArgGlnIleIleAsnGluGln

600
AlaAlaGluTrpAspValGlnHisProIleProGlyProLeuPro

AlaGlyGlnLeuArgGluProArgGlySerAspIleAlaGlyThr

700
ThrSerThrValGluGluGlnIleGluTrpMetPheArgProGln

AsnProValProValGlyAsnIleTyrArgArgTrpIleGlnIle

800
GlyLeuGlnLysCysValArgMetTyrAsnProThrAsnIleLeu

AspIleLysGlnGlyProLysGluProPheGlnSerTyrValAsp

900
ArgPheTyrLysSerLeuArgAlaGluGlnThrAspProAlaVal

LysAsnTrpMetThrGlnThrLeuLeuValGlnAsnAlaAsnPro

AspCysLysLeuValLeuLysGlyLeuGlyMetAsnProThrLeu

1000
GluGluMetLeuThrAlaCysGlnGlyValGlyGlyProGlyGln

LysAlaArgLeuMetAlaGluAlaLeuLysGluValIleGlyPro

1100
AlaProIleProPheAlaAlaAlaGlnGln

53. Nucleic acid according to claim 46, characterised in that it contains a nucleotidic sequence coding for the totality or part of the aminoacid sequence indicated hereafter, the encoded aminoacid sequence having the capacity of giving a specific immunological reaction with antibodies against HIV-2 retrovirus as defined in anyone of claims 1 to 9 when this aminoacid sequence is contacted with a serum of a patient infected with a HIV-2 retrovirus :

XXVII

MetMetAsnGlnLeuLeuIleAlaIleLeuLeuAlaSerAlaCys

LeuValTyrCysThrGlnTyrValThrValPheTyrGlyValPro

ThrTrpLysAsnAlaThrIleProLeuPheCysAlaThrArgAsn

100

ArgAspThrTrpGlyThrIleGlnCysLeuProAspAsnAspAsp

TyrGlnGluIleThrLeuAsnValThrGluAlaPheAspAlaTrp

200
AsnAsnThrValThrGlnGlnAlaIleGluAspValTrpHisLeu

PheGluThrSerIleLysProCysValLysLeuThrProLeuCys

300
ValAlaMetLysCysSerSerThrGluSerSerThrGlyAsnAsn

ThrThrSerLysSerThrSerThrThrThrThrThrProThrAsp

400
GlnGluGlnGluIleSerGluAspThrProCysAlaArgAlaAsp

AsnCysSerGlyLeuGlyGluGlnGluThrIleAsnCysGlnPhe

AsnMetThrGlyLeuGluArgAspLysLysLysGlnTyrAsnGlu

500
ThrTrpTyrSerLysAspValValCysGluThrAsnAsnSerThr

AsnGlnThrGlnCysTyrMetAsnHisCysAsnThrSerValIle

600
ThrGluSerCysAspLysHisTyrTrpAspAlaIleArgPheArg

TyrCysAlaProProGlyTyrAlaLeuLeuArgCysAsnAspThr

700
AsnTyrSerGlyPheAlaProAsnCysSerLysValValAlaSer

ThrCysThrArgMetMetGluThrGlnThrSerThrTrpPheGly

800
PheAsnGlyThrArgAlaGluAsnArgThrTyrIleTyrTrpHis

GlyArgAspAsnArgThrIleIleSerLeuAsnLysTyrTyrAsn

900
LeuSerLeuHisCysLysArgProGlyAsnLysThrValLysGln

IleMetLeuMetSerGlyHisValPheHisSerHisTyrGlnPro

IleAsnLysArgProArgGlnAlaTrpCysTrpPheLysGlyLys

1000
TrpLysAspAlaMetGlnGluValLysThrLeuAlaLysHisPro

90

ArgTyrArgGlyThrAsnAspThrArgAsnIleSerPheAlaAla

1100
ProGlyLysGlySerAspProGlnValAlaTyrMetTrpThrAsn

CysArgGlyGluPheLeuTyrCysAsnMetThrTrpPheLeuAsn

1200
TrpIleGluAsnLysThrHisArgAsnTyrAlaProCysHisIle

LysGlnIleIleAsnThrTrpHisLysValGlyArgAsnValTyr

1300
LeuProProArgGluGlyGluLeuSerCysAsnSerThrValThr

SerIleIleAlaAsnIleAspTrpGlnAsnAsnAsnGlnThrAsn

IleThrPheSerAlaGluValAlaGluLeuTyrArgLeuGluLeu

1400
GlyAspTyrLysLeuValGluIleThrProIleGlyPheAlaPro

ThrLysGluLysArgTyrSerSerAlaHisGlyArgHisThrArg

1500
GlyValPheValLeuGlyPheLeuGlyPheLeuAlaThrAlaGly

SerAlaMetGlyAlaArgAlaSerLeuThrValSerAlaGlnSer

1600
ArgThrLeuLeuAlaGlyIleValGlnGlnGlnGlnGlnLeuLeu

AspValValLysArgGlnGlnGluLeuLeuArgLeuThrValTrp

1700
GlyThrLysAsnLeuGlnAlaArgValThrAlaIleGluLysTyr

LeuGlnAspGlnAlaArgLeuAsnSerTrpGlyCysAlaPheArg

1800
GlnValCysHisThrThrValProTrpValAsnAspSerLeuAla

ProAspTrpAspAsnMetThrTrpGlnGluTrpGluLysGlnVal

ArgTyrLeuGluAlaAsnIleSerLysSerLeuGluGlnAlaGln

1900
IleGlnGlnGluLysAsnMetTyrGluLeuGlnLysLeuAsnSer

TrpAspIlePheGlyAsnTrpPheAspLeuThrSerTrpValLys

2000

```
TyrIleGlnTyrGlyValLeuIleIleValAlaValIleAlaLeu

ArgIleValIleTyrValValGlnMetLeuSerArgLeuArgLys

                              2100
GlyTyrArgProValPheSerSerProProGlyTyrIleGlnGln

IleLisIleLisLysAspArgGlyGlnProAlaAsnGluGlnThr

                              2200
GluGluAspGlyGlySerAsnGlyGlyAspArgTyrTrpProTrp

ProIleAlaTyrIleHisPheLeuIleArgGlnLeuIleArgLeu

LeuThrArgLeuTyrSerIleCysArgAspLeuLeuSerArgSer

2300
PheLeuThrLeuGlnLeuIleTyrGlnAsnLeuArgAspTrpLeu

ArgLeuArgThrAlaPheLeuGlnTyrGlyCysGluTrpIleGln

                2400
GluAlaPheGlnAlaAlaAlaArgAlaThrArgGluThrLeuAla

GlyAlaCysArgGlyLeuTrpArgValLeuGluArgIleGlyArg

                2500
GlyIleLeuAlaValProArgArgIleArgGlnGlyAlaGlnIle

AlaLeuLeu
```

54. Recombinant nucleic acid comprising a cDNA or part of this cDNA according to anyone of claims 46 to 53 and/or a RNA or part of a RNA corresponding to this cDNA inserted in a nucleic acid coming from a vector.

55. Recombinant nucleic acid according to claim 52, characterised in that it is labelled.

56. Process for the detection of a retrovirus capable of having a cytopathogenic effect for man, of the HIV-2 type or of its RNA, in a biological liquid or tissue especially for the in vitro diagnosis in man for the potentially or existence of LAS or AIDS, characterised by contacting nucleic acids contained in this biological liquid or tissue with a probe containing a nucleic acid according to anyone of claims 49 to 53 in stringent hybridization conditions, or containing a DNA presenting a sequence homologie of at least 70 %, preferably of at least 90 % with the RNA of a retrovirus according to anyone of claims 1 to 9 and capable of hybridizing with this one, in non stringent conditions with the « spot blot » technique the above contacting being realised for the time necessary for said hybridization to occur and being followed of the washing of the hybride formed, with a solution ensuring the preservation of said stringent conditions and the detection of the hybride formed.

57. A process for the production of a retrovirus capable of having a cytopathogenic effect for man, of the HIV-2 type characterised by culturing in human T4 lymphocytes or in permanent cell lines derived from said T4 lymphocytes and carrying the T4 phenotype, which lymphocytes or cell lines had previously been infected with an isolate of HIV-2 virus as defined in anyone of claims 1 to 9, particularly when the level of reverse transcriptase activity has reached a determined threshold, recovering and purifying the amounts of virus released in the culture medium of said lymphocytes or cell lines, particularly by differential centrifugation in a gradient of sucrose or metrizamide.

58. A process for the production of specific antigens of the HIV-2 retrovirus characterised by lysing, particularly by means of detergent, such as sodium dodecylsulfate for instance 0.1 % SDS in a RIPA buffer) of the purified virus obtained after realisation of the process according to claim 57 and recovering the lysate containing said antigens.

59. Process for the production of a hybridization probe for the detection of RNA of HIV-2 retrovirus characterised by the insertion of a DNA sequence, particularly according to anyone of claims 46 to 53, in a cloning vector, by in vitro recombination, cloning the modified vector obtained in a competent cellular host, and recovering the DNA-recombinants obtained.

60. The hybridoma secreting the monoclonal antibody according to claim 45.

61. Application of the polyclonal antibodies according to claim 44 or of the monoclonal antibodies according to claim 45, to the detection of HIV-2 retrovirus as defined in anyone of claims 1 to 9 in a biological preparation.

62. Application of the polyclonal antibodies according to claim 44 or of the monoclonal antibodies according to claim 45, to the detection of antigens of the HIV-2 retrovirus as defined in anyone of claims 1 to 9 in a biological preparation.

63. Application of the polyclonal antibodies according to claim 44 or of the monoclonal antibodies according to claim 45 to the purification of viral antigens of HIV-2 retrovirus as defined in anyone of claims 1 to 9.

**Claims** (for the Contracting States : AR, ES, GR)

1. HIV-2 human retrovirus, having the capacity of infecting human T4 lymphocytes and being capable of causing AIDS in man, this retrovirus being constituted of one of the retroviruses deposited with the CNCM under n° I-502, I-532, I-642 and I-643 and with the ECACC under n° 87.011001 and 87.011002 (corresponding to n° I-502 and I-532 respectively), or a variant of one of these retroviruses comprising all the antigens which are recognized by the antibodies raised against the corresponding antigens of anyone of the retroviruses having been deposited with the CNCM or with the ECACC as said above.

2. Human retrovirus according to claim 1, characterised in that its genomic RNA is capable of hybridizating under stringent conditions with the complementary chain of a cDNA or cDNA fragment derived from the genomic RNA of anyone of the retroviruses deposited with the CNCM under n° I-502, I-532, I-642 and I-643 and with the ECACC under n° 87.011001 and 87.011002 (corresponding to n° I 502 and I-532 respectively).

3. Human retrovirus according to claim 1, characterised in that it comprises a set of antigens formed by core proteins having molecular weights of the order or 12000, 16000 and 26000 daltons respectively, proteins or glycoproteins having molecular weights respectively of the order of 36000 daltons and of the order of 42000-45000 daltons and a major envelope glycoprotein having a molecular weight of the order of 130000-140000 daltons.

4. Human purified retrovirus according to claim 1, pathogenic for man and capable of causing AIDS, characterised by the whole following properties :

— the preferred target for the HIV-2 retrovirus consists of human Leu 3 cells (or T4 lymphocytes) and for permanent cell lines derived of said T4 lymphocytes ;

— it is capable of having a cytopathogenic effect for the human T4 lymphocytes which it infects ;

— it has a reverse transcriptase activity which requires the presence of $Mg^{2+}$ ions and has a strong affinity for polyadenylate oligodeocythymidylate (Lpoly(A)-oligo(dT)12-18) ;

— it has a density of approximately 1.16 in a sucrose gradient ;

— it has a mean diameter of 140 nanometres and a core having mean diameter of 41 nanometres ;

— it can be cultivated in permanent cell lines expressing the T4 protein ;

— It is not infectious in T8 lymphocytes ;

— the lysates of this virus contain p26 protein which does not crossreact immunologically with p24 protein of the HTLV-1 virus or of the HTLV-2 ;

— said lysates further contain p16 protein which is not recognized immunologically by p19 protein of HTLV-1 or of HTLV-2 in radioimmunoprecipitation assays ;

— said lysates further contain an envelope glycoprotein having a molecular weight of the order of 130,000-140,000 daltons which does not crossreact immunologically with gp110 of HTLV-1 retrovirus ;

— said lysates further contain a protein or a glycoprotein which can be labelled by $^{35}$S-cystein, having a molecular weight of the order of 36,000 daltons ;

— the genomic RNA of HIV-2 does not hybridize under stringent conditions with the genomic RNA of HIV-1 and does not hybridize under non-stringent conditions either with the <u>env</u> gene, or with the LTRs of HIV-1 ;

— the genomic RNA of HIV-2 hybridizes weakly under non-stringent conditions with nucleotide sequences comprising on the one hand nucleotides 990-1070 and on the other hand nucleotides 990-1260 of the <u>gag</u> region of the HIV-1 genome.

5. Retrovirus according to anyone of claims 1 to 4, characterised in that it does not multiply in chronic fashion in the lymphocytes of macaques rhesus monkeys, when it has been injected in vivo and in working conditions which permit the development of the $STLVIII_{mac}$ virus, as have been described by Letvin et al., Science (1985) vol. 230, 71-75.

6. The retrovirus of any of claims 1 to 5, characterised in that the sequence comprising the R region and the U3 region of the sequence comprising its genomic RNA also contains a nucleotidic sequence which corresponds to the following nucleotide sequence :

GTGGAAGGCGAGACTGAAAGCAAGAGGAATACCATTTAGTTAAAGGACAG

GAACAGCTATACTTGGTCAGGGCAGGAAGTAACTAACAGAAACAGCTGAG

ACTGCAGGGACTTTCCAGAAGGGGCTGTAACCAAGGGAGGGACATGGGAG

GAGCTGGTGGGGAACGCCTCATATTCTCTGTATAATATACCCGCTGCTTG

CATTGTACTTCAGTCGCTCTGCGGAGAGGCTGGCAGATTGAGCCCTGGAG

GATCTCTCCAGCACTAGACGGATGAGCCTGGGTGCCCTGCTAGACTCTCA

CCAGCACTTGGCCGGTGCTGGCAGACGGCCCCACGCTTGCCTGCTTAAAA

ACCTTCCTTAATAAAGCTGCAGTAGAAGCA

7. Retrovirus according to anyone of claims 1 to 5 characterised in that its genomic RNA contains a gag sequence corresponding to the following nucleotide sequence :

GAGRODH .

ATGGGCGCCAGAAACTCCGTCTTGAGAGGGAAAAAAGCAGATGAA

TTAGAAAGAATCAGGTTACGGCCCCGGCGGAAAGAAAAAGTACAGG

CTAAAACATATTGTGTGGGCACCGAATAAAATTGGACAGATTCGGA
100

TTAGCAGAGAGCCTGTTGGAGTCAAAAGAGGGTTGTCAAAAAATT

CTTACAGTTTTAGATCCAATGGTACCGACAGCTTCAGAAAATTTA
200

AAAAGTCTTTTTAATACTGTCTGCGTCATTTGGTGCATACACGCA

GAAGAGAAAGTGAAAGATACTGAAGGAGCAAAACAAATAGTGCGG
300

AGACATCTAGTGGCAGAAACAGGAACTGCAGAGAAAATGCCAACC

ACAAGTAGACCAACAGCACCATCTAGCGAGAAGGGAGGAAATTAC
400

CCAGTGCAACATGTAGGCCGGCAACTACACCCATATACCGCTGACT

CCCCGAACCCTAAATGCCTGGGTAAAATTAGTAGAGGAAAAAAAG

TTCGGGGCAGAAGTAGTGCCAGGATTTCAGGCACTCTCAGAAGGC
500

TGCACGCCCTATGATATCAACCAAATGCTTAATTGTGTGGGCGAC

CATCAAGCAGCCATGCAGATAATCAGGGAGATTATCAATGAGGAA
600

GCAGCAGAATGGGATGTGCAACATCCAATACCAGCCCCCTTACCA

GCGGGGCAGCTTAGAGAGCCAAGGGGATCTGACATAGCAGGGACA
700

ACAAGCACACTACAAGAACACAGATCCAGTCGATGTTTAGGCCACAA

94

AATCCTGTACCAGTAGGAAACATCTATAGAAGATGGATCCAGATA
800

GGATTGCAGAAGTGTGTCAGGATGTACAACCCGACCAACATCCTA

CACATAAAACAGGGACCAAAGGAGCCGTTCCAAAGCTATCTAGAT
900

AGATTCTACAAAAGCTTGAGGGCAGAACAAACAGATCCAGCAGTG

AAGAATTGGATGACCCAAACACTGCTAGTACAAAATGCCAACCCA

GACTGTAAATTAGTGCTAAAGGACTACGGATGAACCCTACCTTA
1000

GAAGAGATGCTGACCGCCTGTCAGGGGGTAGGTGGGCCAGGCCAG

AAAGCTAGATTAATGGCAGAGGCCCTGAAAGAGGTCATAGGACCT
1100

GCCCCTATCCCATTCGCAGCAGCCCAGCAGAGAAAGGCATTTAAA

TGCTGGAACTGTGGAAAGGAAGGGCACTCGGCAAGACAATGCCGA
1200

GCACCTAGAAGGCAGGGCTGCTGGAAGTGTGGTAAGCCAGGACAC

ATCATGACAAACTGCCCAGATAGACAGGCAGGTTTTTTAGGACTG
1300

GCCCCTTGGGGAAAGAAGCCCCGCAACTTCCCCGTGGCCCAAGTT

CCGCAGGGGCTGACACCAACAGCACCCCCAGTGGATCCAGCAGTG

GATCTACTGGAGAAATATATGCAGCAAGGGAAAAGACAGAGAGAG
1400

CAGAGAGAGAGACCATACAAGGAAGTGACAGAGGACTTACTGCAC

CTCGAGCAGGGGGAGACACCATACAGGGAGCCACCAACAGAGGAC
1500

TTGCTGCACCTCAATTCTCTCTTTGGAAAAGACCAG

8. Retrovirus according to anyone of claims 1 to 6 characterised in that its genomic RNA contains a _env_ sequence corresponding to the following nucleotide sequence :

ENVRE

ATGATGAATCAGCTGCTTATTGCCATTTTATTAGCTAGTGCTTGC

TTAGTATATTGCACCCAATATGTAACTGTTTTCTATGGCGTACCC

```
ACGTGGAAAAATGCAACCATTCCCCTCTTTGTGCAACCAGAAAT
            100

ACGGATACTTGGGGAACCATACAGTGCTTGCCTGACAATGATGAT

TATCAGGAAATAACTTTGAATGTAACAGAGGCTTTTGATGCATGG
                   200

AATAATACAGTAACAGAACAAGCAATAGAAGATGTCTGGCATCTA

TTCGAGACATCAATAAAACCATGTGTCAAACTAACACCTTTATGT
                        300

CTAGCAATGAAATGCAGCAGCACAGAGAGCAGCACAGGGAACAAC

ACAACCTCAAAGAGCACAAGCACAACCACAACCACACCCACAGAC
                                       400

CAGGAGCAAGAGATAAGTGAGGATACTCCATGCGCACGCGCAGAC

AACTGCTCAGGATTGGGAGAGGAAGAAACGATCAATTGCCAGTTC

AATATGACAGGATTAGAAAGAGATAAGAAAAAACAGTATAATGAA
            500

ACATGGTACTCAAAAGATGTGGTTTGTGAGACAAATAATAGCACA

AATCAGACCCAGTGTTACATGAACCATTGCAACACATCAGTCATC
                   600

ACAGAATCATGTGACAAGCACTATTGGGATGCTATAAGGTTTAGA

TACTGTGCACCACCGGGTTATGCCCTATTAAGATGTAATGATACC
                         700

AATTATTCACGCCTTTGCACCCAACTGTTCTAAAGTACTAGCTTCT

ACATGCACCAGGATGATGGAAACGCAAACTTCCACATGGTTTGGC
                                        800

TTTAATGGCACTAGAGCAGAGAATAGAACATATATCTATTGGCAT

GGCAGAGATAATAGAACTATCATCAGCTTAAACAAATATTATAAT
                                              900

CTCAGTTTGCATTGTAAGAGGCCAGGGAATAAGACAGTGAAACAA

ATAATGCTTATGTCAGGACATGTGTTTCACTCCCACTACCAGCCG

ATCAATAAAAGACCCAGACAAGCATGGTGCTGGTTCAAAGGCAAA
            1000
```

96

TGCAAAGACGCCATGCAGGAGGTGAAGACCCTTGCAAAACATCCC

AGGTATAGAGGAACCAATGACACAAGGAATATTAGCTTTGCAGCG
1100

CCAGGAAAAGGCTCAGACCCAGAAGTAGCATACATGTGGACTAAC

TGCAGAGGAGAGTTTCTCTACTGCAACATGACTTGGTTCCTCAAT
1200

TGGATAGAGAATAAGACACACCGCAATTATGCACCGTGCCATATA

AAGCAAATAATTAACACATGGCATAAGGTAGGGAGAAATGTATAT
1300

TTGCCTCCCAGGGAAGGGGAGCTGTCCTGCAACTCAACAGTAACC

AGCATAATTGCTAACATTGACTGGCAAAACAATAATCAGACAAAC

ATTACCTTTAGTGCAGAGGTGGCAGAACTATACAGATTGGAGTTG
1400

CCAGATTATAAATTGGTAGAAATAACACCAATTGGCTTCGCACCT

ACAAAAGAAAAAGATACTCCTCTGCTCACGGGAGACATACAAGA
1500

GGTGTGTTCGTGCTAGGGTTCTTGGGTTTTCTCGCAACAGCAGGT

TCTGCAATGGGCGCTCGAGCGTCCCTGACCGTGTCGGCTCAGTCC
1600

CGGACTTTACTGGCCGGGATAGTGCAGCAACAGCAACAGCTGTTG

GACGTGGTCAAGAGACAACAAGAACTGTTGCGACTGACCGTCTGG
1700

GGAACGAAAAACCTCCAGGCAAGAGTCACTGCTATAGAGAAGTAC

CTACAGGACCAGGCGCGGCTAAATTCATGGGGATGTGCGTTTAGA
1800

CAAGTCTGCCACACTACTGTACCATGGGTTAATGATTCCTTAGCA

CCTGACTGGGACAATATGACGTGGCAGGAATGGGAAAAACAAGTC

CGCTACCTGGAGGCAAATATCAGTAAAAGTTTAGAACAGGCACAA
1900

ATTCAGCAAGAGAAAAATATGTATGAACTACAAAAATTAAATAGC

```
TCGGATATTTTTGGCAATTGGTTTGACTTAACCTCCTGGGTCAAG
              2000              .        .     .

TATATTCAATATGGAGTGCTTATAATAGTAGCAGTAATAGCTTTA
    .        .        .        .        .     .

AGAATAGTGATATATGTAGTACAAATGTTAAGTAGGCTTAGAAAG
    .        .        2100              .

GGCTATAGGCCTGTTTTCTCTTCCCCCCCCGGTTATATCCAACAG

ATCCATATCCACAAGGACCGGGGACAGCCAGCCAACGAAGAAACA
    .        .        .        2200

GAAGAAGACGGTGGAAGCAACGGTGGAGACAGATACTGGCCCTGG
    .   .        .        .        .        .

GCGATAGCATATATACATTTCCTGATCCGCCAGCTGATTCGCCTC
        .        .        .        .

TTGACCAGACTATACAGCATCTGCAGGGACTTACTATCCAGGAGC
    2300        .        .        .        .

TTCCTGAUCCTCCAACTCATCTACCAGAATCTCAGAGACTGGCTG
        .        .        .        .

AGACTTAGAACAGCCTTCTTGCAATATGGGTGCGAGTGGATCCAA
    .        2400              .        .     .

GAAGCATTCCAGGCCGCCGCGAGGGCTACAAGAGAGACTCTTGCG
        .        .        .        .

GGCGCGTGCAGGGGCTTGTGGAGGGTATTGGAACGAATCGGGAGG ·
        .        .        2500        .        .

GGAATACTCGCGGTTCCAAGAAGGATCAGACAGGGAGCAGAAATC
    .    .        .        .        .        .

GCCCTCCTG
```

9. Retrovirus according to anyone of claims 1 to 8, characterised in that its RNA does not hybridize under stringent conditions with the genomic RNA of HIV-1 and does not hybridize under non-stringent conditions, with the _env_ gene and the LTR close to it, more particularly with the nucleotidic sequence 5290-9130 of HIV-1 and does not hybridize under non-stringent conditions with sequences of the _pol_ region of the HIV-1 genome, particularly with the nucleotide sequence 2170-2240.

10. A process for the production of specific antigens of the HIV-2 retrovirus characterised by the lysis, particularly by means of detergent, (such as sodium dodecylsulfate for instance 0.1 % SDS in a RIPA buffer) of the purified HIV-2 rétrovirus according to claims 1 to 9 the recovery the lysate containing said antigens.

11. Process according to claim 10, comprising further :
— the separation from the lysate and the purification of one of the antigens consisting of one of the proteins or glycoproteins p12, p16, gp36, p43, gp140.

12. Process according to claim 11, characterised in that the antigen having the immunological characteristics of the HIV-2 p12 protein, recognized by antibodies formed against a retrovirus according to anyone of claims 1 to 9 is separated and purified, this antigen having a molecular weight of the order of 12000 daltons.

13. Process according to claim 11, characterised in that the antigen having the immunological characteristics of the HIV-2 p16 protein, recognized by antibodies formed against a retrovirus according to anyone of claims 1 to 9 is separated and purified, this antigen having a molecular weight of the order of 16000 daltons.

14. Process according to claim 11, characterised in that the antigen having the immunological characteristics of the HIV-2 p26 protein, recognized by antibodies formed against a retrovirus according to anyone of claims 1 to 9 is separated and purified, this antigen having a molecular weight of the order of 26000 daltons.

15. Process according to claim 11, characterised in that the antigen having the immunological characteristics of the HIV-2 gp36 protein, recognized by antibodies formed against a retrovirus according to anyone of claims 1 to 9 is separated and purified, this antigen having a molecular weight of the order of 36000 daltons.

16. Process according to claim 11, characterised in that the antigen having the immunological characteristics of the HIV-2 p42 protein, recognized by antibodies formed against a retrovirus according to anyone of claims 1 to 9 is separated and purified, this antigen having a molecular weight of the order of 42000-45000 daltons.

17. Process according to claim 11, characterised in that the antigen having the immunological characteristics of the HIV-2 gp140 protein, recognized by antibodies formed against a retrovirus according to anyone of claims 1 to 9 is separated and purified, this antigen having a molecular weight of the order of 130000-140000 daltons.

18. Process according to claim 11, characterised in that the antigen separated and purified has the following aminoacid sequence or a part of said sequence, providing that it raises a specific immunological reaction with antibodies against a HIV-2 retrovirus, according to anyone of claims 1 to 9, especially when this antigen is contacted with the serum of a patient infected with HIV-2.

ArgLysAlaPheLys

CysTrpAsnCysGlyLysGluGlyHisSerAlaArgGlnCysArg

1200
AlaProArgArgGlnGlyCysTrpLysCysGlyLysProGlyHis

IleMetThrAsnCysProAspArgGlnAlaGlyPheLeuGlyLeu

1300
GlyProTrpGlyLysLysProArgAsnPheProValAlaGlnVal

ProGlnGlyLeuThrProThrAlaProProValAspProAlaVal

AspLeuLeuGluLysTyrMetGlnGlnGlyLysArgGlnArgGlu

1400
GlnArgGluArgProTyrLysGluValThrGluAspLeuLeuHis

LeuGluGlnGlyGluThrProTyrArgGluProProThrGluAsp

1500
LeuLeuHisLeuAsnSerLeuPheGlyLysAspGln

19. Process according to claim 11, characterised in that the antigen separated and purified has the following aminoacid sequence or a part of said sequence, providing that it raises a specific immunological reaction with the antibodies against a HIV-2 retrovirus, according to anyone of claims 1 to 9, especially when this antigen is contacted with the serum of a patient infected with HIV-2.

MetGlyAlaArgAsnSerValLeuArgGlyLysLysAlaAspGln

LeuGluArgIleArgLeuArgProGlyGlyLysLysLysTyrArg

LeuLysHisIleValTrpAlaAlaAsnLysLeuAspArgPheGly

100
LeuAlaGluSerLeuLeuGluSerLysGluGlyCysGlnLysIle

99

LeuThrValLeuAspProMetValProThrGlySerGluAsnLeu

    200

LysSerLeuPheAsnThrValCysValIleTrpCysIleHisAla

GlnGlnLysValLysAspThrGluGlyAlaLysGlnIleValArg

    300

ArgHisLeuValAlaGluThrGlyThrAlaGluLysMetProSer

ThrSerArgProThrAlaProSerSerGluLysGlyGlyAsnTyr

    400

20. Process according to claim 11, characterised in that the antigen separated and purified has the following aminoacid sequence or a part of said sequence, providing that it raises a specific immunological reaction with the antibodies against a HIV-2 retrovirus, according to anyone of claims 1 to 9, especially when this antigen is contacted with the serum of a patient infect with HIV-2 :

ProValGlnHisValGlyGlyAsnTyrThrHisIleProLeuSer

ProArgThrLeuAsnAlaTrpValLysLeuValGluGluLysLys

PheGlyAlaGluValValProGlyPheGlnAlaLeuSerGluGly

    500

CysThrProTyrAspIleAsnGlnMetLeuAsnCysValGlyAsp

HisGlnAlaAlaMetGlnIleIleArgGluIleIleAsnGluGlu

    600

AlaAlaGluTrpAspValGlnHisProIleProGlyProLeuPro

AlaGlyGlnLeuArgGluProArgGlySerAspIleAlaGlyThr

    700

ThrSerThrValGluGluGlnIleGlnTrpMetPheArgProGln

AsnProValProValGlyAsnIleTyrArgArgTrpIleGlnIle

    800

GlyLeuGlnLysCysValArgMetTyrAsnProThrAsnIleLeu

AspIleLysGlnGlyProLysGluProPheGlnSerTyrValAsp

    900

ArgPheTyrLysSerLeuArgAlaGluGlnThrAspProAlaVal

LysAsnTrpMetThrGlnThrLeuLeuValGlnAsnAlaAsnPro

AspCysLysLeuValLeuLysGlyLeuGlyMetAsnProThrLeu

    1000

GluGlnMetLeuThrAlaCysGlnGlyValGlyGlyProGlyGln

LysAlaArgLeuMetAlaGluAlaLeuLysGluValIleGlyPro

1100

AlaProIleProPheAlaAlaAlaGlnGln

21. Process according to claim 11, characterised in that the antigen separated and purified has the following aminoacid sequence or a part of said sequence, providing that it raises a specific immunological reaction with the anibodies against a HIV-2 retrovirus, according to anyone of claims 1 to 9, especially when this antigen is contacted with the serum of a patient infected with HIV-2 :

ENV2N

MetMetAsnGlnLeuLeuIleAlaIleLeuLeuAlaSerAlaCys

LeuValTyrCysThrGlnTyrValThrValPheTyrGlyValPro

ThrTrpLysAsnAlaThrIleProLeuPheCysAlaThrArgAsn

100

ArgAspThrTrpGlyThrIleGlnCysLeuProAspAsnAspAsp

TyrGlnGluIleThrLeuAsnValThrGluAlaPheAspAlaTrp

200

AsnAsnThrValThrGluGlnAlaIleGluAspValTrpHisLeu

PheGluThrSerIleLysProCysValLysLeuThrProLeuCys

300

ValAlaMetLysCysSerSerThrGluSerSerThrGlyAsnAsn

ThrThrSerLysSerThrSerThrThrThrThrThrProThrAsp

400

GlnGluGlnGluIleSerGluAspThrProCysAlaArgAlaAsp

AsnCysSerGlyLeuGlyGluGluGluThrIleAsnCysGlnPhe

AsnMetThrGlyLeuGluArgAspLysLysLysGlnTyrAsnGlu

500

ThrTrpTyrSerLysAspValValCysGluThrAsnAsnSerThr

AsnGlnThrGlnCysTyrMetAsnHisCysAsnThrSerValIle

600

ThrGluSerCysAspLysHisTyrTrpAspAlaIleArgPheArg

TyrCysAlaProProGlyTyrAlaLeuLeuArgCysAsnAspThr

700

AsnTyrSerGlyPheAlaProAsnCysSerLysValValAlaSer

ThrCysThrArgMetMetGluThrGlnThrSerThrTrpPheGly

800
PheAsnGlyThrArgAlaGluAsnArgThrTyrIleTyrTrpHis

GlyArgAspAsnArgThrIleIleSerLeuAsnLysTyrTyrAsn

900
LeuSerLeuHisCysLysArgProGlyAsnLysThrValLysGln

IleMetLeuMetSerGlyHisValPheHisSerHisTyrGlnPro

IleAsnLysArgProArgGlnAlaTrpCysTrpPheLysGlyLys

1000
TrpLysAspAlaMetGlnGluValLysThrLeuAlaLysHisPro

ArgTyrArgGlyThrAsnAspThrArgAsnIleSerPheAlaAla

1100
ProGlyLysGlySerAspProGluValAlaTyrMetTrpThrAsn

CysArgGlyGluPheLeuTyrCysAsnMetThrTrpPheLeuAsn

1200
TrpIleGluAsnLysThrHisArgAsnTyrAlaProCysHisIle

LysGlnIleIleAsnThrTrpHisLysValGlyArgAsnValTyr

1300
LeuProProArgGluGlyGluLeuSerCysAsnSerThrValThr

SerIleIleAlaAsnIleAspTrpGlnAsnAsnAsnGlnThrAsn

IleThrPheSerAlaGluValAlaGluLeuTyrArgLeuGluLeu

1400
GlyAspTyrLysLeuValGluIleThrProIleGlyPheAlaPro

ThrLysGluLysArgTyrSerSerAlaHisGlyArgHisThrArg

1500
GlyValPheValLeuGlyPheLeuGlyPheLeuAlaThrAlaGly

SerAlaMetGlyAlaArgAlaSerLeuThrValSerAlaGlnSer

1600
ArgThrLeuLeuAlaGlyIleValGlnGlnGlnGlnGlnLeuLeu

AspValValLysArgGlnGlnGluLeuLeuArgLeuThrValTrp

```
                                    1700
GlyThrLysAsnLeuGlnAlaArgValThrAlaIleGluLysTyr

LeuGlnAspGlnAlaArgLeuAsnSerTrpGlyCysAlaPheArg
                                    1800
GlnValCysHisThrThrValProTrpValAsnAspSerLeuAla

ProAspTrpAspAsnMetThrTrpGlnGluTrpGluLysGlnVal

ArgTyrLeuGluAlaAsnIleSerLysSerLeuGluGlnAlaGln
    1900
IleGlnGlnGluLysAsnMetTyrGluLeuGlnLysLeuAsnSer

TrpAspIlePheGlyAsnTrpPheAspLeuThrSerTrpValLys
              2000
TyrIleGlnTyrGlyValLeuIleIleValAlaValIleAlaLeu

ArgIleValIleTyrValValGlnMetLeuSerArgLeuArgLys
                    2100
GlyTyrArgProValPheSerSerProProGlyTyrIleGlnGln

IleHisIleHisLysAspArgGlyGlnProAlaAsnGluGluThr

GluGlnAspGlyGlySerAsnGlyGlyAspArgTyrTrpProTrp
                            2200
ProIleAlaTyrIleHisPheLeuIleArgGlnLeuIleArgLeu

LeuThrArgLeuTyrSerIleCysArgAspLeuIleSerArgSer
2300
PheLeuThrLeuGlnLeuIleTyrGlnAsnLeuArgAspTrpLeu

ArgLeuArgThrAlaPheLeuGlnTyrGlyCysGluTrpIleGln
        2400
GluAlaPheGlnAlaAlaAlaArgAlaThrArgGluThrLeuAla

GlyAlaCysArgGlyLeuTrpArgValLeuGlnArgIleGlyArg
                    2500
GlyIleLeuAlaValProArgArgIleArgGlnGlyAlaGluIle

AlaLeuLeu
```

22. Process for the in vitro detection of the presence of antibodies, induced in man infected with a human HIV-2 retrovirus, in a human biological sample such as a serum and more particularly for the in vitro diagnosis of a potential or existing LAS or AIDS due to such a retrovirus and obtained from the person being diagnosed, characterised in that this biological sample is contacted with an antigen recognized by an antibody induced in the infected mean, by a human HIV-2 retrovirus as defined in claims 1 to 9 in conditions authorizing the formation of an immunological complex between this antigen and said antibody and in that the possibly immunological conjugate formed between this antibody and the antigen used is detected.

23. Method according to claim 22, characterised in that the biological sample is contacted with an antigen obtained by a process according to anyone of claims 12 to 21 in conditions authorizing the formation of an immunological complex between this antigen and said antibodies and in that the immunological conjugate possibly formed between these anti-HIV-2 antibodies and the antigen used is detected.

24. Method according to claim 23, characterised in that said immunological conjugate is detected by a reaction between the possibly formed immunological conjugate and a labelled reagent formed either by anti-human immunoglobulin-antibodies either by bacterial A protein and in that the complex formed between the reagent and the above immunological conjugate is detected.

25. Method for the in vitro detection of the presence of antibodies recognizing antigens of a human retrovirus capable of having a cytopathogenic effect for the T4 human lymphocytes in a human biological sample like a serum and more particularly for the in vitro diagnostis of a potential or existing LAS or AIDS caused by a human retrovirus, characterised in that this biological sample obtained from the person being diagnosed, is contacted with a mixture of antigens capable of giving a specific immunological reaction with the antibodies against, on the one hand a human HIV-2 retrovirus capable of having a cytopathogenic effect, as defined in anyone of claims 1 to 9 on the other hand a human HIV-1 retrovirus and in that the immunological conjugate possibly formed between at least one of these antigens and said antibodies is detected.

26. Method according to claim 25, characterised in that the biological sample obtained from the person being diagnosed is contacted with a mixture of antigens obtained both from a HIV-1 and a HIV-2, according to anyone of claims 1 to 9, especially with antigens obtained by the process according to anyone of claims 11 to 21 and HIV-1 antigens obtained by the same process, except that it is applied to a HIV-1 retrovirus and in that the immunological conjugate possibly formed between at least one of these antigens and said antibodies is detected.

27. Process for the obtention of antibodies specifically recognizing an antigen of a HIV-2 retrovirus comprising :
— the immunization of an animal with a HIV-2 retrovirus according to anyone of claims 1 to 9,
— the recovery of the antibodies formed.

28. Process for the production of a nucleic acid or of a nucleic acid fragment derived from the RNA of a HIV-2 retrovirus according to anyone of claims 1 to 9, comprising :
— the ultracentrifugation of the supernanant of a cells culture infected with an isolate of a HIV-2 retrovirus according to anyone of claims 1 to 9 and the recovery of the centrifugation pellet,
— when necessary the centrifugation of the pellet recovered on a sucrose gradient by the method described in EP-A-0138667 and the recovery of the sedimented pellet containing the genomic RNA,
— the synthesis of the initiator cDNA chain in contact with the RNA of the virus which is purified and sedimented in the presence of an oligo (dT) initiator, in an endogen reaction which is activated by a detergent, according to the technique described in Nature 312 :575-760 (1984)
— when necessary the purification of the RNA-cDNA hybrids by an extraction with a mixture of phenolchloroform, and precipitation with ethanol,
— the manufacture of a second cDNA chain in the presence of DNA polymerase I, RNAseH and of the 4 desoxynucleotides, according to the method described in gene 25 : 263-269 (1983), if necessary after extraction of the proteins present in the reaction mixture,
— the recovery of the nucleic acid obtained.

29. Process according to claim 28, characterised in that a nucleic acid is recovered being labelled if necessary, derived of at least a part of the RNA of the HIV-2 retrovirus as defined in anyone of claims 1 to 9, which hybridises with the RNA of said HIV-2 virus in stringent conditions, and not hybridizing in stringent conditions with the probes 1 to 4 respectively comprising the nucleotides 990-1070, 990-1260, 2170-2240, 3370-3640 of the DNA derived from the HIV-1 genome.

30. Process according to claim 29 characterised in that a nucleic acid is recovered which contains at least a part of a cDNA sequence capable of hybridising with the total genomic RNA of a HIV-2 retrovirus according to anyone of claims 1 to 9 and not hybridising with the probes whose DNA fragments derived from the HIV-1 genome are respectively constituted by fragments delimitated by the nucleotides 990-1070, 990-1260, 2170-2240, 3370-3640 of the DNA derived from the HIV-1 genome.

31. Process according to claim 29, characterised in that a nucleic acid is recovered which contains the nucleotidic sequence :

GTGGAAGGCGAGACTGAAAGCAAGAGGAATACCATTTAGTTAAAGGACAG

GAACAGCTATACTTGGTCAGGGCAGGAAGTAACTAACAGAAACAGCTGAG

ACTGCAGGGACTTTCCAGAAGGGGCTGTAACCAAGGGAGGGACATGGGAG

GAGCTGGTGGGGAACGCCTCATATTCTCTGTATAATATACCCGCTGCTTG
CATTGTACTTCAGTCGCTCTGCGGAGAGGCTGGCAGATTGAGCCCTGGAG
GATCTCTCCAGCACTAGACGGATGAGCCTGGGTGCCCTGCTAGACTCTCA
CCAGCACTTGGCCGGTGCTGGCAGACGGCCCCACGCTTGCCTGCTTAAAA
ACCTTCCTTAATAAAGCTGCAGTAGAAGCA

32. Process according to claim 29, characterised in that a nucleic acid is recovered, which contains a nucleotidic sequence coding for all or part of the aminoacid sequence indicated hereafter, this sequence or part of aminoacid sequence having the capacity of giving a specific immunological reaction with antibodies against a HIV-2 retrovirus as defined in anyone of claims 1 to 9 when this aminoacid sequence is contacted with a serum of a patient infected with a HIV-2 retrovirus :

GAGRODH :

MetGlyAlaArgAsnSerValLeuArgGlyLysLysAlaAspGlu

LeuGluArgIleArgLeuArgProGlyGlyLysLysLysTyrArg

LeuLysHisIleValTrpAlaAlaAsnLysLeuAspArgPheGly

100
LeuAlaGlySerLeuLeuGluSerLysGluGlyCysGlnLysIle

LeuThrValLeuAspProMetValProThrGlySerGluAsnLeu

200
LysSerLeuPheAsnThrValCysValIleTrpCysIleHisAla

GluGluLysValLysAspThrGluGlyAlaLysGlnIleValArg

300
ArgHisLeuValAlaGluThrGlyThrAlaGluLysMetProSer

ThrSerArgProThrAlaProSerSerGluLysGlyGlyAsnTyr

400
ProValGlnHisValGlyGlyAsnTyrThrHisIleProLeuSer

ProArgThrLeuAsnAlaTrpValLysLeuValGluGluLysLys

PheGlyAlaGluValValProGlyPheGlnAlaLeuSerGluGly

500
CysThrProTyrAspIleAsnGlnMetLeuAsnCysValGlyAsp

HisGlnAlaAlaMetGlnIleIleArgGluIleIleAsnGluGlu

600
AlaAlaGluTrpAspValGlnHisProIleProGlyProLeuPro

AlaGlyGluLeuArgGluProArgGlySerAspIleAlaGlyThr

ThrSerThrValGluGluGlnIleGlnTrpMetPheArgProGln
700

AsnProValProValGlyAsnIleTyrArgArgTrpIleGlnIle

GlyLeuGlnLysCysValArgMetTyrAsnProThrAsnIleLeu
800

AspIleLysGlnGlyProLysGluProPheGlnSerTyrValAsp

ArgPheTyrLysSerLeuArgAlaGluGlnThrAspProAlaVal
900

LysAsnTrpMetThrGlnThrLeuLeuValGlnAsnAlaAsnPro

AspCysLysLeuValLeuLysGlyLeuGlyMetAsnProThrLeu

GluGluMetLeuThrAlaCysGlnGlyValGlyGlyProGlyGln
1000

LysAlaArgLeuMetAlaGluAlaLeuLysGluValIleGlyPro

AlaProIleProPheAlaAlaAlaGlnGlnArgLysAlaPheLys
1100

CysTrpAsnCysGlyLysGluGlyHisSerAlaArgGlnCysArg

AlaProArgArgGlnGlyCysTrpLysCysGlyLysProGlyHis
1200

IleMetThrAsnCysProAspArgGlnAlaGlyPheLeuGlyLeu

GlyProTrpGlyLysLysProArgAsnPheProValAlaGlnVal
1300

ProGlnGlyLeuThrProThrAlaProProValAspProAlaVal

AspLeuLeuGluLysTyrMetGlnGlnGlyLysArgGlnArgGlu

GlnArgGluArgProTyrLysGluValThrGluAspLeuLeuHis
1400

LeuGluGlnGlyGluThrProTyrArgGluProProThrGluAsp

LeuLeuHisLeuAsnSerLeuPheGlyLysAspGln
1500

33. Process according to claim 29, characterised in that a nucleic acid is recovered, which contains a nucleotidic sequence coding for part at least of the aminoacid sequence indicated hereafter, this aminoacid sequence being capable of giving a specific immunological reaction with antibodies against a HIV-2 retrovirus as defined in anyone of claims 1 to 9 when this aminoacid sequence is contacted with a serum of a patient infected with a HIV-2 retrovirus :

```
                                          ArgLysAlaPheLys


    CysTrpAsnCysGlyLysGluGlyHisSerAlaArgGlnCysArg

                                1200
    AlaProArgArgGlnGlyCysTrpLysCysGlyLysProGlyHis

    IleMetThrAsnCysProAspArgGlnAlaGlyPheLeuGlyLeu

                                      1300
    GlyProTrpGlyLysLysProArgAsnPheProValAlaGlnVal

    ProGlnGlyLeuThrProThrAlaProProValAspProAlaVal

    AspLeuLeuGluLysTyrMetGlnGlnGlyLysArgGlnArgGlu

    1400
    GlnArgGluArgProTyrLysGluValThrGluAspLeuLeuHis

    LeuGluGlnGlyGluThrProTyrArgGluProProThrGluAsp

                  1500
    LeuLeuHisLeuAsnSerLeuPheGlyLysAspGln
```

34. Process according to claim 29, characterised in that a nucleic acid is recovered, which contains a nucleotidic sequence coding for the totality or part of the aminoacid sequence indicated hereafter, the encoded aminoacid sequence having the capacity of giving a specific immunological reaction with antibodies against HIV-2 retrovirus as defined in anyone of claims 1 to 9 when it is contacted with a serum of a patient infected with a HIV-2 retrovirus :

```
    MetGlyAlaArgAsnSerValLeuArgGlyLysLysAlaAspGln

    LeuGluArgIleArgLeuArgProGlyGlyLysLysLysTyrArg

    LeuLysHisIleValTrpAlaAlaAsnLysLeuAspArgPheGly

              100
    LeuAlaGluSerLeuLeuGluSerLysGluGlyCysGlnLysIle

    LeuThrValLeuAspProMetValProThrGlySerGluAsnLeu

                  200
    LysSerLeuPheAsnThrValCysValIleIleTrpCysIleHisAla
```

```
GluGluLysValLysAspThrGluGlyAlaLysGlnIleValArg
                              300
ArgHisLeuValAlaGluThrGlyThrAlaGluLysMetProSer

ThrSerArgProThrAlaProSerSerGluLysGlyGlyAsnTyr
                                          400
```

35. Process according to claim 29, characterised in that a nucleic acid is recovered, which contains a nucleotidic sequence coding for the totality or part of the aminoacid sequence indicated hereafter, the encoded aminoacid sequence having the capacity of giving a specific immunological reaction with antibodies against a HIV-2 retrovirus as defined in anyone of claims 1 to 9 when it is contacted with a serum of a patient infected specifically with a HIV-2 retrovirus :

```
ProValGlnHisValGlyGlyAsnTyrThrHisIleProLeuSer

ProArgThrLeuAsnAlaTrpValLysLeuValGluGluLysLys

PheGlyAlaGluValValProGlyPheGlnAlaLeuSerGluGly
  500
CysThrProTyrAspIleAsnGlnMetLeuAsnCysValGlyAsp

HisGlnAlaAlaMetGlnIleIleArgGlnIleIleAsnGluGln
          600
AlaAlaGluTrpAspValGlnHisProIleProGlyProLeuPro

AlaGlyGlnLeuArgGluProArgGlySerAspIleAlaGlyThr
                  700
ThrSerThrValGlnGluGlnIleGlnTrpMetPheArgProGln

AsnProValProValGlyAsnIleTyrArgArgTrpIleGlnIle
                          800
GlyLeuGlnLysCysValArgMetTyrAsnProThrAsnIleLeu

AspIleLysGlnGlyProLysGluProPheGlnSerTyrValAsp
                                  900
ArgPheTyrLysSerLeuArgAlaGluGlnThrAspProAlaVal

LysAsnTrpMetThrGlnThrLeuLeuValGlnAsnAlaAsnPro

AspCysLysLeuValLeuLysGlyLeuGlyMetAsnProThrLeu
  1000
GluGlnMetLeuThrAlaCysGlnGlyValGlyGlyProGlyGln
```

LysAlaArgLeuMetAlaGluAlaLeuLysGluValIleGlyPro

1100

AlaProIleProPheAlaAlaAlaGlnGln

36. Process according to claim 29, characterised in that a nucleic acid is recovered, which contains a nucleotidic sequence coding for the totality or part of the aminoacid sequence indicated hereafter, this encoded aminoacid sequence having the capacity of giving a specific immunological reaction with antibodies against a HIV-2 retrovirus as defined in anyone of claims 1 to 9 when this aminoacid sequence is contact with a serum of a patient infect with a HIV-2 retrovirus :

**XIVRI**

MetMetAsnGlnLeuLeuIleAlaIleLeuLeuAlaSerAlaCys

LeuValTyrCysThrGlnTyrValThrValPheTyrGlyValPro

ThrTrpLysAsnAlaThrIleProLeuPheCysAlaThrArgAsn

100
ArgAspThrTrpGlyThrIleGlnCysLeuProAspAsnAspAsp

TyrGlnGluIleThrLeuAsnValThrGluAlaPheAspAlaTrp

200
AsnAsnThrValThrGluGlnAlaIleGluAspValTrpHisLeu

PheGluThrSerIleLysProCysValLysLeuThrProLeuCys

300
ValAlaMetLysCysSerSerThrGluSerSerThrGlyAsnAsn

ThrThrSerLysSerThrSerThrThrThrThrThrProThrAsp

400
GlnGluGlnGluIleSerGluAspThrProCysAlaArgAlaAsp

AsnCysSerGlyLeuGlyGluGluGluThrIleAsnCysGlnPhe

AsnMetThrGlyLeuGluArgAspLysLysLysGlnTyrAsnGlu

500
ThrTrpTyrSerLysAspValValCysGluThrAsnAsnSerThr

AsnGlnThrGlnCysTyrMetAsnHisCysAsnThrSerValIle

600
ThrGluSerCysAspLysHisTyrTrpAspAlaIleArgPheArg

TyrCysAlaProProGlyTyrAlaLeuLeuArgCysAsnAspThr

700
AsnTyrSerGlyPheAlaProAsnCysSerLysValValAlaSer

109

ThrCysThrArgMetMetGluThrGlnThrSerThrTrpPheGly

800
PheAsnGlyThrArgAlaGluAsnArgThrTyrIleTyrTrpHis

GlyArgAspAsnArgThrIleIleSerLeuAsnLysTyrTyrAsn

900
LeuSerLeuHisCysLysArgProGlyAsnLysThrValLysGln

IleMetLeuMetSerGlyHisValPheHisSerHisTyrGlnPro

IleAsnLysArgProArgGlnAlaTrpCysTrpPheLysGlyLys

1000
TrpLysAspAlaMetGlnGluValLysThrLeuAlaLysHisPro

ArgTyrArgGlyThrAsnAspThrArgAsnIleSerPheAlaAla

1100
ProGlyLysGlySerAspProGluValAlaTyrMetTrpThrAsn

CysArgGlyGluPheLeuTyrCysAsnMetThrTrpPheLeuAsn

1200
TrpIleGluAsnLysThrHisArgAsnTyrAlaProCysHisIle

LysGlnIleIleAsnThrTrpHisLysValGlyArgAsnValTyr

1300
LeuProProArgGluGlyGluLeuSerCysAsnSerThrValThr

SerIleIleAlaAsnIleAspTrpGlnAsnAsnAsnGlnThrAsn

IleThrPheSerAlaGluValAlaGluLeuTyrArgLeuGluLeu

1400
GlyAspTyrLysLeuValGluIleThrProIleGlyPheAlaPro

ThrLysGluLysArgTyrSerSerAlaHisGlyArgHisThrArg

1500
GlyValPheValLeuGlyPheLeuGlyPheLeuAlaThrAlaGly

SerAlaMetGlyAlaArgAlaSerLeuThrValSerAlaGlnSer

1600
ArgThrLeuLeuAlaGlyIleValGlnGlnGlnGlnGlnLeuLeu

AspValValLysArgGlnGlnGluLeuLeuArgLeuThrValTrp

1700
GlyThrLysAsnLeuGlnAlaArgValThrAlaIleGluLysTyr

LeuGlnAspGlnAlaArgLeuAsnSerTrpGlyCysAlaPheArg

1800
GlnValCysHisThrThrValProTrpValAsnAspSerLeuAla

ProAspTrpAspAsnMetThrTrpGlnGluTrpGluLysGlnVal

ArgTyrLeuGluAlaAsnIleSerLysSerLeuGluGlnAlaGln

1900
IleGlnGlnGluLysAsnMetTyrGluLeuGlnLysLeuAsnSer

TrpAspIlePheGlyAsnTrpPheAspLeuThrSerTrpValLys

2000
TyrIleGlnTyrGlyValLeuIleIleValAlaValIleAlaLeu

ArgIleValIleTyrValValGlnMetLeuSerArgLeuArgLys

2100
GlyTyrArgProValPheSerSerProProGlyTyrIleGlnGln

IleHisIleHisLysAspArgGlyGlnProAlaAsnGluGluThr

2200
GluGluAspGlyGlySerAsnGlyGlyAspArgTyrTrpProTrp

ProIleAlaTyrIleHisPheLeuIleArgGlnLeuIleArgLeu

LeuThrArgLeuTyrSerIleCysArgAspLeuLeuSerArgSer

2300
PheLeuThrLeuGlnLeuIleTyrGlnAsnLeuArgAspTrpLeu

ArgLeuArgThrAlaPheLeuGlnTyrGlyCysGluTrpIleGln

2400
GluAlaPheGlnAlaAlaAlaArgAlaThrArgGluThrLeuAla

GlyAlaCysArgGlyLeuTrpArgValLeuGluArgIleGlyArg

2500
GlyIleLeuAlaValProArgArgIleArgGlnGlyAlaGluIle

AlaLeuLeu

111

37. Process according to claim 28, characterised in that it further comprises the following steps :
— the action of the T4 DNA polymerase until the obtention of blunt ends on the double-stranded cDNA,
— the insertion of the cDNA containing adaptators and digested by adapted enzymes in the nucleic acid of a vector,
— the recovery of a recombinant nucleic acid comprising a cDNA or a part of this cDNA obtained by a process according to anyone of claims 28 to 36 and/or a RNA or a part of RNA corresponding to this cDNA, inserted in a nucleic acid obtained from said vector.

38. Process according to claim 37 characterised in that the recovered recombinant nucleic acid is labelled.

39. Process for the detection of a retrovirus capable of having a cytopathogenic effect for man, of the HIV-2 type or of its RNA, in a biological liquid or tissue especially for the in vitro diagnosis in man for the potentiality or existence of LAS or AIDS, characterised by contacting the nucleic acids contained in this biological liquid or tissue with a probe containing a nucleic acid obtained according to a process according to anyone of claims 32 to 36 under stringent hybridization conditions, or containing a DNA presenting a sequence homologie of a least 70 %, preferably of at least 90 % with the RNA of a retrovirus according to anyone of claims 1 to 9 and capable of hybridizing with this one, in non stringent conditions with the « spot blot » technique the above contacting being realised for the time necessary for said hybridization to occur and being followed by the washing of the hybride formed, with a solution ensuring the preservation of said stringent conditions and the detection of the hybride formed.

40. A process for the production of a retrovirus capable of having a cytopathogenic effect for man, of the HIV-2 type characterised by culturing in human T4 lymphocytes or in permanent cell lines derived from said T4 lymphocytes and carrying the T4 phenotype, which lymphocytes or cell lines had previously been infected with an isolate of HIV-2 virus as defined in anyone of claims 1 to 9, particularly when the level of reverse transcriptase activity has reached a determined threshold, recovering and purifying the amounts of virus released in the culture medium of said lymphocytes or cell lines, particularly by differential centrifugation in a gradient of sucrose or metrizamide.

41. Process for the production of a hybridization probe for the detection of a RNA of a HIV-2 retrovirus, characterised by the insertion of a DNA sequence, especially a probe obtained by a process according to anyone of claims 28 to 36 in a cloning vector, by in vitro recombination, by cloning the obtained modified vector in a competent cellular host and by the recovery of the obtained recombinant DNA.

42. The hybridoma secreting a monoclonal antibody specifically recognizing an antigen of a HIV-2 retrovirus according to anyone of claims 1 to 9.

43. Application of the polyclonal antibodies obtained by a process according to claim 27 to the detection of HIV-2 retrovirus as defined in anyone of Claims 1 to 9 in a biological preparation.

44. Application of the polyclonal antibodies obtained by a process according to claim 27 to the detection of antigens of the HIV-2 retrovirus as defined in anyone of claims 1 to 9 in a biological preparation.

45. Application of the polyclonal antibodies obtained by a process according to claim 27 to the purification of viral antigens of HIV-2 retrovirus as defined in anyone of claims 1 to 9.

**Patentansprüche** (für die Vertragsstaaten : BE, CH, DE, FR, GB, IT, LI, LU, NL, SE)

1. Menschliches Retrovirus HIV-2, das die Fähigkeit besitzt, menschliche T4-Lymphozyten zu infizieren und geeignet ist, beim Menschen Aids hervorzurufen, wobei dieses Retrovirus eines der bei CNCM unter der Nummer I-502, I-532, I-642 und I-643 und bei ECACC unter der Nummer 87011001 und 87011002 (jeweils den Nummern I-502 und I-532 entsprechend) hinterlegten Retroviren oder eine Variation eines dieser Retroviren darstellt und alle Antigene enthält, die von den gegen die entsprechenden Antigene eines beliebigen Retrovirus, das Gegenstand der genannten Hinterlegungen bei CNCM oder ECACC ist, gebildeten Antikörper erkannt werden.

2. Menschliches Retrovirus nach Anspruch 1, dadurch gekennzeichnet, daß sich seine Genom-RNS unter stringenten Bedingungen mit der Komplementärkette eines c-DNS oder c-DNS-Fragments, gewonnen aus der Genom-RNS eines der bei CNCM unter der Nummer I-502, I-532, I-642 und I-643 und bei ECACC unter der Nummer 87011001 und 87011002 (jeweils den Nummern I-502 und I-532 entsprechend) hinterlegten Retroviren zu kreuzen vermag.

3. Menschliches Retrovirus nach Anspruch 1, dadurch gekennzeichnet, daß es eine Gesamtheit von Antigenen umfaßt, die durch Proteine des Kerns (Core) mit einem Molekulargewicht in der Größenordnung von 12000, 16000 bzw. 26000 Dalton, durch Proteine oder Glycoproteine mit jeweils einem Molekulargewicht in der Größenordnung von 36000 Dalton und in der Größenordnung von 42000 bis 45000 Dalton, und durch ein Hauptglycoprotein der Hülle mit einem Molekulargewicht in der Größenordnung von 130000 bis 140000 Dalton gebildet sind.

4. Gereinigtes menschliches Retrovirus nach Anspruch 1, das pathogen ist für den Menschen und Aids hervorrufen kann, gekennzeichnet durch folgende Gesamtheit von Eigenschaften :

— bevorzugtes Zielobjekt des Retrovirus HIV-2 sind menschliche Leu3-Zellen (oder T4-Lymphozyten) und von diesen T4-Lymphozyten abgeleitete konstante Zellinien ;

— es kann auf menschliche T4-Lymphozyten, die es infiziert, eine zytopathogene Wirkung ausüben ;

— es übt eine Reverse-Transkriptase-Aktivität aus, die die Anwesenheit von $Mg^{2+}$-Ionen erforderlich macht, und zeigt starke Aktivität gegenüber Poly(adenylat-oligodeoxythymidylat)-(poly(A)-oligo(DT) 12-18) ;

— es hat eine Dichte von ca. 1,16 in einem Saccharosegradienten ;

— es hat einen durchschnittlichen Durchmesser von 140 nm und einen Kern mit einem durchschnittlichen Durchmesser von 41 nm ;

— es kann in konstanten, das Protein T4 exprimierenden Linien gezüchtet werden ;

— es ist für die T8-Lymphozyten nicht infektiös ;

— die Lysate dieses Virus enthalten ein Protein p26, das sich immunologisch nicht mit dem Protein p24 des Virus HTLV-1 oder des Virus HTLV-2 kreuzt ;

— diese Lysate enthalten außerdem ein Protein p16, das sich immunologisch nicht mit dem Protein p19 des HTLV-1 oder des HTLV-2 in Radioimmunausfällungstests kreuzt ;

— sie enthalten außerdem ein Hauptglycoprotein der Hülle mit einem Molekulargewicht in der Größenordnung von 130000 bis 140000 Dalton, das sich immunologisch nicht mit dem gp110 des Retrovirus HIV-1 kreuzt ;

— diese Lysate enthalten außerdem ein durch $^{35}$S-Cystein markierbares Protein oder Glycoprotein mit einem Molekulargewicht in der Größenordnung von 36000 Dalton ;

— die Genom-RNS des HIV-2 hybridisiert nicht, unter stringenten Bedingungen, mit der Genom-RNS des HIV-1, und hybridisiert nicht, unter nicht-stringenten Bedingungen, weder mit dem env-Gen noch mit den LTR des HIV-1 ;

— die Genom-RNS des HIV-2 hybridisiert schwach, unter nichtstringenten Bedingungen, mit den Nukleotidsequenzen, die einerseits die Nukleotide 990-1070, andererseits die Nukleotide 990-1260 der gag-Region des Genoms des HIV-1 enthalten.

5. Retrovirus nach einem der Ansprüche 1 bis 4, dadurch gekennzeichnet, daß es sich nicht chronisch in Rhesusaffen-Lymphozyten vermehrt, wenn es in vivo injiziert wurde und unter Arbeitsbedingungen, welche die Entwicklung des Virus STLV-III$_{mac}$, wie von Letvin et al., Science (1985), Band 230, 71-75 beschrieben, ermöglichen.

6. Retrovirus nach einem der Ansprüche 1 bis 5, dadurch gekennzeichnet, daß die Sequenz, welche die R-Region und die U3-Region der ihre Genom-RNS umfassenden Sequenz enthält, eine Nukleotidsequenz aufweist, die der nachfolgenden Nukleotidsequenz entspricht :

GTGGAACGCGAGACTGAAAGCAAGAGGAATACCATTTAGTTAAAGGACAG

GAACAGCTATACTTGGTCAGGGCAGGAAGTAACTAACAGAAACAGCTGAG

ACTGCAGGGACTTTCCAGAAGGGGCTGTAACCAAGGGAGGGACATGGAG

GAGCTGGTGGGGAACGCCTCATATTCTCTGTATAATATACCCGCTGCTTG

CATTGTACTTCAGTCGCTCTGCGGAGAGGCTGGCAGATTGAGCCCTGGAG

GATCTCTCCAGCACTAGACGGATGAGCCTGGGTGCCCTGCTAGACTCTCA

CCAGCACTTGGCCGGTGCTGGCAGACGGCCCCACGCTTGCCTGCTTAAA

ACCTTCCTTAAATAAAGCTGCAGTAGAAGCA

7. Retrovirus nach einem der Ansprüche 1 bis 5, dadurch gekennzeichnet, daß seine Genom-RNS eine gag-Sequenz enthält, die der folgenden Nukleotidsequenz entspricht :

GAGIODI

ATCGGCGCCAGAAACTCCGTCTTCACAGGCUAAAAACCACATGAA

TTACAAACAATCAGCTTACCGCCCCGCCCAAACAAAAGTACACG

CTAAAACATATTGTCTCGGCAGCCAATAAATTGCACAGATTCCGA

100

```
TTAGCAGAGAGCCTGTTCGAGTCAAAACACGGTTCTCAAAAATT

CTTACAGTTTTAGATCCAATGGTACCGACACGTTCAGAAAATTTA
                    200

AAAAGTCTTTTTAATACTGTCTGCGTCATTTCGTCCATACACCCA

CAACAGAAAGTGAAAGATACTGAAGGAGCAAAACAAATAGTGCGG
                              300

AGACATCTAGTGGCAGAAACAGGAACTGCAGAGAAAATGCCAAGC

ACAAGTAGACCAACAGCACCATCTAGCGAGAACGGCACCAAATTAC
                                        400

CCAGTGCAACATGTAGGCGGCAACTACACCCATATACCGCTGAGT

CCCCGAACCCTAAATGCCTGCGTAAAATTAGTAGACCAAAAAAAG

TTCGGGGCAGAAGTAGTGCCAGGATTTCAGGCACTCTCAGAAGGC
500

TGCACGCCCTATGATATCAACCAAATGCTTAATTGTGTGGGCGAC

CATCAAGCAGCCATGCAGATAATCAGGGACATTATCAATGAGGAA
            600

GCAGCAGAATGGGATGTGCAACATCCAATACCACGCCCCTTACCA

GCCCGGCAGCTTAGAGAGCCAAGCGGATCTGACATACCAGGGACA
                    700

ACAAGCACAGTAGAACAACACATCCAGTGCATGTTTACGCCACAA

AATCCTGTACCAGTAGGAAACATCTATAGAAGATGGATCCACATA
                                        800

GGATTGCAGAAGTGTGTCAGGATGTACAACCCGACCAACATCCTA

GACATAAAACAGGCACCAAAGGAGCCGTTCCAAAGCTATGTAGAT
                                        900

AGATTCTACAAAGCTTGAGGGCAGAACAAACAGATCCAGCACTG

AAGAATTCGATGACCCAAACACTGCTAGTACAAAATGCCAACCCA

GACTGTAAATTAGTGCTAAAAGGACTAGGCATGAACCCTACCTTA
    1000
```

114

CAAGAGATGCTGACCGGCTGTCAGCGGGTAGGTGCGCCCAGGCCAG

AAAGCTAGATTAATGGCAGAGGCCCTGAAACAGGTCATAGGACCT
1100

GCCCCTATCCCATTCGCAGCACCCCAGCAGAGAAAGGCATTTAAA

TGCTGGAACTGTCGAAACGAAGGGCACTCGTCAAGACAATGCCGA
1200

GCACCTAGAAGGCAGGGCTGCTGGAACTGTGGTAACCCAGGACAG

ATCATGACAAACTGCCCAGATAGACAGGCAGGTTTTTTAGGACTG
1300

GGCCCTTGGGGAAAGAAGCCCCGCAACTTCCCCGTGCCCCAAGTT

CCGCAGCGGCTGACACCAACAGCACCCCCAGTGGATCCAGCAGTG

GATCTACTGGAGAAATATATGCAGCAAGGGAAAGACAGAGACAG
1400

CAGAGAGAGAGACCATACAAGGAAGTGACAGAGGACTTACTGCAC

CTCCAGCAGGGGGAGACACCATACAGGGAGCCACCAACAGAGGAG
1500

TTGCTGCCACCTCAATTCTCTCTTTTGGAAAACACCAG

8. Retrovirus nach einem der Ansprüche 1 bis 6, dadurch gekennzeichnet, daß seine Genom-RNS eine env-Sequenz enthält, die der folgenden Nukleotidsequenz entspricht:

ENVLI

ATGATGAATCAGCTGCTTATTGCCATTTTATTAGCTAGTGCTTGC

TTAGTATATTGCACCCAATATGTAACTGTTTTCTATGGCCTACCC

ACGTGGAAAAATGCAACCATTCCCCTCTTTTCTGCAACCAGAAAT
100

AGGGATACTTGGGGAACCATACAGTGCTTGCCTCACAATGATGAT

TATCAGGAAATAACTTTGAATGTAACAGAGGCTTTTGATGCATGG
200

AATAATACAGTAACAGAACAAGCAATAGAAGATGTCTGGCATCTA

TTCGAGACATCAATAAAACCATGTCTCAAACTAACACCTTTATGT
300

GTAGCAATGAAATGCAGCAGCACAGAGAGCAGCACAGGGAACAAC

ACAACCTCAAAGAGCACAAGCACAACCACAACCACACCCACAGAC
400

CAGGAGCAAGAGATAAGTGAGGATACTCCATGCGCACGCGCAGAG

AACTGCTCAGGATTGGGAGAGGAACAAACCATCAATTGCCAGTTC

AATATGACAGGATTAGAAAGAGATAAGAAAAACACTATAATGAA
500

ACATGGTACTCAAAAGATGTGGTTTGTCACACAAATAATAGCACA

AATCAGACCCAGTGTTACATGAACCATTGCAACACATCAGTCATC
600

ACAGAATCATGTGACAAGCACTATTGGCATGCTATAAGGTTTAGA

TACTGTGCACCACCGGGTTATGCCCTATTAAGATGTAATGATACC
700

AATTATTCAGGCTTTGCACCCAACTGTTCTAAAGTAGTAGCTTCT

ACATGCACCAGGATGATGGAAACGCAAACTTCCACATGGTTTCGC
800

TTTAATGGCACTAGAGCAGAGAATAGAACATATATCTATTGGCAT

GGCAGAGATAATAGAACTATCATCAGCTTAAACAAATATTATAAT
900

CTCAGTTTGCATTGTAAGAGGCCAGGGAATAAGACACTGAAACAA

ATAATGCTTATGTCAGGACATGTGTTTCACTCCCACTACCAGCCG

ATCAATAAAAGACCCAGACAAGCATGGTGCTGGTTCAAAGGCAAA
1000

TGGAAAGACGCCATGCAGGAGGTGAAGACCCTTGCAAAACATCCC

AGGTATAGAGGAACCAATGACACACAAGGAATATTAGCTTTGCAGCC
1100

CCAGGAAAAGGCTCAGACCCAGAAGTAGCATACATGTGGACTAAC

TGCAGAGGAGAGTTTCTCTACTGCAACATGACTTGGTTCCTCAAT
1200

TGCATACAGAATAAGACACACCGCAATTATCCACCGTGCCATATA

AAGCAAATAATTAACACATGGCATAAGGTAGCCAGAAATGTATAT
1300

TTGCCTCCCACGGAAGGGGAGCTGTCCTGCAACTCAACAGTAACC

AGCATAATTGCTAACATTGACTGGCAAAACAATAATCAGACAAAG

ATTACCTTTACTGCAGAGGTGGCAGAACTATACAGATTGGACTTG
1400

GGACATTATAAATTGGTAGAAATAACACCAATTGGCTTCGCACCT

ACAAAAGAAAAAAGATACTCCTCTGCTCACGGGAGACATACAAGA
1500

GGTGTGTTCGTGCTAGGGTTCTTGGGTTTTCTCGCAACAGCAGGT

TCTGCAATGGGCGGCTCGAGCGTCCCTGACCGTGTCGGCTCAGTCC
1600

CGGACTTTACTGGCCGGGATAGTGCAGCAACAGCAACAGCTGTTG

GACGTGGTCAAGAGACAACAAGAACTGTTGCCACTGACCGTCTGG
1700

GGAACGAAAAACCTCCAGGCAAGAGTCACTGCTATAGAGAAGTAG

CTACAGGACCAGGCGCGGCTAAATTCATGGGGATCTGCCTTTAGA
1800

CAAGTCTGCCACACTACTGTACCATGGGTTAATGATTCCTTAGCA

CCTGACTGGGACAATATGACGTGGCAGGAATGGGAAAAACAAGTC

CGCTACCTGGAGGCAAATATCAGTAAAAGTTTAGAACAGGCACAA
1900

ATTCAGCAAGAGAAAAATATGTATGAACTACAAAAATTAAATAGC

TGGGATATTTTTGGCAATTGGTTTGACTTAACCTCCTGGGTCAAG
2000

117

TATATTCAATATCGAGTGCTTATAATAGTAGCAGTAATAGCTTTA

AGAATAGTGATATATGTAGTACAAATGTTAAGTAGGCTTAGAAG
2100

GGCTATAGGCCTGTTTTCTCTTCCCCCCCCGGTTATATCCAACAG

ATCCATATCCACAAGGACCCGGGACAGCCACCCAACGAAGAAACA
2200

GAAGAAGACGGTGGAAGCAACGGTGGAGACAGATACTGGCCCTGG

GCGATAGCATATATACATTTCCTGATCCCGCCAGCTGATTCGCCTC

TTGACCAGACTATACAGCATCTGCAGGGACTTACTATCCAGGAGG
2300

TTCCTGAGGGTCCAACTCATCTACCAGAATCTCAGAGACTGGCTG

AGACTTAGAACAGCCTTCTTGCAATATCGGTGCGAGTGGATCGAA
2400

GAAGCATTCCAGGCCGCGGCGAGGGCTACAAGAGAGACTCTTGCG

GGCGCGTGCAGGGGCTTGTGGAGGGTATTGGAACGAATCGGGAGG
2500

GGAATACTCGCGGTTCCAAGAAGGATCAGACAGGGAGCAGAAATC

GCCCTCCTG

9. Retrovirus nach einem der Ansprüche 1 bis 8, dadurch gekennzeichnet, daß seine RNS unter stringenten Bedingungen nicht mit der Genom-RNS des HIV-1 hybridisiert, und unter nicht-stringenten Bedingungen nicht mit dem env-Gen und dem angrenzenden LTR hybridisiert, insbesondere nicht mit der Nukleotidsequenz 5290-9130 des HIV-1, und unter nicht-stringenten Bedingungen nicht mit den Sequenzen der pol-Region des Genoms von HIV-1, insbesondere nicht mit der Nukleotidsequenz 2170-2240 hybridisiert.

10. Gereinigtes Antigen des Retrovirus HIV-2 mit den immunologischen Charakteristika des Proteins p12 des HIV-2, das immunologisch durch gegen ein Retrovirus nach einem der Ansprüche 1 bis 9 gebildete Antikörper erkannt wurde und ein Molekulargewicht in der Größenordnung von 12000 Dalton aufweist.

11. Gereinigtes Antigen des Retrovirus HIV-2 mit den immunologischen Charakteristika des Proteins p16 des HIV-2, das immunologisch durch gegen ein Retrovirus nach einem der Ansprüche 1 bis 9 gebildete Antikörper erkannt wurde und ein Molekulargewicht in der Größenordnung bon 16000 Dalton aufweist.

12. Gereinigtes Antigen des Retrovirus HIV-2 mit den immunologischen Charakteristika des Proteins p26 des HIV-2, das immunologisch durch gegen ein Retrovirus nach einem der Ansprüche 1 bis 9 gebildete Antikörper erkannt wurde und ein Molekulargewicht in der Größenordnung von 26000 Dalton aufweist.

13. Gereinigtes Antigen des Retrovirus HIV-2 mit den immunologischen Charakteristika des Glycoproteins gp36 des HIV-2, das immunologisch durch gegen ein Retrovirus nach einem der Ansprüche 1 bis 9 gebildete Antikörper erkannt wurde und ein Molekulargewicht in der Größenordnung von 36000 Dalton aufweist.

14. Gereinigtes Antigen des Retrovirus HIV-2 mit den immunologischen Charakteristika des Proteins p42 des HIV-2, das immunologisch durch gegen ein Retrovirus nach einem der Ansprüche 1 bis 9 gebildete Antikörper erkannt wurde und ein Molekulargewicht in der Größenordnung von 42000 bis 45000 Dalton aufweist.

15. Gereinigtes Antigen des Retrovirus HIV-2 mit den immunologischen Charakteristika des Glycoproteins gp140 des HIV-2, das immunologisch durch gegen ein Retrovirus nach einem der Ansprüche 1 bis 9 gebildete Antikörper erkannt wurde und ein Molekulargewicht in der Größenordnung von 130000 bis 140000 aufweist Dalton aufweist.

16. Antigen, das die die nachfolgend angeführte Aminosäuresequenz oder einen Teil dieser Sequenz enthält, von dem Zeitpunkt an, an dem sie eine spezifische immunologische Reaktion mit Antikörpern gegen ein Retrovirus HIV-2 nach einem der Ansprüche 1 bis 9 auslöst, insbesondere wenn dieses Antigen mit einem Serum eines mit HIV-2 infizierten Patienten in Kontakt gebracht wird :

ArgLysAlaPheLys

CysTrpAsnCysGlyLysGluGlyHisSerAlaArgGluCysArg

1200
AlaProArgArgGlnGlyCysTrpLysCysGlyLysProGlyHis

IleMetThrAsnCysProAspArgGlnAlaGlyPheLeuGlyLeu

1300
GlyProTrpGlyLysLysProArgAsnPheProValAlaGlnVal

ProGlnGlyLeuThrProThrAlaProProValAspProAlaVal

AspLeuLeuGluLysTyrMetGlnGlnGlyLysArgGlnArgGlu

1400
GlnArgGlnArgProTyrLysGluValThrGluAspLeuLeuHis

LeuGluGlnGlyGluThrProTyrArgGluProProThrGluAsp

1500
LeuLeuHisLeuAsnSerLeuPheGlyLysAspGln

17. Antigen, das die nachfolgend angeführte Aminosäuresequenz oder einen Teil dieser Sequenz enthält, von dem Zeitpunkt an, an dem sie eine spezifische immunologische Reaktion mit Antikörpern gegen ein Retrovirus HIV-2 nach einem der Ansprüche 1 bis 9 auslöst, insbesondere wenn dieses Antigen mit einem Serum eines mit HIV-2 infizierten Patienten in Kontakt gebracht wird :

MetGlyAlaArgAsnSerValLeuArgGlyLysLysAlaAspGlu

LeuGluArgIleArgLeuArgProGlyGlyLysLysLysTyrArg

LeuLysHisIleValTrpAlaAlaAsnLysLeuAspArgPheGly

100
LeuAlaGluSerLeuLeuGluSerLysGlnGlyCysGlnLysIle

LeuThrValLeuAspProMetValProThrGlySerGlnAsnLeu

200
LysSerLeuPheAsnThrValCysValIleTrpCysIleHisAla.

119

GluGluLysValLysAspThrGluGlyAlaLysGlnIleValArg

ArgHisLeuValAlaGluThrGlyThrAlaGluLysMetProSer

300

ThrSerArgProThrAlaProSerSerGluLysGlyGlyAsnTyr

400

18. Antigen, das die nachfolgend angeführte Aminosäuresequenz oder einen Teil dieser Sequenz enthält, von dem Zeitpunkt an, an dem sie eine spezifische immunologische Reaktion mit Antikörpern gegen ein Retrovirus HIV-2 nach einem der Ansprüche 1 bis 9 auslöst, insbesondere wenn dieses Antigen mit einem Serum eines HIV-2 infizierten Patienten in Kontakt gebracht wird :

ProValGluHisValGlyGlyAsnTyrThrHisIleProLeuSer

ProArgThrLeuAsnAlaTrpValLysLeuValGluGluLysLys

PheGlyAlaGluValValProGlyPheGlnAlaLeuSerGluGly

500

CysThrProTyrAspIleAsnGlnMetLeuAsnCysValGlyAsp

HisGlnAlaAlaMetGlnIleIleArgGluIleIleAsnGluGlu

600

AlaAlaGluTrpAspValGlnHisProIleProGlyProLeuPro

AlaGlyGlnLeuArgGluProArgGlySerAspIleAlaGlyThr

700

ThrSerThrValGluGluGlnIleGlnTrpMetPheArgProGln

AsnProValProValGlyAsnIleTyrArgArgTrpIleGlnIle

800

GlyLeuGlnLysCysValArgMetTyrAsnProThrAsnIleLeu

AspIleLysGlnGlyProLysGluProPheGlnSerTyrValAsp

900

ArgPheTyrLysSerLeuArgAlaGluGlnThrAspProAlaVal

LysAsnTrpMetThrGluThrLeuLeuValGlnAsnAlaAsnPro

AspCysLysLeuValLeuLysGlyLeuGlyMetAsnProThrLeu

1000

GluGlnMetLeuThrAlaCysGlnGlyValGlyGlyProGlyGln

LysAlaArgLeuMetAlaGluAlaLeuLysGluValIleGlyPro

1100
AlaProIleProPheAlaAlaAlaGlnGln

19. Antigen, das die nachfolgend angeführte Aminosäuresequenz oder einen Teil dieser Sequenz enthält, von dem Zeitpunkt an, and dem sie eine spezifische immunologische Reaktion mit Antikörpern gegen ein Retrovirus HIV-2 nach einem der Ansprüche 1 bis 9 auslöst, insbesondere wenn dieses Antigen ·mit einem Serum eines HIV-2 infizierten Patienten in Kontakt gebracht wird :

ENV

MetMetAsnGlnLeuLeuIleAlaIleLeuLeuAlaSerAlaCys

LeuValTyrCysThrGlnTyrValThrValPheTyrGlyValPro

ThrTrpLysAsnAlaThrIleProLeuPheCysAlaThrArgAsn

100
ArgAspThrTrpGlyThrIleGlnCysLeuProAspAsnAspAsp

TyrGlnGluIleThrLeuAsnValThrGluAlaPheAspAlaTrp

200
AsnAsnThrValThrGluGlnAlaIleGluAspValTrpHisLeu

PheGluThrSerIleLysProCysValLysLeuThrProLeuCys

300
ValAlaMetLysCysSerSerThrGluSerSerThrGlyAsnAsn

ThrThrSerLysSerThrSerThrThrThrThrThrProThrAsp

400
GlnGluGlnGluIleSerGluAspThrProCysAlaArgAlaAsp

AsnCysSerGlyLeuGlyGluGluGluThrIleAsnCysGlnPhe

AsnMetThrGlyLeuGluArgAspLysLysLysGlnTyrAsnGlu

500
ThrTrpTyrSerLysAspValValCysGluThrAsnAsnSerThr

AsnGlnThrGlnCysTyrMetAsnHisCysAsnThrSerValIle

600
ThrGluSerCysAspLysHisTyrTrpAspAlaIleArgPheArg

TyrCysAlaProProGlyTyrAlaLeuLeuArgCysAsnAspThr

700
AsnTyrSerGlyPheAlaProAsnCysSerLysValValAlaSer


ThrCysThrArgMetMetGluThrGlnThrSerThrTrpPheGly

800
PheAsnGlyThrArgAlaGluAsnArgThrTyrIleTyrTrpHis

GlyArgAspAsnArgThrIleIleSerLeuAsnLysTyrTyrAsn

900
LeuSerLeuHisCysLysArgProGlyAsnLysThrValLysGln

IleMetLeuMetSerGlyHisValPheHisSerHisTyrGlnPro

IleAsnLysArgProArgGlnAlaTrpCysTrpPheLysGlyLys

1000
TrpLysAspAlaMetGlnGluValLysThrLeuAlaLysHisPro

ArgTyrArgGlyThrAsnAspThrArgAsnIleSerPheAlaAla

1100
ProGlyLysGlySerAspProGluValAlaTyrMetTrpThrAsn

CysArgGlyGluPheLeuTyrCysAsnMetThrTrpPheLeuAsn

1200
TrpIleGluAsnLysThrHisArgAsnTyrAlaProCysHisIle


LysGlnIleIleAsnThrTrpHisLysValGlyArgAsnValTyr

1300
LeuProProArgGluGlyGluLeuSerCysAsnSerThrValThr

SerIleIleAlaAsnIleAspTrpGlnAsnArgAsnGlnThrAsn

IleThrPheSerAlaGluValAlaGluLeuTyrArgLeuGluLeu

1400
GlyAspTyrLysLeuValGluIleThrProIleGlyPheAlaPro

ThrLysGluLysArgTyrSerSerAlaHisGlyArgHisThrArg

1500
GlyValPheValLeuGlyPheLeuGlyPheLeuAlaThrAlaGly

SerAlaMetGlyAlaArgAlaSerLeuThrValSerAlaGlnSer

1600
ArgThrLeuLeuAlaGlyIleValGlnGlnGlnGlnGlnLeuLeu

AspValValLysArgGlnGlnGluLeuLeuArgLeuThrValTrp

1700
GlyThrLysAsnLeuGlnAlaArgValThrAlaIleGluLysTyr

LeuGlnAspGlnAlaArgLeuAsnSerTrpGlyCysAlaPheArg

1800
GlnValCysHisThrThrValProTrpValAsnAspSerLeuAla

ProAspTrpAspAsnMetThrTrpGlnGluTrpGluLysGlnVal

ArgTyrLeuGluAlaAsnIleSerLysSerLeuGluGlnAlaGln

1900
IleGlnGlnGluLysAsnMetTyrGluLeuGlnLysLeuAsnSer

TrpAspIlePheGlyAsnTrpPheAspLeuThrSerTrpValLys

2000
TyrIleGlnTyrGlyValLeuIleIleValAlaValIleAlaLeu

ArgIleValIleTyrValValGlnMetLeuSerArgLeuArgLys

2100
GlyTyrArgProValPheSerSerProProGlyTyrIleGlnGln

IleHisIleHisLysAspArgGlyGlnProAlaAsnGluGluThr

2200
GluGluAspGlyGlySerAsnGlyGlyAspArgTyrTrpProTrp

ProIleAlaTyrIleHisPheLeuIleArgGlnLeuIleArgLeu

LeuThrArgLeuTyrSerIleCysArgAspLeuLeuSerArgSer

123

```
2300
PheLeuThrLeuGlnLeuIleTyrGlnAsnLeuArgAspTrpLeu

ArgLeuArgThrAlaPheLeuGlnTyrGlyCysGluTrpIleGln

            2400
GluAlaPheGlnAlaAlaAlaArgAlaThrArgGluThrLeuAla

GlyAlaCysArgGlyLeuTrpArgValLeuGluArgIleGlyArg

            2500
GlyIleLeuAlaValProArgArgIleArgGlnGlyAlaGluIle

AlaLeuLeu
```

20. Zusammensetzung für den Nachweis von Antikörpern gegen ein menschliches HIV-2 Virus, das eine zytopathogene Wirkung auf T4-Lymphozyten ausüben kann, in einer biologischen Probe vom Menschen in vitro, dadurch gekennzeichnet, daß sie wenigstens ein Antigen, insbesondere ein Protein oder ein Glycoprotein des Retrovirus HIV-2 nach einem der Ansprüche 1 bis 9 oder wenigstens ein Antigen nach einem der Ansprüche 10 bis 19 enthält.

21. Zusammensetzung nach Anspruch 20, dadurch gekennzeichnet, daß sie aus einem Gesamtextrakt oder einem Lysat des genannten Retrovirus besteht.

22. Zusammensetzung nach Anspruch 20, dadurch gekennzeichnet, daß das Antigen wenigstens aus einem der Innenproteine des Kerns (Core) des Virus besteht, ausgewählt unter den Proteinen oder Glycoproteinen p12, p16 und p26 mit einem Molekulargewicht in der Größenordnung von jeweils 12000, 16000 und 26000 Dalton.

23. Zusammensetzung nach Anspruch 20 oder Anspruch 21, dadurch gekennzeichnet, daß sie ein Glycoprotein gp140 mit einem Molekulargewicht in der Größenordnung bon 130000 bis 140000 Dalton enthält.

24. Zusammensetzung nach Anspruch 20 oder Anspruch 21, dadurch gekennzeichnet, daß sie ein Glycoprotein gp36 mit einem Molekulargewicht in der Größenordnung von 36000 Dalton enthält.

25. Zusammensetzung für den Nachweis von Antikörpern gegen ein menschliches Retrovirus, das eine zytopathogene Wirkung auf die T4-Lymphozyten ausüben kann und wenigstens ein HIV-1-Antigen enthält, in einer biologischen Probe vom Menschen in vitro, dadurch gekennzeichnet, daß sie ebenfalls wenigstens ein HIV-2 Antigen nach einem der Ansprüche 1 bis 9, insbesondere ein Protein oder ein Glycoprotein dieses Retrovirus HIV-2, oder wenigstens ein Antigen nach einem der Ansprüche 10 bis 19 enthält.

26. Zusammensetzung nach Anspruch 25, dardurch gekennzeichnet, daß sie Proteine des Kerns (Core) von HIV-1 und HIV-2 enthält.

27. Zusammensetzung nach Anspruch 26, dadurch gekennzeichnet, daß sie die p25 eines HIV-1 und die p26 eines HIV-2 enthält.

28. Zusammensetzung nach Anspruch 26, dadurch gekennzeichnet, daß sie das p18 eines HIV-1 und das p16 eines HIV-2 enthält.

29. Zusammensetzung nach Anspruch 25, dadurch gekennzeichnet, daß sie Hüllglycoproteine eines HIV-1 und Hüllglycoproteine eines HIV-2 enthält.

30. Zusammensetzung nach Anspruch 29, dadurch gekennzeichnet, daß sie das gp110 des HIV-1 und das gp140 des HIV-2 enthält.

31. Zusammensetzung nach Anspruch 29 oder Anspruch 30, dadurch gekennzeichnet, daß sie das gp42 des HIV-1 und das p36 des HIV-2 enthält.

32. Zusammensetzung nach Anspruch 26, dadurch gekennzeichnet, daß sie Mischungen, einerseits von Antigenen aus Proteinen oder Glycoproteinen von HIV-1 oder beides zusammen, und andererseits Mischungen aus Proteinen und Glycoproteinen von HIV-2 oder von beiden zusammen enthält.

33. Methode zum Nachweis der Anwesenheit von Antikörpern in vitro, die bei einem durch ein menschliches Retrovirus HIV-2 infizierten Menschen induziert wurden, in einer biologischen Probe vom Menschen, wie einem Serum, insbesondere zum Nachweis in vitro von möglicherweise oder tatsächlich vorhandenem SLA oder AIDS aufgrund eines solchen Nachweises, und die von der Person stammt, die Gegenstand des Diagnose ist, dadurch gekennzeichnet, daß man diese biologische Probe mit einem Antigen in Kontakt bringt, das durch einen bei dem durch ein menschliches Retrovirus HIV-2, wie es in einem der Ansprüche 1 bis 9 definiert wurde, infizierten Menschen induzierten Antikörper erkannt wurde, unter Bedingungen, welche die Bildung einer immunologischen Verbindung zwischen diesem Antigen und dem besagten Antikörper ermöglichen, und daß man das gegebenenfalls zwischen diesem Antikörper und dem eingesetzten Antigen gebildete immunologische Konjugat nachweist.

34. Methode nach Anspruch 33, dadurch gekennzeichnet, daß man die biologische Probe mit einem Antigen nach einem der Ansprüche 10 bis 19 oder einer Zusammensetzung nach einem der Ansprüche 20 bis 24 unter Bedingungen in Kontakt bringt, welche die Bildung einer immunologischen Verbindung zwischen diesem Antigen und den besagten Antikörpern ermöglichen, und daß man das gegebenenfalls zwischen diesen Antikörpern Anti-HIV-2 und dem eingesetzten Antigen gebildete immunologische Konjugat nachweist.

35. Methode nach Anspruch 34, dadurch gekennzeichnet, daß man den Nachweis des immunologischen Konjugats so durchführt, daß man das gegebenenfalls gebildete immunologische Konjugat mit einem markierten Reagens, gebildet durch menschliche Antiimmunoglobulin-Antikörper oder durch ein Bakterienprotein A, reagieren läßt und den gebildeten Komplex zwischen dem Reagens und dem genannten immunologischen Konjugat nachweist.

36. Methode zum Nachweis der Anwesenheit von Antikörpern in vitro, die Antigene eines menschlichen Retrovirus erkennen, das auf menschliche T4-Lymphozyten eine zytopathogene Wirkung auszuüben vermag, in einer biologischen Probe vom Menschen, wie einem Serum, insbesondere zum Nachweis in vitro von möglicherweise oder tatsächlich vorhandenem SLA oder AIDS aufgrund eines menschlichen Retrovirus, dadurch gekennzeichnet, daß man diese biologische Probe, die aus der Person stammt, die Gegenstand des Nachweises ist, mit einer Mischung von Antigenen in Kontakt bringt, die mit den Antikörpern einerseits gegen ein menschliches Retrovirus HIV-2, das eine zytopathogene Wirkung auszuüben vermag, wie in einem der Ansprüche 1 bis 9 definiert wird, und andererseits gegen ein menschliches Retrovirus HIV-1 eine spezifische immunologische Reaktion einzugehen vermögen, und daß man das gegebenenfalls zwischen diesem Antikörper und wenigstens einem dieser Antigene gebildete immunologische Konjugat nachweist.

37. Methode nach Anspruch 36, dadurch gekennzeichnet, daß die aus der Person, die Gegenstand des Nachweises darstellt, stammende biologische Probe mit einem Gemisch von gleichzeitig aus HIV-1 und HIV-2 nach einem der Ansprüche 1 bis 9 stammenden Antigenen und insbesondere mit einer Zusammensetzung nach einem der Ansprüche 26 bis 32 in Kontakt gebracht wird, und daß man das gegebenenfalls zwischen diesem Antikörper und wenigstens einem dieser Antigene gebildete immunologische Konjugat nachweist.

38. Stoffkimbination (Kit) zum Nachweis in vitro von Antikörpern gegen ein Retrovirus, das eine zytopathogene Wirkung auf den Menschen auszuüben vermag, vom Typ HIV-2 in einer biologischen Probe, insbesondere gegebenenfalls eines Trägers dieser Antikörper, dadurch gekennzeichnet, daß sie folgendes umfaßt :

— wenigstens ein Antigen nach einem der Ansprüche 10 bis 19, oder eine Zusammensetzung, wie in einem der Ansprüche 20 bis 32 und Anspruch 34 definiert,

— Mittel zum Nachweis des aus der immunologischen Reaktion zwischen dem Antigen und der besagten biologischen Probe resultierenden immunologischen Konjugats.

39. Stoffkombination (Kit) nach Anspruch 38, dadurch gekennzeichnet, daß die Mittel zum Nachweis des gebildeten immunologischen Konjugats menschliche Antiimmunoglobuline oder ein Protein A und Mittel zum Nachweis des zwischen den Antikörpern Anti-HIV-2, die im nachgewiesenen immunologischen Konjugat enthalten sind, gebildeten Komplexes umfassen.

40. Stoffkombination (Kit) zum Nachweis der Anwesenheit von Antikörpern gegen ein für menschliche T4-Lymphozyten zytopathogenes Retrovirus in vitro, insbesondere gegebenenfalls eines Trägers dieser Antikörper, dadurch gekennzeichnet, daß sie folgendes umfaßt :

— ein Gemisch aus wenigstens einem Antigen von HIV-1 und wenigstens einem Antigen von HIV-2, insbesondere eine Zusammensetzung, wie sie in einem der Ansprüche 25 bis 32 definiert ist,

— Mittel zum Nachweis des aus der immunologischen Reaktion zwischen den Antigenen und den Antikörpern der biologischen Flüssigkeit resultierenden immunologischen Konjugats.

41. Immunogene Zusammensetzung, die ein Hüllglycoprotein des menschlichen Retrovirus HIV-2 enthält, wie es in einer der Ansprüche 1 bis 9 definiert wird, wie zum Beispiel das gp140 dieses Retrovirus, oder einen Teil dieses Glycoproteins, der Antikörper gegen HIV-2 in Verbindung mit einem pharmazeutisch verträglichen Träger, der für die Bildung von Impfstoffen gegen HIV-2 geeignet ist, zu induzieren vermag.

42. Zusammensetzung nach Anspruch 41, dadurch gekennzeichnet, daß sie wenigstens einen immunogenen Teil eines Glycoproteins enthält, der ein Proteingerüst mit der nachfolgend angeführten Sequenz enthält, wobei dieser immunogene Teil Antikörper gegen ein menschliches Retrovirus HIV-2, wie in einem Ansprüche 1 bis 9 definiert, zu induzieren vermag :

ENV

MetMetAsnGlnLeuLeuIleAlaIleLeuLeuAlaSerAlaCys

LeuValTyrCysThrGlnTyrValThrValPheTyrGlyValPro

125

ThrTrpLysAsnAlaThrIleProLeuPheCysAlaThrArgAsn

ArgAspThrTrpGlyThrIleGlnCysLeuProAspAsnAspAsp

TyrGlnGluIleThrLeuAsnValThrGluAlaPheAspAlaTrp

AsnAsnThrValThrGluGlnAlaIleGluAspValTrpHisLeu

PheGluThrSerIleLysProCysValLysLeuThrProLeuCys

ValAlaMetLysCysSerSerThrGluSerSerThrGlyAsnAsn

ThrThrSerLysSerThrSerThrThrThrThrThrProThrAsp

GlnGluGlnGluIleSerGluAspThrProCysAlaArgAlaAsp

AsnCysSerGlyLeuGlyGluGluGluThrIleAsnCysGlnPhe

AsnMetThrGlyLeuGluArgAspLysLysLysGlnIleTyrSerGln

ThrTrpTyrSerLysAspValValCysGluThrAsnAsnSerThr

AsnGlnThrGlnCysTyrMetAsnHisCysAsnThrSerValIle

ThrGluSerCysAspLysHisTyrTrpAspAlaIleArgPheArg

TyrCysAlaProProGlyTyrAlaLeuLeuArgCysAsnAspThr

AsnTyrSerGlyPheAlaProAsnCysSerLysValValAlaSer

ThrCysThrArgMetMetGluThrGlnThrSerThrTrpPheGly

PheAsnGlyThrArgAlaGluAsnArgThrTyrIleTyrTrpHis

GlyArgAspAsnArgThrIleIleSerLeuAsnLysTyrTyrAsn

LeuSerLeuHisCysLysArgProGlyAsnLysThrValLysGln

IleMetLeuMetSerGlyHisValPheHisSerHisTyrGlnPro

IleAsnLysArgProArgGlnAlaTrpCysTrpPheLysGlyLys

TrpLysAspAlaMetGlnGluValLysThrLeuAlaLysHisPro

ArgTyrArgGlyThrAsnAspThrArgAsnIleSerPheAlaAla

ProGlyLysGlySerAspProGluValAlaTyrMetTrpThrAsn

CysArgGlyGluPheLeuTyrCysAsnMetThrTrpPheLeuAsn

TrpIleGluAsnLysThrHisArgAsnTyrAlaProCysHisIle

LysGlnIleIleAsnThrTrpHisLysValGlyArgAsnValTyr

LeuProProArgGluGlyGluLeuSerCysAsnSerThrValThr

SerIleIleAlaAsnIleAspTrpGlnAsnAsnAsnGlnThrAsn

IleThrPheSerAlaGluValAlaGluLeuTyrArgLeuGluLeu

GlyAspTyrLysLeuValGluIleThrProIleGlyPheAlaPro

ThrLysGluLysArgTyrSerSerAlaHisGlyArgHisThrArg

GlyValPheValLeuGlyPheLeuGlyPheLeuAlaThrAlaGly

SerAlaMetGlyAlaArgAlaSerLeuThrValSerAlaGlnSer

ArgThrLeuLeuAlaGlyIleValGlnGlnGlnGlnGlnLeuLeu

AspValValLysArgGlnGlnGluLeuLeuArgLeuThrValTrp

GlyThrLysAsnLeuGlnAlaArgValThrAlaIleGluLysTyr

LeuGlnAspGlnAlaArgLeuAsnSerTrpGlyCysAlaPheArg

GlnValCysHisThrThrValProTrpValAsnAspSerLeuAla

ProAspTrpAspAsnMetThrTrpGlnGluTrpGluLysGlnVal

ArgTyrLeuGluAlaAsnIleSerLysSerLeuGluGlnAlaGln

1900
IleGlnGlnGluLysAsnMetTyrGluLeuGlnLysLeuAsnSer

TrpAspIlePheGlyAsnTrpPheAspLeuThrSerTrpValLys

2000
TyrIleGlnTyrGlyValLeuIleIleValAlaValIleAlaLeu

ArgIleValIleTyrValValGlnMetLeuSerArgLeuArgLys

2100
GlyTyrArgProValPheSerSerProProGlyTyrIleGlnGln

IleHisIleHisLysAspArgGlyGlnProAlaAsnGluGluThr

2200
GluGluAspGlyGlySerAsnGlyGlyAspArgTyrTrpProTrp

ProIleAlaTyrIleHisPheLeuIleArgGlnLeuIleArgLeu

LeuThrArgLeuTyrSerIleCysArgAspLeuLeuSerArgSer

2300
PheLeuThrLeuGlnLeuIleTyrGlnAsnLeuArgAspTrpLeu

ArgLeuArgThrAlaPheLeuGlnTyrGlyCysGlnTrpIleGln

2400
GluAlaPheGlnAlaAlaAlaArgAlaThrArgGluThrLeuAla

GlyAlaCysArgGlyLeuTrpArgValLeuGluGlnArgIleGlyArg

2500
GlyIleLeuAlaValProArgArgIleArgGlnGlyAlaGluIle

AlaLeuLeu

43. Immunogene Zusammensetzung nach Anspruch 41 oder Anspruch 42, dadurch gekennzeichnet, daß sie, im Hinblick auf das Antigen so dosiert ist, daß sie die Verabreichung einer Einheitsdosis von 10 bis 500, insbesondere von 50 bis 100 mg/kg Körpergewicht ermöglicht.

44. Antikörper, der ein Antigen eines Retrovirus HIV-2 nach einem der Ansprüche 1 bis 9 spezifisch erkennt.

45. Antikörper nach Anspruch 44, dadurch gekennzeichnet, daß er monoklonal ist.

46. Nukleinsäure, die gegebenenfalls von einem Teil wenigstens der RNS des HIV-2-Virus oder einem seiner Variationen, wie in einem der Ansprüche 1 bis 9 definiert, abgeleitet ist, gekennzeichnet durch ihre Fähigkeit zur Hybridisierung mit der RNS des HIV-2-Virus und durch die fehlende Hybridisierung unter stringenten Bedingungen mit den Sonden 1 bis 4, welche die Nukleotide 990-1070, 990-1260, 2170-2240 bzw. 3370-3640 des DNS-Derivats vom Genom des HIV-1 umfaßt.

47. Nukleinsäure nach Anspruch 46, die einen Teil wenigstens einer c-DNS-Sequenz enthält, der sich mit der gesamten Genom-RNS eines Retrovirus HIV-2 nach einem der Ansprüche 1 bis 9 zu kreuzen vermag, und sich unter stringenten Bedingungen nicht mit den Sonden kreuzt, deren DNS-Fragmente vom Genom des HIV-1 abgeleitet bzw. aus durch die Nukleotide 990-1070, 990-1260, 2170-2240 bzw. 3370-3640 des DNS-Derivats vom Genom des HIV-1 begrenzte Fragmente gebildet sind.

48. Nukleinsäure nach Anspruch 46, dadurch gekennzeichnet, daß sie die folgende Nukleotidsequenz enthält :

GTGGAAGGCGAGACTGAAAGCAAGAGGAATACCATTTAGTTAAAGGACAG

GAACAGCTATACTTGGTCAGGGCAGGAAGTAACTAACAGAAACAGCTGAG

ACTGCAGGGACTTTCCAGAAGGGGCTGTAACCAAGGGAGGGACATGGGAG

GAGCTGGTGGGGAACGCCTCATATTCTCTGTATAATATACCCGCTGCTTG

CATTGTACTTCAGTCGCTCTGCGGAGAGGCTGGCAGATTGAGCCCTGGAG

GATCTCTCCAGCACTAGACGGATGAGCCTGGGTGCCCTGCTAGACTCTCA

CCAGCACTTGGCCGGTGCTGGCAGACGGCCCCACGCTTGCCTGCTTAAAX

ACCTTCCTTAATAAAGCTGCAGTAGAAGCX

49. Nukleinsäure nach Anspruch 46, dadurch gekennzeichnet, daß sie eine Nukleotidsequenz enthält, welche die Gesamtheit oder einen Teil der nachfolgend angeführten Aminosäuresequenz kodiert, wobei diese Aminosäuresequenz oder ein Teil davon eine spezifische immunologische Reaktion mit Antikörpern gegen ein Retrovirus HIV-2, wie in einem der Ansprüche 1 bis 9 definiert, auszulösen vermag, wenn diese Aminosäuresequenz mit einem Serum eines mit einem Retrovirus HIV-2 infizierten Patienten in Kontakt gebracht wird :

GAGRODH

MetGlyAlaArgAsnSerValLeuArgGlyLysLysAlaAspGlu

LeuGluArgIleArgLeuArgProGlyGlyLysLysLysTyrArg

LeuLysHisIleValTrpAlaAlaAsnLysLeuAspArgPheGly

100
LeuAlaGlySerLeuLeuGluSerLysGluGlyCysGlnLysIle

LeuThrValLeuAspProMetValProThrGlySerGluAsnLeu

200
LysSerLeuPheAsnThrValCysValIleTrpCysIleHisAla

300
GluGluLysValLysAspThrGluGlyAlaLysGlnIleValArg

ArgHisLeuValAlaGluThrGlyThrAlaGlyLysMetProSer

ThrSerArgProThrAlaProSerSerGluLysGlyGlyAsnTyr

400
ProValGlnHisValGlyGlyAsnTyrThrHisIleProLeuSer

ProArgThrLeuAsnAlaTrpValLysLeuValGluGluLysLys

129

```
PheGlyAlaGluValValProGlyPheGlnAlaLeuSerGluGly
        500
CysThrProTyrAspIleAsnGlnMetLeuAsnCysValGlyAsp

IleGlnAlaAlaMetGlnIleIleArgGluIleIleAsnGluGlu
                600
AlaAlaGlyTrpAspValGlnHisProIleProGlyProLeuPro

AlaGlyGlyLeuArgGluProArgGlySerAspIleAlaGlyThr
                        700
ThrSerThrValGluGluGlnIleGlnTrpMetPheArgProGln


AsnProValProValGlyAsnIleTyrArgArgTrpIleGlnIle
                                800
GlyLeuGlnLysCysValArgMetTyrAsnProThrAsnIleLeu

AspIleLysGlnGlyProLysGluProPheGlnSerTyrValAsp
                                        900
ArgPheTyrLysSerLeuArgAlaGluGlnThrAspProAlaVal

LysAsnTrpMetThrGlnThrLeuLeuValGlnAsnAlaAsnPro

AspCysLysLeuValLeuLysGlyLeuGlyMetAsnProThrLeu
        1000
GluGluMetLeuThrAlaCysGlnGlyValGlyGlyProGlyGln

LysAlaArgLeuMetAlaGluAlaLeuLysGlnValIleGlyPro
                1100
AlaProIleProPheAlaAlaAlaGlnGlnArgLysAlaPheLys

CysTrpAsnCysGlyLysGluGlyHisSerAlaArgGlnCysArg
                        1200
AlaProArgArgGlnGlyCysTrpLysCysGlyLysProGlyHis

IleMetThrAsnCysProAspArgGlnAlaGlyPheLeuGlyLeu
                                1300
GlyProTrpGlyLysLysProArgAsnPheProValAlaGlnVal

ProGlnGlyLeuThrProThrAlaProProValAspProAlaVal
```

130

AspLeuLeuGluLysTyrMetGlnGlnGlyLysArgGlnArgGlu

1400
GlnArgGluArgProTyrLysGluValThrGlnAspLeuLeuHis

LeuGluGlnGlyGluThrProTyrArgGluProProThrGluAsp

1500
LeuLeuHisLeuAsnSerLeuPheGlyLysAspGln

50. Nukleinsäure nach Anspruch 46, dadurch gekennzeichnet, daß sie eine Nukleotidsequenz enthält, welche wenigstens einen Teil der nachfolgend angeführten Aminosäuresequenz kodiert, wobei diese Aminosäuresequenz eine spezifische immunologische Reaktion mit Antikörpern gegen ein Retrovirus HIV-2, wie in einem der Ansprüche 1 bis 9 definiert, auszulösen vermag, wenn diese Aminosäuresequenz mit einem Serum eines mit Retrovirus HIV-2 infizierten Patienten in Kontakt gebracht wird :

ArgLysAlaPheLys

CysTrpAsnCysGlyLysGluGlyHisSerAlaArgGlnCysArg

1200
AlaProArgArgGlnGlyCysTrpLysCysGlyLysProGlyHis

IleMetThrAsnCysProAspArgGlnAlaGlyPheLeuGlyLeu

1300
GlyProTrpGlyLysLysProArgAsnPheProValAlaGlnVal

ProGlnGlyLeuThrProThrAlaProProValAspProAlaVal

AspLeuLeuGluLysTyrMetGlnGlnGlyLysArgGlnArgGlu

1400
GlnArgGluArgProTyrLysGluValThrGluAspLeuLeuHis

LeuGluGlnGlyGluThrProTyrArgGlnProProThrGluAsp

1500
LeuLeuHisLeuAsnSerLeuPheGlyLysAspGln

51. Nukleinsäure nach Anspruch 46, dadurch gekennzeichnet, daß sie eine Nukleotidsequenz enthält, welche die Gesamtheit oder einen Teil der nachfolgend angeführten Aminosäuresequenz kodiert, wobei die kodierte Aminosäuresequenz eine spezifische immunologische Reaktion mit Antikörpern gegen ein Retrovirus HIV-2, wie in einem der Ansprüche 1 bis 9 definiert, auszulösen vermag, wenn diese Aminosäuresequenz mit einem Serum eines mit Retrovirus HIV-2 infizierten Patienten in Kontakt gebracht wird :

MetGlyAlaArgAsnSerValLeuArgGlyLysLysAlaAspGln

LeuGluArgIleArgLeuArgProGlyGlyLysLysLysTyrArg

EP 0 239 425 B1

LeuLysHisIleValTrpAlaAlaAsnLysLeuAspArgPheGly

100
LeuAlaGluSerLeuLeuGluSerLysGluGlyCysGlnLysIle

LeuThrValLeuAspProMetValProThrGlySerGluAsnLeu

200
LysSerLeuPheAsnThrValCysValIleTrpCysIleHisAla

GlnGlyLysValLysAspThrGluGlyAlaLysGlnIleValArg

300
ArgHisLeuValAlaGluThrGlyThrAlaGluLysMetProSer

ThrSerArgProThrAlaProSerSerGluLysGlyGlyAsnTyr

400

52. Nukleinsäure nach Anspruch 46, dadurch gekennzeichnet, daß sie eine Nukleotidsequenz enthält, welche die Gesamtheit oder einen Teil der nachfolgend angeführten Aminosäuresequenz kodiert, wobei die kodierte Aminosäuresequenz eine spezifische immunologische Reaktion mit Antikörpern gegen ein Retrovirus HIV-2, wie in einem der Ansprüche 1 bis 9 definiert, auszulösen vermag, wenn diese Aminosäuresequenz mit einem Serum eines mit einem Retrovirus HIV-2 infizierten Patienten in Kontakt gebracht wird :

ProValGlnHisValGlyGlyAsnTyrThrHisIleProLeuSer

ProArgThrLeuAsnAlaTrpValLysLeuValGluGluLysLys

PheGlyAlaGluValValProGlyPheGlnAlaLeuSerGluGly

500
CysThrProTyrAspIleAsnGlnMetLeuAsnCysValGlyAsp

HisGlnAlaAlaMetGlnIleIleArgGluIleIleAsnGluGlu

600
AlaAlaGluTrpAspValGlnHisProIleProGlyProLeuPro

AlaGlyGlnLeuArgGluProArgGlySerAspIleAlaGlyThr

700
ThrSerThrValGlnGluGlnIleGlnTrpMetPheArgProGln

AsnProValProValGlyAsnIleTyrArgArgTrpIleGlnIle

800
GlyLeuGlnLysCysValArgMetTyrAsnProThrAsnIleLeu

AspIleLysGlnGlyProLysGluProPheGlnSerTyrValAsp

132

```
                                                                900
ArgPheTyrLysSerLeuArgAlaGluGlnThrAspProAlaVal

LysAsnTrpMetThrGlnThrLeuLeuValGlnAsnAlaAsnPro.

AspCysLysLeuValLeuLysGlyLeuGlyMetAsnProThrLeu
        1000
GluGluMetLeuThrAlaCysGlnGlyValGlyGlyProGlyGln

LysAlaArgLeuMetAlaGluAlaLeuLysGluValIleGlyPro
                1100
AlaProIleProPheAlaAlaAlaGlnGln
```

53. Nukleinsäure nach Anspruch 46, dadurch gekennzeichnet, daß sie eine Nukleotidsequenz enthält, welche die Gesamtheit oder einen Teil der nachfolgend angeführten Aminosäuresequenz kodiert, wobei die kodierte Aminosäuresequenz eine spezifische immunologische Reaktion mit Antikörpern gegen ein Retrovirus HIV-2, wie in einem der Ansprüche 1 bis 9 definiert, auszulösen vermag, wenn diese Aminosäuresequenz mit einem Serum eines mit einem Retrovirus HIV-2 infizierten Patienten in Kontakt gebracht wird :

```
ENVR2


MetMetAsnGlnLeuLeuIleAlaIleLeuLeuAlaSerAlaCys

LeuValTyrCysThrGlnTyrValThrValPheTyrGlyValPro

ThrTrpLysAsnAlaThrIleProLeuPheCysAlaThrArgAsn
        100
ArgAspThrTrpGlyThrIleGlnCysLeuProAspAsnAspAsp

TyrGlnGluIleThrLeuAsnValThrGluAlaPheAspAlaTrp
                200
AsnAsnThrValThrGluGlnAlaIleGluAspValTrpHisLeu

PheGluThrSerIleLysProCysValLysLeuThrProLeuCys
                300
ValAlaMetLysCysSerSerThrGluSerSerThrGlyAsnAsn

ThrThrSerLysSerThrSerThrThrThrThrThrProThrAsp
                        400
GlnGluGlnGluIleSerGluAspThrProCysAlaArgAlaAsn

AsnCysSerGlyLeuGlyGluGluGluThrIleAsnCysGlnPhe
```

AsnMetThrGlyLeuGluArgAspLysLysLysGluTyrAsnGln

500
ThrTrpTyrSerLysAspValValCysGluThrAsnAsnSerThr

AsnGlnThrGlnCysTyrMetAsnHisCysAsnThrSerValIle

600
ThrGluSerCysAspLysIleTyrTrpAspAlaIleArgPheArg

TyrCysAlaProProGlyTyrAlaLeuLeuArgCysAsnAspThr

700
AsnTyrSerGlyPheAlaProAsnCysSerLysValValAlaSer

ThrCysThrArgMetMetGluThrGlnThrSerThrTrpPheGly

300
PheAsnGlyThrArgAlaGluAsnArgThrTyrIleTyrTrpHis

GlyArgAspAsnArgThrIleIleSerLeuAsnLysTyrTyrAsn

900
LeuSerLeuHisCysLysArgProGlyAsnLysThrValLysGln

IleMetLeuMetSerGlyHisValPheHisSerHisTyrGlnPro

IleAsnLysArgProArgGlnAlaTrpCysTrpPheLysGlyLys

1000
TrpLysAspAlaMetGlnGluValLysThrLeuAlaLysHisPro

ArgTyrArgGlyThrAsnAspThrArgAsnIleSerPheAlaAla

1100
ProGlyLysGlySerAspProGluValAlaTyrMetTrpThrAsn

CysArgGlyGluPheLeuTyrCysAsnMetThrTrpPheLeuAsn

1200
TrpIleGluAsnLysThrHisArgAsnTyrAlaProCysHisIle

LysGlnIleIleAsnThrTrpHisLysValGlyArgAsnValTyr

1300
LeuProProArgGluGlyGluLeuSerCysAsnSerThrValThr

SerIleIleAlaAsnIleAspTrpGlnAsnAsnAsnGlnThrAsn

134

IleThrPheSerAlaGluValAlaGluLeuTyrArgLeuGluLeu

1400
GlyAspTyrLysLeuValGluIleThrProIleGlyPheAlaPro

ThrLysGluLysArgTyrSerSerAlaHisGlyArgHisThrArg

1500
GlyValPheValLeuGlyPheLeuGlyPheLeuAlaThrAlaGly

SerAlaMetGlyAlaArgAlaSerLeuThrValSerAlaGlnSer

1600
ArgThrLeuLeuAlaGlyIleValGlnGlnGlnGlnLeuLeu

AspValValLysArgGlnGlnGluLeuLeuArgLeuThrValTrp

1700
GlyThrLysAsnLeuGlnAlaArgValThrAlaIleGluLysTyr

LeuGlnAspGlnAlaArgLeuAsnSerTrpGlyCysAlaPheArg

1800
GlnValCysHisThrThrValProTrpValAsnAspSerLeuAla

ProAspTrpAspAsnMetThrTrpGlnGluTrpGluLysGlnVal

ArgTyrLeuGluAlaAsnIleSerLysSerLeuGluGlnAlaGln

1900
IleGlnGlnGluLysAsnMetTyrGluLeuGlnLysLeuAsnSer

TrpAspIlePheGlyAsnTrpPheAspLeuThrSerTrpValLys

2000
TyrIleGlnTyrGlyValLeuIleIleValAlaValIleAlaLeu

ArgIleValIleTyrValValGlnMetLeuSerArgLeuArgLys

2100
GlyTyrArgProValPheSerSerProProGlyTyrIleGlnGln

IleHisIleHisLysAspArgGlyGlnProAlaAsnGluGluThr

2200
GluGluAspGlyGlySerAsnGlyGlyAspArgTyrTrpProTrp

ProIleAlaTyrIleHisPheLeuIleArgGlnLeuIleArgLeu

LeuThrArgLeuTyrSerIleCysArgAspLeuLeuSerArgSer

```
       2300           •           •           •           •
     PheLeuThrLeuGlnLeuIleTyrGlnAsnLeuArgAspTrpLeu

     ArgLeuArgThrAlaPheLeuGlnTyrGlyCysGluTrpIleGln

                  2400           •           •           •
     GluAlaPheGlnAlaAlaAlaArgAlaThrArgGluThrLeuAla

     GlyAlaCysArgGlyLeuTrpArgValLeuGlnArgIleGlyArg

                              2500           •           •
     GlyIleLeuAlaValProArgArgIleArgGlnGlyAlaGluIle

     AlaLeuLeu
```

54. Rekombinante Nukleinsäure die eine c-DNS oder einen Teil dieser c-DNS nach einem der Ansprüche 46 bis 53, und/oder eine RNS oder einen Teil einer RNS, der dieser c-DNS entspricht, die in eine aus einem Vektor stammende Nukleinsäure inseriert ist.

55. Rekombinante Nukleinsäure nach Anspruch 52, dadurch gekennzeichnet, daß sie markiert ist.

56. Verfahren zum Nachweis eines Retrovirus, das auf den Menschen eine zytopathogene Wirkung ausüben kann, vom Typ HIV-2, oder seiner RNS in einer biologischen Flüssigkeit oder einem biologischen Gewebe, insbesondere im Hinblick auf den Nachweis des möglichen oder tatsächlichen Vorliegens von SLA oder AIDS in vitro beim Menschen, gekennzeichnet durch die Kontaktierung von in dieser biologischen Flüssigkeit oder diesem biologischen Gewebe enthaltenen Nukleinsäuren mit einer Sonde, die eine Nukleinsäure nach einem der Ansprüche 49 bis 53 enthält, unter stringenten Hybridisierungsbedingungen, oder eine DNS enthält, die eine Sequenzhomologie von wenigstens 70 %, vorzugsweise wenigstens 90 % mit der RNS eines Retrovirus nach einem der Ansprüche 1 bis 9 aufweist, und zur Hybridisierung mit dieser befähigt ist, unter nicht-stringenten Bedingungen, wobei die Kontaktierung nach der « Spot-Blot-Technik » während der für die Durchführung der Hybridisierung erforderlichen Zeitdauer durchgeführt wird, wonach das gebildete Hybrid mit einer Lösung gewaschen wird, welche die Beibehaltung der stringenten Bedingungen gewährleistet, sowie durch den Nachweis des gebildeten Hybrids.

57. Verfahren zur Herstellung eines Retrovirus, das auf den Menschen eine zytopathogene Wirkung auszuüben vermag, vom Typ HIV-2, gekennzeichnet durch die Züchtung in menschlichen T4-Lymphozyten oder in von T4-Lymphozyten abgeleiteten konstanten Zellinien, wobei diese den Phänotyp T4 aufweisen, und diese Lymphozyten oder Zellinien vorgängig mit einem Isolat des Virus HIV-2, wie in einem der Ansprüche 1 bis 9 definiert, infiziert worden waren, und insbesondere wenn das Aktivitätsniveau der Reverse-Transkriptase eine vorgegebene Schwelle erreicht hat, und durch Abtrennung und Reinigung der im Medium für die Züchtung dieser Lymphozyten oder Linien freigesetzten Virenmengen, insbesondere durch differentielle Zentrifugierung in einem Saccharose- oder Metrizamidgradienten.

58. Verfahren zur Herstellung von spezifischen Antigenen des Retrovirus HIV-2, gekennzeichnet durch die Lyse, insbesondere mit Hilfe von Detergentien wie Natriumdodecylsulfat (z.B. 0,1 % SDS in einem Puffer RIPA) des am Ende des Verfahrens nach Anspruch 37 erhaltenen gereinigten Virus, und die Abtrennung des, die genannten Antigene enthaltenen Lysats.

59. Verfahren zur Herstellung einer Hybridisierungssonde zum Nachweis einer RNS des Retrovirus HIV-2, gekennzeichnet durch die Inserierung einer DNS-Sequenz, insbesondere nach einem der Ansprüche 46 bis 53, in einen Klonierungsvektor durch Rekombination in vitro, durch die Klonierung des in einer kompetenten Wirtszelle erhaltenen modifizierten Vektors, sowie durch die Abtrennung der erhaltenen DNS-Rekombinanten.

60. Die sekretorischen Hybridome des monoklonalen Antikörpers nach Anspruch 45.

61. Verwendung der polyklonalen Antikörper nach Anspruch 44, oder der monoklonalen Antikörper nach Anspruch 45, zum Nachweis des Retrovirus HIV-2, wie in einem der Ansprüche 1 bis 9 definiert, in einem biologischen Präparat.

62. Verwendung der polyklonalen Antikörper nach Anspruch 44, oder der monoklonalen Antikörper nach Anspruch 45, zum Nachweis der Antigene des Retrovirus HIV-2, wie in einem der Ansprüche 1 bis 9 definiert, in einem biologischen Präparat.

63. Verwendung der polyklonalen Antikörper nach Anspruch 44, oder der monoklonalen Antikörper nach Anspruch 45, zur Reinigung der Virusantigene eines Retrovirus HIV-2, wie in einem der Ansprüche 1 bis 9 definiert.

EP 0 239 425 B1

**Patentansprüche** (für die Vertragsstaaten : AT, ES, GR)

1. Menschliches Retrovirus HIV-2, das die Fähigkeit besitzt, menschliche T4-Lymphozyten zu infizieren und geeignet ist, beim Menschen Aids hervorzurufen, wobei dieses Retrovirus eines der bei CNCM unter der Nummer I-502, I-532, I-642 und I-643 und bei ECACC unter der Nummer 87011001 und 87011002 (jeweils den Nummern I-502 und I-532 entsprechend) hinterlegten Retroviren oder eine Variation eines dieser Retroviren darstellt und alle Antigene enthält, die von den gegen die entsprechenden Antigene eines beliebigen Retrovirus, das Gegenstand der genannten Hinterlegungen bei CNCM oder ECACC ist, gebildeten Antikörper erkannt werden.

2. Menschliches Retrovirus nach Anspruch 1, dadurch gekennzeichnet, daß sich seine Genom-RNS unter stringenten Bedingungen mit der Komplementärkette eines c-DNS oder c-DNS-Fragments, gewonnen aus der Genom-RNS eines der bei CNCM unter der Nummer I-502, I-532, I-642 und I-643 und bei ECACC unter der Nummer 87011001 und 87011002 (jeweils den Nummern I-502 und I-532 entsprechend) hinterlegten Retroviren zu kreuzen vermag.

3. Menschliches Retrovirus nach Anspruch 1, dadurch gekennzeichnet, daß es eine Gesamtheit von Antigenen umfaßt, die durch Proteine des Kerns (Core) mit einem Molekulargewicht in der Größenordnung von 12000, 16000 bzw. 26000 Dalton, durch Proteine oder Glycoproteine mit jeweils einem Molekulargewicht in der Größenordnung von 36000 Dalton und in der Größenordnung von 42000 bis 45000 Dalton, und durch ein Hauptglycoprotein der Hülle mit einem Molekulargewicht in der Größenordnung von 130000 bis 140000 Dalton gebildet sind.

4. Gereinigtes menschliches Retrovirus nach Anspruch 1, das pathogen ist für den Menschen und Aids hervorrufen kann, gekennzeichnet durch folgende Gesamtheit von Eigenschaften :

— bevorzugtes Zielobjekt des Retrovirus HIV-2 sind menschliche Leu3-Zellen (oder T4-Lymphozyten) und von diesen T4-Lymphozyten abgeleitete konstante Zellinien ;

— es kann auf menschliche T4-Lymphozyten, die es infiziert eine zytopathogene Wirkung ausüben ;

— es übt eine Reverse-Transkriptase-Aktivität aus, die die Anwesenheit von $Mg^{2+}$-Ionen erforderlich macht, und zeigt dann starke Aktivität gegenüber Poly(adenylat-oligodeoxythymidylat)-(poly(A)-oligo(DT) 12-18 ;

— es hat eine Dichte von ca. 1,16 in einem Saccharosegradienten ;

— es hat einen durchschnittlichen Durchmesser von 140 nm und einen Kern mit einem durchschnittlichen Durchmesser von 41 nm ;

— es kann in konstanten, das Protein T4 exprimierenden Zellinien gezüchtet werden ;

— es ist für die T8-Lymphozyten nicht infektiös ;

— die Lysate dieses Virus enthalten ein Protein p26, das sich immunologisch nicht mit dem Protein p24 des Virus HTLV-1 oder des Virus HTLV-2 kreuzt ;

— diese Lysate enthalten außerdem ein Protein p16, das sich immunologisch nicht mit dem Protein p19 des HTLV-1 oder des HTLV-2 in Radioimmunausfällungstests kreuzt ;

— sie enthalten außerdem ein Hauptglycoprotein der Hülle mit einem Molekulargewicht in der Größenordnung von 130000 bis 140000 Dalton, das sich immunologisch nicht mit dem gp110 des Retrovirus HIV-1 kreuzt ;

— diese Lysate enthalten außerdem ein durch $^{35}$S-Cystein markierbares Protein oder Glycoprotein mit einem Molekulargewicht in der Größenordnung von 36000 Dalton ;

— die Genom-RNS des HIV-2 hybridisiert nicht, unter nicht-stringenten Bedingungen, mit der Genom-RNS des HIV-1, und hybridisiert nicht, unter nicht-stringenten Bedingungen, weder mit dem env-Gen noch mit den LTR des HIV-1 ;

— die Genom-RNS des HIV-2 hybridisiert schwach, unter nichtstringenten Bedingungen, mit den Nukleotidsequenzen, die einerseits die Nukleotide 990-1070, andererseits die Nukleotide 990-1260 der gag-Region des Genom-RNS des HIV-1 enthalten.

5. Retrovirus nach einem der Ansprüche 1 bis 4, dadurch gekennzeichnet, daß es sich nicht chronisch in Rhesusaffen-Lymphozyten vermehrt, wenn es in vivo injiziert wurde und unter Arbeitsbedingungen, welche die Entwicklung des Virus STLV-III$_{mac}$, wie von Letvin et al., Science (1985), Band 230, 71-75 beschrieben, ermöglichen.

6. Retrovirus nach einem der Ansprüche 1 bis 5, dadurch gekennzeichnet, daß die Sequenz, welche die R-Region und die U3-Region der ihre Genom-RNS umfassenden Sequenz enthält, eine Nukleotidsequenz aufweist, die der nachfolgenden Nukleotidsequenz entspricht :

GTGGAACGCGAGACTGAAAGCAAGAGGAATACCATTTAGTTAAAGGACAG

GAACAGCTATACTTGGTCAGGGCAGGAAGTAACTAACAGAAACAGCTGAG

ACTGCAGGGACTTTCCAGAAGGGGCTGTAACCAAGGGAGGGACATGGGAG

GAGCTGGTGGGGAACGCCTCATATTCTCTGTATAATATACCCGCTGCTTG

CATTGTACTTCAGTCGCTCTGCGGAGAGGCTGGCAGATTGAGCCCTGGAG

137

GATCTCTCCAGCACTAGACGGATGAGCCTGGGTGCCCTGCTAGACTCTCA
CCAGCACTTGGCCGGTGCTGGCAGACGGCCCCACGCTTGCCTGCTTAAA
ACCTTCCTTAATAAAGCTGCAGTAGAAGCA

7. Retrovirus nach einem der Ansprüche 1 bis 5, dadurch gekennzeichnet, daß seine Genom-RNS eine gag-Sequenz enthält, die der folgenden Nukleotidsequenz entspricht:

GAGRODE .

ATGGGCGCCGAGAAACTCCGTGTTGAGAGGGGTAAAAAGCAGATGAA

TTACAAAGAATCAGGTTACGGCCCCGCGGCAAAGAAAAAGTACAGG

CTAAAACATATTGTGTGGGCAGCCGAATAAATTGGACAGATTCGGA
100

TTAGCACAGACCCTGTTCGAGTCAAAGACGGTTCTCAAAAAATT

CTTACAGTTTTAGATCCAATGGTACCGACAGGTTCAGAAAATTTA
200

AAAGTCTTTTTAATACTGTCTGCGTCATTTGGTGCATACACGCA

GAACAGAAAGTCAAAGATACTCAAGGAGCCAAAACAAATAGTGCGG
300

AGACATCTAGTGGCAGAAACAGGAACTGCAGAGAAAATGCCAAGC

ACAAGTAGACCAACAGCACCATCTAGCCGACAAGGGACGAAATTAC
400

CCAGTGCAACATGTAGGCCGGCAACTACACCCATATACCGCTGAGT

CCCCGAACCCTAAATGCCTGGGTAAAATTACTAGAGGAAAAAAAG

TTCCGGCAGAAGTAGTGCCACGGATTTCAGGCACTCTCAGAAGGC
500

TGCACGCCCTATGATATCAACCAAATGCTTAATTGTGTGGGCCAC

CATCAAGCAGCCATGCAGATAATCAGGGAGATTATCAATGAGGAA
600

GCAGCAGAATGGGATGTGCCAACATCCAATACCAGCCCCCTTACCA

GCGGGCAGCTTAGAGAGCCAAGGGGATCTGACATAGCAGGGACA
700

ACAAGCACAGTACAACAACAGATCCAGTGCATGTTTACGCCACAA

138

EP 0 239 425 B1

AATCCTGTACCACTAGGAAACATCTATAGAACATGCATCCACATA
800

GGATTGCAGAAGTGTGTCAGGATGTACAACCCGACCAACATCCTA

CACATAAACAGGGACCAAAGGAGCCCGTTCCAAAGCTATGTACAT
900

AGATTCTACAAAAGCTTGAGGGCAGAACAAACAGATCCAGCAGTG

AAGAATTCGATGACCCAAACACTGCTAGTACAAAATCCCAACCCA

GACTCTAAATTACTGCTAAAAGGACTAGGGATGAACCCTACCTTA
1000

GAAGAGATGCTGACCGCCTGTCAGGGGGTAGGTGGGCCACGCCAG

AAAGCTAGATTAATGGCAGAGGCCCTGAAAGAGGTCATACGACCT
1100

GCCCCTATCCCATTCGCAGCAGCCCAGCAGAGAAAGGCATTTAAA

TGCTGGAACTGTGGAAAGGAAGGGCACTCGGuuAGACAATGCCCGA
1200

GCACCTAGAAGGCAGGGCTGCTGGAAGTGTGGTAAGCCAGGACAC

ATCATGACAAACTGCCCAGATAGACAGGCAGGTTTTTTAGGACTG
1300

GGCCCTTGGGGAAAGAAGCCCCGCAACTTCCCCGTGGCCCAAGTT

CCGCAGGGGCTGACACCAACAGCACCCCCAGTGGATCCAGCAGTG

GATCTACTGGACAAATATATGCAGCAAGGGAAAAGACAGACACAG
1400

CAGAGAGAGACACCATACAAGGAAGTGACAGAGGACTTACTGCAC.

CTGCAGCAGGGGCACACACCATACACGGACCCACCAACAGACGAG
1500

TTCCTGCACCTCAATTCTCTCTTTGGAAAAGACCAG

8. Retrovirus nach einem der Ansprüche 1 bis 6, dadurch gekennzeichnet, daß seine Genom-RNS eine env-Sequenz enthält, die der folgenden Nukleotidsequenz entspricht :

ENVII

ATGATGAATCAGCTGCTTATTGCCATTTTATTAGCTAGTGCTTGC

139

TTAGTATATTGCACCCAATATGTAACTGTTTTCTATGGCGTACCC

ACGTGGAAAAATGCAACCATTCCCCTCTTTTGTGCAACCAGAAT
100

ACGGATACTTGGGCAACCATACAGTGCTTGCCTGACAATGATGAT

TATCAGGAAATAACTTTGAATGTAACAGACGGCTTTTGATGCATGG
200

AATAATACAGTAACAGAACAAGCAATAGAAGATGTCTGGCATCTA

TTCGAGACATCAATAAAACCATGTGTCAAACTAACACCTTTATGT
300

GTAGCAATGAAATGCAGCAGCACAGAGAGCAGCACAGGGAACAAC

ACAACCTCAAAGAGCACAAGCACAACCACAACCACACCCACACAC
400

CACGAGCAAGAGATAAGTCAGGATACTCCATGCGCACGGGCAGAG

AACTGCTCAGGATTCGGACAGGAAGAAACGATCAATTGCCAGTTC

AATATGACAGGATTAGAAAGAGATAAGAAAAAACACTATAATGAA
500

ACATGGTACTCAAAAGATGTGGTTTGTGAGACAAATAATAGCACA

AATCAGACCCAGTGTTACATGAACCATTGCAACACATCAGTCATC
600

ACAGAATCATGTGACAAGCACTATTGGGATGCTATAAGGTTTACA

TACTGTGCACCACCGGGTTATGCCCTATTAAGATGTAATGATACC
700

AATTATTCACGGCTTTGCACCCAACTGTTCTAAAGTAGTAGCTTCT

ACATGCACCAGGATGATGGAAACGCAAACTTCCACATGGTTTGGC
800

TTTAATGGCACTAGAGCAGAGAATAGAACATATATCTATTGGCAT

GGCAGAGATAATACAACTATCATCAGCTTAAACAAATATTATAAT
900

CTCAGTTTGCATTGTAAGAGGCCAGGGAATAAGACAGTGAAACAA

ATAATGCTTATGTCAGGACATGTGTTTCACTCCCACTACCAGCCG

ATCAATAAAAGACCCAGACAAGCATGGTGCTGCTTCAAAGGCAAA
1000

TGGAAAGACGCCATGCAGGAGGTGAAGACCCTTGCAAAACATCCC

AGGTATAGACGAACCAATGACACAAGGAATATTAGCTTTGCAGCG
1100

CCAGGAAAAGGCTCAGACCCAGAAGTAGCATACATGTGGACTAAC

TGCAGAGGAGAGTTTCTCTACTGCAACATGACTTGGTTCCTCAAT
1200

TGGATAGAGAATAAGACACACCGCAATTATGCACCGTGCCATATA

AAGCAAATAATTAACACATGGCATAAGGTAGGCAGAAATGTATAG
1300

TTGCCTCCCAGGGAAGGGCAGCTGTCCTGCAACTCAACAGTAACC

AGCATAATTGCTAACATTGACTGGCAAAACAATAATCAGACAAAC

ATTACCTTTAGTGCAGAGGTGGCAGAACTATACAGATTGGAGTTG
1400

GGACATTATAAATTCGTAGAAATAACACCAATTGGCTTCGCACCT

ACAAAGAAAAAGATACTCCTCTGCTCACGGGAGACATACAAGA
1500

GGTGTGTTCGTGCTAGGGTTCTTGGGTTTTCTCGCAACAGCACGT

TCTGCAATGGGCGCTCGAGCGTCCCTGACCGTGTCGGCTCAGTCC
1600

CGGACTTTACTGGCCGGGATAGTGCAGCAACAGCAACAGCTCTTG

GACGTGGTCAAGACACAACAAGAACTGTTGCGACTGACCGTCTGG
1700

GGAACGAAAAACCTCCAGGCAAGAGTCACTGCTATAGACAAGTAC

CTACAGGACCAGGCGCGGCTAAATTCATGGGGATGTGCCGTTAGA
1800

CAAGTCTGCCACACTACTGTACCATGGGTTAATGATTCGTTAGCA

```
CCTGACTGGGACAATATGACGTGGCAGGAATGGGAAAAACAAGTC

CGCTACCTGGAGGCAAATATCAGTAAAAGTTTACAACAGGCACAA
            1900

ATTCAGCAAGAGAAAAATATGTATGAACTACAAAAATTAAATAGC

TGGGATATTTTTGGCAATTGGTTTGACTTAACCTCCTGGGTCAAG
              2000

TATATTCAATATGGAGTGCTTATAATAGTAGCAGTAATAGCTTTA

AGAATAGTGATATATGTAGTACAAATGTTAAGTAGGCTTAGAAAG
                      2100

GGCTATAGGCCTGTTTTCTCTTCCCCCCCCGGTTATATCCAACAG

ATCCATATCCACAAGGACCGGGACAGCCACCCAACGAAGAAACA
                              2200

GAAGAAGACGGTGGAAGCAACGGTGCAGACAGATACTGGCCCTGG

GCGATAGCATATATACATTTCCTGATCCGCCAGCTGATTCGCCTC

TTGACCAGACTATACAGCATCTGCAGGGACTTACTATCCAGGAGC
2300

TTCCTGAUUUTCCAACTCATCTACCAGAATCTCAGAGACTGGCTG

AGACTTAGAACAGCCTTCTTGCAATATGGGTGCGAGTGGATCCAA
            2400

GAAGCATTCCACGCCGCCGCGAGCGGCTACAAGAGAGACTCTTGCG

GGCGCGTGCAGGGGCTTGTGGAGGGTATTGGAACGAATCCCGAGG
                      2500

GGAATACTCCGCGGTTCCAAGAACGATCAGACACGGACCAGAAATC

GCCCTCCTG
```

9. Retrovirus nach einem der Ansprüche 1 bis 8, dadurch gekennzeichnet, daß seine RNS unter stringenten Bedingungen nicht mit der Genom-RNS des HIV-1 hybridisiert, und unter nicht-stringenten Bedingungen nicht mit dem env-Gen und dem angrenzenden LTR hybridisiert, insbesondere nicht mit der Nukleotidsequenz 5290-9130 des HIV-1, und unter nicht-stringenten Bedingungen nicht mit den Sequenzen der pol-Region des Genoms von HIV-1, insbesondere nicht mit der Nukleotidsequenz 2170-2240 hybridisiert.

10. Verfahren zur Herstellung von spezifischen Antigenen eines Retrovirus HIV-2, gekennzeichnet durch die Lyse, insbesondere mit Hilfe von Detergentien wie Natriumdodecylsulfat (z.B. 0,1 % SDS in einem Puffer RIPA) des Retrovirus HIV-2 nach einem der Ansprüche 1 bis 9, sowie durch die Abtrennung des Lysats, worin die genannten Antigene enthalten sind.

11. Verfahren nach Anspruch 10, das außerdem die Trennung vom Lysat und die Reinigung eines der Antigene umfaßt, welches aus einem der Proteine oder Glycoproteine p12, p16, p26, gp36, p42, gp140 besteht.

12. Verfahren nach Anspruch 11, dadurch gekennzeichnet, daß das Antigen mit den immunologischen Charakteristika des Proteins p12 des HIV-2, das immunologisch durch gegen ein Retrovirus nach einem der Ansprüche 1 bis 9 gebildete Antikörper erkannt wurde, und ein Molekulargewicht in der Größenordnung von 12000 Dalton aufweist, abgetrennt und gereinigt wird.

13. Verfahren nach Anspruch 11, dadurch gekennzeichnet, daß das Antigen mit den immunologischen Charakteristika des Proteins p16 des HIV-2, das immunologisch durch gegen ein Retrovirus nach einem der Ansprüche 1 bis 9 gebildete Antikörper erkannt wurde und ein Molekulargewicht in der Größenordnung von 16000 Dalton aufweist, abgetrennt und gereinigt wird.

14. Verfahren nach Anspruch 11, dadurch gekennzeichnet, daß das Antigen mit den immunologischen Charakteristika des Proteins p26 des HIV-2, das immunologisch durch gegen ein Retrovirus nach einem der Ansprüche 1 bis 9 gebildete Antikörper erkannt wurde und ein Molekulargewicht in der Größenordnung von 26000 Dalton aufweist, abgetrennt und gereinigt wird.

15. Verfahren nach Anspruch 11, dadurch gekennzeichnet, daß das Antigen mit den immunologischen Charakteristika des Glycoproteins gp36 des HIV-2, das immunologisch durch gegen ein Retrovirus nach einem der Ansprüche 1 bis 9 gebildete Antikörper erkannt wurde und ein Molekulargewicht in der Größenordnung von 36000 Dalton aufweist, abgetrennt und gereinigt wird.

16. Verfahren nach Anspruch 11, dadurch gekennzeichnet, daß das Antigen mit den immunologischen Charakteristika des Proteins p42 des HIV-2, das immunologisch durch gegen ein Retrovirus nach einem der Ansprüche 1 bis 9 gebildete Antikörper erkannt wurde und ein Molekulargewicht in der Größenordnung von 42000 bis 45000 Dalton aufweist, abgetrennt und gereinigt wird.

17. Verfahren nach Anspruch 11, dadurch gekennzeichnet, daß das Antigen mit den immunologischen Charakteristika des Glycoproteins gp140 des HIV-2, das immunologisch durch gegen ein Retrovirus nach einem der Ansprüche 1 bis 9 gebildete Antikörper erkannt wurde und ein Molekulargewicht in der Größenordnung von 130000 bis 140000 Dalton aufweist, abgetrennt und gereinigt wird.

18. Verfahren nach Anspruch 11, dadurch gekennzeichnet, daß das Antigen, das die nachfolgend angeführte Aminosäuresequenz oder einen Teil dieser Sequenz enthält, von dem Zeitpunkt an, an dem sie eine spezifische immunologische Reaktion mit den Antikörpern gegen ein Retrovirus HIV-2 nach einem der Ansprüche 1 bis 9 auslöst, insbesondere wenn dieses Antigen mit einem Serum eines mit HIV-2 infizierten Patienten in Kontakt gebracht wird, abgetrennt und gereinigt wird.

ArgLysAlaPheLys

CysTrpAsnCysGlyLysGluGlyHisSerAlaArgGlnCysArg

1200
AlaProArgArgGlnGlyCysTrpLysCysGlyLysProGlyHis

IleMetThrAsnCysProAspArgGlnAlaGlyPheLeuGlyLeu

1300
GlyProTrpGlyLysLysProArgAsnPheProValAlaGlnVal

ProGlnGlyLeuThrProThrAlaProProValAspProAlaVal

AspLeuLeuGluLysTyrMetGlnGlnGlyLysArgGlnArgGlu

1400
GlnArgGluArgProTyrLysGluValThrGluAspLeuLeuHis

LeuGluGlnGlyGlnThrProTyrArgGluProProThrGluAsp

1500
LeuLeuIleLeuAsnSerLeuPheGlyLysAspGln

19. Verfahren nach Anspruch 11, dadurch gekennzeichnet, daß das Antigen, das die nachfolgend angeführte Aminosäuresequenz oder einen Teil dieser Sequenz enthält, von dem Zeitpunkt an, an dem sie eine spezifische immunologische Reaktion mit den Antikörpern gegen ein Retrovirus HIV-2 nach einem der Ansprüche 1 bis 9 auslöst, insbesondere wenn dieses Antigen mit einem Serum eines mit HIV-2 infizierten Patienten in Kontakt gebracht wird, abgetrennt und gereinigt wird.

```
MetGlyAlaArgAsnSerValLeuArgGlyLysLysAlaAspGln

LeuGluArgIleArgLeuArgProGlyGlyLysLysLysTyrArg

LeuLysHisIleValTrpAlaAlaAsnLysLeuAspArgPheGly
             100
LeuAlaGluSerLeuLeuGluSerLysGlnGlyCysGlnLysIle

LeuThrValLeuAspProMetValProThrGlySerGluAsnLeu
             200
LysSerLeuPheAsnThrValCysValIleTrpCysIleHisAla

GluGlnLysValLysAspThrGluGlyAlaLysGlnIleValArg
                    300
ArgHisLeuValAlaGlnThrGlyThrAlaGluLysMetProSer

ThrSerArgProThrAlaProSerSerGluLysGlyGlyAsnTyr
                              400
```

20. Verfahren nach Anspruch 11, dadurch gekennzeichnet, daß das Antigen, das die nachfolgend angeführte Aminosäuresequenz oder einen Teil dieser Sequenz enthält, von dem Zeitpunkt an, an dem sie eine spezifische immunologische Reaktion mit Antikörpern gegen ein Retrovirus HIV-2 nach einem der Ansprüche 1 bis 9 auslöst, insbesondere wenn dieses Antigen mit einem Serum eines HIV-2 infizierten Patienten in Kontakt gebracht wird, abgetrennt und gereinigt wird.

```
ProValGlnHisValGlyGlyAsnTyrThrHisIleProLeuSer

ProArgThrLeuAsnAlaTrpValLysLeuValGluGluLysLys

PheGlyAlaGluValValProGlyPheGlnAlaLeuSerGluGly
    500
CysThrProTyrAspIleAsnGlnMetLeuAsnCysValGlyAsp

HisGlnAlaAlaMetGlnIleIleArgGluIleIleAsnGluGlu
              600
AlaAlaGluTrpAspValGlnHisProIleProGlyProLeuPro

AlaGlyGlnLeuArgGluProArgGlySerAspIleAlaGlyThr
                    700
ThrSerThrValGluGluGlnIleGlnTrpMetPheArgProGln

AsnProValProValGlyAsnIleTyrArgArgTrpIleGlnIle
```

```
                                             800
GlyLeuGlnLysCysValArgMetTyrAsnProThrAsnIleLeu

AspIleLysGlnGlyProLysGluProPheGlnSerTyrValAsp

                                             900
ArgPheTyrLysSerLeuArgAlaGluGlnThrAspProAlaVal

LysAsnTrpMetThrGlnThrLeuLeuValGlnAsnAlaAsnPro

AspCysLysLeuValLeuLysGlyLeuGlyMetAsnProThrLeu

          1000
GluGluMetLeuThrAlaCysGlnGlyValGlyGlyProGlyGln

LysAlaArgLeuMetAlaGluAlaLeuLysGluValIleGlyPro

                 1100
AlaProIleProPheAlaAlaAlaGlnGln
```

21. Verfahren nach Anspruch 11, dadurch gekennzeichnet, daß das Antigen, das die nachfolgend angeführte Aminosauresequenz oder einen Teil dieser Sequenz enthält, von dem Zeitpunkt an, an dem sie eine spezifische immunologische Reaktion mit Antikörpern gegen ein Retrovirus HIV-2 nach einem der Ansprüche 1 bis 9 auslöst, insbesondere wenn dieses Antigen mit einem Serum eines HIV-2 infizierten Patienten in Kontakt gebracht wird, abgetrennt und gereinigt wird.

```
HIV-2

MetMetAsnGlnLeuLeuIleAlaIleLeuLeuLeuAlaSerAlaCys

LeuValTyrCysThrGlnTyrValThrValPheTyrGlyValPro

ThrTrpLysAsnAlaThrIleProLeuPheCysAlaThrArgAsn

    100
ArgAspThrTrpGlyThrIleGlnCysLeuProAspAsnAspAsp

TyrGlnGluIleThrLeuAsnValThrGluAlaPheAspAlaTrp

            200
AsnAsnThrValThrGluGlnAlaIleGluAspValTrpHisLeu

PheGluThrSerIleLysProCysValLysLeuThrProLeuCys

                    300
ValAlaMetLysCysSerSerThrGluSerSerThrGlyAsnAsn

ThrThrSerLysSerThrSerThrThrThrThrThrProThrAsp

                    400
```

145

GlnGluGlnGluIleSerGluAspThrProCysAlaArgAlaAsn

AsnCysSerGlyLeuGlyGluGluGluThrIleAsnCysGlnPhe

AsnMetThrGlyLeuGluArgAspLysLysLysGlnTyrAsnGlu

500
ThrTrpTyrSerLysAspValValCysGluThrAsnAsnSerThr

AsnGlnThrGlnCysTyrMetAsnHisCysAsnThrSerValIle

600
ThrGluSerCysAspLysHisTyrTrpAspAlaIleArgPheArg

TyrCysAlaProProGlyTyrAlaLeuLeuArgCysAsnAspThr

700
AsnTyrSerGlyPheAlaProAsnCysSerLysValValAlaSer

ThrCysThrArgMetMetGluThrGlnThrSerThrTrpPheGly

800
PheAsnGlyThrArgAlaGluAsnArgThrTyrIleTyrTrpHis

GlyArgAspAsnArgThrIleIleSerLeuAsnLysTyrTyrAsn

900
LeuSerLeuHisCysLysArgProGlyAsnLysThrValLysGln

IleMetLeuMetSerGlyHisValPheHisSerHisTyrGlnPro

IleAsnLysArgProArgGlnAlaTrpCysTrpPheLysGlyLys

1000
TrpLysAspAlaMetGlnGluValLysThrLeuAlaLysHisPro

ArgTyrArgGlyThrAsnAspThrArgAsnIleSerPheAlaAla

1100
ProGlyLysGlySerAspProGluValAlaTyrMetTrpThrAsn

CysArgGlyGluPheLeuTyrCysAsnMetThrTrpPheLeuAsn

1200
TrpIleGluAsnLysThrHisArgAsnTyrAlaProCysHisIle

LysGlnIleIleAsnThrTrpHisLysValGlyArgAsnValTyr

146

1300
LeuProProArgGluGlyGluLeuSerCysAsnSerThrValThr

SerIleIleAlaAsnIleAspTrpGlnAsnAsnAsnGlnThrAsn

IleThrPheSerAlaGluValAlaGluLeuTyrArgLeuGluLeu
1400
GlyAspTyrLysLeuValGluIleThrProIleGlyPheAlaPro

ThrLysGluLysArgTyrSerSerAlaHisGlyArgHisThrArg

1500
GlyValPheValLeuGlyPheLeuGlyPheLeuAlaThrAlaGly

SerAlaMetGlyAlaArgAlaSerLeuThrValSerAlaGlnSer

1600
ArgThrLeuLeuAlaGlyIleValGlnGlnGlnGlnGlnLeuLeu

AspValValLysArgGlnGlnGluLeuLeuArgLeuThrValTrp

1700
GlyThrLysAsnLeuGlnAlaArgValThrAlaIleGluLysTyr

LeuGlnAspGlnAlaArgLeuAsnSerTrpGlyCysAlaPheArg

1800
GlnValCysHisThrThrValProTrpValAsnAspSerLeuAla

ProAspTrpAspAsnMetThrTrpGlnGluTrpGluLysGlnVal

ArgTyrLeuGluAlaAsnIleSerLysSerLeuGluGlnAlaGln

1900
IleGlnGlnGluLysAsnMetTyrGluLeuGlnLysLeuAsnSer

TrpAspIlePheGlyAsnTrpPheAspLeuThrSerTrpValLys

2000
TyrIleGlnTyrGlyValLeuIleIleValAlaValIleAlaLeu

ArgIleValIleTyrValValGlnMetLeuSerArgLeuArgLys

2100
GlyTyrArgProValPheSerSerProProGlyTyrIleGlnGln

IleHisIleHisLysAspArgGlyGlnProAlaAsnGluGluThr

EP 0 239 425 B1

```
                              •          • .      2200
GluGluAspGlyGlySerAsnGlyGlyAspArgTyrTrpProTrp

PzoIleAlaTyrIleHisPheLeuIleArgGlnLeuIleArgLeu

LeuThrArgLeuTyrSerIleCysArgAspLeuLeuSerArgSer

2300
PheLeuThrLeuGlnLeuIleTyrGlnAsnLeuArgAspTrpLeu

ArgLeuArgThrAlaPheLeuGlnTyrGlyCysGluTrpIleGln

           2400
GluAlaPheGlnAlaAlaAlaArgAlaThrArgGluThrLeuAla

GlyAlaCysArgGlyLeuTrpArgValLeuGluArgIleGlyArg

                     2500
GlyIleLeuAlaValProArgArgIleArgGlnGlyAlaGluIle

AlaLeuLeu
```

22. Methode zum Nachweis der Anwesenheit von Antikörpern in vitro, die bei einem durch ein menschliches Retrovirus HIV-2 infizierten Menschen induziert wurden, in einer biologischen Probe vom Menschen, wie einem Serum, insbesondere zum Nachweis in vitro von möglicherweise oder tatsächlich vorhandenem SLA oder AIDS aufgrund eines solchen Retrovirus, und die von der Person stammt, die Gegenstand des Nachweises ist, dadurch gekennzeichnet, daß man diese biologische Probe mit einem Antigen in Kontakt bringt, das durch einen bei dem durch ein menschliches Retrovirus HIV-2, wie es in einem der Ansprüche 1 bis 9 definiert wurde, infizierten Menschen induzierten Antikörper erkannt wurde, unter Bedingungen, welche die Bildung einer immunologischen Verbindung zwischen diesem Antigen und dem besagten Antikörper ermöglichen, und daß man das gegebenenfalls zwischen diesem Antikörper und dem eingesetzten Antigen gebildete immunologische Konjugat nachweist.

23. Methode nach Anspruch 22, dadurch gekennzeichnet, daß man die biologische Probe mit einem, durch das Verfahren nach einem der Ansprüche 12 bis 22 erhaltene Antigen, oder einer Mischung dieser Antigene, unter Bedingungen in Kontakt bringt, welche die Bildung einer immunologischen Verbindung zwischen diesem Antigen und dem besagten Antikörper ermöglichen, und daß man das gegebenenfalls zwischen diesem Antikörper Anti-HIV-2 und dem eingesetzten Antigen gebildete immunologische Konjugat nachweist.

24. Methode nach Anspruch 23, dadurch gekennzeichnet, daß man den Nachweis des immunologischen Konjugats so durchführt, daß man das gegebenenfalls gebildete immunologische Konjugat mit einem markierten Reagens, gebildet durch menschliche Antiimmunoglobulin-Antikörper oder durch ein Bakterienprotein A, reagieren läßt und den gebildeten Komplex zwischen dem Reagens und dem genannten immunologischen Konjugat nachweist.

25. Methode zum Nachweis der Anwesenheit von Antikörpern in vitro, die Antigene eines menschlichen Retrovirus erkennen, das auf menschliche T4-Lymphozyten eine zytopathogene Wirkung auszuüben vermag, in einer biologischen Probe vom Menschen, wie einem Serum, insbesondere zum Nachweis in vitro von möglicherweise oder tatsächlich vorhandenem SLA oder AIDS aufgrund eines menschlichen Retrovirus, dadurch gekennzeichnet, daß man diese biologische Probe, die aus der Person stammt, die Gegenstand des Nachweises ist, mit einer Mischung von Antigenen in Kontakt bringt, die mit den Antikörpern einerseits gegen ein menschliches Retrovirus HIV-2, das eine zytopathogene Wirkung auszuüben vermag, wie in einem der Ansprüche 1 bis 9 definiert wird, und andererseits gegen ein menschliches Retrovirus HIV-1 eine spezifische immunologische Reaktion einzugehen vermögen, und daß man das gegebenenfalls zwischen diesen Antikörpern und wenigstens einem dieser Antigene gebildete immunologische Konjugat nachweist.

26. Methode nach Anspruch 25, dadurch gekennzeichnet, daß die aus der Person, die Gegenstand des Nachweises darstellt, stammende biologische Probe mit einem Gemisch von gleichzeitig aus HIV-1 und HIV-2 nach einem der Ansprüche 1 bis 9 stammenden Antigenen, und insbesondere mit den durch das Verfahren nach einem der Ansprüche 11 bis 21 erhaltenen Antigenen, und den durch dasselbe Verfahren erhaltenen HIV-1-Antigenen, wenn es nur bei einem Retrovirus HIV-1 angewendet wird, in Kontakt gebracht wird, und daß man das gegebenenfalls zwischen diesen Antikörpern und wenigstens einem dieser Antigene gebildete immunologische Konjugat nachweist.

27. Verfahren zum Erhalt von Antikörpern, die spezifisch ein Antigen eines HIV-2 Retrovirus erkennen, das

— die Immunisierung eines Tieres mit einem HIV-2 Retrovirus nach einem der Ansprüche 1 bis 9 umfaßt, sowie

— das Abtrennen der gebildeten Antikörper.

28. Verfahren zur Herstellung einer Nukleinsäure oder eines Nukleinsäurefragments der RNS eines HIV-2-Retrovirus nach einem der Ansprüche 1 bis 9, das

— die Ultra-Zentrifugierung des Überstandes einer Zellkultur, die durch ein Isolat des HIV-2 Retrovirus nach einem der Ansprüche 1 bis 9 infiziert worden ist, sowie das Abtrennen des Zentrifugierungsrückstands,

— gegebenenfalls die Zentrifugierung des abgetrennten Rückstands in einem Saccharosegradienten durch die in der EP-A 0 138 667 beschriebenen Methode, und das Abtrennen des Rückstandsediments, das das RNS-Genom enthält,

— die Synthese eines c-DNS-Initiatorstranges in Kontakt mit der RNS des gereinigten, sedimentierten Virus in Anwesenheit eines Oligo (dT)-Initiators während einer endogenen Reaktion, aktiviert durch ein Detergents, gemäß dem Verfahren, wie es in « Nature 312 : 757-760 (1984) » beschrieben ist,

— gegebenenfalls die Reinigung der RNS-c-DNS-Hybride durch Extraktion durch eine Phenol-Chloroformmischung und Ausfällung mit Ethanol,

— die Herstellung eines zweiten c-DNS-Stranges in Anwesenheit von DNS-Polymerase I, RNaseH und von 4 Desoxynukleotiden, gemäß dem in « Gene, 25 : 263-269 (1983) » beschriebenen Verfahren, falls erforderlich nach der Extraktion der Proteine die im Reaktionsmedium vorhanden sind,

— sowie das Abtrennen der so erhaltenen Nukleinsäure umfaßt.

29. Verfahren nach Anspruch 28, dadurch gekennzeichnet, daß man eine gegebenenfalls markierte Nukleinsäure abtrennt, die von wenigstens einem Teil der RNS HIV-2-Virus, wie in einem der Ansprüche 1 bis 9 definiert, stammt, der sich unter stringenten Bedingungen mit der RNS des genannten HIV-2 Virus kreuzt, und sich unter stringenten Bedingungen nicht mit den Sonden 1 bis 4 zu kreuzt, die jeweils die Nukleotide 990-1070, 990-1260, 2170-2240 bzw. 3370-3640 der RNS des HIV-1-Genoms umfassen.

30. Verfahren nach Anspruch 29, dadurch gekennzeichnet, daß eine Nukleinsäure, die einen Teil wenigstens einer c-DNS-Sequenz enthält, der sich mit der gesamten Genom-RNS eines Retrovirus HIV-2 nach einem der Ansprüche 1 bis 9 zu kreuzen vermag, und sich unter stringenten Bedingungen nicht mit den Sonden kreuzt, deren DNS-Fragmente vom Genom des HIV-1 abgeleitet bzw. aus durch die Nukleotide 990-1070, 990-1260, 2170-2240 bzw. 3370-3640 des RNS-Derivats vom Genom des HIV-1 begrenzte Fragmente gebildet sind, abgetrennt wird.

31. Verfahren nach Anspruch 29, dadurch gekennzeichnet, daß man eine Nukleinsäure abtrennt, die nachfolgend angeführte Nukleotidsequenz enthält :

GTGGAAGGCGAGACTGAAAGCAAGAGGAATACCATTTAGTTAAAGGACAG

GAACAGCTATACTTGGTCAGGGCAGGAAGTAACTAACAGAAACAGCTGAG

ACTGCAGGGACTTTCCAGAAGGGGCTGTAACCAAGGGAGGGACATGGGAG

GAGCTGGTGGGGAACGCCTCATATTCTCTGTATAATATACCCGCTGCTTG

CATTGTACTTCAGTCGCTCTGCGGAGAGGCTGGCAGATTGAGCCCTGGAG

GATCTCTCCAGCACTAGACGGATGAGCCTGGGTGCCCTGCTAGACTCTCA

CCAGCACTTGGCCGGTGCTGGCAGACGGCCCCACGCTTGCCTGCTTAAAA

ACCTTCCTTAATAAAGCTGCAGTAGAAGCA

32. Verfahren nach Anspruch 29, dadurch gekennzeichnet, daß man eine Nukleinsäure abtrennt, die eine Nukleotidsequenz enthält, welche die Gesamtheit oder einen Teil der nachfolgend angeführten Aminosäuresequenz kodiert, wobei diese Aminosäuresequenz oder ein Teil davon eine spezifische immunologische Reaktion mit Antikörpern gegen ein Retrovirus HIV-2, wie in einem der Ansprüche 1 bis 9 definiert, auszulösen vermag, wenn diese Aminosäuresequenz mit einem Serum eines mit einem Retrovirus HIV-2 infizierten Patienten in Kontakt gebracht wird :

CACRODH

MetGlyAlaArgAsnSerValLeuArgGlyLysLysAlaAspGlu

LeuGluArgIleArgLeuArgProGlyGlyLysLysLysTyrArg

LeuLysHisIleValTrpAlaAlaAsnLysLeuAspArgPheGly

100
LeuAlaGlySerLeuLeuGluSerLysGluGlyCysGlnLysIle

LeuThrValLeuAspProMetValProThrGlySerGluAsnLeu

200
LysSerLeuPheAsnThrValCysValIleTrpCysIleHisAla

GluGluLysValLysAspThrGluGlyAlaLysGlnIleValArg

300
ArgHisLeuValAlaGluThrGlyThrAlaGlnLysMetProSer

ThrSerArgProThrAlaProSerSerGluLysGlyGlyAsnTyr

400
ProValGlnHisValGlyGlyAsnTyrThrHisIleProLeuSer

ProArgThrLeuAsnAlaTrpValLysLeuValGluGluLysLys

PheGlyAlaGluValValProGlyPheGlnAlaLeuSerGluGly

500
CysThrProTyrAspIleAsnGlnMetLeuAsnCysValGlyAsp

HisGlnAlaAlaMetGlnIleIleArgGlyIleIleAsnGluGlu

600
AlaAlaGluTrpAspValGlnHisProIleProGlyProLeuPro

AlaGlyGlnLeuArgGluProArgGlySerAspIleAlaGlyThr

700
ThrSerThrValGluGluGlnIleGlnTrpMetPheArgProGln

AsnProValProValGlyAsnIleTyrArgArgTrpIleGlnIle

800
GlyLeuGlnLysCysValArgMetTyrAsnProThrAsnIleLeu

AspIleLysGlnGlyProLysGluProPheGlnSerTyrValAsp

900
ArgPheTyrLysSerLeuArgAlaGluGlnThrAspProAlaVal

LysAsnTrpMetThrGlnThrLeuLeuValGlnAsnAlaAsnPro

AspCysLysLeuValLeuLysGlyLeuGlyMetAsnProThrLeu

1000
GluGluMetLeuThrAlaCysGlnGlyValGlyGlyProGlyGln

LysAlaArgLeuMetAlaGluAlaLeuLysGluValIleGlyPro

1100
AlaProIleProPheAlaAlaAlaGlnGlnArgLysAlaPheLys

CysTrpAsnCysGlyLysGluGlyHisSerAlaArgGlnCysArg

1200
AlaProArgArgGlnGlyCysTrpLysCysGlyLysProGlyHis

IleMetThrAsnCysProAspArgGlnAlaGlyPheLeuGlyLeu

1300
GlyProTrpGlyLysLysProArgAsnPheProValAlaGlnVal

ProGlnGlyLeuThrProThrAlaProProValAspProAlaVal

AspLeuLeuGluLysTyrMetGlnGlnGlyLysArgGlnArgGlu

1400
GlnArgGluArgProTyrLysGluValThrGluAspLeuLeuHis

LeuGluGlnGlyGluThrProTyrArgGluProProThrGluAsp

1500
LeuLeuHisLeuAsnSerLeuPheGlyLysAspGln

33. Verfahren nach Anspruch 29, dadurch gekennzeichnet, daß man eine Nukleinsäure abtrennt, die eine Nukleotidsequenz enthält, welche wenigstens einen Teil der nachfolgend angeführten Aminosäuresequenz kodiert, wobei diese Aminosäuresequenz eine spezifische immunologische Reaktion mit Antikörpern gegen ein Retrovirus HIV-2, wie in einem der Ansprüche 1 bis 9 definiert, auszulösen vermag, wenn diese Aminosäuresequenz mit einem Serum eines mit einem Retrovirus HIV-2 infizierten Patienten in Kontakt gebracht wird :

ArgLysAlaPheLys

CysTrpAsnCysGlyLysGluGlyHisSerAlaArgGlnCysArg

1200
AlaProArgArgGlnGlyCysTrpLysCysGlyLysProGlyHis

IleMetThrAsnCysProAspArgGlnAlaGlyPheLeuGlyLeu

```
                                              1300
GlyProTrpGlyLysLysProArgAsnPheProValAlaGlnVal

ProGlnGlyLeuThrProThrAlaProProValAspProAlaVal

AspLeuLeuGluLysTyrMetGlnGlnGlyLysArgGlnArgGln
   1400
GlnArgGluArgProTyrLysGluValThrGluAspLeuLeuHis

LeuGlnGlnGlyGluThrProTyrArgGluProProThrGluAsp
          1500
LeuLeuHisLeuAsnSerLeuPheGlyLysAspGln
```

34. Verfahren nach Anspruch 29, dadurch gekennzeichnet, daß man eine Nukleinsäure abtrennt, die eine Nukleotidsequenz enthält, welche die Gesamtheit der nachfolgend angeführten Aminosäuresequenz kodiert, wobei die kodierte Aminosäuresequenz eine spezifische immunologische Reaktion mit Antikörpern gegen ein Retrovirus HIV-2, wie in einem der Ansprüche 1 bis 9 definiert, auszulösen vermag, wenn diese Aminosäuresequenz mit einem Serum eines mit einem Retrovirus HIV-2 infizierten Patienten in Kontakt gebracht wird :

```
MetGlyAlaArgAsnSerValLeuArgGlyLysLysAlaAspGln

LeuGluArgIleArgLeuArgProGlyGlyLysLysLysTyrArg

LeuLysHisIleValTrpAlaAlaAsnLysLeuAspArgPheGly
   100
LeuAlaGluSerLeuLeuGluSerLysGluGlyCysGlnLysIle

LeuThrValLeuAspProMetValProThrGlySerGluAsnLeu
          200
LysSerLeuPheAsnThrValCysValIleTrpCysIleHisAla

GluGlnLysValLysAspThrGluGlyAlaLysGlnIleValArg
                        300
ArgHisLeuValAlaGluThrGlyThrAlaGluLysMetProSer

ThrSerArgProThrAlaProSerSerGluLysGlyGlyAsnTyr
                              400
```

35. Verfahren nach Anspruch 29, dadurch gekennzeichnet, daß man eine Nukleinsäure abtrennt, die eine Nukleotidsequenz enthält, welche die Gesamtheit oder· einen Teil der nachfolgend angeführten Aminosäuresequenz kodiert, wobei die kodierte Aminosäuresequenz eine spezifische immunologische Reaktion mit Antikörpern gegen ein Retrovirus HIV-2, wie in einem der Ansprüche 1 bis 9 definiert, auszulösen vermag, wenn diese Aminosäuresequenz mit einem Serum eines mit einem Retrovirus HIV-2 infizierten Patienten in Kontakt gebracht wird :

ProValGluHisValGlyGlyAsnTyrThrHisIleProLeuSer

ProArgThrLeuAsnAlaTrpValLysLeuValGluGluLysLys

PheGlyAlaGluValValProGlyPheGluAlaLeuSerGluGly

500
CysThrProTyrAspIleAsnGlnMetLeuAsnCysValGlyAsp

HisGlnAlaAlaMetGlnIleIleArgGluIleIleAsnGluGln

600
AlaAlaGluTrpAspValGlnHisProIleProGlyProLeuPro

AlaGlyGlnLeuArgGluProArgGlySerAspIleAlaGlyThr

700
ThrSerThrValGluGluGlnIleGlnTrpMetPheArgProGln

AsnProValProValGlyAsnIleTyrArgArgTrpIleGlnIle

800
GlyLeuGlnLysCysValArgMetTyrAsnProThrAsnIleLeu

AspIleLysGlnGlyProLysGluProPheGlnSerTyrValAsp

900
ArgPheTyrLysSerLeuArgAlaGluGlnThrAspProAlaVal

LysAsnTrpMetThrGlnThrLeuLeuValGlnAsnAlaAsnPro

AspCysLysLeuValLeuLysGlyLeuGlyMetAsnProThrLeu

1000
GluGlnMetLeuThrAlaCysGlnGlyValGlyGlyProGlyGln

LysAlaArgLeuMetAlaGluAlaLeuLysGluValIleGlyPro

1100
AlaProIleProPheAlaAlaAlaGlnGln

36. Verfahren nach Anspruch 29, dadurch gekennzeichnet, daß man eine Nukleinsäure abtrennt, die eine Nukleotidsequenz enthält, welche die Gesamtheit oder einen Teil der nachfolgend angeführten Aminosäuresequenz kodiert, wobei die kodierte Aminosäuresequenz eine spezifische immunologische Reaktion mit Antikörpern gegen ein Retrovirus HIV-2, wie in einem der Ansprüche 1 bis 9 definiert, auszulösen vermag, wenn diese Aminosäuresequenz mit einem Serum eines mit einem Retrovirus HIV-2 infizierten Patienten in Kontakt gebracht wird :

XIVLI

MetMetAsnGlnLeuLeuIleAlaIleLeuLeuAlaSerAlaCys

LeuValTyrCysThrGlnTyrValThrValPheTyrGlyValPro

ThrTrpLysAsnAlaThrIleProLeuPheCysAlaThrArgAsn

100
ArgAspThrTrpGlyThrIleGlnCysLeuProAspAsnAspAsp

TyrGlnGluIleThrLeuAsnValThrGluAlaPheAspAlaTrp

200
AsnAsnThrValThrGluGlnAlaIleGluAspValTrpHisLeu

PheGluThrSerIleLysProCysValLysLeuThrProLeuCys

300
ValAlaMetLysCysSerSerThrGluSerSerThrGlyAsnAsn

ThrThrSerLysSerThrSerThrThrThrThrThrProThrAsp

400
GluGluGlnGluIleSerGluAspThrProCysAlaArgAlaAsn

AsnCysSerGlyLeuGlyGluGluGluThrIleAsnCysGlnPhe

AsnMetThrGlyLeuGluArgAspLysLysLysGlnTyrAsnGlu

500
ThrTrpTyrSerLysAspValValCysGluThrAsnAsnSerThr

AsnGlnThrGlnCysTyrMetAsnHisCysAsnThrSerValIle

600
ThrGluSerCysAspLysHisTyrTrpAspAlaIleArgPheArg

TyrCysAlaProProGlyTyrAlaLeuLeuArgCysAsnAspThr

700
AsnTyrSerGlyPheAlaProAsnCysSerLysValValAlaSer

ThrCysThrArgMetMetGluThrGlnThrSerThrTrpPheGly

800
PheAsnGlyThrArgAlaGluAsnArgThrTyrIleTyrTrpHis

GlyArgAspAsnArgThrIleIleSerLeuAsnLysTyrTyrAsn

LeuSerLeuHisCysLysArgProGlyAsnLysThrValLysGln
900

IleMetLeuMetSerGlyHisValPheHisSerHisTyrGlnPro

IleAsnLysArgProArgGlnAlaTrpCysTrpPheLysGlyLys

1000
TrpLysAspAlaMetGlnGluValLysThrLeuAlaLysHisPro

ArgTyrArgGlyThrAsnAspThrArgAsnIleSerPheAlaAla

1100
ProGlyLysGlySerAspProGluValAlaTyrMetTrpThrAsn

CysArgGlyGluPheLeuTyrCysAsnMetThrTrpPheLeuAsn

1200
TrpIleGluAsnLysThrHisArgAsnTyrAlaProCysHisIle

LysGlnIleIleAsnThrTrpHisLysValGlyArgAsnValTyr

1300
LeuProProArgGluGlyGluLeuSerCysAsnSerThrValThr

SerIleIleAlaAsnIleAspTrpGlnAsnAsnAsnGlnThrAsn

IleThrPheSerAlaGluValAlaGluLeuTyrArgLeuGluLeu

1400
GlyAspTyrLysLeuValGluIleThrProIleGlyPheAlaPro

ThrLysGluLysArgTyrSerSerAlaHisGlyArgHisThrArg

1500
GlyValPheValLeuGlyPheLeuGlyPheLeuAlaThrAlaGly

SerAlaMetGlyAlaArgAlaSerLeuThrValSerAlaGlnSer

1600
ArgThrLeuLeuAlaGlyIleValGlnGlnGlnGlnGlnLeuLeu

AspValValLysArgGlnGlnGluLeuLeuArgLeuThrValTrp

1700
GlyThrLysAsnLeuGlnAlaArgValThrAlaIleGluLysTyr

LeuGlnAspGlnAlaArgLeuAsnSerTrpGlyCysAlaPheArg

GlnValCysHisThrThrValProTrpValAsnAspSerLeuAla

ProAspTrpAspAsnMetThrTrpGlnGlnTrpGluLysGlnVal

ArgTyrLeuGluAlaAsnIleSerLysSerLeuGluGlnAlaGln

1900
IleGlnGlnGluLysAsnMetTyrGluLeuGlnLysLeuAsnSer

TrpAspIlePheGlyAsnTrpPheAspLeuThrSerTrpValLys

2000
TyrIleGlnTyrGlyValLeuIleIleValAlaValIleAlaLeu

ArgIleValIleTyrValValGlnMetLeuSerArgLeuArgLys

2100
GlyTyrArgProValPheSerSerProProGlyTyrIleGlnGln

IleHisIleHisLysAspArgGlyGlnProAlaAsnGluGluThr

2200
GluGluAspGlyGlySerAsnGlyGlyAspArgTyrTrpProTrp

ProIleAlaTyrIleHisPheLeuIleArgGlnLeuIleArgLeu

LeuThrArgLeuTyrSerIleCysArgAspLeuLeuSerArgSer

2300
PheLeuThrLeuGlnLeuIleTyrGlnAsnLeuArgAspTrpLeu

ArgLeuArgThrAlaPheLeuGlnTyrGlyCysGlnTrpIleGln

2400
GluAlaPheGlnAlaAlaAlaArgAlaThrArgGluThrLeuAla

GlyAlaCysArgGlyLeuTrpArgValLeuGlnArgIleGlyArg

2500
GlyIleLeuAlaValProArgArgIleArgGlnGlyAlaGluIle

AlaLeuLeu

37. Verfahren nach Anspruch 28, dadurch gekennzeichnet, daß es folgende Schritte umfaßt:

— die Einwirkung der T4-DNS-Polymerase bis zum Erhalt der freien Enden der Doppelkette der c-DNS,

— die Inserierung der mit Adaptoren versehenen und durch die adequaten Enzyme in einer Nukleinsäure eines Vektors verdauten c-DNA,

— das Abtrennen einer rekombinanten Nukleinsäure, die eine nach einem Verfahren nach einem der Ansprüche 28 bis 36 erhaltenen c-DNS oder einen Teil dieser c-DNS umfaßt, und/oder eine RNS oder einen Teil einer RNS, der dieser c-DNS entspricht, die in eine aus einem Vektor stammende Nukleinsäure inseriert ist.

38. Verfahren nach Anspruch 37, dadurch gekennzeichnet, daß die abgetrennte rekombinante Nukleinsäure markiert ist.

39. Verfahren zum Nachweis eines Retrovirus, das auf den Menschen eine zytopathogene Wirkung ausüben kann, vom Typ HIV-2, oder seiner RNS in einer biologischen Flüssigkeit oder einem biologischen Gewebe, insbesondere im Hinblick auf den Nachweis des möglichen oder tatsächlichen Vorliegens von SLA oder AIDS in vitro beim Menschen, gekennzeichnet durch die Kontaktierung von in dieser biologischen Flüssgikeit oder diesem biologischen Gewebe enthaltenen Nukleinsäuren mit einer Sonde, die eine durch ein Verfahren nach einem der Ansprüche 32 bis 36 erhaltenen Nukleinsäure enthält, unter stringenten Hybridisierungsbedingungen, oder eine DNS enthält, die eine Sequenz-homologie von wenigstens 70 %, vorzugsweise wenigstens 90 % mit der RNS eines Retrovirus nach einem der Ansprüche 1 bis 9 aufweist, und zur Hybridisierung mit dieser befähigt ist, unter nicht-stringenten Bedingungen, wobei die Kontaktierung nach der « Spot-Blot-Technik » während der für die Durchführung der Hybridisierung erforderlichen Zeitdauer durchgeführt wird, wonach das gebildete Hybrid mit einer Lösung gewaschen wird, welche die Beibehaltung der stringenten Bedingungen gewährleistet, sowie durch den Nachweis des gebildeten Hybrids.

40. Verfahren zur Herstellung eines Retrovirus, das auf den Menschen eine zytopathogene Wirkung auszuüben vermag, vom Typ HIV-2, gekennzeichnet durch die Züchtung in menschlichen T4-Lymphozyten oder in von T4-Lymphozyten abgeleiteten konstanten Zellinien, wobei diese den Phänotyp T4 aufweisen, und diese Lymphozyten oder Zellinien vorgängig mit einem Isolat des Virus HIV-2, wie in einem der Ansprüche 1 bis 9 definiert, infiziert worden waren, und insbesondere wenn das Aktivitätsniveau der Reverse-Transkriptase eine vorgegebene Schwelle erreicht hat, und durch Abtrennung und Reinigung der im Medium für die Züchtung dieser Lymphozyten oder Linien freigesetzten Virenmengen, insbesondere durch differentielle Zentrifugierung in einem Saccharose- oder Metrizamidgradienten.

41. Verfahren zur Herstellung einer Hybridisierungssonde zum Nachweis einer RNS des Retrovirus HIV-2, gekennzeichnet durch die Inserierung einer DNS-Sequenz, insbesondere eine nach einem Verfahren nach einem der der Ansprüche 28 bis 36, in einen Klonierungsvektor durch Rekombination in vitro, durch die Klonierung des in einer kompetenten Wirtszelle erhaltenen modifizierten Vektors, sowie durch die Abtrennung der erhaltenen DNS-Rekombinanten.

42. Die sekretorischen Hybridome eines monoklonalen Antikörpers, die spezifisch ein Antigen eines HIV-2 Retrovirus nach einem der Ansprüche 1 bis 9 erkennen.

43. Verwendung der durch ein Verfahren nach Anspruch 27 erhaltenen polyklonalen Antikörper zum Nachweis des Retrovirus HIV-2, wie in einem der Ansprüche 1 bis 9 definiert, in einem biologischen Präparat.

44. Verwendung der durch ein Verfahren nach Anspruch 27 erhaltenen polyklonalen Antikörper, zum Nachweis der Antigene des Retrovirus HIV-2, wie in einem der Ansprüche 1 bis 9 definiert, in einem biologischen Präparat.

45. Verwendung der durch ein Verfahren nach Anspruch 27 erhaltenen polyklonalen Antikörper zur Reinigung der Virusantigene eines Retrovirus HIV-2, wie in einem der Ansprüche 1 bis 9 definiert.

FIG.1

FIG.2

FIG.3

CARTE DE RESTRICTION

FIG.4

FIG.5

# FIG.6

EP 0 239 425 B1

```
        10          20          30          40          50          60          70          80          90         100
GT GGAAGG CGAGACT GAAAGCAAGAGGAATACCATT TAGTT AAAGGACAGGAACAGCTATAC TTGGTCAGGGCAGGAAGTAACTAACAGAAACAGCTGAG
              MNLI                                      ALUI                      MAEIII      PVUII
                                                                                              ALUI
                                                                                                 ODEI


        110         120         130         140         150         160         170         180         190         200
ACTGCA GGGACTTTCCAGAA GGGGCTGTAACCAAGGGAGGGACA TGGGAGGAGCTGGTGGGGAACGCCTCATATTCTCTGTATAATATACCCGCTGCTTG
   PSTI                     MAEIII    MNLI   NLAIII   ALUI              MNLI                      BBVI
                              STYI                MNLI                                           FNU4HI
                                                                                                THIIIIII


        210         220         230         240         250         260         270         280         290         300
CATTGTACTTCAGTCGCTCTGCGGAGAGGCTGGCAGATTGAGCCCTGGAGGATCTCTCCAGCACTAGACGGATGAGCCTGGGTGCCCTGCTAGACTCTCA
   RSAI                    MNLI          BANII   MNLI              MAEI           APYI BSP1286 MAEI     HPHI
                                         BSP1286   XHOII                          BSTNI            HINFI
                                         APYI       DPNI                          ECORII
                                         BSTNI      MBOI                          SCRFI
                                         ECORII     NDEII                            BANI
                                         SCRFI      SAUIIIA


        310         320         330         340         350         360         370         380
CCAGCACTTGGCCGGTGCTGGCAGACGGCCCCACGCTTGCCTGCTTAAAAACCTTCCTTAATAAAGCTGCAGTAGAAGCA
       HAEIII                  HAEIII                            ALUI
          HAPII                SAU96A                            BBVI
          HPAII                                                  FNU4HI
          MSPI
```

# FIG.7

gag     pol    Q    env    F

## FIG.7A

HIV-1 sonde

HIV-2 ADNc(E2)

1 kb

H    H

Pv   Pv

(A)n

B Ps Ps

## FIG.7B

**B**

                                PvuI           PstI

HIV.2    AGTAACTAACAGAA------ACAGCTGAGACTGC----AGGGACTTTCCAGAAGGGGCTG

HIV.1    AGT-ACTTCAAGAACTGCTGACATC-GAGCTTGC TACAAGGGACTTTCCGCTGGGGACTT
               9000         9010       9020      9030     9040

HIV.2    TAACCAA-------------GGGAGGGACATGGGAG----GAGCTGGTGGGGAACGCCTC

HIV.1    T--CCAGGGAGGCGTGGCCTGGGCGGGACTGGGGAGTGGCGAGC-----------CCTC
        9050      9060      9070      9000      9090

HIV.2    ATATTCTCTG⌐TATAAA⌐TATACCCGCTGCTTGCATTGTACTTCAGTCGCTCTGCGGAGAGG

HIV.1    AGATG--CTGCA⌐TATAAG⌐CAGCTGCTTTTTGCC-TGTACTGG-GTCTCTCTGGTTAGAC-
           9100      9110       9120       9130      9140
                                                  U3 R

HIV.2    CTGGCAGATTGAGCCCTGGGAGGTTCTCTCCAGCACTAGCAGGTAGAGCCTGGGTGTCCC

HIV.1    -----CAGATTTGAGCCTGGGAGC-TCTCTGGCTAACTAGGGAACCCAC----------
          9150      9160       9170      9180      9190

HIV.2    TGCTAGACTCTCACCAGCACTTGGCCGGTGCTGGGCAGACGGCCCCACGCTTGCTTGCTT

HIV.1    ------------------------------------------------------TGCTT

HIV.2    AAAAACCTCCTT⌐AATAAA⌐GCT-GCC---AGTAGAAGCA

HIV.1    AAG-----CCT⌐AATAAA⌐GCTTGCCTTGAGTGCTTCAA
                   9210      9220
                  HindIII              R

FIG.8

FIG.9